# EUROPEAN PATENT SPECIFICATION

(11) **EP 4 110 779 B1**
(45) Date of publication and mention of the grant of the patent: **21.10.2026**
(21) Application number: 21712651.5
(22) Date of filing: 23.02.2021
(51) Int. Cl.: C07D 471/04, C07D 401/14, C07D 405/14, A61P 35/00, A61K 31/4375

(54) **COMPOUNDS AND METHODS OF USE**
VERBINDUNGEN UND ANWENDUNGSVERFAHREN
COMPOSÉS ET PROCÉDÉS D'UTILISATION

(30) Priority: 24.02.2020 US 202062980954 P; 04.12.2020 US 202063121418 P
(43) Date of publication of application: 04.01.2023
(73) Proprietor: Exelixis, Inc., Alameda, CA 94502 (US)
(72) Inventor: BANNEN, Lynne, Alameda, California 94502 (US); XU, Wei, Alameda, California 94502 (US); WANG, Yong, Alameda, California 94502 (US); RAUB, Andrew, Alameda, California 94502 (US); SPANGLER, Benjamin, Alameda, California 94502 (US); SALVANT, Justin, Alameda, California 94502 (US); TSO, Kin, Alameda, California 94502 (US); JIANG, Faming, Alameda, California 94502 (US)
(74) Representative: J A Kemp LLP
(86) International application number: PCT/US2021/019294
(87) International publication number: WO 2021/173591

(56) References cited:
- EP-A1- 3 239 147
- CN-A- 104 072 480
- CN-A- 109 896 997

## Description

### CROSS REFERENCE TO RELATED APPLICATIONS

This application claims the benefit under 35 U.S.C. §119(e) of U.S. Provisional Application No. 62/980,954, filed February 24, 2020, and U.S. Provisional Application No. 63/121,418, filed December 4, 2020.

### FIELD

Provided herein are compounds and pharmaceutical compositions suitable as modulators of protein kinases, and their use in methods of treating disorders mediated, at least in part by, protein kinases.

### BACKGROUND

Human Axl belongs to the TAM subfamily of receptor tyrosine kinases that includes Mer. TAM kinases are characterized by an extracellular ligand binding domain consisting of two immunoglobulin-like domains and two fibronectin type III domains. Axl is overexpressed in a number of tumor cell types and was initially cloned from patients with chronic myelogenous leukemia. When overexpressed, Axl exhibits transforming potential. Axl signaling is believed to cause tumor growth through activation of proliferative and anti-apoptotic signaling pathways. Axl has been associated with cancers including, but not limiting to lung cancer, myeloid leukemia, uterine cancer, ovarian cancer, gliomas, melanoma, thyroid cancer, renal cell carcinoma, osteosarcoma, gastric cancer, prostate cancer, and breast cancer. The over-expression of Axl results in a poor prognosis for patients with the indicated cancers.

Activation of Mer, like Axl, conveys downstream signaling pathways that cause tumor growth and activation. Mer binds ligands such as the soluble protein Gas-6. Gas-6 binding to Mer induces autophosphorylation of Mer on its intracellular domain, resulting in downstream signal activation. Over-expression of Mer in cancer cells leads to increased metastasis, most likely by generation of soluble Mer extracellular domain protein as a decoy receptor. Tumor cells secrete a soluble form of the extracellular Mer receptor which reduces the ability of soluble Gas-6 ligand to activate Mer on endothelial cells, leading to cancer progression.

c-Met, is the prototypic member of a subfamily of heterodimeric receptor tyrosine kinases (RTKs) which include Met, Ron and Sea. Expression of c-Met occurs in a wide variety of cell types including epithelial, endothelial and mesenchymal cells where activation of the receptor induces cell migration, invasion, proliferation and other biological activities associated with invasive cell growth. Signal transduction through c-Met receptor activation is responsible for many of the characteristics of tumor cells.

Therefore, a need exists for new compounds that modulate Axl, Mer and c-Met kinases for the treatment of cancers.

CN 109896997 A discusses N-acylaniline derivatives with c-Met inhibitory activity. CN 104072480 A discusses quinoline derivatives with c-Met inhibitory activity. EP 3239147 A1 discusses quinoline derivatives with Axl inhibitory activity.

### SUMMARY

Provided herein are compounds that inhibit c-Met, Axl, Mer and/or KDR. The compounds are of Formula (II) or a pharmaceutically acceptable salt, stereoisomer, or mixture of stereoisomers thereof, as described herein.

Also provided herein are pharmaceutical compositions comprising a compound as described herein, or a pharmaceutically acceptable salt, stereoisomer, or mixture of stereoisomers thereof, and a pharmaceutically acceptable carrier or excipient.

Also provided herein is a compound of the invention, or a pharmaceutically acceptable salt or stereoisomer thereof, or a pharmaceutical composition of the invention, for use in treating a disease, disorder, or syndrome in a subject in need thereof, said use comprising administering to the subject a therapeutically effective amount of said compound or pharmaceutical composition, wherein the disease, disorder, or syndrome is mediated at least in part by modulating in vivo activity of a protein kinase; preferably wherein the protein kinase is AXL, KDR, Mer, or Met.

Also provided herein are compositions, including pharmaceutical compositions, kits that include the compounds, and methods of making the compounds. Also provided herein are compounds and/or compositions for use in a method of treating a disease, disorder, or condition that is mediated, at least in part, by c-Met, Axl, Mer and/or KDR activity.

### DETAILED DESCRIPTION

### Definitions

As used in the present specification, the following words, phrases and symbols are generally intended to have the meanings as set forth below, except to the extent that the context in which they are used indicates otherwise.

A dash ("-") that is not between two letters or symbols is used to indicate a point of attachment for a substituent. For example, -C(O)NH₂ is attached through the carbon atom. A dash at the front or end of a chemical group is a matter of convenience; chemical groups may be depicted with or without one or more dashes without losing their ordinary meaning. A wavy line or a dashed line drawn through or perpendicular across the end of a line in a structure indicates a specified point of attachment of a group. Unless chemically or structurally required, no directionality or stereochemistry is indicated or implied by the order in which a chemical group is written or named.

The prefix "Cᵤ₋ᵥ" indicates that the following group has from u to v carbon atoms. For example, "C₁₋₆ alkyl" indicates that the alkyl group has from 1 to 6 carbon atoms.

Reference to "about" a value or parameter herein includes (and describes) embodiments that are directed to that value or parameter per se. In certain embodiments, the term "about" includes the indicated amount ± 10%. In other embodiments, the term "about" includes the indicated amount ± 5%. In certain other embodiments, the term "about" includes the indicated amount ± 1%. Also, to the term "about X" includes description of "X". Also, the singular forms "a" and "the" include plural references unless the context clearly dictates otherwise. Thus, e.g., reference to "the compound" includes a plurality of such compounds and reference to "the assay" includes reference to one or more assays and equivalents thereof known to those skilled in the art.

"Alkyl" refers to an unbranched or branched saturated hydrocarbon chain. As used herein, alkyl has 1 to 20 carbon atoms (i.e., C₁₋₂₀ alkyl), 1 to 12 carbon atoms (i.e., C₁₋₁₂ alkyl), 1 to 8 carbon atoms (i.e., C₁₋₈ alkyl), 1 to 6 carbon atoms (i.e., C₁₋₆ alkyl) or 1 to 4 carbon atoms (i.e., C₁₋₄ alkyl). Examples of alkyl groups include, e.g., methyl, ethyl, propyl, isopropyl, n-butyl, sec-butyl, iso-butyl, tert-butyl, pentyl, 2-pentyl, isopentyl, neopentyl, hexyl, 2-hexyl, 3-hexyl and 3-methylpentyl. When an alkyl residue having a specific number of carbons is named by chemical name or identified by molecular formula, all positional isomers having that number of carbons may be encompassed; thus, for example, "butyl" includes n-butyl (i.e., -(CH₂)₃CH₃), sec-butyl (i.e., -CH(CH₃)CH₂CH₃), isobutyl (i.e., -CH₂CH(CH₃)₂) and tert-butyl (i.e., -C(CH₃)₃); and "propyl" includes n-propyl (i.e., -(CH₂)₂CH₃) and isopropyl (i.e., -CH(CH₃)₂).

"Alkenyl" refers to an alkyl group containing at least one carbon-carbon double bond and having from 2 to 20 carbon atoms (i.e., C₂₋₂₀ alkenyl), 2 to 8 carbon atoms (i.e., C₂₋₈ alkenyl), 2 to 6 carbon atoms (i.e., C₂₋₆ alkenyl) or 2 to 4 carbon atoms (i.e., C₂₋₄ alkenyl). Examples of alkenyl groups include, e.g., ethenyl, propenyl, butadienyl (including 1,2-butadienyl and 1,3-butadienyl).

"Alkynyl" refers to an alkyl group containing at least one carbon-carbon triple bond and having from 2 to 20 carbon atoms (i.e., C₂₋₂₀ alkynyl), 2 to 8 carbon atoms (i.e., C₂₋₈ alkynyl), 2 to 6 carbon atoms (i.e., C₂₋₆ alkynyl) or 2 to 4 carbon atoms (i.e., C₂₋₄ alkynyl). The term "alkynyl" also includes those groups having one triple bond and one double bond.

"Alkoxy" refers to the group "alkyl-O-". Examples of alkoxy groups include, e.g., methoxy, ethoxy, n-propoxy, iso-propoxy, n-butoxy, tert-butoxy, sec-butoxy, n-pentoxy, n-hexoxy and 1,2-dimethylbutoxy.

"Alkylthio" refers to the group "alkyl-S-". "Alkylsulfinyl" refers to the group "alkyl-S(O)-". "Alkylsulfonyl" refers to the group "alkyl-S(O)₂-". "Alkylsulfonylalkyl" refers to -alkyl-S(O)₂-alkyl.

"Acyl" refers to a group -C(O)R^{y}, wherein R^{y} is hydrogen, alkyl, alkenyl, alkynyl, cycloalkyl, heterocycloalkyl, aryl, heteroalkyl or heteroaryl; each of which may be optionally substituted, as defined herein. Examples of acyl include, e.g., formyl, acetyl, cyclohexylcarbonyl, cyclohexylmethyl-carbonyl and benzoyl.

"Amido" refers to both a "C-amido" group which refers to the group -C(O)NR^{y}R^{z} and an "N-amido" group which refers to the group -NR^{y}C(O)R^{z}, wherein R^{y} and R^{z} are independently hydrogen, alkyl, alkenyl, alkynyl, cycloalkyl, heterocycloalkyl, aryl, heteroalkyl or heteroaryl; each of which may be optionally substituted, as defined herein, or R^{y} and R^{z} are taken together to form a cycloalkyl or heterocycloalkyl; each of which may be optionally substituted, as defined herein.

"Amino" refers to the group -NR^{y}R^{z} wherein R^{y} and R^{z} are independently hydrogen, alkyl, alkenyl, alkynyl, cycloalkyl, heterocycloalkyl, aryl, heteroalkyl or heteroaryl; each of which may be optionally substituted, as defined herein.

"Amidino" refers to -C(NR^{y})(NR^{z}₂), wherein R^{y} and R^{z} are independently hydrogen, alkyl, alkenyl, alkynyl, cycloalkyl, heterocycloalkyl, aryl, heteroalkyl or heteroaryl; each of which may be optionally substituted, as defined herein.

"Aryl" refers to an aromatic carbocyclic group having a single ring (e.g., monocyclic) or multiple rings (e.g., bicyclic or tricyclic) including fused systems. As used herein, aryl has 6 to 20 ring carbon atoms (i.e., C₆₋₂₀ aryl), 6 to 12 carbon ring atoms (i.e., C₆₋₁₂ aryl), or 6 to 10 carbon ring atoms (i.e., C₆₋₁₀ aryl). Examples of aryl groups include, e.g., phenyl, naphthyl, fluorenyl and anthryl. Aryl, however, does not encompass or overlap in any way with heteroaryl defined below. If one or more aryl groups are fused with a heteroaryl, the resulting ring system is heteroaryl. If one or more aryl groups are fused with a heterocycloalkyl, the resulting ring system is heterocycloalkyl.

"Arylalkyl" or "Aralkyl" refers to the group "aryl-alkyl-".

"Carbamoyl" refers to -C(O)NR^{y}R^{z}. "O-carbamoyl" refers to -O-C(O)NR^{y}R^{z} and "N-carbamoyl" refers to -NR^{y}C(O)OR^{z}, wherein R^{y} and R^{z} are independently hydrogen, alkyl, alkenyl, alkynyl, cycloalkyl, heterocycloalkyl, aryl, heteroalkyl or heteroaryl; each of which may be optionally substituted, as defined herein.

"Carboxyl ester" or "ester" refer to both -OC(O)R^{x} and -C(O)OR^{x}, wherein R^{x} is alkyl, alkenyl, alkynyl, cycloalkyl, heterocycloalkyl, aryl, heteroalkyl or heteroaryl; each of which may be optionally substituted, as defined herein.

"Cycloalkyl" refers to a saturated or partially unsaturated cyclic alkyl group having a single ring or multiple rings including fused, bridged and spiro ring systems. The term "cycloalkyl" includes cycloalkenyl groups (i.e., the cyclic group having at least one double bond) and carbocyclic fused ring systems having at least one sp³ carbon atom (i.e., at least one non-aromatic ring). As used herein, cycloalkyl has from 3 to 20 ring carbon atoms (i.e., C₃₋₂₀ cycloalkyl), 3 to 12 ring carbon atoms (i.e., C₃₋₁₂ cycloalkyl), 3 to 10 ring carbon atoms (i.e., C₃₋₁₀ cycloalkyl), 3 to 8 ring carbon atoms (i.e., C₃₋₈ cycloalkyl), or 3 to 6 ring carbon atoms (i.e., C₃₋₆ cycloalkyl). Monocyclic groups include, for example, cyclopropyl, cyclobutyl, cyclopentyl, cyclohexyl, cycloheptyl and cyclooctyl. Polycyclic groups include, for example, bicyclo[2.2.1]heptanyl, bicyclo[2.2.2]octanyl, adamantyl, norbornyl, decalinyl, 7,7-dimethyl-bicyclo[2.2.1]heptanyl and the like. In some embodiments, one or more ring carbons of "cycloalkyl" can be optionally replaced by a carbonyl group. Examples of such cycloalkyl include cyclohexanone-4-yl, and the like. Further, the term cycloalkyl is intended to encompass moieties that have one or more aromatic ring fused (i.e., having a bond in common with) to the cycloalkyl ring, e.g., benzo or thienyl derivatives of cyclopentane, cyclohexane, and the like. A cycloalkyl group containing a fused aromatic ring can be attached through any ring-forming atom including a ring-forming atom of the fused aromatic ring. Still further, cycloalkyl also includes "spirocycloalkyl" when there are two positions for substitution on the same carbon atom, for example spiro[2.5]octanyl, spiro[4.5]decanyl, or spiro[5.5]undecanyl.

"Cycloalkylalkyl" refers to the group "cycloalkyl-alkyl-".

"Guanidino" refers to -NR^{y}C(=NR^{z})(NR^{y}R^{z}), wherein each R^{y} and R^{z} are independently hydrogen, alkyl, alkenyl, alkynyl, cycloalkyl, heterocycloalkyl, aryl, heteroalkyl or heteroaryl; each of which may be optionally substituted, as defined herein.

"Hydrazino" refers to -NHNH₂.

"Imino" refers to a group -C(NR^{y})R^{z}, wherein R^{y} and R^{z} are each independently hydrogen, alkyl, alkenyl, alkynyl, cycloalkyl, heterocycloalkyl, aryl, heteroalkyl or heteroaryl; each of which may be optionally substituted, as defined herein.

"Imido" refers to a group -C(O)NR^{y}C(O)R^{z}, wherein R^{y} and R^{z} are each independently hydrogen, alkyl, alkenyl, alkynyl, cycloalkyl, heterocycloalkyl, aryl, heteroalkyl or heteroaryl; each of which may be optionally substituted, as defined herein.

"Halogen" or "halo" refers to atoms occupying group VIIA of the periodic table, such as fluoro, chloro, bromo or iodo.

"Haloalkyl" refers to an unbranched or branched alkyl group as defined above, wherein one or more (e.g., 1 to 6 or 1 to 3) hydrogen atoms are replaced by a halogen. For example, where a residue is substituted with more than one halogen, it may be referred to by using a prefix corresponding to the number of halogen moieties attached. Dihaloalkyl and trihaloalkyl refer to alkyl substituted with two ("di") or three ("tri") halo groups, which may be, but are not necessarily, the same halogen. Examples of haloalkyl include, e.g., trifluoromethyl, difluoromethyl, fluoromethyl, trichloromethyl, 2,2,2-trifluoroethyl, 1,2-difluoroethyl, 3-bromo-2-fluoropropyl, 1,2-dibromoethyl and the like.

"Haloalkoxy" refers to an alkoxy group as defined above, wherein one or more (e.g., 1 to 6 or 1 to 3) hydrogen atoms are replaced by a halogen.

"Hydroxyalkyl" refers to an alkyl group as defined above, wherein one or more (e.g., 1 to 6 or 1 to 3) hydrogen atoms are replaced by a hydroxy group.

"Heteroalkyl" refers to an alkyl group in which one or more of the carbon atoms (and any associated hydrogen atoms) are each independently replaced with the same or different heteroatomic group, provided the point of attachment to the remainder of the molecule is through a carbon atom. The term "heteroalkyl" includes unbranched or branched saturated chain having carbon and heteroatoms. By way of example, 1, 2 or 3 carbon atoms may be independently replaced with the same or different heteroatomic group. Heteroatomic groups include, but are not limited to, -NR^{y}-, -O-, -S-, -S(O)-, -S(O)₂-, and the like, wherein R^{y} is hydrogen, alkyl, alkenyl, alkynyl, cycloalkyl, heterocycloalkyl, aryl, heteroalkyl or heteroaryl; each of which may be optionally substituted, as defined herein. Examples of heteroalkyl groups include, e.g., ethers (e.g., -CH₂OCH₃, - CH(CH₃)OCH₃, -CH₂CH₂OCH₃, -CH₂CH₂OCH₂CH₂OCH₃, etc.), thioethers (e.g., -CH₂SCH₃, - CH(CH₃)SCH₃, -CH₂CH₂SCH₃, -CH₂CH₂SCH₂CH₂SCH₃, etc.), sulfones (e.g., -CH₂S(O)₂CH₃, - CH(CH₃)S(O)₂CH₃, -CH₂CH₂S(O)₂CH₃, -CH₂CH₂S(O)₂CH₂CH₂OCH₃, etc.) and amines (e.g., - CH₂NR^{y}CH₃, -CH(CH₃)NR^{y}CH₃, -CH₂CH₂NR^{y}CH₃, -CH₂CH₂NR^{y}CH₂CH₂NR^{y}CH₃, etc., where R^{y} is hydrogen, alkyl, alkenyl, alkynyl, cycloalkyl, heterocycloalkyl, aryl, heteroalkyl, or heteroaryl; each of which may be optionally substituted, as defined herein). As used herein, heteroalkyl includes 1 to 10 carbon atoms, 1 to 8 carbon atoms, or 1 to 4 carbon atoms; and 1 to 3 heteroatoms, 1 to 2 heteroatoms, or 1 heteroatom.

"Heteroaryl" refers to an aromatic group having a single ring, multiple rings or multiple fused rings, with one or more ring heteroatoms independently selected from nitrogen, oxygen, boron, phosphorus and sulfur. As used herein, heteroaryl includes 1 to 20 ring carbon atoms (i.e., C₁₋₂₀ heteroaryl), 3 to 12 ring carbon atoms (i.e., C₃₋₁₂ heteroaryl), or 3 to 8 carbon ring atoms (i.e., C₃₋₈ heteroaryl), and 1 to 5 ring heteroatoms, 1 to 4 ring heteroatoms, 1 to 3 ring heteroatoms, 1 to 2 ring heteroatoms, or 1 ring heteroatom independently selected from nitrogen, oxygen and sulfur. In certain instances, heteroaryl includes 5-10 membered ring systems, 5-7 membered ring systems, or 5-6 membered ring systems, each independently having 1 to 4 ring heteroatoms, 1 to 3 ring heteroatoms, 1 to 2 ring heteroatoms, or 1 ring heteroatom independently selected from nitrogen, oxygen and sulfur. In some embodiments, the heteroaryl has 5-14 ring atoms including carbon atoms and 1, 2, 3 or 4 heteroatom ring members independently selected from nitrogen, sulfur and oxygen. In some embodiments, the heteroaryl has 5-14, or 5-10 ring atoms including carbon atoms and 1, 2, 3 or 4 heteroatom ring members independently selected from nitrogen, sulfur and oxygen. In some embodiments, the heteroaryl has 5-6 ring atoms and 1 or 2 heteroatom ring members independently selected from nitrogen, sulfur and oxygen. In some embodiments, the heteroaryl is a five-membered or six-membered heteroaryl ring. In other embodiments, the heteroaryl is an eight-membered, nine-membered or ten-membered fused bicyclic heteroaryl ring. Examples of heteroaryl groups include, e.g., acridinyl, benzimidazolyl, benzothiazolyl, benzindolyl, benzofuranyl, benzothiazolyl, benzothiadiazolyl, benzonaphthofuranyl, benzoxazolyl, benzothienyl (benzothiophenyl), benzotriazolyl, benzo[4,6]imidazo[1,2-a]pyridyl, carbazolyl, cinnolinyl, dibenzofuranyl, dibenzothiophenyl, furanyl, isothiazolyl, imidazolyl, indazolyl, indolyl, indazolyl, isoindolyl, isoquinolyl, isoxazolyl, naphthyridinyl, oxadiazolyl, oxazolyl, 1-oxidopyridinyl, 1-oxidopyrimidinyl, 1-oxidopyrazinyl, 1-oxidopyridazinyl, phenazinyl, phthalazinyl, pteridinyl, purinyl, pyrrolyl, pyrazolyl, pyridinyl, pyrazinyl, pyrimidinyl, pyridazinyl, quinazolinyl, quinoxalinyl, quinolinyl, quinuclidinyl, isoquinolinyl, thiazolyl, thiadiazolyl, triazolyl, tetrazolyl and triazinyl. In some embodiments, any ring-forming N in a heteroaryl moiety can be an N-oxide.

In certain instances, a fused heteroaryl refers to a heteroaryl ring fused to another heteroaryl ring. Examples of the fused-heteroaryl rings include, but are not limited to, benzo[d]thiazolyl, quinolinyl, isoquinolinyl, benzo[b]thiophenyl, indazolyl, benzo[d]imidazolyl, pyrazolo[1,5-a]pyridinyl and imidazo[1,5-a]pyridinyl, where the heteroaryl can be bound *via* either ring of the fused system. Any aromatic ring, having a single or multiple fused rings, containing at least one heteroatom, is considered a heteroaryl regardless of the attachment to the remainder of the molecule (i.e., through any one of the fused rings). Heteroaryl does not encompass or overlap with aryl as defined above.

"Heteroarylalkyl" refers to the group "heteroaryl-alkyl-".

"Heterocycloalkyl" or "heterocyclyl" refers to a saturated or partially unsaturated cyclic alkyl group, with one or more ring heteroatoms independently selected from boron, phosphorus, nitrogen, oxygen and sulfur. The term "heterocycloalkyl" includes heterocycloalkenyl groups (i.e., the heterocycloalkyl group having at least one double bond), bridged-heterocycloalkyl groups, fused-heterocycloalkyl groups and spiro-heterocycloalkyl groups. A heterocycloalkyl may be a single ring or multiple rings wherein the multiple rings may be fused, bridged or spiro. One or more ring carbon atoms and ring heteroatoms of a heterocycloalkyl group can be optionally oxidized to form an oxo or sulfido group or other oxidized linkage (*e.g.,* C(O), S(O), C(S) or S(O)₂, *N-*oxide *etc*.) or a nitrogen atom can be quaternized. The heterocycloalkyl group can be attached through a ring carbon atom or a ring heteroatom. Any non-aromatic ring containing at least one heteroatom is considered a heterocycloalkyl, regardless of the attachment (i.e., can be bound through a carbon atom or a heteroatom). As used herein, heterocycloalkyl has 2 to 20 ring carbon atoms (i.e., C₂₋₂₀ heterocycloalkyl), 2 to 12 ring carbon atoms (i.e., C₂₋₁₂ heterocycloalkyl), 2 to 10 ring carbon atoms (i.e., C₂₋₁₀ heterocycloalkyl), 2 to 8 ring carbon atoms (i.e., C₂₋₈ heterocycloalkyl), 3 to 12 ring carbon atoms (i.e., C₃₋₁₂ heterocycloalkyl), 3 to 8 ring carbon atoms (i.e., C₃₋₈ heterocyclyl), or 3 to 6 ring carbon atoms (i.e., C₃₋₆ heterocycloalkyl); having 1 to 5 ring heteroatoms, 1 to 4 ring heteroatoms, 1 to 3 ring heteroatoms, 1 to 2 ring heteroatoms, or 1 ring heteroatom independently selected from nitrogen, sulfur or oxygen. Examples of heterocycloalkyl groups include, e.g., azetidinyl, azepinyl, benzodioxolyl, benzo[b][1,4]dioxepinyl, 1,4-benzodioxanyl, benzopyranyl, benzodioxinyl, benzopyranonyl, benzofuranonyl, dioxolanyl, dihydropyranyl, hydropyranyl, thienyl[1,3]dithianyl, decahydroisoquinolyl, furanonyl, imidazolinyl, imidazolidinyl, indolinyl, isoindolinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, oxiranyl, oxetanyl, phenothiazinyl, phenoxazinyl, piperidinyl, piperazinyl, 4-piperidonyl, pyrrolidinyl, pyrazolidinyl, quinuclidinyl, thiazolidinyl, tetrahydrofuryl, tetrahydropyranyl, trithianyl, tetrahydroquinolinyl, thiophenyl (i.e., thienyl), tetrahydropyranyl, thiomorpholinyl, thiamorpholinyl, 1-oxo-thiomorpholinyl and 1,1-dioxo-thiomorpholinyl. The term "heterocycloalkyl" also includes "spiroheterocycloalkyl" when there are two positions for substitution on the same carbon atom. Examples of the spiro-heterocycloalkyl rings include, e.g., bicyclic and tricyclic ring systems, such as 2-oxa-7-azaspiro[3.5]nonanyl, 2-oxa-6-azaspiro[3.4]octanyl and 6-oxa-1-azaspiro[3.3]heptanyl.

Further, the term heterocycloalkyl is intended to encompass any non-aromatic ring containing at least one heteroatom, which ring is fused to one or more aryl or heteroaryl rings, regardless of the attachment to the remainder of the molecule (i.e., a heterocycloalkyl group containing a fused aromatic ring can be attached through any ring atom including a ring atom of the fused aromatic ring). Examples of the fused-heterocycloalkyl rings include, but are not limited to, 1,2,3,4-tetrahydroisoquinolinyl, 4,5,6,7-tetrahydrothieno[2,3-c]pyridinyl, indolinyl and isoindolinyl, where the heterocycloalkyl can be bound *via* either ring of the fused system.

"Heterocycloalkylalkyl" refers to the group "heterocycloalkyl-alkyl-."

"Oxime" refers to the group -CR^{y}(=NOH) wherein R^{y} is hydrogen, alkyl, alkenyl, alkynyl, cycloalkyl, heterocycloalkyl, aryl, heteroalkyl or heteroaryl; each of which may be optionally substituted, as defined herein.

"Sulfonyl" refers to the group -S(O)₂R^{y}, where R^{y} is hydrogen, alkyl, alkenyl, alkynyl, cycloalkyl, heterocycloalkyl, aryl, heteroalkyl or heteroaryl; each of which may be optionally substituted, as defined herein. Examples of sulfonyl are methylsulfonyl, ethylsulfonyl, phenylsulfonyl and toluene sulfonyl.

"Sulfinyl" refers to the group -S(O)R^{y}, where R^{y} is hydrogen, alkyl, alkenyl, alkynyl, cycloalkyl, heterocycloalkyl, aryl, heteroalkyl or heteroaryl; each of which may be optionally substituted, as defined herein. Examples of sulfinyl are methylsulfinyl, ethylsulfinyl, phenylsulfinyl and toluenesulfinyl.

"Sulfonamido" refers to the groups -SO₂NR^{y}R^{z} and -NR^{y}SO₂R^{z}, where R^{y} and R^{z} are each independently hydrogen, alkyl, alkenyl, alkynyl, cycloalkyl, heterocycloalkyl, aryl, heteroalkyl or heteroaryl; each of which may be optionally substituted, as defined herein.

The terms "optional" or "optionally" means that the subsequently described event or circumstance may or may not occur and that the description includes instances where said event or circumstance occurs and instances in which it does not. Also, the term "optionally substituted" refers to any one or more (e.g., 1 to 5 or 1 to 3) hydrogen atoms on the designated atom or group may or may not be replaced by a moiety other than hydrogen.

The term "substituted" used herein means any of the above groups (i.e., alkyl, alkenyl, alkynyl, alkylene, alkoxy, haloalkyl, haloalkoxy, cycloalkyl, aryl, heterocycloalkyl, heteroaryl, and/or heteroalkyl) wherein at least one (e.g., 1 to 5 or 1 to 3) hydrogen atom is replaced by a bond to a non-hydrogen atom such as, but not limited to alkyl, alkenyl, alkynyl, alkoxy, alkylthio, acyl, amido, amino, amidino, aryl, aralkyl, azido, carbamoyl, carboxyl, carboxyl ester, cyano, cycloalkyl, cycloalkylalkyl, guanidino, halo, haloalkyl, haloalkoxy, hydroxyalkyl, heteroalkyl, heteroaryl, heteroarylalkyl, heterocycloalkyl, heterocycloalkylalkyl, -NHNH₂, =NNH₂, imino, imido, hydroxy, oxo, oxime, nitro, sulfonyl, sulfinyl, alkylsulfonyl, alkylsulfinyl, thiocyanate, -S(O)OH, -S(O)₂OH, sulfonamido, thiol, thioxo, N-oxide or -Si(R^{y})₃, wherein each R^{y} is independently hydrogen, alkyl, alkenyl, alkynyl, heteroalkyl, cycloalkyl, aryl, heteroaryl or heterocycloalkyl.

In certain embodiments, "substituted" includes any of the above alkyl, alkenyl, alkynyl, cycloalkyl, heterocycloalkyl, aryl or heteroaryl groups in which one or more (e.g., 1 to 5 or 1 to 3) hydrogen atoms are independently replaced with deuterium, halo, cyano, nitro, azido, oxo, alkyl, alkenyl, alkynyl, haloalkyl, cycloalkyl, heterocycloalkyl, aryl, heteroaryl, -NR^{k}R^{h}, -NR^{k}C(=O)R^{h}, -NR^{k}C(=O)NR^{k}R^{h}, -NR^{k}C(=O)OR^{h}, -NR^{k}S(=O)₁₋₂R^{h}, -C(=O)R^{k}, -C(=O)OR^{k}, -OC(=O)OR^{k}, -OC(=O)R^{k}, -C(=O)NR^{k}R^{h}, -OC(=O)NR^{k}R^{h}, -OR^{k}, -SR^{k}, -S(=O)R^{k}, -S(=O)₂R^{k}, -OS(=O)₁₋₂R^{k}, -S(=O)₁₋₂OR^{k}, -NR^{k}S(=O)₁₋₂NR^{k}R^{h}, =NSO₂R^{k}, =NOR^{k}, -S(=O)₁₋₂NR^{k}R^{h}, -SF₅, -SCF₃ or -OCF₃. In certain embodiments, "substituted" also means any of the above groups in which one or more (e.g., 1 to 5 or 1 to 3) hydrogen atoms are replaced with -C(=O)R^{k}, -C(=O)OR^{k}, -C(=O)NR^{k}R^{h}, -CH₂SO₂R^{k}, or -CH₂SO₂NR^{k}R^{h}. In the foregoing, R^{k} and R^{h} are the same or different and independently hydrogen, alkyl, alkenyl, alkynyl, alkoxy, thioalkyl, aryl, aralkyl, cycloalkyl, cycloalkylalkyl, haloalkyl, heterocycloalkyl, heterocycloalkylalkyl, heteroaryl, and/or heteroarylalkyl. In certain embodiments, "substituted" also means any of the above groups in which one or more (e.g., 1 to 5 or 1 to 3) hydrogen atoms are replaced by a bond to an amino, cyano, hydroxyl, imino, nitro, oxo, thioxo, halo, alkyl, alkoxy, alkylamino, thioalkyl, aryl, aralkyl, cycloalkyl, cycloalkylalkyl, haloalkyl, heterocycloalkyl, N-heterocycloalkyl, heterocycloalkylalkyl, heteroaryl, and/or heteroarylalkyl, or two of R^{k} and R^{h} and Rⁱ are taken together with the atoms to which they are attached to form a heterocycloalkyl ring optionally substituted with oxo, halo or alkyl optionally substituted with oxo, halo, amino, hydroxyl, or alkoxy.

Certain commonly used alternative chemical names may be used. For example, a divalent group such as a divalent "alkyl" group, a divalent "aryl" group, etc., may also be referred to as an "alkylene" group or an "alkylenyl" group, an "arylene" group or an "arylenyl" group, respectively. Also, unless indicated explicitly otherwise, where combinations of groups are referred to herein as one moiety, e.g., arylalkyl or aralkyl, the last mentioned group contains the atom by which the moiety is attached to the rest of the molecule.

Polymers or similar indefinite structures arrived at by defining substituents with further substituents appended ad infinitum (e.g., a substituted aryl having a substituted alkyl which is itself substituted with a substituted aryl group, which is further substituted by a substituted heteroalkyl group, etc.) are not intended for inclusion herein. Unless otherwise noted, the maximum number of serial substitutions in compounds described herein is three. For example, serial substitutions of substituted aryl groups with two other substituted aryl groups are limited to ((substituted aryl)substituted aryl) substituted aryl. Similarly, the above definitions are not intended to include impermissible substitution patterns (e.g., methyl substituted with 5 fluorines or heteroaryl groups having two adjacent oxygen ring atoms). Such impermissible substitution patterns are well known to the skilled artisan. When used to modify a chemical group, the term "substituted" may describe other chemical groups defined herein.

In certain embodiments, as used herein, the phrase "one or more" refers to one to five. In certain embodiments, as used herein, the phrase "one or more" refers to one to three.

Any compound or structure given herein, is intended to represent unlabeled forms as well as isotopically labeled forms (isotopologues) of the compounds. These forms of compounds may also be referred to as and include "isotopically enriched analogs." Isotopically labeled compounds have structures depicted herein, except that one or more atoms are replaced by an atom having a selected atomic mass or mass number. Examples of isotopes that can be incorporated into the disclosed compounds include isotopes of hydrogen, carbon, nitrogen, oxygen, phosphorous, fluorine, chlorine and iodine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ³⁶Cl, ¹²³I, and ¹²⁵I, respectively. Various isotopically labeled compounds of the present disclosure, for example those into which radioactive isotopes such as ³H and ¹⁴C are incorporated. Such isotopically labelled compounds may be useful in metabolic studies, reaction kinetic studies, detection or imaging techniques, such as positron emission tomography (PET) or single-photon emission computed tomography (SPECT) including drug or substrate tissue distribution assays or in radioactive treatment of patients.

The term "isotopically enriched analogs" includes "deuterated analogs" of compounds described herein in which one or more hydrogens is/are replaced by deuterium, such as a hydrogen on a carbon atom. Such compounds exhibit increased resistance to metabolism and are thus useful for increasing the half-life of any compound when administered to a mammal, particularly a human. See, for example, Foster, "Deuterium Isotope Effects in Studies of Drug Metabolism," Trends Pharmacol. Sci. 5(12):524-527 (1984). Such compounds are synthesized by means well known in the art, for example by employing starting materials in which one or more hydrogens have been replaced by deuterium.

Deuterium labelled or substituted therapeutic compounds of the disclosure may have improved DMPK (drug metabolism and pharmacokinetics) properties, relating to distribution, metabolism and excretion (ADME). Substitution with heavier isotopes such as deuterium may afford certain therapeutic advantages resulting from greater metabolic stability, for example increased in vivo half-life, reduced dosage requirements and/or an improvement in therapeutic index (see e.g., A. Kerekes et.al. J. Med. Chem. 2011, 54, 201-210; R. Xu et.al. J. Label Compd. Radiopharm. 2015, 58, 308-312). An ¹⁸F, ³H, ¹¹C labeled compound may be useful for PET or SPECT or other imaging studies. Isotopically labeled compounds of this disclosure and prodrugs thereof can generally be prepared by carrying out the procedures disclosed in the schemes or in the examples and preparations described below by substituting a readily available isotopically labeled reagent for a non-isotopically labeled reagent. It is understood that deuterium in this context is regarded as a substituent in a compound described herein.

One or more constituent atoms of the compounds presented herein can be replaced or substituted with isotopes of the atoms in natural or non-natural abundance. In some embodiments, the compound includes at least one deuterium atom. For example, one or more hydrogen atoms in a compound presented herein can be replaced or substituted by deuterium (e.g., one or more hydrogen atoms of a C₁₋₆ alkyl group can be replaced by deuterium atoms, such as -CH₃ being replaced for - CD₃). In some embodiments, the compound includes two or more deuterium atoms. In some embodiments, the compound includes 1, 2, 3, 4, 5, 6, 7, 8, 9, 10, 11, or 12 deuterium atoms. In some embodiments, all of the hydrogen atoms in a compound can be replaced or substituted by deuterium atoms. Synthetic methods for including isotopes into organic compounds are known in the art (Deuterium Labeling in Organic Chemistry by Alan F. Thomas (New York, NY., Appleton-Century-Crofts, 1971; The Renaissance of H/D Exchange by Jens Atzrodt, Volker Derdau, Thorsten Fey and Jochen Zimmermann, Angew. Chem. Int. Ed. 2007, 7744-7765; The Organic Chemistry of Isotopic Labelling by James R. Hanson, Royal Society of Chemistry, 2011). Isotopically labeled compounds can be used in various studies such as NMR spectroscopy, metabolism experiments, and/or assays.

The concentration of such a heavier isotope, specifically deuterium, may be defined by an isotopic enrichment factor. In the compounds of this disclosure any atom not specifically designated as a particular isotope is meant to represent any stable isotope of that atom. Unless otherwise stated, when a position is designated specifically as "H" or "hydrogen", the position is understood to have hydrogen at its natural abundance isotopic composition. Accordingly, in the compounds of this disclosure any atom specifically designated as a deuterium (D) is meant to represent deuterium. Further, in some embodiments, the corresponding deuterated analog is provided.

In many cases, the compounds of this disclosure are capable of forming acid and/or base salts by virtue of the presence of amino and/or carboxyl groups or groups similar thereto.

Provided also are a pharmaceutically acceptable salt, isotopically enriched analog, deuterated analog, isomer (such as a stereoisomer), mixture of isomers (such as a mixture of stereoisomers), and prodrug of the compounds described herein.

"Pharmaceutically acceptable" or "physiologically acceptable" refer to compounds, salts, compositions, dosage forms and other materials which are useful in preparing a pharmaceutical composition that is suitable for veterinary or human pharmaceutical use.

The term "pharmaceutically acceptable salt" of a given compound refers to salts that retain the biological effectiveness and properties of the given compound and which are not biologically or otherwise undesirable. "Pharmaceutically acceptable salts" or "physiologically acceptable salts" include, for example, salts with inorganic acids and salts with an organic acid. In addition, if the compounds described herein are obtained as an acid addition salt, the free base can be obtained by basifying a solution of the acid salt. Conversely, if the product is a free base, an addition salt, particularly a pharmaceutically acceptable addition salt, may be produced by dissolving the free base in a suitable organic solvent and treating the solution with an acid, in accordance with conventional procedures for preparing acid addition salts from base compounds. Those skilled in the art will recognize various synthetic methodologies that may be used to prepare nontoxic pharmaceutically acceptable addition salts. Pharmaceutically acceptable acid addition salts may be prepared from nontoxic inorganic and organic acids. The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, non-aqueous media like ether, ethyl acetate, alcohols (e.g., methanol, ethanol, iso-propanol or butanol) or acetonitrile (MeCN) are preferred. Lists of suitable salts are found in Remington's Pharmaceutical Sciences , 17th Ed., (Mack Publishing Company, Easton, 1985), p. 1418, Berge et al., J. Pharm. Sci., 1977, 66(1), 1-19 and in Stahl et al., Handbook of Pharmaceutical Salts: Properties, Selection, and Use, (Wiley, 2002).

Some of the compounds exist as tautomers. Tautomers are in equilibrium with one another. For example, amide containing compounds may exist in equilibrium with imidic acid tautomers. Regardless of which tautomer is shown and regardless of the nature of the equilibrium among tautomers, the compounds are understood by one of ordinary skill in the art to comprise both amide and imidic acid tautomers. Thus, the amide containing compounds are understood to include their imidic acid tautomers. Likewise, the imidic acid containing compounds are understood to include their amide tautomers.

The compounds of the invention, or their pharmaceutically acceptable salts include an asymmetric center and may thus give rise to enantiomers, diastereomers, and other stereoisomeric forms that may be defined, in terms of absolute stereochemistry, as (R)- or (S)- or, as (D)- or (L)- for amino acids. The present invention is meant to include all such possible isomers, as well as their racemic and optically pure forms. Optically active (+) and (-), (R)- and (S)-, or (D)- and (L)- isomers may be prepared using chiral synthons or chiral reagents, or resolved using conventional techniques, for example, chromatography and fractional crystallization. Conventional techniques for the preparation/isolation of individual enantiomers include chiral synthesis from a suitable optically pure precursor or resolution of the racemate (or the racemate of a salt or derivative) using, for example, chiral high pressure liquid chromatography (HPLC). When the compounds described herein contain olefinic double bonds or other centers of geometric asymmetry, and unless specified otherwise, it is intended that the compounds include both E and Z geometric isomers.

A "stereoisomer" refers to a compound made up of the same atoms bonded by the same bonds but having different three-dimensional structures, which are not interchangeable. The present invention contemplates various stereoisomers and mixtures thereof and includes "enantiomers," which refers to two stereoisomers whose molecules are nonsuperimposeable mirror images of one another.

"Diastereomers" are stereoisomers that have at least two asymmetric atoms, but which are not mirror-images of each other.

Relative centers of the compounds as depicted herein are indicated graphically using the "thick bond" style (bold or parallel lines) and absolute stereochemistry is depicted using wedge bonds (bold or parallel lines).

"Prodrugs" means any compound which releases an active parent drug according to a structure described herein in vivo when such prodrug is administered to a mammalian subject. Prodrugs of a compound described herein are prepared by modifying functional groups present in the compound described herein in such a way that the modifications may be cleaved in vivo to release the parent compound. Prodrugs may be prepared by modifying functional groups present in the compounds in such a way that the modifications are cleaved, either in routine manipulation or in vivo, to the parent compounds. Prodrugs include compounds described herein wherein a hydroxy, amino, carboxyl, or sulfhydryl group in a compound described herein is bonded to any group that may be cleaved in vivo to regenerate the free hydroxy, amino, or sulfhydryl group, respectively. Examples of prodrugs include, but are not limited to esters (e.g., acetate, formate and benzoate derivatives), amides, guanidines, carbamates (e.g., N,N-dimethylaminocarbonyl) of hydroxy functional groups in compounds described herein and the like. Preparation, selection and use of prodrugs is discussed in T. Higuchi and V. Stella, "Pro-drugs as Novel Delivery Systems," Vol. 14 of the A.C.S. Symposium Series; "Design of Prodrugs," ed. H. Bundgaard, Elsevier, 1985; and in Bioreversible Carriers in Drug Design, ed. Edward B. Roche, American Pharmaceutical Association and Pergamon Press, 1987.

The term "leaving group" refers to an atom or a group of atoms that is displaced in a chemical reaction as stable species taking with it the bonding electrons. The non-limiting examples of a leaving group include, halo, methanesulfonyloxy, p-toluenesulfonyloxy, trifluoromethanesulfonyloxy, nonafluorobutanesulfonyloxy, (4-bromo-benzene)sulfonyloxy, (4-nitrobenzene)sulfonyloxy, (2-nitro-benzene)-sulfonyloxy, (4-isopropyl-benzene)sulfonyloxy, (2,4,6-triisopropyl-benzene)-sulfonyloxy, (2,4,6-trimethyl-benzene)sulfonyloxy, *(4-tert-butyl*benzene)sulfonyloxy, benzenesulfonyloxy, (4-methoxy-benzene)sulfonyloxy, and the like.

The term "amide coupling conditions" refers to the reaction conditions under which an amine and a carboxylic acid couple to form an amide using a coupling reagent in presence of a base. The non-limiting examples of coupling reagents include 1-Ethyl-3-(3-dimethylaminopropyl) carbodiimide (EDC) with hydroxybenzotriazole monohydrate (HOBt), O-(7-Azabenzotriazole-1-yl)-*N*,*N*,*N*,*N'*-tetramethyluronium hexafluorophosphate (HATU), 1-hydroxy-7-azabenzotriazole, and the like. The non-limiting examples of the base include N-methylmorpholine, pyridine, morpholine, imidazole, and the like.

The term "protecting group" refers to a moiety of a compound that masks or alters the properties of a functional group or the properties of the compound as a whole. The chemical substructure of a protective group varies widely. One function of a protective group is to serve as an intermediate in the synthesis of the parental drug substance. Chemical protective groups and strategies for protection/deprotection are well known in the art. See: "Protective Groups in Organic Chemistry", Theodora W. Greene (John Wiley & Sons, Inc., New York, 1991. Protective groups are often utilized to mask the reactivity of certain functional groups, to assist in the efficiency of desired chemical reactions, e.g., making and breaking chemical bonds in an ordered and planned fashion. Protection of functional groups of a compound alters other physical properties besides the reactivity of the protected functional group, such as the polarity, lipophilicity (hydrophobicity), and other properties which can be measured by common analytical tools. Chemically protected intermediates may themselves be biologically active or inactive.

The non-limiting examples of protective groups for a hydroxy (i.e. a "hydroxy protecting group") include methoxymethyl ether, tetrahydropyranyl ether, t-butyl ether, allyl ether, benzyl ether, t-butyldiphenylsilyl ether, acetate ester, pivalate ester, benzoate ester, benzylidene acetal, acetonide, silyl ether, and the like.

### List of Abbreviations and Acronyms

| **Abbreviation** | **Meaning** |
|---|---|
| Amphos₂PdCl₂ | bis(di-tert-butyl(4- |
| | dimethylaminophenyl)phosphine)dichloropalladium(II) |
| anhyd. | anhydrous |
| aq | aqueous |
| δ | Chemical shift (ppm) |
| DCE | dicholoroethane |
| DCM | dichloromethane |
| DIEA | diisopropylethylamine |
| DMAP | Dimethylaminopyridine |
| DMF | Dimethylformamide |
| DMSO | Dimethylsulfoxide |
| dppf | 1,1'-Bis(diphenylphosphino)ferrocene |
| DMAC | Dimethylacetamide |
| TEMED or TMEDA | Tetramethylethylenediamine |
| DMFDEA | N,N-dimethylformamide diethyl acetal |
| eq or equiv. | Equivalent(s) |
| Et | Ethyl |
| EtOAc | ethyl acetate |
| EtOH | ethanol |
| HATU | N-[(dimethylamino)-1H-1,2,3-triazolo-[4,5-b]pyridin-1-ylmethylene]-N-methylmethanaminium hexafluorophosphate N-oxide |
| HPLC | High performance liquid chromatography |
| LC-MS | Liquid chromatography - mass spectrometry |
| MeOH | methanol |
| MS | Mass spectrometry |
| MW or mw | microwaved |
| m/z | Mass to charge ratio |
| NBS | N-bromosuccinimide |
| NMR | Nuclear magnetic resonance spectroscopy |
| o/n | overnight |
| Ph | phenyl |
| Pd(PPh₃)₄ or tetrakis | tetrakis(triphenylphosphine)palladium(0) |
| Prep | preparatory |
| THF | tetrahydrofuran |
| TLC | Thin layer chromatography |

### Compounds

Provided herein is a compound of Formula (II): 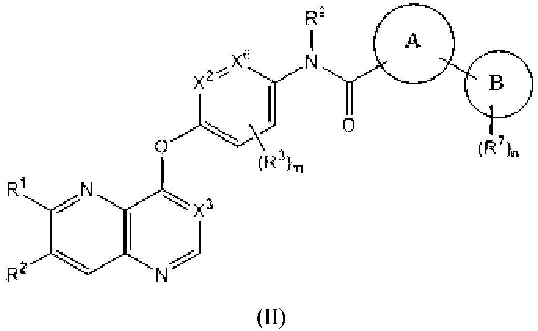 or a pharmaceutically acceptable salt or stereoisomer thereof, wherein:
ring A is 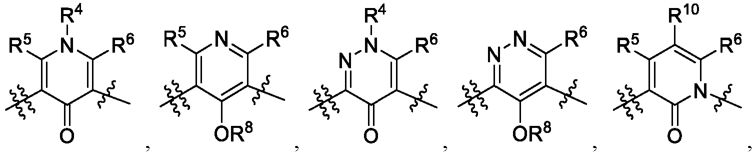 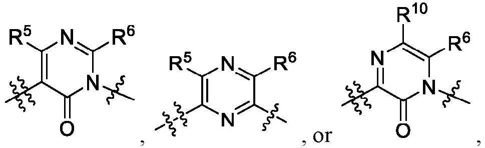 wherein the single wavy line indicates the point of attachment to the ring B and the double wavy line indicates the point of attachment to the rest of the molecule;
ring B is 5-membered heteroaryl having 1, 2 or 3 heteroatoms as ring members selected from N, O and S; 9-10-membered heteroaryl having 1, 2 or 3 heteroatoms as ring members selected from N, O and S; or 6-membered heteroaryl having 1 or 2 nitrogen atoms as ring members;
X² is CH or CR³;
X³ is CH;
X⁶ is CH or CR³;
R¹ and R² are each independently selected from H, C₁₋₆ alkoxy, and C₁₋₆ alkoxy-C₁₋₆ alkoxy;
each R³ is halo;
R⁴ is selected from H, halo, C₁₋₆ alkyl, C₂₋₆ alkenyl, C₂₋₆ alkynyl, C₁₋₆ haloalkyl, C₁₋₆ haloalkoxy, C₆₋₁₀ aryl, C₃₋₁₄ cycloalkyl, 5-14 membered heteroaryl, 4-14 membered heterocycloalkyl, C₆₋₁₀ aryl-C₁₋₄ alkylene-, C₃₋₁₄ cycloalkyl-C₁₋₄ alkylene-, (5-14 membered heteroaryl)-C₁₋₄ alkylene-, (4-14 membered heterocycloalkyl)-C₁₋₄ alkylene-, CN, NO₂, OR^{a}, SR^{a}, NHOR^{a}, C(O)R^{a}, C(O)NR^{a}R^{a}, C(O)OR^{a}, C(O)NR^{a}S(O)₂R^{a}, OC(O)R^{a}, OC(O)NR^{a}R^{a}, NHR^{a}, NR^{a}R^{a}, NR^{a}C(O)R^{a}, N=C(NR^{a}R^{a})₂, NR^{a}C(=NR^{a})R^{a}, NR^{a}C(O)OR^{a}, NR^{a}C(O)NR^{a}R^{a}, C(=NR^{a})R^{a}, C(=NOH)R^{a}, C(=NOH)NR^{a}, C(=NCN)NR^{a}R^{a} , NR^{a}C(=NCN)NR^{a}R", C(=NR^{a})NR^{a}R^{a}, NR^{a}C(=NR^{a})NR^{a}R^{a}, NR^{a}S(O)R^{a}, NR^{a}S(O)₂R^{a}, NR^{a}S(O)₂NR^{a}R^{a}, S(O)R^{a}, S(O)NR^{a}R^{a}, S(O)₂R^{a}, S(O)₂NR^{a}C(O)R^{a}, P(O)R^{a}R^{a}, P(O)(OR^{a})(OR^{a}), B(OH)₂, B(OR^{a})₂, and S(O)₂NR^{a}R^{a}, wherein the C₁₋₆ alkyl, C₂₋₆ alkenyl, C₂₋₆ alkynyl, C₆₋₁₀ aryl, C₃₋₁₄ cycloalkyl, 5-14 membered heteroaryl, 4-14 membered heterocycloalkyl, C₆₋₁₀ aryl-C₁₋₄ alkylene-, C₃₋₁₄ cycloalkyl-C₁₋₄ alkylene-, (5-14 membered heteroaryl)-C₁₋₄ alkylene-, and (4-14 membered heterocycloalkyl)-C₁₋₄ alkylene- of R⁴ are each optionally substituted with 1, 2, 3, 4 or 5 independently selected R^{b} substituents;
R⁵, R⁶ and R¹⁰ are each independently H, halo, C₁₋₆ alkyl, C₂₋₆ alkenyl, C₁₋₆ alkoxy, C₁₋₆ alkylthio, CN, C₁₋₄ haloalkyl, C₁₋₄ haloalkoxy, OH, C₁₋₄alkyl-C(O)-, C₁₋₄alkyl-OC(O)-, -CONH(C₁₋₄ alkyl), NH₂, -NHC₁₋₄alkyl, or -N(C₁₋₄ alkyl)₂, wherein the C₁₋₆ alkyl, C₂₋₆ alkenyl, C₁₋₆ alkoxy, C₁₋₆ alkylthio, C₁₋₆ alkyl-C(O)- and C₁₋₄ alkyl of -NH(C₁₋₄alkyl), or -N(C₁₋₄ alkyl)₂ of R⁵, R⁶ and R¹⁰ are each optionally substituted with 1 or 2 independently selected R^{g} substituents;
each R⁷ is independently selected from halo, OH, C(O)OR^{a}, C(O)R^{a}, C(O)NR^{a}R^{a}, CN, NH₂, - NH(C₁₋C₆ alkyl), -N(C₁₋C₆ alkyl)₂, C₁₋C₆ alkyl, C₂₋₆ alkenyl, C₁₋C₆ alkoxy, C₁₋C₆ haloalkyl, C₁₋C₆ haloalkoxy, C(O)NR^{a}R^{a}, NR^{a}C(O)R^{a}, NR^{a}C(O)NR^{a}R^{a}, SO₂R^{a}, NR^{a}S(O)₂R^{a}, NR^{a}S(O)₂NR^{a}R^{a}, C₃₋C₆ cycloalkyl, 4- to 6-membered heterocycloalkyl, phenyl, 5- or 6-membered heteroaryl, C₃₋C₆ cycloalkyl-C₁₋C₄ alkylene-, (4- to 6-membered heterocycloalkyl)-C₁₋C₄ alkylene-, phenyl-C₁₋C₂ alkylene, and (5- or 6-membered heteroaryl)-C₁₋C₄ alkylene-; wherein the C₁₋C₆ alkyl, C₁₋₆ alkenyl, C₁₋C₆ alkoxy, C₃₋C₆ cycloalkyl, 4- to 6-membered heterocycloalkyl, phenyl, 5- or 6-membered heteroaryl, C₃₋C₆ cycloalkyl-C₁₋C₄ alkylene-, (4- to 6-membered heterocycloalkyl)-C₁₋C₄ alkylene-, phenyl-C₁₋C₂ alkylene, and (5- or 6-membered heteroaryl)-C₁₋C₄ alkylene- of R⁷ are each optionally substituted with 1, 2, or 3 independently selected R^{f} substituents;
R⁸ is H or C₁₋₆ alkyl optionally substituted with 1 or 2 R^{g} substituents or a hydroxy protecting group;
R⁹ is H or C₁₋₆ alkyl optionally substituted with 1, 2, or 3 independently selected R^{g} substituents;
or R⁴ and R⁵ taken together with the atoms to which they are attached form 4- to 7-membered fused heterocycloalkyl or 5- to 6-membered fused heteroaryl, wherein the 4- to 7-membered fused heterocycloalkyl and 5- to 6-membered fused heteroaryl are each optionally substituted with 1 or 2 independently selected R^{g} substituents;
or R¹⁰ and R⁵ taken together with the atoms to which they are attached form fused C₃₋₇ cycloalkyl, 4- to 6-membered fused heterocycloalkyl, fused 5- or 6-membered heteroaryl or fused phenyl, wherein the fused C₃₋₇ cycloalkyl, 4- to 6-membered fused heterocycloalkyl, 5- or 6-membered heteroaryl, or fused phenyl is each optionally substituted with 1 or 2 independently selected R^{g} substituents and wherein one or two ring carbon atoms of the fused C₃₋₇ cycloalkyl or fused heterocycloalkyl are optionally replaced by a carbonyl group;
or R⁶ and R⁵ taken together with the atoms to which they are attached form fused C₃₋₇ cycloalkyl, 4- to 6-membered fused heterocycloalkyl, 5- to 6-membered fused heteroaryl, or fused phenyl, wherein the fused C₃₋₇ cycloalkyl, 4- to 6-membered fused heterocycloalkyl, 5- to 6-membered fused heteroaryl, and fused phenyl are each optionally substituted with 1 or 2 independently selected R^{g} substituents and wherein one or two ring carbon atoms of the fused C₃₋₇ cycloalkyl or 4- to 6-membered fused heterocycloalkyl are optionally replaced by a carbonyl;
or R⁶ and R⁴ taken together with the atoms to which they are attached form 4- to 7-membered fused heterocycloalkyl or 5- to 6-membered fused heteroaryl, wherein the 4- to 7-membered fused heterocycloalkyl and 5- to 6-membered fused heteroaryl are each optionally substituted with 1 or 2 independently selected R^{g} substituents;
or R⁶ and R¹⁰ taken together with the atoms to which they are attached form fused C₃₋₇ cycloalkyl, 4- to 6-membered fused heterocycloalkyl, 5- to 6-membered fused heteroaryl, or fused heteroaryl, wherein the fused C₃₋₇ cycloalkyl, 4- to 6-membered fused heterocycloalkyl, 5- to 6-membered fused heteroaryl, and fused heteroaryl are each optionally substituted with 1 or 2 independently selected R^{g} substituents and wherein one or two ring carbon atoms of the fused C₃₋₇ cycloalkyl or 4- to 6-membered fused heterocycloalkyl are optionally replaced by a carbonyl;
each R^{a} is independently selected from the group consisting of H, CN, C₁₋C₆ alkyl, C₁₋C₆ haloalkyl, C₂-C₆ alkenyl, C₂-C₆ alkynyl, C₆-C₁₀ aryl, C₃-C₁₀ cycloalkyl, 5-14 membered heteroaryl, 4-14 membered heterocycloalkyl, C₆-C₁₀ aryl-C₁₋C₄ alkylene-, C₃-C₁₀ cycloalkyl-C₁₋C₄ alkylene-, (5-14 membered heteroaryl)-C₁₋C₄ alkylene-, and (4-14 membered heterocycloalkyl)-C₁₋C₄ alkylene-; wherein the C₁₋C₆ alkyl, C₁₋C₆ haloalkyl, C₂-C₆ alkenyl, C₂-C₆ alkynyl, C₆-C₁₀ aryl, C₃-C₁₀ cycloalkyl, 5-14 membered heteroaryl, 4-14 membered heterocycloalkyl, C₆-C₁₀ aryl-C₁₋C₄ alkylene-, C₃-C₁₀ cycloalkyl-C₁₋C₄ alkylene-, (5-14 membered heteroaryl)-C₁₋C₄ alkylene-, and (4-14 membered heterocycloalkyl)-C₁₋C₄ alkylene- of R^{a} are each optionally substituted with 1, 2, 3, 4, or 5 independently selected R^{d} substituents;
or any two R^{a} substituents together with the nitrogen atom to which they are attached form 4-, 5-, 6-, 7-, 8-, 9-, or 10-membered heterocycloalkyl, each of which is optionally substituted with 1, 2, or 3 independently selected R^{f} substituents;
each R^{b} is independently selected from the group consisting of halo, C₁₋C₆ alkyl, C₂-C₆ alkenyl, C₂-C₆ alkynyl, C₁₋C₆ haloalkyl, C₁₋C₆ haloalkoxy, C₆-C₁₀ aryl, C₃-C₁₀ cycloalkyl, 5-10 membered heteroaryl, 4-10 membered heterocycloalkyl, C₆₋C₁₀ aryl-C₁₋C₄ alkylene-, C₃-C₁₀ cycloalkyl-C₁₋C₄ alkylene-, (5-10 membered heteroaryl)-C₁₋C₄ alkylene-, (4-10 membered heterocycloalkyl)-C₁₋C₄ alkylene-, CN, OH, NH₂, NO₂, NHOR^{c}, OR^{c}, SR^{c}, C(O)R^{c}, C(O)NR^{c}R^{c}, C(O)OR^{a}, C(O)NR^{c}S(O)₂R^{c}, OC(O)R^{c}, OC(O)NR^{c}R^{c}, C(=NOH)R^{c}, C(=NOH)NR^{c}, C(=NCN)NR^{c}R^{c}, NR^{c}C(=NCN)NR^{c}R^{c}, C(=NR^{c})NR^{c}R^{c}, NR^{c}C(=NR^{c})NR^{c}R^{c}, NHR^{c}, NR^{c}R^{c}, NR^{c}C(O)R^{c}, NR^{c}C(=NR^{c})R^{c}, NR^{c}C(O)OR^{c}, NR^{c}C(O)NR^{c}R^{c}, NR^{c}S(O)R^{c}, NR^{c}S(O)₂R^{c}, NR^{c}S(O)₂NR^{c}R^{c}, S(O)R^{c}, S(O)NR^{c}R^{c}, S(O)₂R^{c}, S(O)₂NR^{c}C(O)R^{c}, Si(R^{c})₃, P(O)R^{c}R^{c}, P(O)(OR^{c})(OR^{c}), B(OH)₂, B(OR^{c})₂, and S(O)₂NR^{c}R^{c}; wherein the C₁₋C₆ alkyl, C₂-C₆ alkenyl, C₂-C₆ alkynyl, C₆-C₁₀ aryl, C₃-C₁₀ cycloalkyl, 5-10 membered heteroaryl, 4-10 membered heterocycloalkyl, C₆-C₁₀ aryl-C₁₋C₄ alkylene-, C₃-C₁₀ cycloalkyl-C₁₋C₄alkylene-, (5-10 membered heteroaryl)-C₁₋C₄ alkylene-, and (4-10 membered heterocycloalkyl)-C₁₋C₄ alkylene- of R^{b} are each further optionally substituted with 1, 2, or 3 independently selected R^{d} substituents;
each R^{c} is independently selected from the group consisting of H, C₁₋C₆ alkyl, C₁₋C₆ haloalkyl, C₂-C₆ alkenyl, C₂-C₆ alkynyl, C₆-C₁₀ aryl, C₃-C₁₀ cycloalkyl, 5-10 membered heteroaryl, 4-10 membered heterocycloalkyl, C₆-C₁₀ aryl-C₁₋C₄ alkylene-, C₃-C₁₀ cycloalkyl-C₁₋C₄ alkylene-, (5-10 membered heteroaryl)-C₁₋C₄ alkylene-, and (4-10 membered heterocycloalkyl)-C₁₋C₄ alkylene-; wherein the C₁₋C₆ alkyl, C₂-C₆ alkenyl, C₂-C₆ alkynyl, C₆-C₁₀ aryl, C₃-C₁₀ cycloalkyl, 5-10 membered heteroaryl, 4-10 membered heterocycloalkyl, C₆-C₁₀ aryl-C₁₋C₄ alkylene-, C₃-C₁₀ cycloalkyl-C₁₋C₄ alkylene-, (5-10 membered heteroaryl)-C₁₋C₄ alkylene-, and (4-10 membered heterocycloalkyl)-C₁₋C₄ alkylene- of R^{c} are each optionally substituted with 1, 2, 3, 4, or 5 independently selected R^{f} substituents;
or any two R^{c} substituents together with the nitrogen atom to which they are attached form 4-, 5-, 6-, 7-, 8-, 9-, or 10-membered heterocycloalkyl, each of which is optionally substituted with 1, 2, or 3 independently selected R^{f} substituents;
each R^{d} is independently selected from the group consisting of C₁₋C₆ alkyl, C₁₋C₆ haloalkyl, halo, C₆₋C₁₀ aryl, 5-10 membered heteroaryl, C₃-C₁₀ cycloalkyl, 4-10 membered heterocycloalkyl, C₆₋C₁₀ aryl-C₁₋C₄ alkylene-, C₃-C₁₀ cycloalkyl-C₁₋C₄ alkylene-, (5-10 membered heteroaryl)-C₁₋C₄ alkylene-, (4-10 membered heterocycloalkyl)-C₁₋C₄ alkylene-, CN, NH₂, NHOR^{e}, OR^{e}, SR^{e}, C(O)R^{e}, C(O)NR^{e}R^{e}, C(O)OR^{e}, OC(O)R^{e}, OC(O)NR^{e}R^{e}, NHR^{e}, NR^{e}R^{e}, NR^{e}C(O)R^{e}, NR^{e}C(O)NR^{e}R^{e}, NR^{e}C(O)OR^{e}, C(=NR^{e})NR^{e}R^{e}, NR^{e}C(=NR^{e})NR^{e}R^{e}, NR^{e}C(=NOH)NR^{e}R^{e}, NR^{e}C(=NCN)NR^{e}R^{e}, S(O)R^{e}, S(O)NR^{e}R^{e}, S(O)₂R^{e}, NR^{e}S(O)₂R^{e}, NR^{e}S(O)₂NR^{e}R^{e}, and S(O)₂NR^{e}R^{e}; wherein the C₁₋C₆ alkyl, C₆₋C₁₀ aryl, 5-10 membered heteroaryl, C₃-C₁₀ cycloalkyl, 4-10 membered heterocycloalkyl, C₆₋C₁₀ aryl-C₁₋C₄ alkylene-, C₃-C₁₀ cycloalkyl-C₁₋C₄ alkylene-, (5-10 membered heteroaryl)-C₁₋C₄ alkylene-, and (4-10 membered heterocycloalkyl)-C₁₋C₄ alkylene- of R^{d} are each optionally substituted with 1, 2, or 3 independently selected R^{f} substituents;
each R^{e} is independently selected from the group consisting of H, C₁₋C₆ alkyl, C₁₋C₆ alkoxy, C₃₋C₆ cycloalkyl, C₃₋C₆ cycloalkyl-C₁₋C₄ alkylene-, C₆-C₁₀ aryl, C₆₋C₁₀ aryl-C₁₋C₄ alkylene-, 5- or 6-membered heteroaryl, (5- or 6-membered heteroaryl)-C₁₋C₄ alkylene-, 4-7-membered heterocycloalkyl, (4-7-membered heterocycloalkyl)-C₁₋C₄ alkylene-, C₁₋C₆ haloalkyl, C₁₋C₆ haloalkoxy, C₂₋C₄ alkenyl, and C₂₋C₄ alkynyl, wherein the C₁₋C₆ alkyl, C₁₋C₆ alkoxy, C₃₋C₆ cycloalkyl, C₆₋C₁₀ aryl, 5 or 6-membered heteroaryl, 4-7-membered heterocycloalkyl, C₆₋C₁₀ aryl-C₁₋C₄ alkylene-, (5- or 6-membered heteroaryl)-C₁₋C₄ alkylene-, (4-7-membered heterocycloalkyl)-C₁₋C₄ alkylene-, C₂₋C₄ alkenyl, and C₂₋C₄ alkynyl of R^{e} are each optionally substituted with 1, 2, or 3 R^{f} substituents;
or any two R^{e} substituents together with the nitrogen atom to which they are attached form a 4-, 5-, 6-, 7-, 8-, 9-, or 10-membered heterocycloalkyl, each of which is optionally substituted with 1, 2, or 3 independently selected R^{f} substituents;
each R^{f} is independently selected from the group consisting of halo, OH, CN, C(O)OH, NH₂, -NH(C₁₋C₆ alkyl), -N(C₁₋C₆ alkyl)₂, C₁₋C₆ alkyl, vinyl, C₁₋C₆ alkoxy, C₁₋C₆ alkylthio, C₁₋C₆ haloalkyl, C₁₋C₆ haloalkoxy, phenyl, 5-6 membered heteroaryl, 4-6 membered heterocycloalkyl, and C₃₋C₆ cycloalkyl, wherein the C₁₋C₆ alkyl, C₁₋C₆ alkoxy, C₁₋C₆ alkylthio, phenyl, C₃₋C₆ cycloalkyl, 4-6 memberd heterocycloalkyl, and 5-6 membered heteroaryl of R^{f} are each optionally substituted with 1, 2, or 3 substituents selected from halo, OH, CN, -C(O)OH, -NH₂, C₁₋C₄ alkyl, C₁₋C₄ alkoxy, C₁₋C₄ haloalkyl, C₁₋C₄ haloalkoxy, phenyl, C₃-C₁₀ cycloalkyl, 5-6 membered heteroaryl, and 4-6 membered heterocycloalkyl
each R^{g} is independently selected from the group consisting of halo, OH, CN, C(O)OH, - C(O)O-C₁₋C₄ alkyl, NH₂, -NH(C₁₋C₆ alkyl), -N(C₁₋C₆ alkyl)₂, C₁₋C₆ alkyl, C₁₋C₆ alkoxy, C₁₋C₆ alkylthio, C₁₋C₆ haloalkyl, C₁₋C₆ haloalkoxy, phenyl, 5-6 membered heteroaryl, 4-6 membered heterocycloalkyl, and C₃₋C₆ cycloalkyl;
a ring nitrogen atom in Formula (II) is optionally oxidized;
the subscript m is 0, 1 or 2; and
the subscript n is 0, 1, 2, 3 or 4.

In some embodiments of Formula (II), or a pharmaceutically acceptable salt or stereoisomer thereof, ring B is 6-membered heteroaryl having 1 or 2 nitrogen atoms as ring members.

In some embodiments of Formula (II) or subformulas thereof, any ring nitrogen atom in Formula (II) or subformulas thereof is optionally oxidized.

In some embodiments, a compound of Formula (II) is a compound having the structure of formula (IIa): 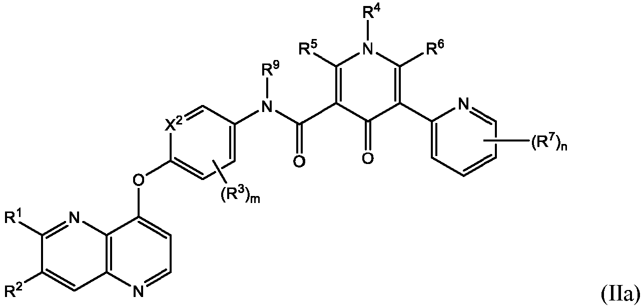 or a pharmaceutically acceptable salt or stereoisomer thereof.

In some embodiments, a compound of Formula (II) is a compound having the structure of formula (Ia-1): 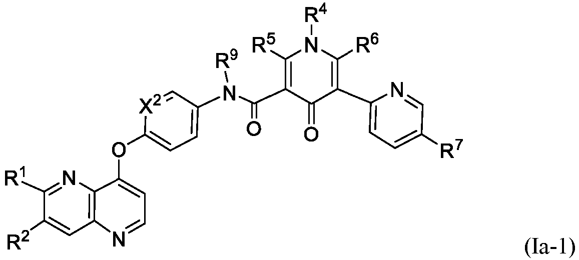 or a pharmaceutically acceptable salt or stereoisomer thereof.

In some embodiments, a compound of Formula (II) is a compound having the structure of formula (IIb): 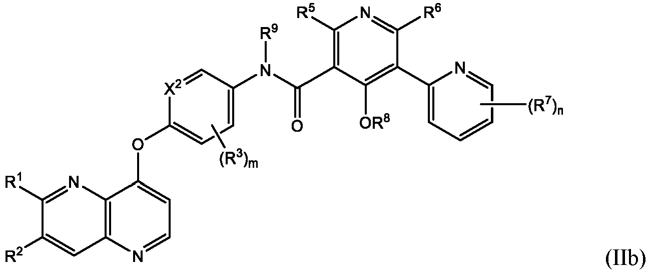 or a pharmaceutically acceptable salt or stereoisomer thereof.

In some embodiments, a compound of Formula (II) is a compound having the structure of formula (Ib-1): 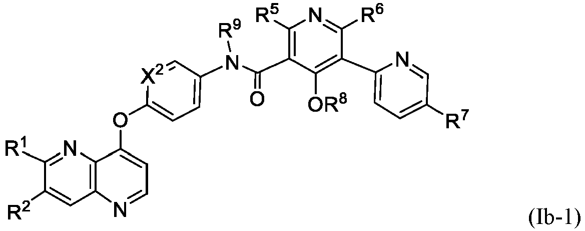 or a pharmaceutically acceptable salt or stereoisomer thereof.

In some or any embodiments described herein, a compound of Formula (II) is a compound having the structure of formula (IIc): 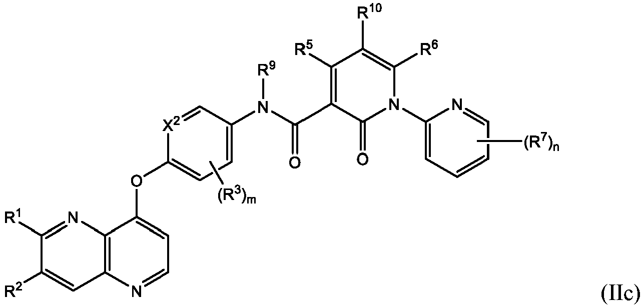 or a pharmaceutically acceptable salt or stereoisomer thereof.

In some or any embodiments described herein, a compound of Formula (II) is a compound having the structure of formula (Ic-1): 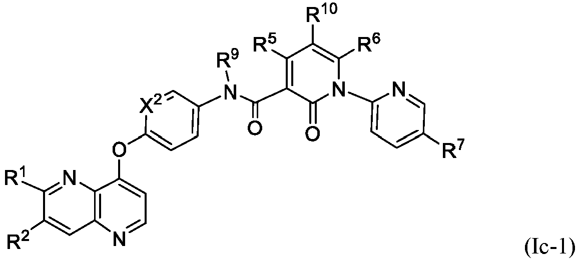 or a pharmaceutically acceptable salt or stereoisomer thereof.

In some or any embodiments described herein, a compound of Formula (II) is a compound having the structure of formula (Id): 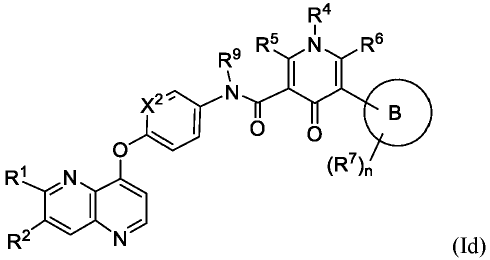 or a pharmaceutically acceptable salt or stereoisomer thereof.

In some or any embodiments described herein, a compound of Formula (II) is a compound having the structure of formula (Ie): 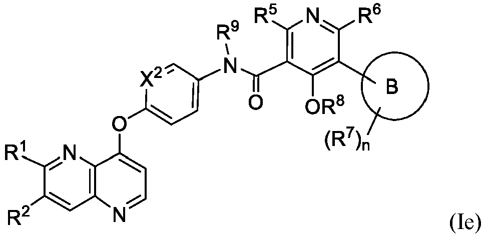 or a pharmaceutically acceptable salt or stereoisomer thereof.

In some or any embodiments described herein, a compound of Formula (II) is a compound having the structure of formula (If): 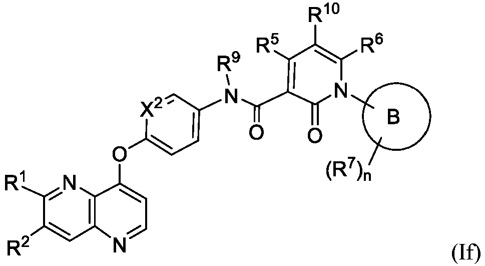 or a pharmaceutically acceptable salt or stereoisomer thereof.

In some or any embodiments described herein, a compound of Formula (II) is a compound having the structure of formula (Ij): 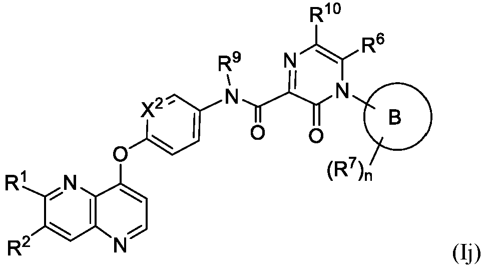 or a pharmaceutically acceptable salt or stereoisomer thereof.

In some or any embodiments described herein, a compound of Formula (II) is a compound having the structure of formula (Ik): 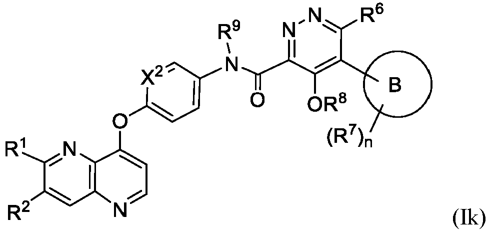 or a pharmaceutically acceptable salt or stereoisomer thereof.

In some or any embodiments described herein, a compound of Formula (II) is a compound having the structure of formula (Im): 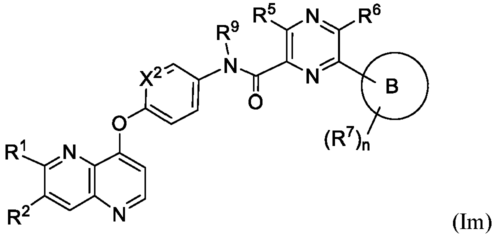 or a pharmaceutically acceptable salt or stereoisomer thereof.

Some embodiments provide a compound of any formula described herein, or a pharmaceutically acceptable salt or stereoisomer thereof, wherein ring B is 2-pyridyl, 3, pyridyl, 4-pyridyl or 5-membered heteroaryl having 1, 2 or 3 heteroatoms as ring members selected from N, O and S.

Some embodiments provide a compound of any formula described herein, or a pharmaceutically acceptable salt or stereoisomer thereof, wherein ring B is 2-thienyl, 3-thienyl, 2-furyl, 3-furyl, 2-benzofuranyl, 3-benzofuranyl, 1H-pyrazol-3-yl, 1H-pyrazol-4-yl, 1H-pyrazol-5-yl or pyrazol-1-yl.

Some embodiments provide a compound of any formula described herein, or a pharmaceutically acceptable salt or stereoisomer thereof, wherein ring B is 2-thienyl, 3-thienyl, 2-furyl, 3-furyl, 1H-pyrazol-3-yl, 1H-pyrazol-4-yl, 1H-pyrazol-5-yl or pyrazol-1-yl.

As used herein, embodiments of "any of the preceding formulas" or "any formula described herein" refers to embodiments of formulas (IIa), (Ia-1), (IIb), (Ib-1), (IIc), (Ic-1), (Id), (Ie), (Ij), (Ik), and/or (Im), and/or any combinations thereof.

Some embodiments provide a compound of any formula described herein, or pharmaceutically acceptable salt or stereoisomer thereof, wherein R¹ and R² are each independently selected from H, methoxy or methoxyethoxy.

Some embodiments provide a compound of any formula described herein, or a pharmaceutically acceptable salt or stereoisomer thereof, wherein n is 0, or wherein n is 1, 2, 3, or 4, each R⁷ is independently selected from halo, C₁₋₆ alkyl, and C₁₋₆ alkoxy.

Some embodiments provide a compound of any formula described herein, or a pharmaceutically acceptable salt or stereoisomer thereof, wherein R⁹ is H or methyl. In some embodiments, R⁹ is H.

Some embodiments provide a compound of any formula described herein, or a pharmaceutically acceptable salt or stereoisomer thereof, wherein X² is CF. Some embodiments provide a compound of any formula described herein, or a pharmaceutically acceptable salt or stereoisomer thereof, wherein X² is CH.

Some embodiments provide a compound of any formula described herein, or a pharmaceutically acceptable salt or stereoisomer thereof, wherein each R⁴ is independently selected from H, C₁₋₆ alkyl, C₁₋₆ alkoxy, OH, NH₂, -NH(C₁₋₆ alkyl), -N(C₁₋₆ alkyl)₂, C₃₋₆ cycloalkyl, C₁₋₆ haloalkyl, C₃₋₆ cycloalkyl-C₁₋₄ alkylene-, 4-6 membered heterocycloalkyl, (4-6 membered heterocycloalkyl)-C₁₋₄ alkylene-, 5-6 membered heteroaryl, (5-6 membered heteroaryl)-C₁₋₄ alkylene-, and N=C[N(C₁₋₆ alkyl)(C₁₋₆ alkyl)]₂, wherein the C₁₋₆ alkyl, C₁₋₆ alkoxy, -NH(C₁₋₆ alkyl), -N(C₁₋₆ alkyl)₂, C₃₋₆ cycloalkyl, C₃₋₆ cycloalkyl-C₁₋₄ alkylene-, 4-6 membered heterocycloalkyl, (4-6 membered heterocycloalkyl)-C₁₋₄ alkylene-, 5-6 membered heteroaryl, (5-6 membered heteroaryl)-C₁₋₄ alkylene-, and N=C[N(C₁₋₆ alkyl)(C₁₋₆ alkyl)]₂ of R⁴ are each optionally substituted with 1 or 2 independently selected R^{b} or R^{g} substituents. In some embodiments, R⁴ is selected from H or methyl.

Some embodiments provide a compound of any formula described herein, or a pharmaceutically acceptable salt or stereoisomer thereof, wherein R⁵, R⁶, and R¹⁰ are each independently selected from H, CH₃, propen-2-yl, Br, Cl, CN, methoxy, 2-fluoroethyl, isopropyl, CH₃C(O)-, OH, t-butyl, ethyl, hydroxymethyl, isopropylthio, and methoxymethyl. In some embodiments, R⁵ and R⁶ are each independently selected from H or methyl. In some embodiments, R¹⁰ is selected from H, CN, halo, or CH₃C(O)-.

Some embodiments provide a compound of any formula described herein, or a pharmaceutically acceptable salt or stereoisomer thereof, wherein each R⁸ is independently H or C₁₋₆ alkyl.

Any of the embodiments described above are applicable to Formula (II) and sub-formulas thereof in any combination. As used herein, "Formula (II) and sub-formulas thereof" refers to Formula (II), (IIa), (Ia-1), (IIb), (Ib-1), (IIc), (Ic-1), (Id), (Ie), (If), (Ij), (Ik), (Im), and/or any combinations thereof.

In some embodiments, provided is a compound, or a pharmaceutically acceptable salt or stereoisomer thereof, selected from Table 1A. In some embodiments, provided is a compound, or a pharmaceutically acceptable salt or stereoisomer thereof, selected from Table 1B. In some embodiments, provided is a compound, or a pharmaceutically acceptable salt or stereoisomer thereof, selected from Table 1C. In some embodiments, provided is a compound, or a pharmaceutically acceptable salt or stereoisomer thereof, selected from Table 1A and/or Table 1B and/or Table 1C.

**Table 1A**

| **Compound No.** | **Structure** | **Compound Name** |
|---|---|---|
| 41 | 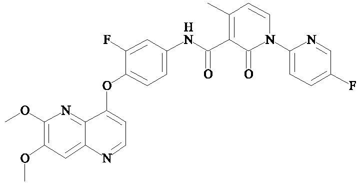 | N-[4-[(6,7-dimethoxy-1,5-naphthyridin-4-yl)oxy]-3-fluorophenyl]-1-(5-fluoropyridin-2-yl)-4-methyl-2-oxopyridine-3-carboxamide |
| 42 | 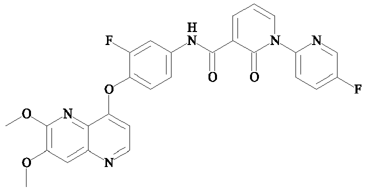 | N-[4-[(6,7-dimethoxy-1,5-naphthyridin-4-yl)oxy]-3-fluorophenyl]-1-(5-fluoropyridin-2-yl)-2-oxopyridine-3-carboxamide |
| 43 | 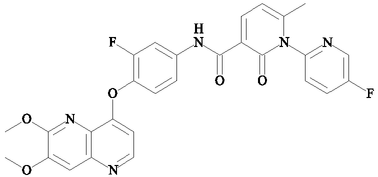 | N-[4-[(6,7-dimethoxy-1,5-naphthyridin-4-yl)oxy]-3-fluorophenyl]-1-(5-fluoropyridin-2-yl)-6-methyl-2-oxopyridine-3-carboxamide |
| 44 | 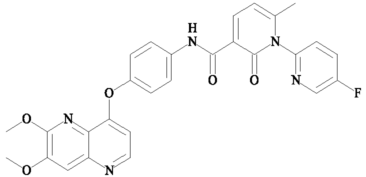 | N-[4-[(6,7-dimethoxy-1,5-naphthyridin-4-yl)oxy]phenyl]-1-(5-fluoropyridin-2-yl)-6-methyl-2-oxopyridine-3-carboxamide |
| 45 | 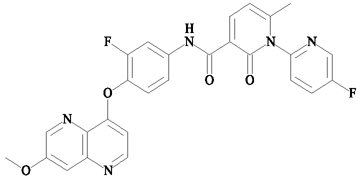 | N-[3-fluoro-4-[(7-methoxy-1,5-naphthyridin-4-yl)oxy]phenyl]-1-(5-fluoropyridin-2-yl)-6-methyl-2-oxopyridine-3-carboxamide |
| 46 | 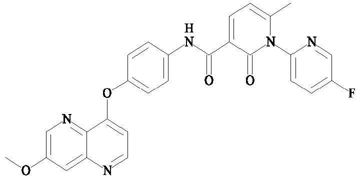 | 1-(5-fluoropyridin-2-yl)-N-[4-[(7-methoxy-1,5-naphthyridin-4-yl)oxy]phenyl]-6-methyl-2-oxopyridine-3-carboxamide |
| 47 | 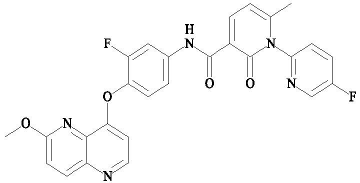 | N-[3-fluoro-4-[(6-methoxy-1,5-naphthyridin-4-yl)oxy]phenyl]-1-(5-fluoropyridin-2-yl)-6-methyl-2-oxopyridine-3-carboxamide |
| 48 | 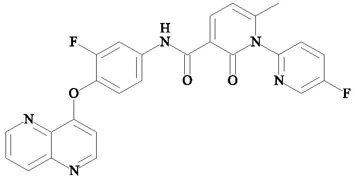 | N-[3-fluoro-4-(1,5-naphthyridin-4-yloxy)phenyl]-1-(5-fluoropyridin-2-yl)-6-methyl-2-oxopyridine-3-carboxamide |
| 50 | 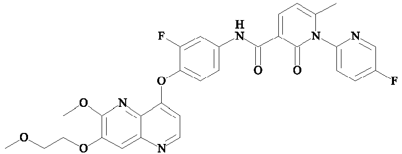 | N-[3-fluoro-4-[[6-methoxy-7-(2-methoxyethoxy)-1,5-naphthyridin-4-yl]oxy]phenyl]-1-(5-fluoropyridin-2-yl)-6-methyl-2-oxopyridine-3-carboxamide |
| 51 | 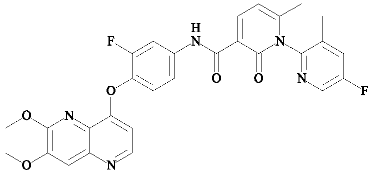 | N-[4-[(6,7-dimethoxy-1,5-naphthyridin-4-yl)oxy]-3-fluorophenyl]-1-(5-fluoro-3-methylpyridin-2-yl)-6-methyl-2-oxopyridine-3-carboxamide |
| 52 | 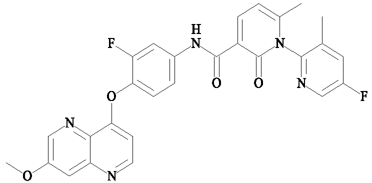 | N-[3-fluoro-4-[(7-methoxy-1,5-naphthyridin-4-yl)oxy]phenyl]-1-(5-fluoro-3-methylpyridin-2-yl)-6-methyl-2-oxopyridine-3-carboxamide |
| 53 | 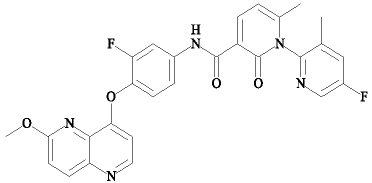 | N-[3-fluoro-4-[(6-methoxy-1,5-naphthyridin-4-yl)oxy]phenyl]-1-(5-fluoro-3-methylpyridin-2-yl)-6-methyl-2-oxopyridine-3-carboxamide |
| 55 | 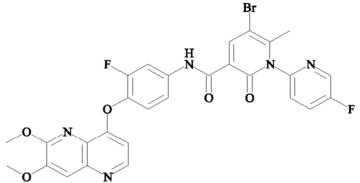 | 5-bromo-N-[4-[(6,7-dimethoxy-1,5-naphthyridin-4-yl)oxy]-3-fluorophenyl]-1-(5-fluoropyridin-2-yl)-6-methyl-2-oxopyridine-3-carboxamide |
| 56 | 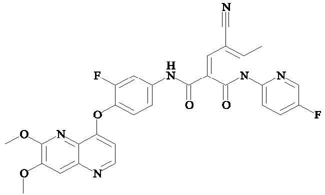 | 5-cyano-N-[4-[(6,7-dimethoxy-1,5-naphthyridin-4-yl)oxy]-3-fluorophenyl]-1-(5-fluoropyridin-2-yl)-6-methyl-2-oxopyridine-3-carboxamide |
| 59 | 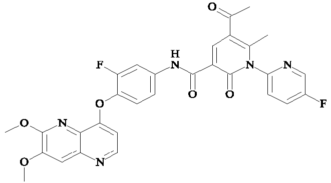 | 5-acetyl-N-[4-[(6,7-dimethoxy-1,5-naphthyridin-4-yl)oxy]-3-fluorophenyl]-1-(5-fluoropyridin-2-yl)-6-methyl-2-oxopyridine-3-carboxamide |
| 68 | 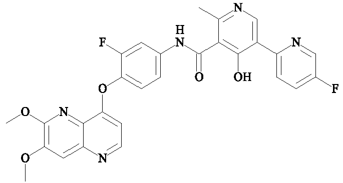 | N-[4-[(6,7-dimethoxy-1,5-naphthyridin-4-yl)oxy]-3-fluorophenyl]-5-(5-fluoropyridin-2-yl)-4-hydroxy-2-methylpyridine-3-carboxamide |
| 69 | 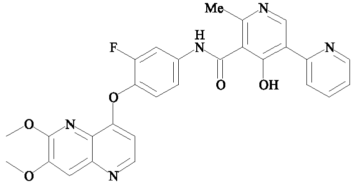 | N-[4-[(6,7-dimethoxy-1,5-naphthyridin-4-yl)oxy]-3-fluorophenyl]-4-hydroxy-2-methyl-5-pyridin-2-ylpyridine-3-carboxamide |
| 70 | 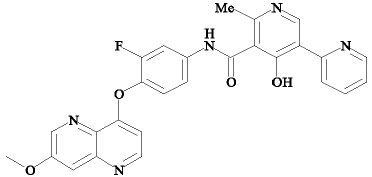 | N-[3-fluoro-4-[(7-methoxy-1,5-naphthyridin-4-yl)oxy]phenyl]-4-hydroxy-2-methyl-5-pyridin-2-ylpyridine-3-carboxamide |
| 71 | 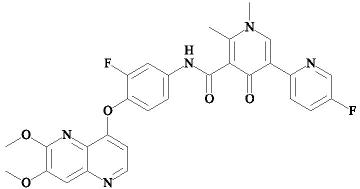 | N-[4-[(6,7-dimethoxy-1,5-naphthyridin-4-yl)oxy]-3-fluorophenyl]-5-(5-fluoropyridin-2-yl)-1,2-dimethyl-4-oxopyridine-3-carboxamide |
| 72 | 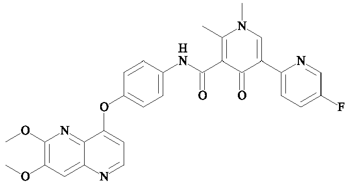 | N-[4-[(6,7-dimethoxy-1,5-naphthyridin-4-yl)oxy]phenyl]-5-(5-fluoropyridin-2-yl)-1,2-dimethyl-4-oxopyridine-3-carboxamide |
| 73 | 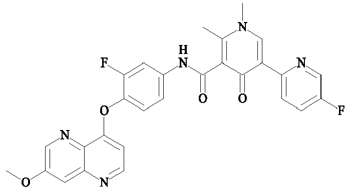 | N-[3-fluoro-4-[(7-methoxy-1,5-naphthyridin-4-yl)oxy]phenyl]-5-(5-fluoropyridin-2-yl)-1,2-dimethyl-4-oxopyridine-3-carboxamide |
| 85 | 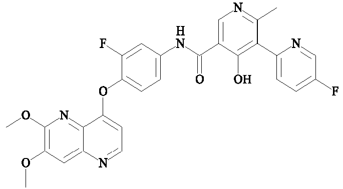 | N-[4-[(6,7-dimethoxy-1,5-naphthyridin-4-yl)oxy]-3-fluorophenyl]-5-(5-fluoropyridin-2-yl)-4-hydroxy-6-methylpyridine-3-carboxamide |

or a pharmaceutically acceptable salt, a stereoisomer, or a mixture of stereoisomers thereof.

**Table 1B**

| **Compound No.** | **Structure** | **Compound Name** |
|---|---|---|
| 86 | 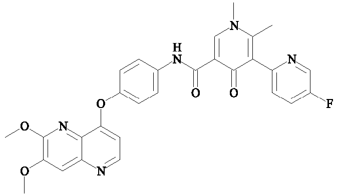 | N-[4-[(6,7-dimethoxy-1,5-naphthyridin-4-yl)oxy]phenyl]-5-(5-fluoropyridin-2-yl)-1,6-dimethyl-4-oxopyridine-3-carboxamide |
| 89 | 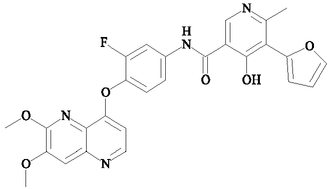 | N-[4-[(6,7-dimethoxy-1,5-naphthyridin-4-yl)oxy]-3-fluorophenyl]-5-(furan-2-yl)-4-hydroxy-6-methylpyridine-3-carboxamide |
| 90 | 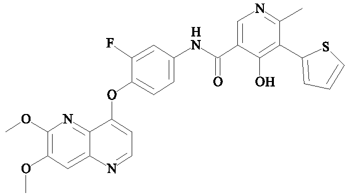 | N-[4-[(6,7-dimethoxy-1,5-naphthyridin-4-yl)oxy]-3-fluorophenyl]-4-hydroxy-6-methyl-5-thiophen-2-ylpyridine-3-carboxamide |
| 91 | 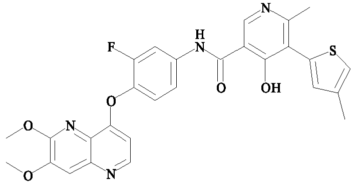 | N-[4-[(6,7-dimethoxy-1,5-naphthyridin-4-yl)oxy]-3-fluorophenyl]-4-hydroxy-6-methyl-5-(4-methylthiophen-2-yl)pyridine-3-carboxamide |
| 92 | 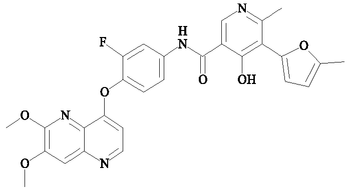 | N-[4-[(6,7-dimethoxy-1,5-naphthyridin-4-yl)oxy]-3-fluorophenyl]-4-hydroxy-6-methyl-5-(5-methylfuran-2-yl)pyridine-3-carboxamide |
| 93 | 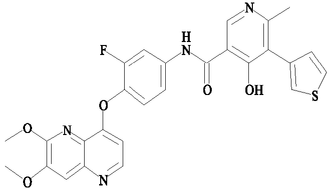 | N-[4-[(6,7-dimethoxy-1,5-naphthyridin-4-yl)oxy]-3-fluorophenyl]-4-hydroxy-6-methyl-5-thiophen-3-ylpyridine-3-carboxamide |
| 94 | 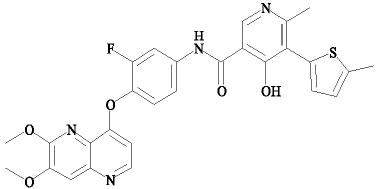 | N-[4-[(6,7-dimethoxy-1,5-naphthyridin-4-yl)oxy]-3-fluorophenyl]-4-hydroxy-6-methyl-5-(5-methylthiophen-2-yl)pyridine-3-carboxamide |
| 95 | 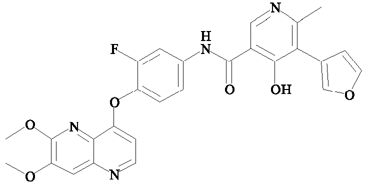 | N-[4-[(6,7-dimethoxy-1,5-naphthyridin-4-yl)oxy]-3-fluorophenyl]-5-(furan-3-yl)-4-hydroxy-6-methylpyridine-3-carboxamide |
| 96 | 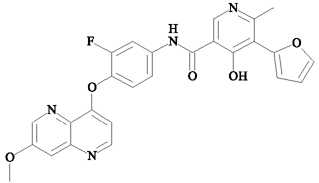 | N-[3-fluoro-4-[(7-methoxy-1,5-naphthyridin-4-yl)oxy]phenyl]-5-(furan-2-yl)-4-hydroxy-6-methylpyridine-3-carboxamide |
| 97 | 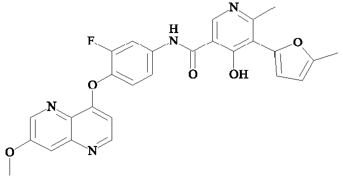 | N-[3-fluoro-4-[(7-methoxy-1,5-naphthyridin-4-yl)oxy]phenyl]-4-hydroxy-6-methyl-5-(5-methylfuran-2-yl)pyridine-3-carboxamide |
| 98 | 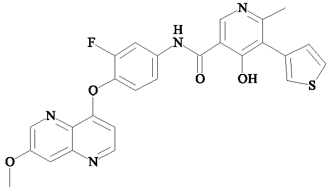 | N-[3-fluoro-4-[(7-methoxy-1,5-naphthyridin-4-yl)oxy]phenyl]-4-hydroxy-6-methyl-5-thiophen-3-ylpyridine-3-carboxamide |
| 99 | 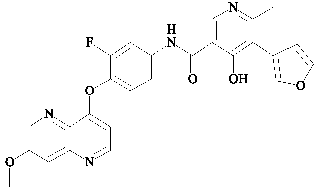 | N-[3-fluoro-4-[(7-methoxy-1,5-naphthyridin-4-yl)oxy]phenyl]-5-(furan-3-yl)-4-hydroxy-6-methylpyridine-3-carboxamide |
| 100 | 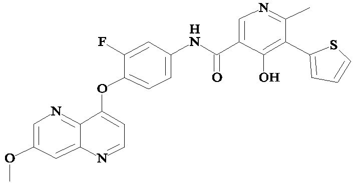 | N-[3-fluoro-4-[(7-methoxy-1,5-naphthyridin-4-yl)oxy]phenyl]-4-hydroxy-6-methyl-5-thiophen-2-ylpyridine-3-carboxamide |
| 101 | 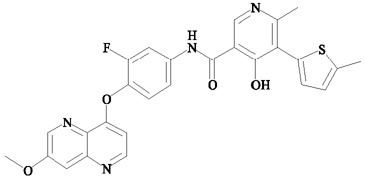 | N-[3-fluoro-4-[(7-methoxy-1,5-naphthyridin-4-yl)oxy]phenyl]-4-hydroxy-6-methyl-5-(5-methylthiophen-2-yl)pyridine-3-carboxamide |
| 102 | 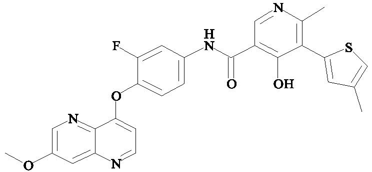 | N-[3-fluoro-4-[(7-methoxy-1,5-naphthyridin-4-yl)oxy]phenyl]-4-hydroxy-6-methyl-5-(4-methylthiophen-2-yl)pyridine-3-carboxamide |
| 103 | 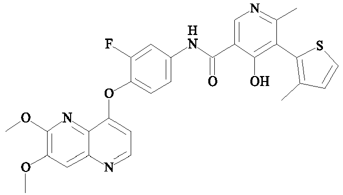 | N-[4-[(6,7-dimethoxy-1,5-naphthyridin-4-yl)oxy]-3-fluorophenyl]-4-hydroxy-6-methyl-5-(3-methylthiophen-2-yl)pyridine-3-carboxamide |
| 104 | 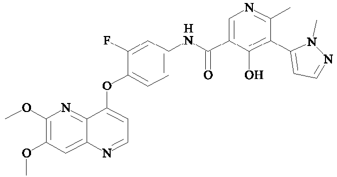 | N-[4-[(6,7-dimethoxy-1,5-naphthyridin-4-yl)oxy]-3-fluorophenyl]-4-hydroxy-6-methyl-5-(2-methylpyrazol-3-yl)pyridine-3-carboxamide |
| 105 | 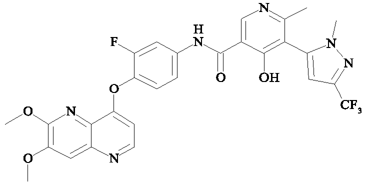 | N-[4-[(6,7-dimethoxy-1,5-naphthyridin-4-yl)oxy]-3-fluorophenyl]-4-hydroxy-6-methyl-5-[2-methyl-5-(trifluoromethyl)pyrazol-3-yl]pyridine-3-carboxamide |
| 106 | 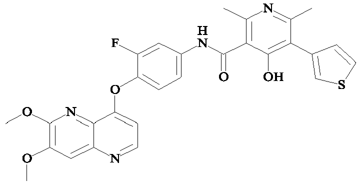 | N-[4-[(6,7-dimethoxy-1,5-naphthyridin-4-yl)oxy]-3-fluorophenyl]-4-hydroxy-2,6-dimethyl-5-thiophen-3-ylpyridine-3-carboxamide |
| 107 | 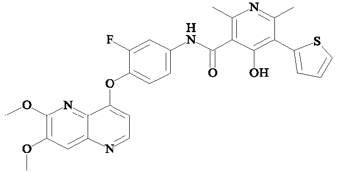 | N-[4-[(6,7-dimethoxy-1,5-naphthyridin-4-yl)oxy]-3-fluorophenyl]-4-hydroxy-2,6-dimethyl-5-thiophen-2-ylpyridine-3-carboxamide |
| 108 | 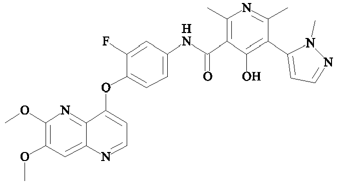 | N-[4-[(6,7-dimethoxy-1,5-naphthyridin-4-yl)oxy]-3-fluorophenyl]-4-hydroxy-2,6-dimethyl-5-(2-methylpyrazol-3-yl)pyridine-3-carboxamide |
| 109 | 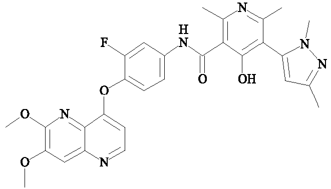 | N-[4-[(6,7-dimethoxy-1,5-naphthyridin-4-yl)oxy]-3-fluorophenyl]-5-(2,5-dimethylpyrazol-3-yl)-4-hydroxy-2,6-dimethylpyridine-3-carboxamide |
| 110 | 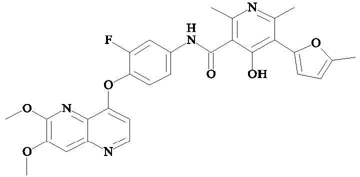 | N-[4-[(6,7-dimethoxy-1,5-naphthyridin-4-yl)oxy]-3-fluorophenyl]-4-hydroxy-2,6-dimethyl-5-(5-methylfuran-2-yl)pyridine-3-carboxamide |
| 111 | 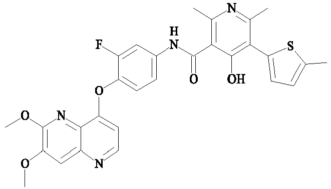 | N-[4-[(6,7-dimethoxy-1,5-naphthyridin-4-yl)oxy]-3-fluorophenyl]-4-hydroxy-2,6-dimethyl-5-(5-methylthiophen-2-yl)pyridine-3-carboxamide |
| 112 | 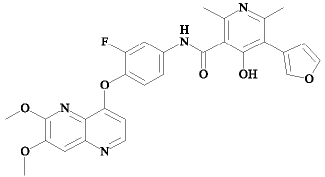 | N-[4-[(6,7-dimethoxy-1,5-naphthyridin-4-yl)oxy]-3-fluorophenyl]-5-(furan-3-yl)-4-hydroxy-2,6-dimethylpyridine-3-carboxamide |
| 115 | 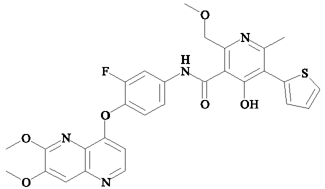 | N-[4-[(6,7-dimethoxy-1,5-naphthyridin-4-yl)oxy]-3-fluorophenyl]-4-hydroxy-2-(methoxymethyl)-6-methyl-5-thiophen-2-ylpyridine-3-carboxamide |
| 116 | 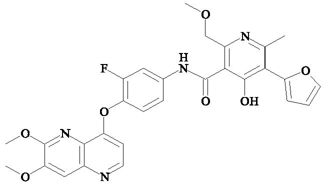 | N-[4-[(6,7-dimethoxy-1,5-naphthyridin-4-yl)oxy]-3-fluorophenyl]-5-(furan-2-yl)-4-hydroxy-2-(methoxymethyl)-6-methylpyridine-3-carboxamide |
| 117 | 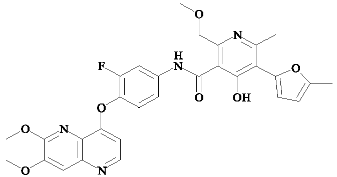 | N-[4-[(6,7-dimethoxy-1,5-naphthyridin-4-yl)oxy]-3-fluorophenyl]-4-hydroxy-2-(methoxymethyl)-6-methyl-5-(5-methylfuran-2-yl)pyridine-3-carboxamide |
| 118 | 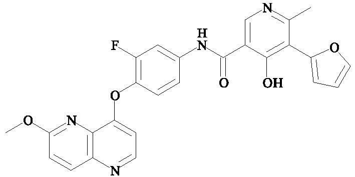 | N-[3-fluoro-4-[(6-methoxy-1,5-naphthyridin-4-yl)oxy]phenyl]-5-(furan-2-yl)-4-hydroxy-6-methylpyridine-3-carboxamide |
| 119 | 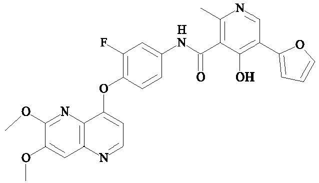 | N-[4-[(6,7-dimethoxy-1,5-naphthyridin-4-yl)oxy]-3-fluorophenyl]-5-(furan-2-yl)-4-hydroxy-2-methylpyridine-3-carboxamide |
| 120 | 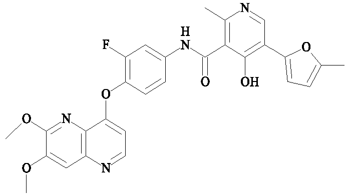 | N-[4-[(6,7-dimethoxy-1,5-naphthyridin-4-yl)oxy]-3-fluorophenyl]-4-hydroxy-2-methyl-5-(5-methylfuran-2-yl)pyridine-3-carboxamide |
| 121 | 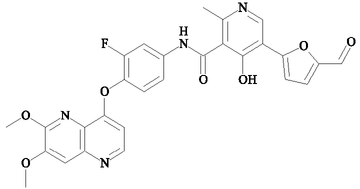 | N-[4-[(6,7-dimethoxy-1,5-naphthyridin-4-yl)oxy]-3-fluorophenyl]-5-(5-formylfuran-2-yl)-4-hydroxy-2-methylpyridine-3-carboxamide |
| 122 | 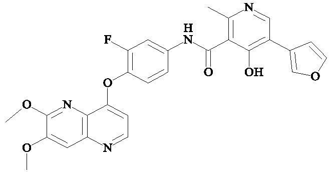 | N-[4-[(6,7-dimethoxy-1,5-naphthyridin-4-yl)oxy]-3-fluorophenyl]-5-(furan-3-yl)-4-hydroxy-2-methylpyridine-3-carboxamide |
| 123 | 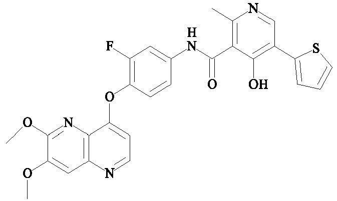 | N-[4-[(6,7-dimethoxy-1,5-naphthyridin-4-yl)oxy]-3-fluorophenyl]-4-hydroxy-2-methyl-5-thiophen-2-ylpyridine-3-carboxamide |
| 124 | 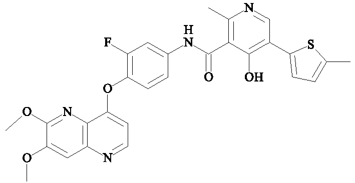 | N-[4-[(6,7-dimethoxy-1,5-naphthyridin-4-yl)oxy]-3-fluorophenyl]-4-hydroxy-2-methyl-5-(5-methylthiophen-2-yl)pyridine-3-carboxamide |
| 125 | 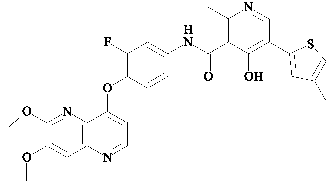 | N-[4-[(6,7-dimethoxy-1,5-naphthyridin-4-yl)oxy]-3-fluorophenyl]-4-hydroxy-2-methyl-5-(4-methylthiophen-2-yl)pyridine-3-carboxamide |
| 126 | 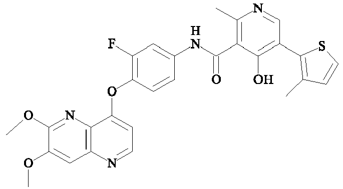 | N-[4-[(6,7-dimethoxy-1,5-naphthyridin-4-yl)oxy]-3-fluorophenyl]-4-hydroxy-2-methyl-5-(3-methylthiophen-2-yl)pyridine-3-carboxamide |
| 127 | 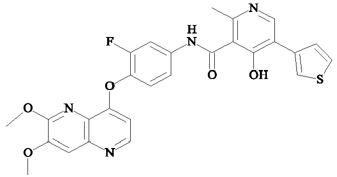 | N-[4-[(6,7-dimethoxy-1,5-naphthyridin-4-yl)oxy]-3-fluorophenyl]-4-hydroxy-2-methyl-5-thiophen-3-ylpyridine-3-carboxamide |
| 128 | 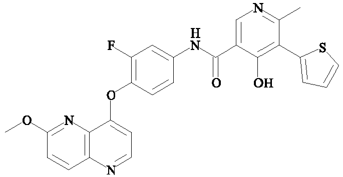 | N-[3-fluoro-4-[(6-methoxy-1,5-naphthyridin-4-yl)oxy]phenyl]-4-hydroxy-6-methyl-5-thiophen-2-ylpyridine-3-carboxamide |
| 129 | 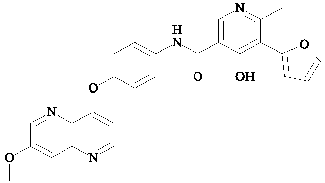 | 5-(furan-2-yl)-4-hydroxy-N-[4-[(7-methoxy-1,5-naphthyridin-4-yl)oxy]phenyl]-6-methylpyridine-3-carboxamide |
| 135 | 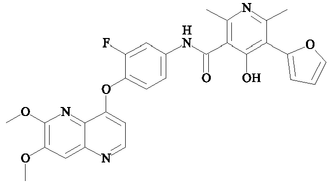 | N-[4-[(6,7-dimethoxy-1,5-naphthyridin-4-yl)oxy]-3-fluorophenyl]-5-(furan-2-yl)-4-hydroxy-2,6-dimethylpyridine-3-carboxamide |
| 136 | 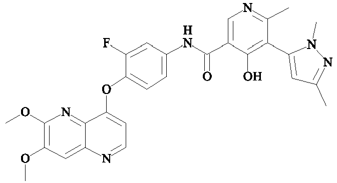 | N-[4-[(6,7-dimethoxy-1,5-naphthyridin-4-yl)oxy]-3-fluorophenyl]-5-(2,5-dimethylpyrazol-3-yl)-4-hydroxy-6-methylpyridine-3-carboxamide |
| 137 | 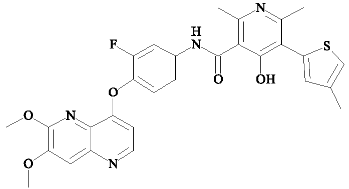 | N-[4-[(6,7-dimethoxy-1,5-naphthyridin-4-yl)oxy]-3-fluorophenyl]-4-hydroxy-2,6-dimethyl-5-(4-methylthiophen-2-yl)pyridine-3-carboxamide |
| 138 | 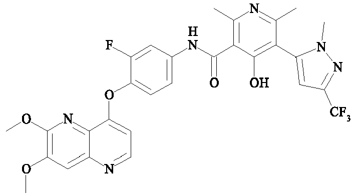 | N-[4-[(6,7-dimethoxy-1,5-naphthyridin-4-yl)oxy]-3-fluorophenyl]-4-hydroxy-2,6-dimethyl-5-[2-methyl-5-(trifluoromethyl)pyrazol-3-yl]pyridine-3-carboxamide |
| 139 | 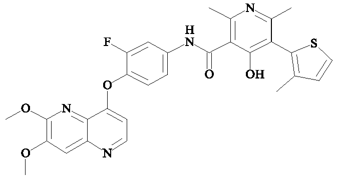 | N-[4-[(6,7-dimethoxy-1,5-naphthyridin-4-yl)oxy]-3-fluorophenyl]-4-hydroxy-2,6-dimethyl-5-(3-methylthiophen-2-yl)pyridine-3-carboxamide |
| 140 | 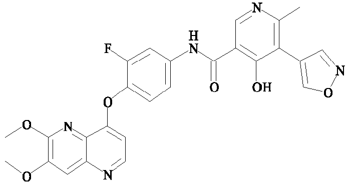 | N-[4-[(6,7-dimethoxy-1,5-naphthyridin-4-yl)oxy]-3-fluorophenyl]-4-hydroxy-6-methyl-5-(1,2-oxazol-4-yl)pyridine-3-carboxamide |
| 141 | 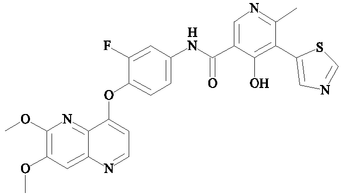 | N-[4-[(6,7-dimethoxy-1,5-naphthyridin-4-yl)oxy]-3-fluorophenyl]-4-hydroxy-6-methyl-5-(1,3-thiazol-5-yl)pyridine-3-carboxamide |
| 142 | 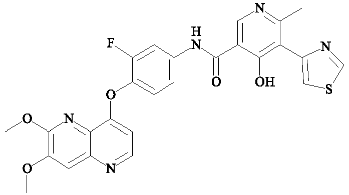 | N-[4-[(6,7-dimethoxy-1,5-naphthyridin-4-yl)oxy]-3-fluorophenyl]-4-hydroxy-6-methyl-5-(1,3-thiazol-4-yl)pyridine-3-carboxamide |
| 143 | 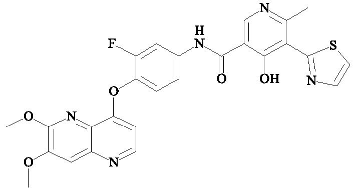 | N-[4-[(6,7-dimethoxy-1,5-naphthyridin-4-yl)oxy]-3-fluorophenyl]-4-hydroxy-6-methyl-5-(1,3-thiazol-2-yl)pyridine-3-carboxamide |
| 147 | 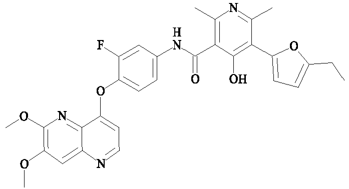 | N-[4-[(6,7-dimethoxy-1,5-naphthyridin-4-yl)oxy]-3-fluorophenyl]-5-(5-ethylfuran-2-yl)-4-hydroxy-2,6-dimethylpyridine-3-carboxamide |
| 148 | 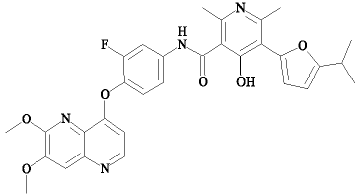 | N-[4-[(6,7-dimethoxy-1,5-naphthyridin-4-yl)oxy]-3-fluorophenyl]-4-hydroxy-2,6-dimethyl-5-(5-propan-2-ylfuran-2-yl)pyridine-3-carboxamide |
| 151 | 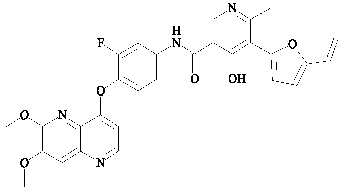 | N-[4-[(6,7-dimethoxy-1,5-naphthyridin-4-yl)oxy]-3-fluorophenyl]-5-(5-ethenylfuran-2-yl)-4-hydroxy-6-methylpyridine-3-carboxamide |
| 152 | 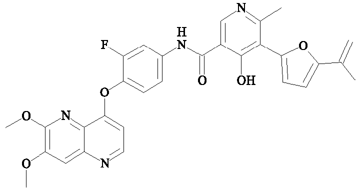 | N-[4-[(6,7-dimethoxy-1,5-naphthyridin-4-yl)oxy]-3-fluorophenyl]-4-hydroxy-6-methyl-5-(5-prop-1-en-2-ylfuran-2-yl)pyridine-3-carboxamide |
| 153 | 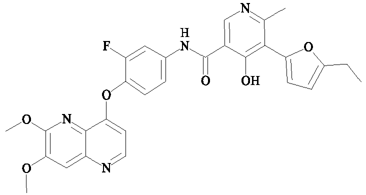 | N-[4-[(6,7-dimethoxy-1,5-naphthyridin-4-yl)oxy]-3-fluorophenyl]-5-(5-ethylfuran-2-yl)-4-hydroxy-6-methylpyridine-3-carboxamide |
| 154 | 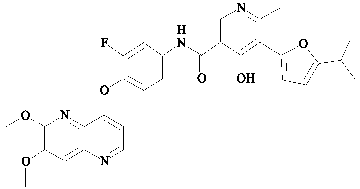 | N-[4-[(6,7-dimethoxy-1,5-naphthyridin-4-yl)oxy]-3-fluorophenyl]-4-hydroxy-6-methyl-5-(5-propan-2-ylfuran-2-yl)pyridine-3-carboxamide |
| 155 | 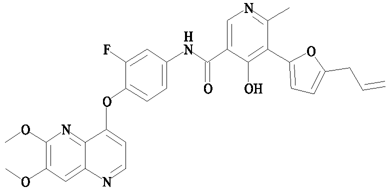 | N-[4-[(6,7-dimethoxy-1,5-naphthyridin-4-yl)oxy]-3-fluorophenyl]-4-hydroxy-6-methyl-5-(5-prop-2-enylfuran-2-yl)pyridine-3-carboxamide |
| 160 | 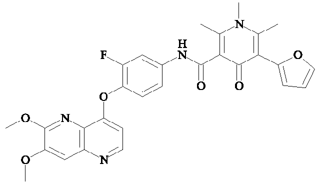 | N-[4-[(6,7-dimethoxy-1,5-naphthyridin-4-yl)oxy]-3-fluorophenyl]-5-(furan-2-yl)-1,2,6-trimethyl-4-oxopyridine-3-carboxamide |

or a pharmaceutically acceptable salt, a stereoisomer, or a mixture of stereoisomers thereof.

**Table 1C**

| **Compound No.** | **Strucutre** | **Compound Name** |
|---|---|---|
| 155A | 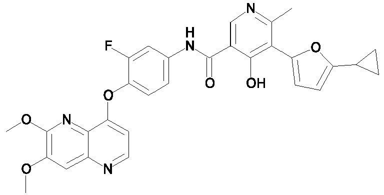 | 5-(5-cyclopropylfuran-2-yl)-N-[4-[(6,7-dimethoxy-1,5-naphthyridin-4-yl)oxy]-3-fluorophenyl]-4-hydroxy-6-methylpyridine-3-carboxamide |
| 155B | 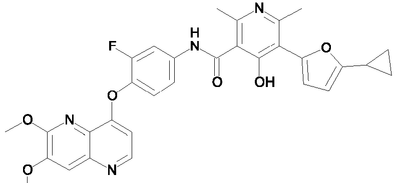 | 5-(5-cyclopropylfuran-2-yl)-N-[4-[(6,7-dimethoxy-1,5-naphthyridin-4-yl)oxy]-3-fluorophenyl]-4-hydroxy-2,6-dimethylpyridine-3-carboxamide |
| 161 | | 5-(furan-2-yl)-N-[4-[(7-methoxy-1,5-naphthyridin-4-yl)oxy]phenyl]-1,2,6-trimethyl-4-oxopyridine-3-carboxamide |
| | 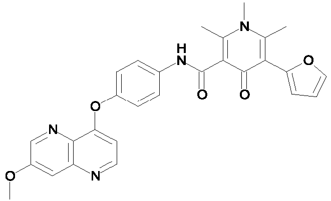 | |
| 162 | 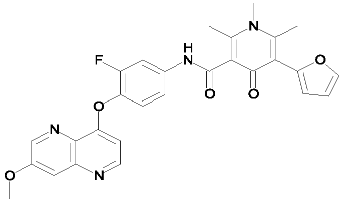 | N-[3-fluoro-4-[(7-methoxy-1,5-naphthyridin-4-yl)oxy]phenyl]-5-(furan-2-yl)-1,2,6-trimethyl-4-oxopyridine-3-carboxamide |
| 163 | 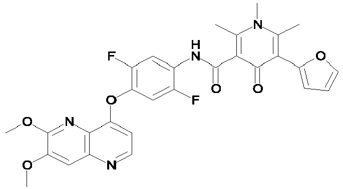 | N-[4-[(6,7-dimethoxy-1,5-naphthyridin-4-yl)oxy]-2,5-difluorophenyl]-5-(furan-2-yl)-1,2,6-trimethyl-4-oxopyridine-3-carboxamide |
| 164 | 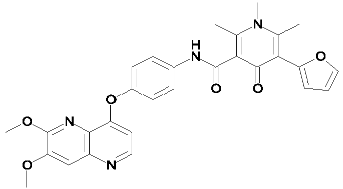 | N-[4-[(6,7-dimethoxy-1,5-naphthyridin-4-yl)oxy]phenyl]-5-(furan-2-yl)-1,2,6-trimethyl-4-oxopyridine-3-carboxamide |
| 165 | 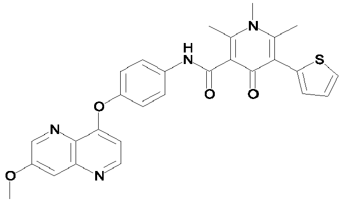 | N-[4-[(7-methoxy-1,5-naphthyridin-4-yl)oxy]phenyl]-1,2,6-trimethyl-4-oxo-5-thiophen-2-ylpyridine-3-carboxamide |
| 166 | 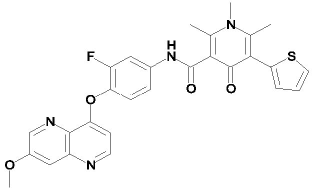 | N-[3-fluoro-4-[(7-methoxy-1,5-naphthyridin-4-yl)oxy]phenyl]-1,2,6-trimethyl-4-oxo-5-thiophen-2-ylpyridine-3-carboxamide |
| 167 | 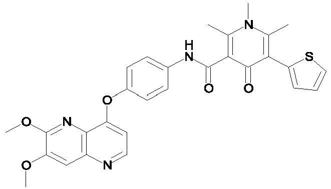 | N-[4-[(6,7-dimethoxy-1,5-naphthyridin-4-yl)oxy]phenyl]-1,2,6-trimethyl-4-oxo-5-thiophen-2-ylpyridine-3-carboxamide |
| 168 | 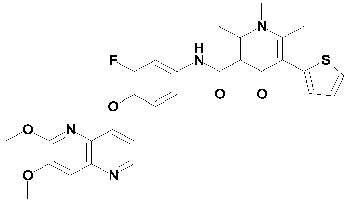 | N-[4-[(6,7-dimethoxy-1,5-naphthyridin-4-yl)oxy]-3-fluorophenyl]-1,2,6-trimethyl-4-oxo-5-thiophen-2-ylpyridine-3-carboxamide |
| 195 | 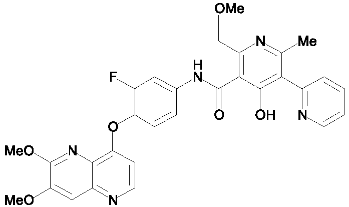 | N-[4-[(6,7-dimethoxy-1,5-naphthyridin-4-yl)oxy]-3-fluorophenyl]-4-hydroxy-2-(methoxymethyl)-6-methyl-5-pyridin-2-ylpyridine-3-carboxamide |
| 196 | 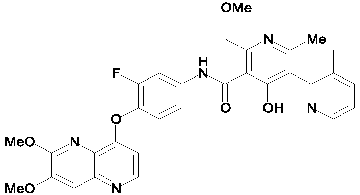 | N-[4-[(6,7-dimethoxy-1,5-naphthyridin-4-yl)oxy]-3-fluorophenyl]-4-hydroxy-2-(methoxymethyl)-6-methyl-5-(3-methylpyridin-2-yl)pyridine-3-carboxamide |
| 197 | 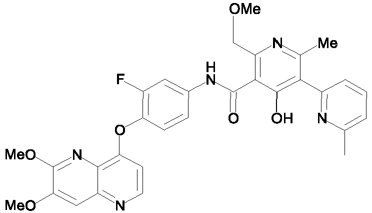 | N-[4-[(6,7-dimethoxy-1,5-naphthyridin-4-yl)oxy]-3-fluorophenyl]-4-hydroxy-2-(methoxymethyl)-6-methyl-5-(6-methylpyridin-2-yl)pyridine-3-carboxamide |
| 198 | 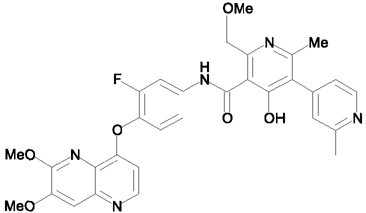 | N-[4-[(6,7-dimethoxy-1,5-naphthyridin-4-yl)oxy]-3-fluorophenyl]-4-hydroxy-2-(methoxymethyl)-6-methyl-5-(2-methylpyridin-4-yl)pyridine-3-carboxamide |
| 200 | 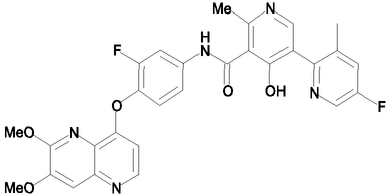 | N-[4-[(6,7-dimethoxy-1,5-naphthyridin-4-yl)oxy]-3-fluorophenyl]-5-(5-fluoro-3-methylpyridin-2-yl)-4-hydroxy-2-methylpyridine-3-carboxamide |
| 212 | 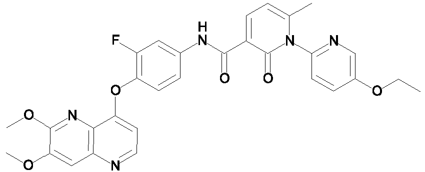 | N-[4-[(6,7-dimethoxy-1,5-naphthyridin-4-yl)oxy]-3-fluorophenyl]-1-(5-ethoxypyridin-2-yl)-6-methyl-2-oxopyridine-3-carboxamide |
| 213 | 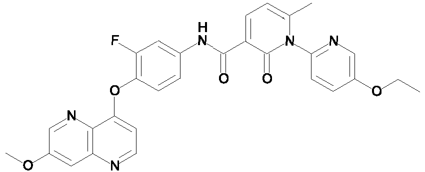 | 1-(5-ethoxypyridin-2-yl)-N-[3-fluoro-4-[(7-methoxy-1,5-naphthyridin-4-yl)oxy]phenyl]-6-methyl-2-oxopyridine-3-carboxamide |
| 214 | 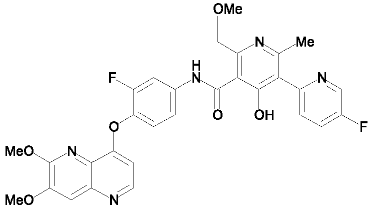 | N-[4-[(6,7-dimethoxy-1,5-naphthyridin-4-yl)oxy]-3-fluorophenyl]-5-(5-fluoropyridin-2-yl)-4-hydroxy-2-(methoxymethyl)-6-methylpyridine-3-carboxamide |
| 215 | 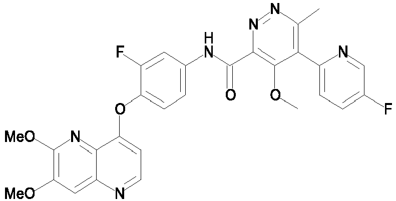 | N-[4-[(6,7-dimethoxy-1,5-naphthyridin-4-yl)oxy]-3-fluorophenyl]-5-(5-fluoropyridin-2-yl)-4-methoxy-6-methylpyridazine-3-carboxamide |
| 216 | 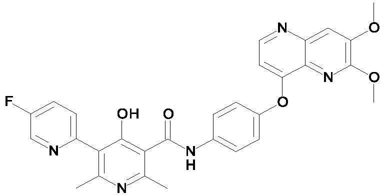 | N-[4-[(6,7-dimethoxy-1,5-naphthyridin-4-yl)oxy]phenyl]-5-(5-fluoropyridin-2-yl)-4-hydroxy-2,6-dimethylpyridine-3-carboxamide |
| 217 | 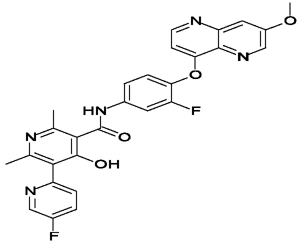 | N-[3-fluoro-4-[(7-methoxy-1,5-naphthyridin-4-yl)oxy]phenyl]-5-(5-fluoropyridin-2-yl)-4-hydroxy-2,6-dimethylpyridine-3-carboxamide |
| 218 | 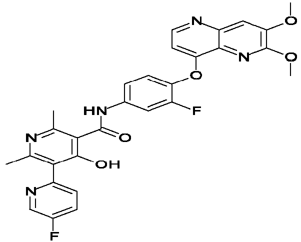 | N-[4-[(6,7-dimethoxy-1,5-naphthyridin-4-yl)oxy]-3-fluorophenyl]-5-(5-fluoropyridin-2-yl)-4-hydroxy-2,6-dimethylpyridine-3-carboxamide |
| 219 | 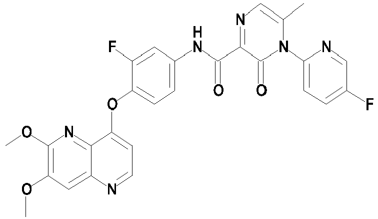 | N-[4-[(6,7-dimethoxy-1,5-naphthyridin-4-yl)oxy]-3-fluorophenyl]-4-(5-fluoropyridin-2-yl)-5-methyl-3-oxopyrazine-2-carboxamide |
| 220 | 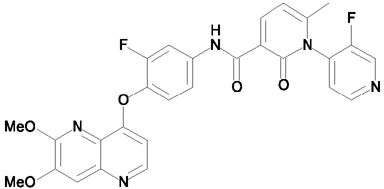 | N-[4-[(6,7-dimethoxy-1,5-naphthyridin-4-yl)oxy]-3-fluorophenyl]-1-(3-fluoropyridin-4-yl)-6-methyl-2-oxopyridine-3-carboxamide |
| 221 | 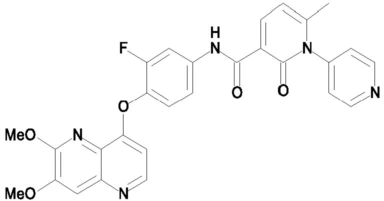 | N-[4-[(6,7-dimethoxy-1,5-naphthyridin-4-yl)oxy]-3-fluorophenyl]-6-methyl-2-oxo-1-pyridin-4-ylpyridine-3-carboxamide |
| 222 | 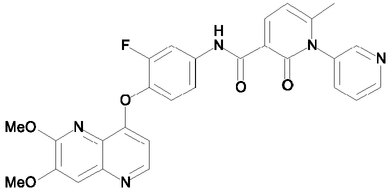 | N-[4-[(6,7-dimethoxy-1,5-naphthyridin-4-yl)oxy]-3-fluorophenyl]-6-methyl-2-oxo-1-pyridin-3-ylpyridine-3-carboxamide |
| 224 | 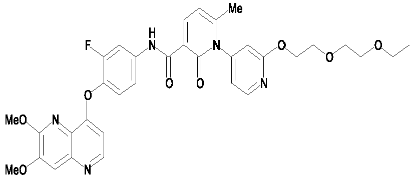 | N-[4-[(6,7-dimethoxy-1,5-naphthyridin-4-yl)oxy]-3-fluorophenyl]-1-[2-[2-(2-ethoxyethoxy)ethoxy]pyridin-4-yl]-6-methyl-2-oxopyridine-3-carboxamide |
| 225 | 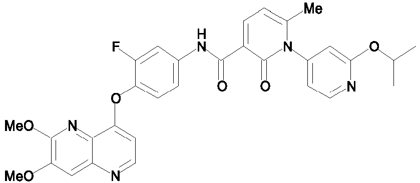 | N-[4-[(6,7-dimethoxy-1,5-naphthyridin-4-yl)oxy]-3-fluorophenyl]-6-methyl-2-oxo-1-(2-propan-2-yloxypyridin-4-yl)pyridine-3-carboxamide |
| 226 | 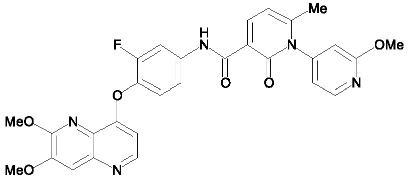 | N-[4-[(6,7-dimethoxy-1,5-naphthyridin-4-yl)oxy]-3-fluorophenyl]-1-(2-methoxypyridin-4-yl)-6-methyl-2-oxopyridine-3-carboxamide |
| 227 | 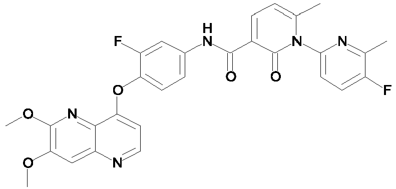 | N-[4-[(6,7-dimethoxy-1,5-naphthyridin-4-yl)oxy]-3-fluorophenyl]-1-(5-fluoro-6-methylpyridin-2-yl)-6-methyl-2-oxopyridine-3-carboxamide |
| 228 | 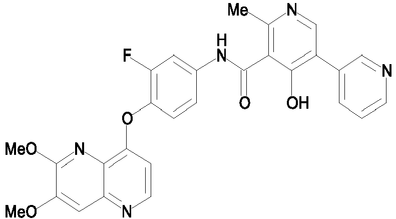 | N-[4-[(6,7-dimethoxy-1,5-naphthyridin-4-yl)oxy]-3-fluorophenyl]-4-hydroxy-2-methyl-5-pyridin-3-ylpyridine-3-carboxamide |
| 229 | 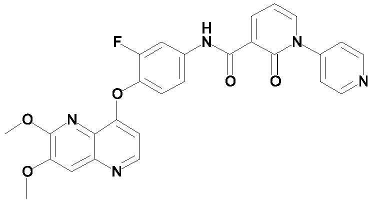 | N-[4-[(6,7-dimethoxy-1,5-naphthyridin-4-yl)oxy]-3-fluorophenyl]-2-oxo-1-pyridin-4-ylpyridine-3-carboxamide |
| 230 | 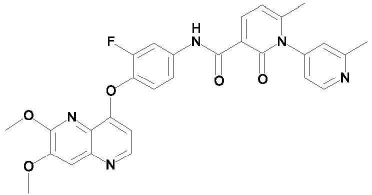 | N-[4-[(6,7-dimethoxy-1,5-naphthyridin-4-yl)oxy]-3-fluorophenyl]-6-methyl-1-(2-methylpyridin-4-yl)-2-oxopyridine-3-carboxamide |
| 231 | 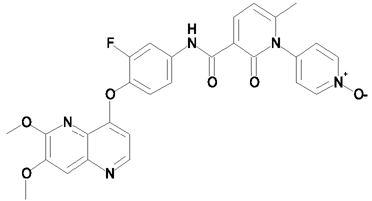 | N-[4-[(6,7-dimethoxy-1,5-naphthyridin-4-yl)oxy]-3-fluorophenyl]-6-methyl-1-(1-oxidopyridin-1-ium-4-yl)-2-oxopyridine-3-carboxamide |
| 232 | 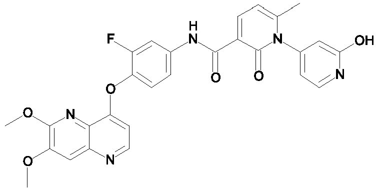 | N-[4-[(6,7-dimethoxy-1,5-naphthyridin-4-yl)oxy]-3-fluorophenyl]-1-(2-hydroxypyridin-4-yl)-6-methyl-2-oxopyridine-3-carboxamide |
| 233 | 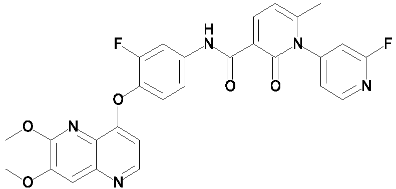 | N-[4-[(6,7-dimethoxy-1,5-naphthyridin-4-yl)oxy]-3-fluorophenyl]-1-(2-fluoropyridin-4-yl)-6-methyl-2-oxopyridine-3-carboxamide |
| 234 | 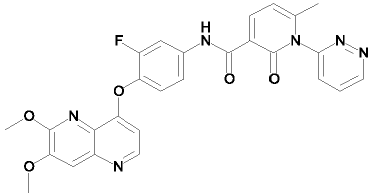 | N-[4-[(6,7-dimethoxy-1,5-naphthyridin-4-yl)oxy]-3-fluorophenyl]-6-methyl-2-oxo-1-pyridazin-3-ylpyridine-3-carboxamide |
| 235 | 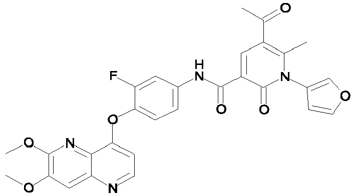 | 5-acetyl-N-[4-[(6,7-dimethoxy-1,5-naphthyridin-4-yl)oxy]-3-fluorophenyl]-1-(furan-3-yl)-6-methyl-2-oxopyridine-3-carboxamide |
| 236 | 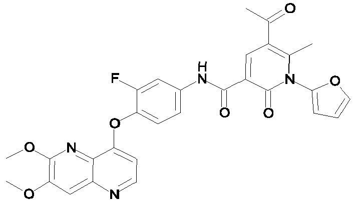 | 5-acetyl-N-[4-[(6,7-dimethoxy-1,5-naphthyridin-4-yl)oxy]-3-fluorophenyl]-1-(furan-2-yl)-6-methyl-2-oxopyridine-3-carboxamide |
| 237 | 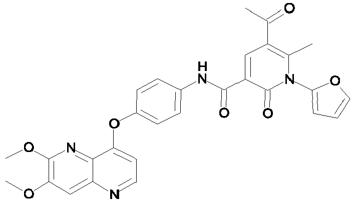 | 5-acetyl-N-[4-[(6,7-dimethoxy-1,5-naphthyridin-4-yl)oxy]phenyl]-1-(furan-2-yl)-6-methyl-2-oxopyridine-3-carboxamide |
| 238 | 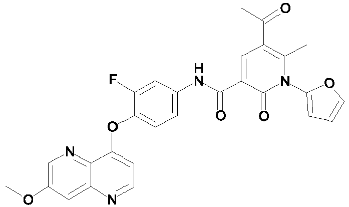 | 5-acetyl-N-[3-fluoro-4-[(7-methoxy-1,5-naphthyridin-4-yl)oxy]phenyl]-1-(furan-2-yl)-6-methyl-2-oxopyridine-3-carboxamide |
| 239 | 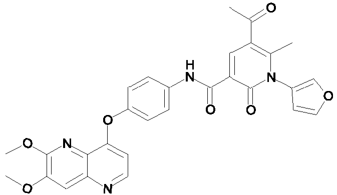 | 5-acetyl-N-[4-[(6,7-dimethoxy-1,5-naphthyridin-4-yl)oxy]phenyl]-1-(furan-3-yl)-6-methyl-2-oxopyridine-3-carboxamide |
| 240 | 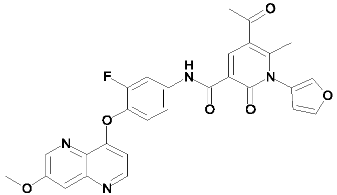 | 5-acetyl-N-[3-fluoro-4-[(7-methoxy-1,5-naphthyridin-4-yl)oxy]phenyl]-1-(furan-3-yl)-6-methyl-2-oxopyridine-3-carboxamide |
| 241 | 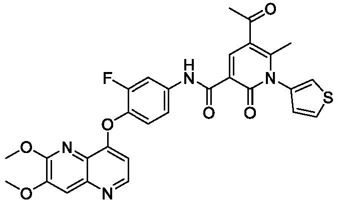 | 5-acetyl-N-[4-[(6,7-dimethoxy-1,5-naphthyridin-4-yl)oxy]-3-fluorophenyl]-6-methyl-2-oxo-1-thiophen-3-ylpyridine-3-carboxamide |
| 242 | 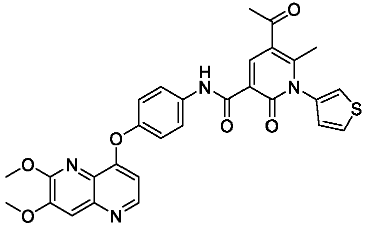 | 5-acetyl-N-[4-[(6,7-dimethoxy-1,5-naphthyridin-4-yl)oxy]phenyl]-6-methyl-2-oxo-1-thiophen-3-ylpyridine-3-carboxamide |
| 243 | 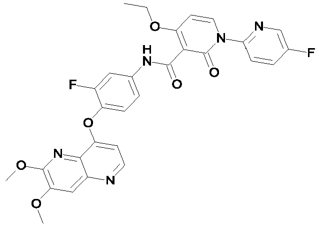 | N-[4-[(6,7-dimethoxy-1,5-naphthyridin-4-yl)oxy]-3-fluorophenyl]-4-ethoxy-1-(5-fluoropyridin-2-yl)-2-oxopyridine-3-carboxamide |
| 244 | 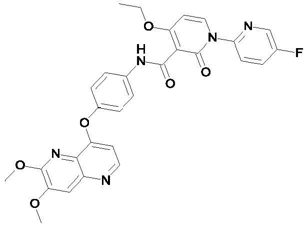 | N-[4-[(6,7-dimethoxy-1,5-naphthyridin-4-yl)oxy]phenyl]-4-ethoxy-1-(5-fluoropyridin-2-yl)-2-oxopyridine-3-carboxamide |
| 245 | 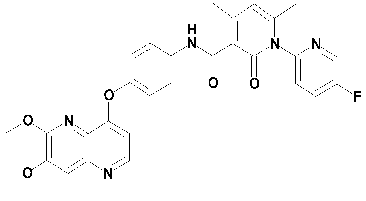 | N-[4-[(6,7-dimethoxy-1,5-naphthyridin-4-yl)oxy]phenyl]-1-(5-fluoropyridin-2-yl)-4,6-dimethyl-2-oxopyridine-3-carboxamide |
| 246 | 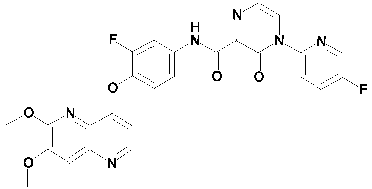 | N-[4-[(6,7-dimethoxy-1,5-naphthyridin-4-yl)oxy]-3-fluorophenyl]-4-(5-fluoropyridin-2-yl)-3-oxopyrazine-2-carboxamide |
| 247 | 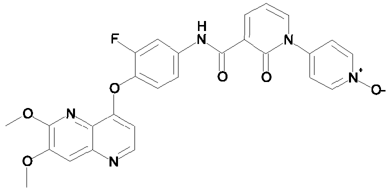 | N-[4-[(6,7-dimethoxy-1,5-naphthyridin-4-yl)oxy]-3-fluorophenyl]-1-(1-oxidopyridin-1-ium-4-yl)-2-oxopyridine-3-carboxamide |
| 248 | 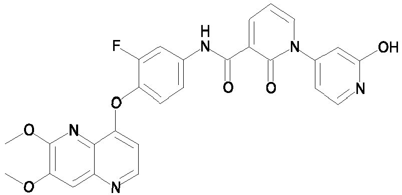 | N-[4-[(6,7-dimethoxy-1,5-naphthyridin-4-yl)oxy]-3-fluorophenyl]-1-(2-hydroxypyridin-4-yl)-2-oxopyridine-3-carboxamide |
| 254 | 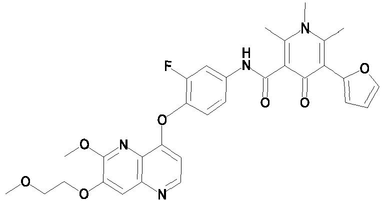 | N-[3-fluoro-4-[[6-methoxy-7-(2-methoxyethoxy)-1,5-naphthyridin-4-yl]oxy]phenyl]-5-(furan-2-yl)-1,2,6-trimethyl-4-oxopyridine-3-carboxamide |
| 255 | 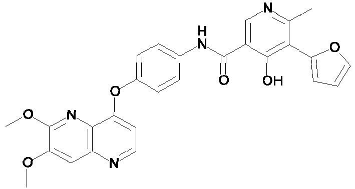 | N-[4-[(6,7-dimethoxy-1,5-naphthyridin-4-yl)oxy]phenyl]-5-(furan-2-yl)-4-hydroxy-6-methylpyridine-3-carboxamide |
| 256 | 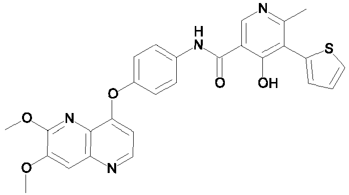 | N-[4-[(6,7-dimethoxy-1,5-naphthyridin-4-yl)oxy]phenyl]-4-hydroxy-6-methyl-5-thiophen-2-ylpyridine-3-carboxamide |
| 257 | 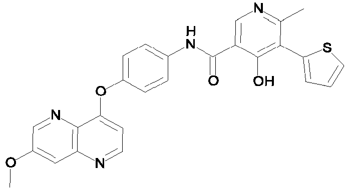 | 4-hydroxy-N-[4-[(7-methoxy-1,5-naphthyridin-4-yl)oxy]phenyl]-6-methyl-5-thiophen-2-ylpyridine-3-carboxamide |
| 260 | 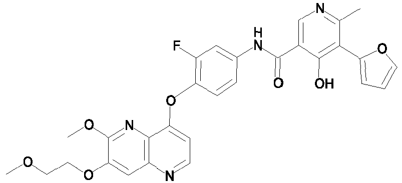 | N-[3-fluoro-4-[[6-methoxy-7-(2-methoxyethoxy)-1,5-naphthyridin-4-yl]oxy]phenyl]-5-(furan-2-yl)-4-hydroxy-6-methylpyridine-3-carboxamide |
| 261 | 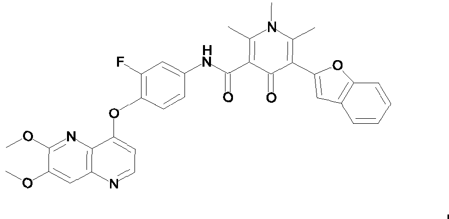 | 5-(1-benzofuran-2-yl)-N-[4-[(6,7-dimethoxy-1,5-naphthyridin-4-yl)oxy]-3-fluorophenyl]-1,2,6-trimethyl-4-oxopyridine-3-carboxamide |
| 262 | 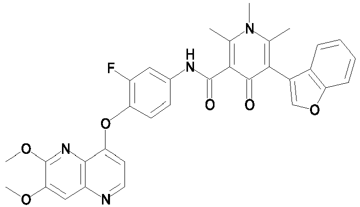 | 5-(1-benzofuran-3-yl)-N-[4-[(6,7-dimethoxy-1,5-naphthyridin-4-yl)oxy]-3-fluorophenyl]-1,2,6-trimethyl-4-oxopyridine-3-carboxamide |
| 263 | 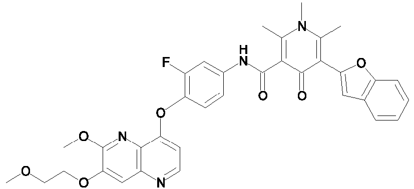 | 5-(1-benzofuran-2-yl)-N-[3-fluoro-4-[[6-methoxy-7-(2-methoxyethoxy)-1,5-naphthyridin-4-yl]oxy]phenyl]-1,2,6-trimethyl-4-oxopyridine-3-carboxamide |
| 264 | 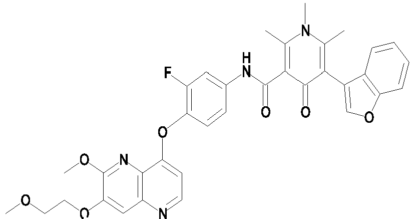 | 5-(1-benzofuran-3-yl)-N-[3-fluoro-4-[[6-methoxy-7-(2-methoxyethoxy)-1,5-naphthyridin-4-yl]oxy]phenyl]-1,2,6-trimethyl-4-oxopyridine-3-carboxamide |
| 265 | 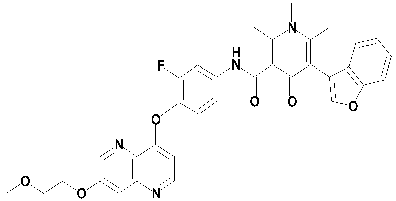 | 5-(1-benzofuran-3-yl)-N-[3-fluoro-4-[[7-(2-methoxyethoxy)-1,5-naphthyridin-4-yl]oxy]phenyl]-1,2,6-trimethyl-4-oxopyridine-3-carboxamide |
| 266 | 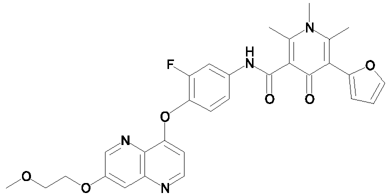 | N-[3-fluoro-4-[[7-(2-methoxyethoxy)-1,5-naphthyridin-4-yl]oxy]phenyl]-5-(furan-2-yl)-1,2,6-trimethyl-4-oxopyridine-3-carboxamide |
| 269 | 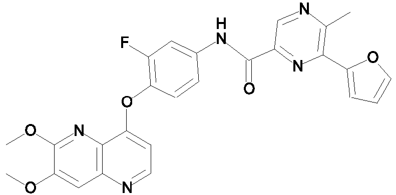 | N-[4-[(6,7-dimethoxy-1,5-naphthyridin-4-yl)oxy]-3-fluorophenyl]-6-(furan-2-yl)-5-methylpyrazine-2-carboxamide |
| 275 | 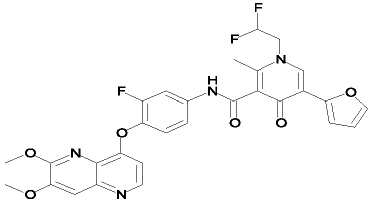 | 1-(2,2-difluoroethyl)-N-[4-[(6,7-dimethoxy-1,5-naphthyridin-4-yl)oxy]-3-fluorophenyl]-5-(furan-2-yl)-2-methyl-4-oxopyridine-3-carboxamide |
| 297 | 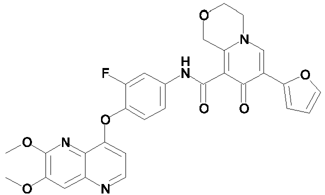 | N-[4-[(6,7-dimethoxy-1,5-naphthyridin-4-yl)oxy]-3-fluorophenyl]-7-(furan-2-yl)-8-oxo-3,4-dihydro-1H-pyrido[2,1-c][1,4]oxazine-9-carboxamide |
| 298 | 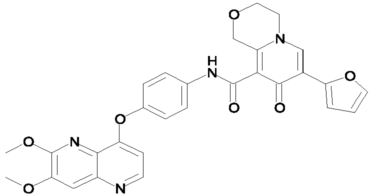 | N-[4-[(6,7-dimethoxy-1,5-naphthyridin-4-yl)oxy]phenyl]-7-(furan-2-yl)-8-oxo-3,4-dihydro-1H-pyrido[2, 1-c][1,4]oxazine-9-carboxamide |
| 299 | 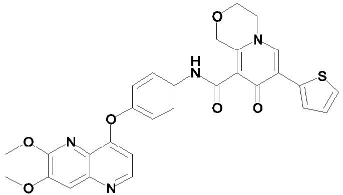 | N-[4-[(6,7-dimethoxy-1,5-naphthyridin-4-yl)oxy]phenyl]-8-oxo-7-thiophen-2-yl-3,4-dihydro-1H-pyrido[2,1-c][1,4]oxazine-9-carboxamide |
| 300 | 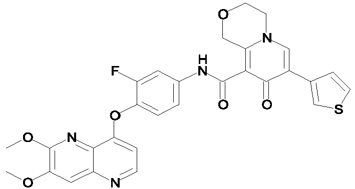 | N-[4-[(6,7-dimethoxy-1,5-naphthyridin-4-yl)oxy]-3-fluorophenyl]-8-oxo-7-thiophen-3-yl-3,4-dihydro-1H-pyrido[2,1-c][1,4]oxazine-9-carboxamide |
| 301 | 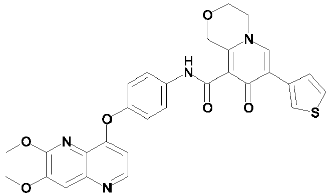 | N-[4-[(6,7-dimethoxy-1,5-naphthyridin-4-yl)oxy]phenyl]-8-oxo-7-thiophen-3-yl-3,4-dihydro-1H-pyrido[2,1-c][1,4]oxazine-9-carboxamide |
| 302 | 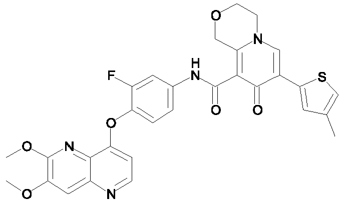 | N-[4-[(6,7-dimethoxy-1,5-naphthyridin-4-yl)oxy]-3-fluorophenyl]-7-(4-methylthiophen-2-yl)-8-oxo-3,4-dihydro-1H-pyrido[2,1-c][1,4]oxazine-9-carboxamide |
| 303 | 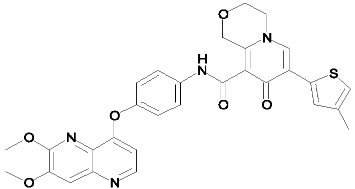 | N-[4-[(6,7-dimethoxy-1,5-naphthyridin-4-yl)oxy]phenyl]-7-(4-methylthiophen-2-yl)-8-oxo-3,4-dihydro-1H-pyrido[2,1-c][1,4]oxazine-9-carboxamide |
| 304 | 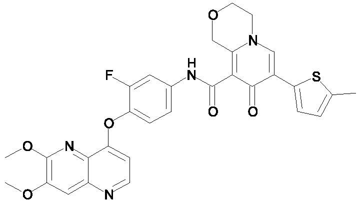 | N-[4-[(6,7-dimethoxy-1,5-naphthyridin-4-yl)oxy]-3-fluorophenyl]-7-(5-methylthiophen-2-yl)-8-oxo-3,4-dihydro-1H-pyrido[2,1-c][1,4]oxazine-9-carboxamide |
| 305 | 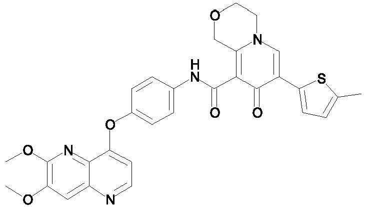 | N-[4-[(6,7-dimethoxy-1,5-naphthyridin-4-yl)oxy]phenyl]-7-(5-methylthiophen-2-yl)-8-oxo-3,4-dihydro-1H-pyrido[2,1-c][1,4]oxazine-9-carboxamide |
| 306 | 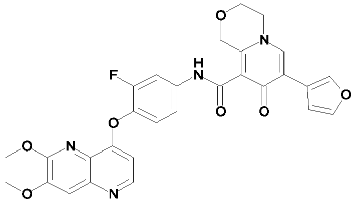 | N-[4-[(6,7-dimethoxy-1,5-naphthyridin-4-yl)oxy]-3-fluorophenyl]-7-(furan-3-yl)-8-oxo-3,4-dihydro-1H-pyrido[2, 1-c][1,4]oxazine-9-carboxamide |
| 307 | 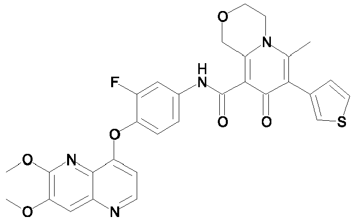 | N-[4-[(6,7-dimethoxy-1,5-naphthyridin-4-yl)oxy]-3-fluorophenyl]-6-methyl-8-oxo-7-thiophen-3-yl-3,4-dihydro-1H-pyrido[2,1-c][1,4]oxazine-9-carboxamide |
| 308 | 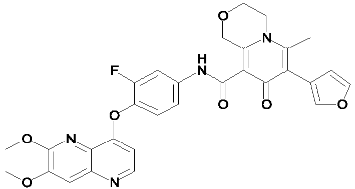 | N-[4-[(6,7-dimethoxy-1,5-naphthyridin-4-yl)oxy]-3-fluorophenyl]-7-(furan-3-yl)-6-methyl-8-oxo-3,4-dihydro-1H-pyrido[2,1-c][1,4]oxazine-9-carboxamide |
| 309 | 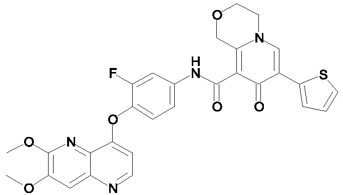 | N-[4-[(6,7-dimethoxy-1,5-naphthyridin-4-yl)oxy]-3-fluorophenyl]-8-oxo-7-thiophen-2-yl-3,4-dihydro-1H-pyrido[2,1-c][1,4]oxazine-9-carboxamide |
| 310 | 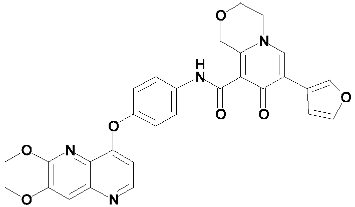 | N-[4-[(6,7-dimethoxy-1,5-naphthyridin-4-yl)oxy]phenyl]-7-(furan-3-yl)-8-oxo-3,4-dihydro-1H-pyrido[2, 1-c][1,4]oxazine-9-carboxamide |
| 311 | 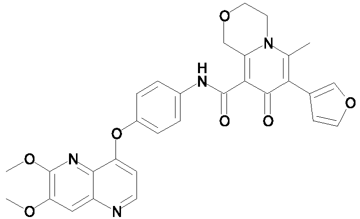 | N-[4-[(6,7-dimethoxy-1,5-naphthyridin-4-yl)oxy]phenyl]-7-(furan-3-yl)-6-methyl-8-oxo-3,4-dihydro-1H-pyrido[2,1-c][1,4]oxazine-9-carboxamide |
| 312 | 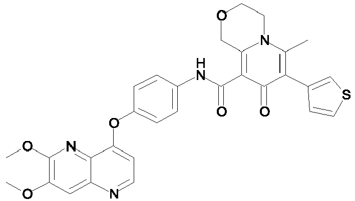 | N-[4-[(6,7-dimethoxy-1,5-naphthyridin-4-yl)oxy]phenyl]-6-methyl-8-oxo-7-thiophen-3-yl-3,4-dihydro-1H-pyrido[2,1-c][1,4]oxazine-9-carboxamide |
| 313 | 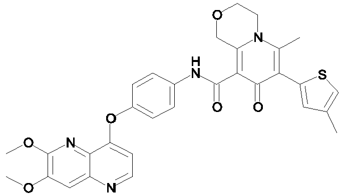 | N-[4-[(6,7-dimethoxy-1,5-naphthyridin-4-yl)oxy]phenyl]-6-methyl-7-(4-methylthiophen-2-yl)-8-oxo-3,4-dihydro-1H-pyrido[2,1-c][1,4]oxazine-9-carboxamide |
| 314 | 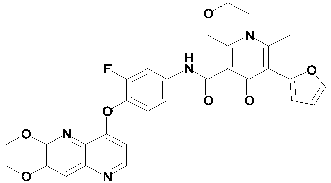 | N-[4-[(6,7-dimethoxy-1,5-naphthyridin-4-yl)oxy]-3-fluorophenyl]-7-(furan-2-yl)-6-methyl-8-oxo-3,4-dihydro-1H-pyrido[2,1-c][1,4]oxazine-9-carboxamide |
| 315 | 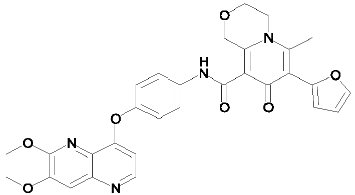 | N-[4-[(6,7-dimethoxy-1,5-naphthyridin-4-yl)oxy]phenyl]-7-(furan-2-yl)-6-methyl-8-oxo-3,4-dihydro-1H-pyrido[2,1-c][1,4]oxazine-9-carboxamide |
| 316 | 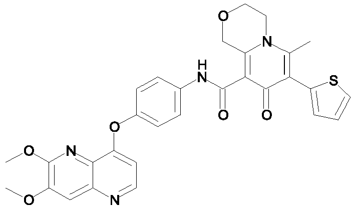 | N-[4-[(6,7-dimethoxy-1,5-naphthyridin-4-yl)oxy]phenyl]-6-methyl-8-oxo-7-thiophen-2-yl-3,4-dihydro-1H-pyrido[2,1-c][1,4]oxazine-9-carboxamide |
| 317 | 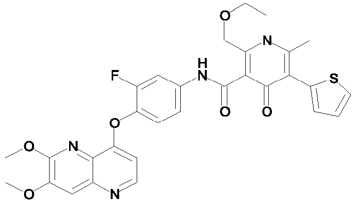 | N-[4-[(6,7-dimethoxy-1,5-naphthyridin-4-yl)oxy]-3-fluorophenyl]-6-methyl-8-oxo-7-thiophen-2-yl-3,4-dihydro-1H-pyrido[2,1-c][1,4]oxazine-9-carboxamide |
| 318 | 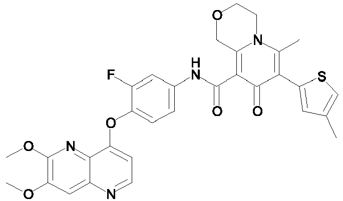 | N-[4-[(6,7-dimethoxy-1,5-naphthyridin-4-yl)oxy]-3-fluorophenyl]-6-methyl-7-(4-methylthiophen-2-yl)-8-oxo-3,4-dihydro-1H-pyrido[2,1-c][1,4]oxazine-9-carboxamide |
| 324 | 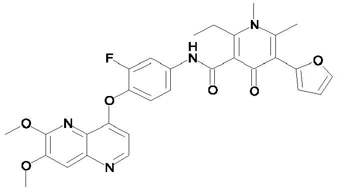 | N-[4-[(6,7-dimethoxy-1,5-naphthyridin-4-yl)oxy]-3-fluorophenyl]-2-ethyl-5-(furan-2-yl)-1,6-dimethyl-4-oxopyridine-3-carboxamide |
| 325 | 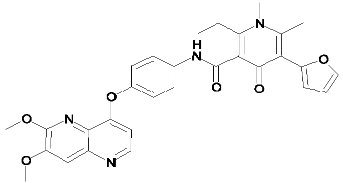 | N-[4-[(6,7-dimethoxy-1,5-naphthyridin-4-yl)oxy]phenyl]-2-ethyl-5-(furan-2-yl)-1,6-dimethyl-4-oxopyridine-3-carboxamide |
| 326 | 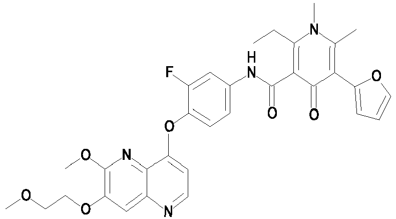 | 2-ethyl-N-[3-fluoro-4-[[6-methoxy-7-(2-methoxyethoxy)-1,5-naphthyridin-4-yl]oxy]phenyl]-5-(furan-2-yl)-1,6-dimethyl-4-oxopyridine-3-carboxamide |
| 327 | 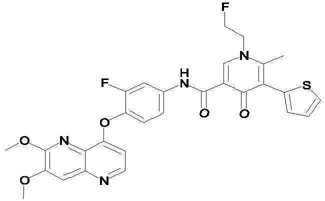 | N-[4-[(6,7-dimethoxy-1,5-naphthyridin-4-yl)oxy]-3-fluorophenyl]-1-(2-fluoroethyl)-6-methyl-4-oxo-5-thiophen-2-ylpyridine-3-carboxamide |
| 328 | 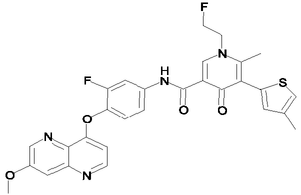 | 1-(2-fluoroethyl)-N-[3-fluoro-4-[(7-methoxy-1,5-naphthyridin-4-yl)oxy]phenyl]-6-methyl-5-(4-methylthiophen-2-yl)-4-oxopyridine-3-carboxamide |
| 329 | 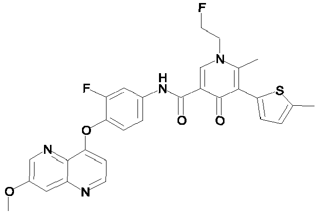 | 1-(2-fluoroethyl)-N-[3-fluoro-4-[(7-methoxy-1,5-naphthyridin-4-yl)oxy]phenyl]-6-methyl-5-(5-methylthiophen-2-yl)-4-oxopyridine-3-carboxamide |
| 330 | 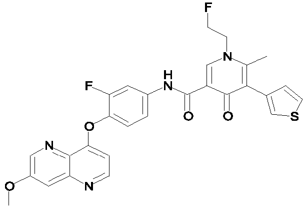 | 1-(2-fluoroethyl)-N-[3-fluoro-4-[(7-methoxy-1,5-naphthyridin-4-yl)oxy]phenyl]-6-methyl-4-oxo-5-thiophen-3-ylpyridine-3-carboxamide |
| 331 | 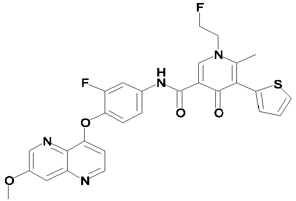 | 1-(2-fluoroethyl)-N-[3-fluoro-4-[(7-methoxy-1,5-naphthyridin-4-yl)oxy]phenyl]-6-methyl-4-oxo-5-thiophen-2-ylpyridine-3-carboxamide |
| 332 | 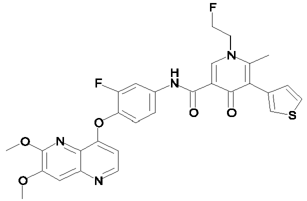 | N-[4-[(6,7-dimethoxy-1,5-naphthyridin-4-yl)oxy]-3-fluorophenyl]-1-(2-fluoroethyl)-6-methyl-4-oxo-5-thiophen-3-ylpyridine-3-carboxamide |
| 333 | 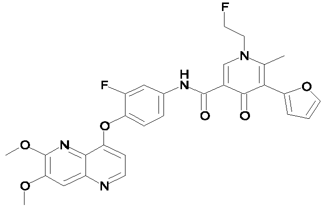 | N-[4-[(6,7-dimethoxy-1,5-naphthyridin-4-yl)oxy]-3-fluorophenyl]-1-(2-fluoroethyl)-5-(furan-2-yl)-6-methyl-4-oxopyridine-3-carboxamide |
| 334 | 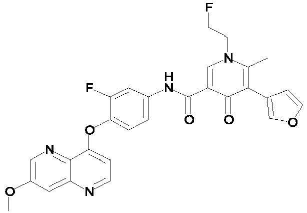 | 1-(2-fluoroethyl)-N-[3-fluoro-4-[(7-methoxy-1,5-naphthyridin-4-yl)oxy]phenyl]-5-(furan-3-yl)-6-methyl-4-oxopyridine-3-carboxamide |
| 337 | 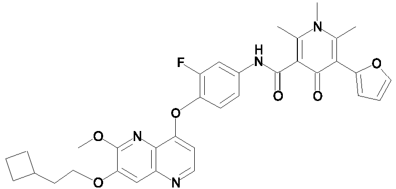 | N-[4-[[7-(2-cyclobutylethoxy)-6-methoxy-1,5-naphthyridin-4-yl]oxy]-3-fluorophenyl]-5-(furan-2-yl)-1,2,6-trimethyl-4-oxopyridine-3-carboxamide |

or a pharmaceutically acceptable salt, a stereoisomer, or a mixture of stereoisomers thereof.

### Treatment Methods and Uses

"Treatment" or "treating" is an approach for obtaining beneficial or desired results including clinical results. Beneficial or desired clinical results may include one or more of the following: a) inhibiting the disease or condition (e.g., decreasing one or more symptoms resulting from the disease or condition, and/or diminishing the extent of the disease or condition); b) slowing or arresting the development of one or more clinical symptoms associated with the disease or condition (e.g., stabilizing the disease or condition, preventing or delaying the worsening or progression of the disease or condition, and/or preventing or delaying the spread (e.g., metastasis) of the disease or condition); and/or c) relieving the disease, that is, causing the regression of clinical symptoms (e.g., ameliorating the disease state, providing partial or total remission of the disease or condition, enhancing effect of another medication, delaying the progression of the disease, increasing the quality of life, and/or prolonging survival.

"Prevention" or "preventing" means any treatment of a disease or condition that causes the clinical symptoms of the disease or condition not to develop. Compounds may be administered to a subject (including a human) who is at risk or has a family history of the disease or condition.

"Subject" refers to an animal, such as a mammal (including a human), that has been or will be the object of treatment, observation or experiment. The methods described herein may be useful in human therapy and/or veterinary applications. The subject may be a mammal. The subject may be a human.

The term "therapeutically effective amount" or "effective amount" of a compound described herein or a pharmaceutically acceptable salt, tautomer, stereoisomer, mixture of stereoisomers, prodrug, or deuterated analog thereof means an amount sufficient to effect treatment when administered to a subject, to provide a therapeutic benefit such as amelioration of symptoms or slowing of disease progression. For example, a therapeutically effective amount may be an amount sufficient to decrease a symptom of a sickle cell disease. The therapeutically effective amount may vary depending on the subject, and disease or condition being treated, the weight and age of the subject, the severity of the disease or condition, and the manner of administering, which can readily be determined by one or ordinary skill in the art.

The methods described herein may be applied to cell populations in vivo or ex vivo. "In vivo" means within a living individual, as within an animal or human. In this context, the methods described herein may be used therapeutically in an individual. "Ex vivo" means outside of a living individual. Examples of ex vivo cell populations include in vitro cell cultures and biological samples including fluid or tissue samples obtained from individuals. Such samples may be obtained by methods well known in the art. Exemplary biological fluid samples include blood, cerebrospinal fluid, urine, and saliva. In this context, the compounds and compositions described herein may be used for a variety of purposes, including therapeutic and experimental purposes. For example, the compounds and compositions described herein may be used *ex vivo* to determine the optimal schedule and/or dosing of administration of a compound of the present disclosure for a given indication, cell type, individual, and other parameters. Information gleaned from such use may be used for experimental purposes or in the clinic to set protocols for in vivo treatment. Other *ex vivo* uses for which the compounds and compositions described herein may be suited are described below or will become apparent to those skilled in the art. The selected compounds may be further characterized to examine the safety or tolerance dosage in human or non-human subjects. Such properties may be examined using commonly known methods to those skilled in the art.

The method may be a method of modulating in vivo activity of a protein kinase in a subject, the method comprising administering to the subject a therapeutically effective amount of a compound of Formula (II) or a subformula thereof (e.g., formula (IIa), (Ia-1), (IIb), (Ib-1), (IIc), (Ic-1), (Id), (Ie), (Ij), (Ik), and/or (Im), and/or any combinations thereof), or a pharmaceutically acceptable salt or stereoisomer thereof, or a pharmaceutical composition described herein.

The method may be a method of treating a disease, disorder, or syndrome in a subject, the method comprising administering to the subject in need thereof a therapeutically effective amount of a compound of Formula (II) or a subformula thereof (e.g., formula (IIa), (Ia-1), (IIb), (Ib-1), (IIc), (Ic-1), (Id), (Ie), (Ij), (Ik), and/or (Im), and/or any combinations thereof), or a pharmaceutically acceptable salt or stereoisomer thereof, or a pharmaceutical composition described herein, wherein the disease, disorder, or syndrome is mediated at least in part by modulating in vivo activity of a protein kinase. In some instances, the protein kinase is AXL, KDR, Mer, or Met.

The method may be a method for treating cancer.

"Cancer" includes tumor types such as tumor types including breast, colon, renal, lung, squamous cell myeloid leukemia, hemangiomas, melanomas, astrocytomas, and glioblastomas as well as other cellular-proliferative disease states, including but not limited to: Cardiac: sarcoma (angiosarcoma, fibrosarcoma, rhabdomyosarcoma, liposarcoma), myxoma, rhabdomyoma, fibroma, lipoma and teratoma; Lung: bronchogenic carcinoma (squamous cell, undifferentiated small cell, undifferentiated large cell, adenocarcinoma), alveolar (bronchiolar) carcinoma, bronchial adenoma, sarcoma, lymphoma, chondromatous hanlartoma, inesothelioma; Gastrointestinal: esophagus (squamous cell carcinoma, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid tumors, vipoma), small bowel (adenocarcinoma, lymphoma, carcinoid tumors, Karposi's sarcoma, leiomyoma, hemangioma, lipoma, neurofibroma, fibroma), large bowel (adenocarcinoma, tubular adenoma, villous adenoma, hamartoma, leiomyoma); Genitourinary tract: kidney (adenocarcinoma, Wilm's tumor [nephroblastoma], lymphoma, leukemia, renal cell carcinoma), bladder and urethra (squamous cell carcinoma, transitional cell carcinoma, adenocarcinoma), prostate (adenocarcinoma, sarcoma, small cell carcinoma of the prostate), testis (seminoma, teratoma, embryonal carcinoma, teratocarcinoma, choriocarcinoma, sarcoma, interstitial cell carcinoma, fibroma, fibroadenoma, adenomatoid tumors, lipoma); Liver: hepatoma (hepatocellular carcinoma), cholangiocarcinoma, hepatoblastoma, angiosarcoma, hepatocellular adenoma, hemangioma; Bone: osteogenic sarcoma (osteosarcoma), fibrosarcoma, malignant fibrous histiocytoma, chondrosarcoma, Ewing's sarcoma, malignant lymphoma (reticulum cell sarcoma), malignant giant cell tumor chordoma, osteochronfroma (osteocartilaginous exostoses), benign chondroma, chondroblastoma, chondromyxofibroma, osteoid osteoma and giant cell tumors; Nervous system: skull (osteoma, hemangioma, granuloma, xanthoma, osteitis defornians), meninges (meningioma, meningiosarcoma, gliomatosis), brain (astrocytoma, medulloblastoma, glioma, ependymoma, germinoma (pinealoma), glioblastoma multiform, oligodendroglioma, schwannoma, retinoblastoma, congenital tumors), spinal cord neurofibroma, meningioma, glioma, sarcoma); Gynecological: uterus (endometrial carcinoma), cervix (cervical carcinoma, pre-tumor cervical dysplasia), ovaries (ovarian carcinoma [serous cystadenocarcinoma, mucinous cystadenocarcinoma, unclassified carcinoma], granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma], fallopian tubes (carcinoma); Hematologic: blood (myeloid leukemia [acute and chronic], acute lymphoblastic leukemia, chronic lymphocytic leukemia, myeloproliferative diseases, multiple myeloma, myelodysplastic syndrome), Hodgkin's disease, non-Hodgkin's lymphoma [malignant lymphoma]; Skin: malignant melanoma, basal cell carcinoma, squamous cell carcinoma, Karposi's sarcoma, moles dysplastic nevi, lipoma, angioma, dermatofibroma, keloids, psoriasis; and Adrenal glands: neuroblastoma; as well as cancers of the thyroid including medullary thyroid cancer. Thus, the term "cancerous cell," as provided herein, includes a cell afflicted by any one of the above-identified conditions.

The cancer may be selected from ovarian cancer, prostate cancer, lung cancer, medullary thyroid cancer, liver cancer, gastrointestinal cancer, pancreatic cancer, bone cancer, hematologic cancer, skin cancer, kidney cancer, breast cancer, colon cancer, and fallopian tube cancer.

The disease or disorder may be ovarian cancer.

The disease or disorder may be prostate cancer.

The disease or disorder may be lung cancer.

The disease or disorder may be medullary thyroid cancer.

The disease or disorder may be liver cancer.

The disease or disorder may be gastrointestinal cancer.

The disease or disorder may be pancreatic cancer.

The disease or disorder may be bone cancer.

The disease or disorder may be hematologic cancer.

The disease or disorder may be skin cancer.

The disease or disorder may be kidney cancer.

The disease or disorder may be breast cancer.

The disease or disorder may be colon cancer. The disease or disorder may be fallopian cancer. The disease or disorder may be liver cancer, wherein the liver cancer is hepatocellular carcinoma, cholangiocarcinoma, hepatoblastoma, angiosarcoma, hepatocellular adenoma, or hemagioma.

The disease or disorder may be gastrointestinal cancer, wherein the gastrointestinal cancer is cancer of the esophagus which is squamous cell carcinoma, adenocarcinoma, or leiomyosarcoma; cancer of the stomach which is carcinoma, or lymphoma; cancer of the pancreas, which is ductal adenocarcinoma, insulinoma, gucagonoma, gastrinoma, carcinoid tumors, or vipoma; cancer of the small bowel, which is adenocarcinoma, lymphoma, carcinoid tumors, Karposi's sarcoma, leiomyoma, hemagioma, lipoma, or cancer of the large bowel, which is adenocarcinoma, tubular adenoma, villous adenoma, hamartoma, or leiomyoma.

The disease or disorder may be cancer of the pancreas, wherein the cancer of the pancreas is ductal adenocarcinoma, insulinoma, gucagonoma, gastrinoma, carcinoid tumors, or vipoma.

The disease or disorder may be bone cancer, wherein the bone cancer is osteosarcoma, fibrosarcoma, malignant fibrous histiocytoma, chondrosarcoma, Ewing's sarcoma, malignant reticulum cell sarcoma, multiple myeloma, malignant giant cell tumor chordoma, osteocartiliginous exostoses, chondroblastoma, chondromyxofibroma, or osteoid osteoma.

The disease or disorder may be hematologic cancer, wherein the hematologic cancer is myeloid leukemia, acute lymphoblastic leukemia, chronic lymphocytic leukemia, myeloproliferative diseases, multiple myeloma, or myelodysplastic syndrome.

The disease or disorder may be skin cancer, wherein the skin cancer is malignant melanoma, basal cell carcinoma, squamous cell carcinoma, or Karposi's sarcoma.

The disease or disorder may be a renal tumor or renal cell carcinoma.

The disease or disorder may be breast cancer.

The disease or disorder may be a colon cancer tumor.

The disease or disorder may be fallopian tube carcinoma.

### Combination Therapies

A compound as disclosed herein can be administered as a single therapy or in combination ("co-administered") with one or more additional therapies for the treatment of a disease or disorder, for instance a disease or disorder associated with hyper-proliferation such as cancer. Therapies that may be used in combination with a compound disclosed herein include: (i) surgery; (ii) radiotherapy (for example, gamma radiation, neutron beam radiotherapy, electron beam radiotherapy, proton therapy, brachytherapy, and systemic radioactive isotopes); (iii) endocrine therapy; (iv) adjuvant therapy, immunotherapy, CAR T- cell therapy; and (v) other chemotherapeutic agents.

The term" co-administered" ("co-administering") refers to either simultaneous administration, or any manner of separate sequential administration, of a compound as described herein, and a further active pharmaceutical ingredient or ingredients, including cytotoxic agents and radiation treatment. If the administration is not simultaneous, the compounds are administered in a close time proximity to each other. Furthermore, it does not matter if the compounds are administered in the same dosage form, e.g. one compound may be administered topically and another compound may be administered orally.

The treatment method may include the co-administration of a compound as disclosed herein, or a pharmaceutically acceptable salt, stereoisomer, or mixture of stereoisomers thereof, and at least one immunotherapy. Immunotherapy (also called biological response modifier therapy, biologic therapy, biotherapy, immune therapy, or biological therapy) is treatment that uses parts of the immune system to fight disease. Immunotherapy can help the immune system recognize cancer cells, or enhance a response against cancer cells. Immunotherapies include active and passive immunotherapies. Active immunotherapies stimulate the body's own immune system while passive immunotherapies generally use immune system components created outside of the body.

Examples of active immunotherapies include, but are not limited to vaccines including cancer vaccines, tumor cell vaccines (autologous or allogeneic), dendritic cell vaccines, antigen vaccines, anti-idiotype vaccines, DNA vaccines, viral vaccines, or Tumor- Infiltrating Lymphocyte (TIL) Vaccine with Interleukin-2 (IL-2) or Lymphokine- Activated Killer (LAK) Cell Therapy.

Further examples of therapeutic antibodies that can be used include, but are not limited to, trastuzumab; abciximab; daclizumab; BEC2; IMC-C22; vitaxin; Campath 1H/LDP-03; Smart M195; epratuzumab; bectumomab; visilizumab; CM3, a humanized anti-ICAM3 antibody; IDEC-l 14; ibritumomab tiuxetan; IDEC-131; IDEC-151; IDEC-152; SMART anti-CD3; eculizumab; adalimumab; certolizumab; IDEC-l 51; MDX-CD4; CD20-sreptdavidin; CDP571; LDP-02; OrthoClone OKT4A; ruplizumab; natalizumab; and lerdelimumab.

Immunotherapies that can be used in combination with a compound as disclosed herein include adjuvant immunotherapies. Examples include cytokines, such as granulocyte- macrophage colony-stimulating factor (GM-CSF), granulocyte-colony stimulating factor (G-CSF), macrophage inflammatory protein (MIP)-l -alpha, interleukins (including IL-1, IL-2, IL-4, IL-6, IL-7, IL-12, IL-15, IL-18, IL-21, and IL-27), tumor necrosis factors (including TNF-alpha), and interferons (including IFN-alpha, IFN-beta, and IFN-gamma); aluminum hydroxide (alum); Bacille Calmette-Guerin (BCG); Keyhole limpet hemocyanin (KLH); Incomplete Freund's adjuvant (IF A); QS-21; DETOX; Levamisole; and Dinitrophenyl (DNP), and combinations thereof, such as, for example, combinations of, interleukins, for example, IL-2 with other cytokines, such as IFN-alpha.

The immunotherapeutic agent may be an agent that modulates immune responses, for example, a checkpoint inhibitor or a checkpoint agonist. The immunotherapeutic agent may be an antibody modulator that targets PD-1, PD-L1, PD-L2, CEACAM (e g., CEACAM-l, -3 and/or -5), CTLA-4, TIM-3, LAG-3, VISTA, BTLA, TIGIT, LAIR1, CD160, 2B4, TGF beta, OX40, 41BB, LIGHT, CD40, GITR, TGF-beta, TIM-3, SIRP-alpha, VSIG8, BTLA, SIGLEC7, SIGLEC9, ICOS, B7H3, B7H4, FAS, and/or BTNL2 among others known in the art. The immunotherapeutic agent may be an agent that increases natural killer (NK) cell activity. The immunotherapeutic agent may be an agent that inhibits suppression of an immune response. The immunotherapeutic agent may be an agent that inhibits suppressor cells or suppressor cell activity. The immunotherapeutic agent may be an agent or therapy that inhibits Treg activity. The immunotherapeutic agent may be an agent that inhibits the activity of inhibitory immune checkpoint receptors.

The immunotherapeutic agent may include a T cell modulator chosen from an agonist or an activator of a costimulatory molecule. The agonist of the costimulatory molecule may be chosen from an agonist (e.g., an agonistic antibody or antigen-binding fragment thereof, or a soluble fusion) of GITR, OX40, ICOS, SLAM (e.g., SLAMF7), HVEM, LIGHT, CD2, CD27, CD28, CDS, ICAM-l, LFA-l (CD1 la/CDl8), ICOS (CD278), 4-1BB (CD137), CD30, CD40, BAFFR, CD7, NKG2C, NKp80, CD160, B7-H3, or CD83 ligand. The effector cell combination may include a bispecific T cell engager (e.g., a bispecific antibody molecule that binds to CD3 and a tumor antigen (e.g., EGFR, PSCA, PSMA, EpCAM, HER2 among others).

The treatment method may include the co-administration of a compound as disclosed herein or a pharmaceutically acceptable salt thereof and at least one cytotoxic agent. The term "cytotoxic agent" as used herein refers to a substance that inhibits or prevents a cellular function and/or causes cell death or destruction. Cytotoxic agents include, but are not limited to, radioactive isotopes (e.g., At²¹¹, 1¹³¹, 1¹²⁵, Y⁹⁰, Re¹⁸⁶, Re¹⁸⁸, Sm¹⁵³, Bi²¹², P³², Pb²¹² and radioactive isotopes of Lu); chemotherapeutic agents; growth inhibitory agents; enzymes and fragments thereof such as nucleolytic enzymes; and toxins such as small molecule toxins or enzymatically active toxins of bacterial, fungal, plant or animal origin, including fragments and/or variants thereof.

Exemplary cytotoxic agents can be selected from anti-microtubule agents, platinum coordination complexes, alkylating agents, antibiotic agents, topoisomerase II inhibitors, antimetabolites, topoisomerase I inhibitors, hormones and hormonal analogues, signal transduction pathway inhibitors, non-receptor tyrosine kinase angiogenesis inhibitors, immunotherapeutic agents, proapoptotic agents, inhibitors of LDH-A; inhibitors of fatty acid biosynthesis; cell cycle signaling inhibitors; HDAC inhibitors, proteasome inhibitors; and inhibitors of cancer metabolism.

"Chemotherapeutic agents" include chemical compounds useful in the treatment of cancer. Examples of chemotherapeutic agents include erlotinib, bortezomib, disulfiram, epigallocatechin gallate, salinosporamide A, carfilzomib, l7-AAG (geldanamycin), radicicol, lactate dehydrogenase A (LDH-A), fulvestrant, sunitib, letrozole, imatinib mesylate, finasunate, oxaliplatin, 5-FET (5-fluorouracil), leucovorin, Rapamycin, Lapatinib, Lonafamib (SCH 66336), sorafenib, Bayer Labs), gefitinib, AG1478; alkylating agents such as thiotepa and CYTOXAN^{®}; cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylomelamine; acetogenins (especially bullatacin and bullatacinone); a camptothecin (including topotecan and irinotecan); bryostatin; cally statin; CC-1065 (including its adozelesin, carzelesin and bizelesin synthetic analogs); cryptophycins (particularly cryptophycin 1 and cryptophycin 8); adrenocorticosteroids (including prednisone and prednisolone); cyproterone acetate; 5 alpha-reductases including finasteride and dutasteride); vorinostat, romidepsin, panobinostat, valproic acid, mocetinostat dolastatin; aldesleukin, talc duocarmycin (including the synthetic analogs, KW-2189 and CB1-TM1); eleutherobin; pancrati statin; a sarcodictyin; spongistatin; nitrogen mustards such as chlorambucil, chlomaphazine, chlorophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (e.g., calicheamicin, especially calicheamicin gamma II and calicheamicin omega I (Angew Chem. Inti. Ed. Engl. 1994 33: 183-186); dynemicin, including dynemicin A; bisphosphonates, such as clodronate; an esperamicin; as well as neocarzinostatin chromophore and related chromoprotein enediyne antibiotic chromophores), aclacinomysins, actinomycin, authramycin, azaserine, bleomycins, cactinomycin, carabicin, caminomycin, carzinophilin, chromomycinis, dactinomycin, daunorubicin, detorubicin, 6-diazo-5-oxo-L- norleucine, doxorubicin, morpholino-doxorubicin, cyanomorpholino- doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogs such as denopterin, methotrexate, pteropterin, trimetrexate; purine analogs such as fludarabine, 6- mercaptopurine, thiamiprine, thioguanine; pyrimidine analogs such as ancitabine, azacitidine, azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfomithine; elliptinium acetate; an epothilone; etoglucid; gallium nitrate; hydroxyurea; lentinan; lonidainine; maytansinoids such as maytansine and ansamitocins; mitoguazone; mitoxantrone; mopidamnol; nitraerine; pentostatin; phenamet; pirarubicin; losoxantrone; podophyllinic acid; 2-ethylhydrazide; procarbazine; PSK^{®} polysaccharide complex (JHS Natural Products, Eugene, Ore.); razoxane; rhizoxin; sizofuran; spirogermanium; tenuazonic acid; triaziquone; 2,2',2"-trichlorotriethylamine; trichothecenes especially T-2 toxin, verracurin A, roridin A and anguidine); urethan; vindesine; dacarbazine; mannomustine; mitobronitol; mitolactol; pipobroman; gacytosine; arabinoside "Ara-C"); cyclophosphamide; thiotepa; taxoids, e.g., paclitaxel, ABRAXANE^{®} (Cremophor-free), albumin-engineered nanoparticle formulations of paclitaxel (American Pharmaceutical Partners, Schaumberg, Ill.), and docetaxel/doxetaxel; chloranmbucil; gemcitabine; 6-thioguanine; mercaptopurine; methotrexate; platinum analogs such as cisplatin and carboplatin; vinblastine; etoposide (VP-16); ifosfamide; mitoxantrone; vincristine; vinorelbine; novantrone; teniposide; edatrexate; daunomycin; aminopterin; capecitabine; ibandronate; CPT-11; topoisomerase inhibitor RFS 2000; difluorom ethyl ornithine (DMFO); retinoids such as retinoic acid; and pharmaceutically acceptable salts, acids and derivatives of any of the above.

Chemotherapeutic agent also includes (i) anti-hormonal agents that act to regulate or inhibit hormone action on tumors such as anti-estrogens and selective estrogen receptor modulators (SERMs), including, for example, tamoxifen (including tamoxifen citrate), raloxifene, droloxifene, iodoxyfene, 4-hydroxytamoxifen, trioxifene, keoxifene, LY117018, onapristone, and toremifme citrate; (ii) aromatase inhibitors that inhibit the enzyme aromatase, which regulates estrogen production in the adrenal glands, such as, for example, 4(5)-imidazoles, aminoglutethimide, megestrol acetate, exemestane, formestanie, fadrozole, vorozole, letrozole, and anastrozole; (iii) anti-androgens such as flutamide, nilutamide, bicalutamide, leuprolide and goserelin; buserelin, tripterelin, medroxyprogesterone acetate, diethylstilbestrol, premarin, fluoxymesterone, all transretionic acid, fenretinide, as well as troxacitabine (a l,3-dioxolane nucleoside cytosine analog); (iv) protein kinase inhibitors; (v) lipid kinase inhibitors; (vi) antisense oligonucleotides, particularly those which inhibit expression of genes in signaling pathways implicated in aberrant cell proliferation, such as, for example, PKC-alpha, Ralf and H-Ras; (vii) ribozymes such as VEGF expression inhibitors (e.g., ANGIOZYME^{®}) and HER2 expression inhibitors; (viii) vaccines such as gene therapy vaccines, for example, ALLOVECTIN^{®}, LEUVECTIN^{®}, and VAXID^{®}; PROLEUKIN^{®}, rIL-2; a topoisomerase 1 inhibitor such as LEIRTOTECAN^{®}; ABARELIX^{®}; and (ix) pharmaceutically acceptable salts, acids and derivatives of any of the above.

Chemotherapeutic agents also include antibodies, as described above, including alemtuzumab, bevacizumab; cetuximab; panitumumab, rituximab, pertuzumab, tositumomab, and the antibody drug conjugate, gemtuzumab ozogamicin. Additional humanized monoclonal antibodies with therapeutic potential as agents in combination with the compounds of the invention include: apolizumab, aselizumab, atlizumab, bapineuzumab, bivatuzumab mertansine, cantuzumab mertansine, cedelizumab, certolizumab pegol, cidfusituzumab, cidtuzumab, daclizumab, eculizumab, efalizumab, epratuzumab, erlizumab, felvizumab, fontolizumab, gemtuzumab ozogamicin, inotuzumab ozogamicin, ipilimumab, labetuzumab, lintuzumab, matuzumab, mepolizumab, motavizumab, motovizumab, natalizumab, nimotuzumab, nivolumab, nolovizumab, numavizumab, ocrelizumab, omalizumab, palivizumab, pascolizumab, pecfusituzumab, pectuzumab, pexelizumab, ralivizumab, ranibizumab, reslivizumab, reslizumab, resyvizumab, rovelizumab, ruplizumab, sibrotuzumab, siplizumab, sontuzumab, tacatuzumab tetraxetan, tadocizumab, talizumab, tefibazumab, tocilizumab, toralizumab, tucotuzumab celmoleukin, tucusituzumab, umavizumab, urtoxazumab, ustekinumab, visilizumab, and the anti-interleukin-l2 (ABT-8744695, Wyeth Research and Abbott Laboratories) which is a recombinant exclusively human-sequence, full-length IgG₁λ antibody genetically modified to recognize interleukin- 12 p40 protein. Chemotherapeutic agents also include dexamethasone, interferons, colchicine, metoprine, cyclosporine, amphotericin, metronidazole, alemtuzumab, alitretinoin, allopurinol, amifostine, arsenic trioxide, asparaginase, BCG live, bevacuzimab, bexarotene, cladribine, clofarabine, darbepoetin alfa, denileukin, dexrazoxane, epoetin alfa, elotinib, filgrastim, histrelin acetate, ibritumomab, interferon alfa-2a, interferon alfa-2b, lenalidomide, levamisole, mesna, methoxsalen, nandrolone, nelarabine, nofetumomab, oprelvekin, palifermin, pamidronate, pegademase, pegaspargase, pegfilgrastim, pemetrexed disodium, plicamycin, porfimer sodium, quinacrine, rasburicase, sargramostim, temozolomide, VM-26, 6-TG, toremifene, tretinoin, ATRA, valrubicin, zoledronate, and zoledronic acid, and pharmaceutically acceptable salts thereof.

Chemotherapeutic agents also include hydrocortisone, hydrocortisone acetate, cortisone acetate, tixocortol pivalate, triamcinolone acetonide, triamcinolone alcohol, mometasone, amcinonide, budesonide, desonide, fluocinonide, fluocinolone acetonide, betamethasone, betamethasone sodium phosphate, dexamethasone, dexamethasone sodium phosphate, fluocortolone, hydrocortisone-17-butyrate, hydrocortisone- 17-valerate, aclometasone dipropionate, betamethasone valerate, betamethasone dipropionate, prednicarbate, clobetasone-l 7-butyrate, clobetasol-l 7-propionate, fluocortolone caproate, fluocortolone pivalate and fluprednidene acetate; immune selective antiinflammatory peptides (ImSAIDs) such as phenylalanine-glutamine-glycine (FEG) and its D-isomeric form (feG) (IMULAN BioTherapeutics, LLC); anti-rheumatic drugs such as azathioprine, ciclosporin (cyclosporine A), D-penicillamine, gold salts, hydroxychloroquine, leflunomideminocycline, sulfasalazine, tumor necrosis factor alpha (TNF alpha) blockers such as etanercept, infliximab, adalimumab, certolizumab pegol, golimumab (Simponi), Interleukin 1 (IL-1) blockers such as anakinra, T cell costimulation blockers such as abatacept, Interleukin 6 (IL-6) blockers such as tocilizumab; Interleukin 13 (IL-l3) blockers such as lebrikizumab; Interferon alpha (IFN) blockers such as Rontalizumab; Beta 7 integrin blockers such as rhuMAb Beta7; IgE pathway blockers such as Anti-Ml prime; Secreted homotrimeric LTa3 and membrane bound heterotrimer LTal/l32 blockers such as Anti-lymphotoxin alpha (LTa); miscellaneous investigational agents such as thioplatin, PS-341, phenylbutyrate, ET- I8-OCH3, or famesyl transferase inhibitors (L-739749, L-744832); polyphenols such as quercetin, resveratrol, piceatannol, epigallocatechine gallate, theaflavins, flavanols, procyanidins, betulinic acid and derivatives thereof; autophagy inhibitors such as chloroquine; delta-9-tetrahydrocannabinol (dronabinol); beta-lapachone; lapachol; colchicines; betulinic acid; acetylcamptothecin, scopolectin, and 9- aminocamptothecin); podophyllotoxin; tegafur; bexarotene; bisphosphonates such as clodronate, etidronate, NE-58095, zoledronic acid/zoledronate, alendronate, pamidronate, tiludronate, or risedronate; and epidermal growth factor receptor (EGF-R); vaccines such as THERATOPE^{®} vaccine; perifosine, COX-2 inhibitor (e.g. celecoxib or etoricoxib), proteosome inhibitor (e.g. PS341); CCI-779; tipifamib (R11577); orafenib, ABT510; Bcl-2 inhibitor such as oblimersen sodium pixantrone; farnesyltransferase inhibitors such as lonafamib (SCH 6636); and pharmaceutically acceptable salts, acids or derivatives of any of the above; as well as combinations of two or more of the above such as CHOP, an abbreviation for a combined therapy of cyclophosphamide, doxorubicin, vincristine, and prednisolone; and FOLFOX, an abbreviation for a treatment regimen with oxaliplatin combined with 5-FU and leucovorin. Chemotherapeutic agents also include Poly ADP ribose polymerase (PARP) inhibitors: olaparib, rucaprib niraparib, talzoparib.

Compounds as disclosed herein can be used in combination therapy with any of the kinase inhibitors disclosed herein for the treatment of diseases such as cancer. Exemplary kinase inhibitors include imatinib, baricitinib gefitinib, erlotinib, sorafenib, dasatinib, sunitinib, lapatinib, nilotinib, pirfenidone, pazopanib, crizotinib, vemurafenib, vandetanib, ruxolitinib, axitinib, bosutinib, regorafenib, tofacitinib, cabozantinib, ponatinib, trametinib, dabrafenib, afatinib, ibrutinib, ceritinib, idelalisib, nintedanib, palbociclib, lenvatinib, cobimetinib, abemaciclib, acalabrutinib, alectinib, binimetinib, brigatinib, encorafenib, erdafitinib, everolimus, fostamatinib, gilter, larotrectinib, lorlatinib, netarsudil, osimertinib, pemigatinib, pexidartinib, ribociclib, temsirolimus, XL-092, XL-147, XL-765, XL-499, and XL-880. A compound as described herein can be used in combination with a HSP90 inhibitor (e.g., XL888), liver X receptor (LXR) modulators, retinoid-related orphan receptor gamma (RORy) modulators, checkpoint inhibitors such as a CK1 inhibitor or aCK1α inhibitor, a Wnt pathway inhibitor (e.g., SST-215), or a mineralocorticoid receptor inhibitor, (e.g., esaxerenone) or XL-888 for the treatment of a disease disclosed herein such as cancer. The compounds as disclosed herein can be combined with one or more inhibitors of the following kinases for the treatment of cancer: Akt1, Akt2, Akt3, TGF-βR, PKA, PKG, PKC, CaM-kinase, phosphorylase kinase, MEKK, ERK, MAPK, mTOR, EGFR, HER2, HER3, HER4, 1NS-R, IGF-1R, IR-R, PDGFαR, PDGFβ/R, CSFIR, KIT, FLK-II, KDR/FLK-1, FLK-4, flt-1, FGFR1, FGFR2, FGFR3, FGFR4, Ron, Sea, TRKA, TRKB, TRKC, FLT3, VEGFR/Flt2, Flt4, EphA1, EphA2, EphA3, EphB2, EphB4, Tie2, Src, Fyn, Lck, Fgr, Btk, Fak, SYR, FRK, JAK (JAK1 and or JAK2), ABL, ALK, CDK7, CDK12, KRAS, and B-Raf.

Non-limiting examples of inhibitors that can be combined with the compounds of the present disclosure for treatment of cancer and infections include an FGFR inhibitor (FGFR1, FGFR2, FGFR3 or FGFR4, e.g., pemigatinib, an EGFR inhibitor (also known as ErB-1 or HER-1; e.g. erlotinib, gefitinib, vandetanib, orsimertinib, cetuximab, necitumumab, or panitumumab), a VEGFR inhibitor or pathway blocker (e.g. bevacizumab, pazopanib, sunitinib, sorafenib, axitinib, regorafenib, ponatinib, vandetanib, ramucirumab, lenvatinib, ziv-aflibercept), a PARP inhibitor (e.g. olaparib, rucaparib, veliparib or niraparib), a JAK inhibitor (e.g., ruxolitinib, baricitinib, itacitinib), an IDO inhibitor (e.g., epacadostat, NLG919, or BMS-986205, MK7162), an LSD1 inhibitor, a TDO inhibitor, a PI3K-delta inhibitor (e.g., parsaclisib), a PI3K-gamma inhibitor such as PI3K-gamma selective inhibitor, a Pim inhibitor, a CSF1R inhibitor, a TAM receptor tyrosine kinases (Tyro-3, Axl, and Mer), an adenosine receptor antagonist (e.g., A2a/A2b receptor antagonist), an HPK1 inhibitor, a chemokine receptor inhibitor (e.g. CCR2 or CCR5 inhibitor), a SHP1/2 phosphatase inhibitor, a histone deacetylase inhibitor (HDAC) such as an HDAC8 inhibitor, an angiogenesis inhibitor, an interleukin receptor inhibitor, bromo and extra terminal family members inhibitors (for example, bromodomain inhibitors or BET inhibitors, or combinations thereof.

For treatment of cancer, compounds as disclosed herein can be used in combination with inhibitors of PD-1 or inhibitors of PD-L1, e.g., an anti-PD-1 monoclonal antibody or an anti -PD-L 1 monoclonal antibody, for example, nivolumab (Opdivo), pembrolizumab (Keytruda, MK-3475), atezolizumab, avelumab, cemiplimab, spartalizumab, camrelizumab, cetrelimab, toripalimab, sintilimab, AB122, JTX-4014, BGB-108, BCD-100, BAT1306, LZM009, AK105, HLX10, and TSR-042, AMP-224, AMP- 514, PDR001, durvalumab, pidilizumab (Imfinzi^{®}, CT-011), CK-301, BMS 936559, and MPDL3280A. The anti-PD-1 monoclonal antibody may be nivolumab, pembrolizumab, pidilizumab, PDR001, MGA012, PDR001, AB122, or AMP-224. The anti-PD-1 monoclonal antibody may be nivolumab or pembrolizumab. The anti-PD1 antibody may be pembrolizumab. The anti-PD1 antibody may be nivolumab.

For treatment of cancer, compounds as disclosed herein can be used in combination with inhibitors of PD-L1. Antibodies that bind to human PD-L1 include atezolizumab, avelumab, durvalumab, tislelizumab, BMS-935559, MEDI4736, FAZ053, KN035, CS1001, CBT-502, A167, STI-A101, CK-301, BGB-A333, MSB-2311, HLX20, KN035, AUNP12, CA-170, BMS-986189 and LY3300054. The anti-PD-Ll monoclonal antibody may be BMS-935559, MEDI4736, MPDL3280A, or MSB0010718C. The anti-PD-L1 monoclonal antibody may be atezolizumab, avelumab, durvalumab.

CTLA-4 inhibitors, e.g., an anti-CTLA-4 antibody, for example, ipilimumab (Yervoy), tremelimumab and AGEN1884; and phosphatidylserine inhibitors, for example, bavituximab (PGN401); antibodies to cytokines (IL-10, TGF-b, and the like); other anti-cancer agents such as cemiplimab. The inhibitor of an immune checkpoint molecule may be an inhibitor of PD-L1 and CTLA-4, e.g., an anti-PD -LI /CTLA-4 bispecific antibody or an anti-PD-1/CTLA-4 bispecific antibody. Bispecific antibodies that bind to PD-L1 and CTLA-4 include AK104.

The compounds of the present disclosure can be used in combination with bispecific antibodies. One of the domains of the bispecific antibody may target PD-1, PD-L1, CTLA-4, GITR, OX40, TIM3, LAG3, CD137, ICOS, CD3 or TGF(i receptor. The bispecific antibody may bind to PD-1 and PD-L1. The bispecific antibody that binds to PD-1 and PD-L1 may be MCLA-136. The bispecific antibody may bind to PD-L1 and CTLA-4. The bispecific antibody that binds to PD-L1 and CTLA-4 may be AK104. The bispecific antibody may bind to PD-L1 and CD137. The bispecific antibody that binds to PD-L1 and CD 137 may be MCLA-145.

### Pharmaceutical Compositions and Modes of Administration

Compounds provided herein are usually administered in the form of pharmaceutical compositions. Thus, provided herein are also pharmaceutical compositions that comprise one or more of the compounds described herein or a pharmaceutically acceptable salt, a stereoisomer, or a mixture of stereoisomers thereof and one or more pharmaceutically acceptable vehicles selected from carriers, adjuvants and excipients. Suitable pharmaceutically acceptable vehicles may include, for example, inert solid diluents and fillers, diluents, including sterile aqueous solution and various organic solvents, permeation enhancers, solubilizers and adjuvants. Such compositions are prepared in a manner well known in the pharmaceutical art. See, e.g., Remington's Pharmaceutical Sciences, Mace Publishing Co., Philadelphia, Pa. 17th Ed. (1985); and Modern Pharmaceutics, Marcel Dekker, Inc. 3rd Ed. (G.S. Banker & C.T. Rhodes, Eds.).

The pharmaceutical compositions may be administered in either single or multiple doses. The pharmaceutical composition may be administered by various methods including, for example, rectal, buccal, intranasal and transdermal routes. In certain embodiments, the pharmaceutical composition may be administered by intra-arterial injection, intravenously, intraperitoneally, parenterally, intramuscularly, subcutaneously, orally, topically, or as an inhalant.

One mode for administration is parenteral, for example, by injection. The forms in which the pharmaceutical compositions described herein may be incorporated for administration by injection include, for example, aqueous or oil suspensions, or emulsions, with sesame oil, corn oil, cottonseed oil, or peanut oil, as well as elixirs, mannitol, dextrose, or a sterile aqueous solution, and similar pharmaceutical vehicles.

Oral administration may be another route for administration of the compounds described herein. Administration may be via, for example, capsule or enteric coated tablets. In making the pharmaceutical compositions that include at least one compound described herein or a pharmaceutically acceptable salt, a stereoisomer, or a mixture of stereoisomers thereof, the active ingredient is usually diluted by an excipient and/or enclosed within such a carrier that can be in the form of a capsule, sachet, paper or other container. When the excipient serves as a diluent, it can be in the form of a solid, semi-solid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing, for example, up to 10% by weight of the active compound, soft and hard gelatin capsules, sterile injectable solutions, and sterile packaged powders.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, sterile water, syrup, and methyl cellulose. The formulations can additionally include lubricating agents such as talc, magnesium stearate, and mineral oil; wetting agents; emulsifying and suspending agents; preserving agents such as methyl and propylhydroxy-benzoates; sweetening agents; and flavoring agents.

The compositions that include at least one compound described herein or a pharmaceutically acceptable salt, a stereoisomer, or a mixture of stereoisomers thereof can be formulated so as to provide quick, sustained or delayed release of the active ingredient after administration to the subject by employing procedures known in the art. Controlled release drug delivery systems for oral administration include osmotic pump systems and dissolutional systems containing polymer-coated reservoirs or drug-polymer matrix formulations. Examples of controlled release systems are given in U.S. Patent Nos. 3,845,770; 4,326,525; 4,902,514; and 5,616,345. Another formulation for use in the methods disclosed herein employ transdermal delivery devices ("patches"). Such transdermal patches may be used to provide continuous or discontinuous infusion of the compounds described herein in controlled amounts. The construction and use of transdermal patches for the delivery of pharmaceutical agents is well known in the art. See, e.g., U.S. Patent Nos. 5,023,252, 4,992,445 and 5,001,139. Such patches may be constructed for continuous, pulsatile, or on demand delivery of pharmaceutical agents.

For preparing solid compositions such as tablets, the principal active ingredient may be mixed with a pharmaceutical excipient to form a solid preformulation composition containing a homogeneous mixture of a compound described herein or a pharmaceutically acceptable salt, a stereoisomer, or a mixture of stereoisomers thereof. When referring to these preformulation compositions as homogeneous, the active ingredient may be dispersed evenly throughout the composition so that the composition may be readily subdivided into equally effective unit dosage forms such as tablets, pills and capsules.

The tablets or pills of the compounds described herein may be coated or otherwise compounded to provide a dosage form affording the advantage of prolonged action, or to protect from the acid conditions of the stomach. For example, the tablet or pill can include an inner dosage and an outer dosage component, the latter being in the form of an envelope over the former. The two components can be separated by an enteric layer that serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

Compositions for inhalation or insufflation may include solutions and suspensions in pharmaceutically acceptable, aqueous or organic solvents, or mixtures thereof, and powders. The liquid or solid compositions may contain suitable pharmaceutically acceptable excipients as described herein. In some embodiments, the compositions are administered by the oral or nasal respiratory route for local or systemic effect. In other embodiments, compositions in pharmaceutically acceptable solvents may be nebulized by use of inert gases. Nebulized solutions may be inhaled directly from the nebulizing device or the nebulizing device may be attached to a facemask tent, or intermittent positive pressure breathing machine. Solution, suspension, or powder compositions may be administered, preferably orally or nasally, from devices that deliver the formulation in an appropriate manner.

### Dosing

The specific dose level of a compound of the present application for any particular subject will depend upon a variety of factors including the activity of the specific compound employed, the age, body weight, general health, sex, diet, time of administration, route of administration, and rate of excretion, drug combination and the severity of the particular disease in the subject undergoing therapy. For example, a dosage may be expressed as a number of milligrams of a compound described herein per kilogram of the subject's body weight (mg/kg). Dosages of between about 0.1 and 150 mg/kg may be appropriate. In some embodiments, about 0.1 and 100 mg/kg may be appropriate. In other embodiments a dosage of between 0.5 and 60 mg/kg may be appropriate. Normalizing according to the subject's body weight is particularly useful when adjusting dosages between subjects of widely disparate size, such as occurs when using the drug in both children and adult humans or when converting an effective dosage in a non-human subject such as dog to a dosage suitable for a human subject.

### Synthesis of the Compounds

The compounds may be prepared using the methods disclosed herein and routine modifications thereof, which will be apparent given the disclosure herein and methods well known in the art. Conventional and well-known synthetic methods may be used in addition to the teachings herein. The synthesis of typical compounds described herein may be accomplished as described in the following examples. If available, reagents may be purchased commercially, *e*.*g*., from Sigma Aldrich or other chemical suppliers.

Typical embodiments of compounds described herein may be synthesized using the general reaction schemes described below. It will be apparent given the description herein that the general schemes may be altered by substitution of the starting materials with other materials having similar structures to result in products that are correspondingly different. Descriptions of syntheses follow to provide numerous examples of how the starting materials may vary to provide corresponding products. Given a desired product for which the substituent groups are defined, the necessary starting materials generally may be determined by inspection. Starting materials are typically obtained from commercial sources or synthesized using published methods. For synthesizing compounds which are embodiments described in the present disclosure, inspection of the structure of the compound to be synthesized will provide the identity of each substituent group. The identity of the final product will generally render apparent the identity of the necessary starting materials by a simple process of inspection, given the examples herein. In general, compounds described herein are typically stable and isolatable at room temperature and pressure.

Preparation of compounds as disclosed herein can involve the protection and deprotection of various chemical groups. The need for protection and deprotection, and the selection of appropriate protecting groups, can be readily determined by one skilled in the art. The chemistry of protecting groups is described, *e*.*g*., in Kocienski, Protecting Groups, (Thieme, 2007); Robertson, Protecting Group Chemistry, (Oxford University Press, 2000); Smith et al., March's Advanced Organic Chemistry: Reactions, Mechanisms, and Structure, 6th Ed. (Wiley, 2007); Peturssion et al., "Protecting Groups in Carbohydrate Chemistry," J. Chem. Educ., 1997, 74(11), 1297; and Wuts et al., Protective Groups in Organic Synthesis, 4th Ed., (Wiley, 2006).

The Schemes below provide general guidance in connection with preparing the compounds of the invention. One skilled in the art would understand that the preparations shown in the Schemes can be modified or optimized using general knowledge of organic chemistry to prepare various compounds of the invention.

Compounds of Formula (II) or any subformulas as disclosed herein and certain intermediates can be prepared, for example, using a process as illustrated in Schemes 1-4. The variables employed in the Schemes below are as defined throughout the specification.

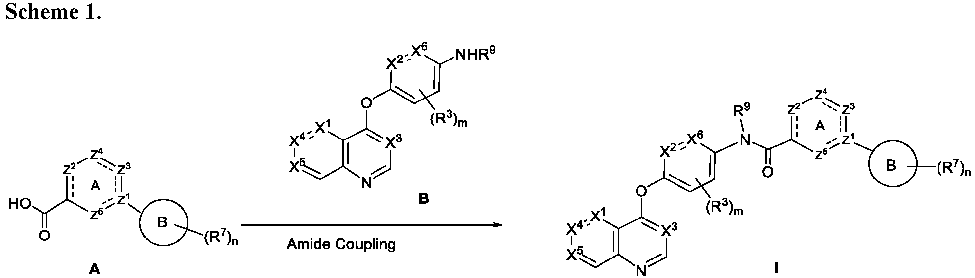

As shown in Scheme 1, a compound of formula I can be synthesized from carboxylic acid **A** and aniline **B** by standard methods to form amide bonds using coupling agents appropriate for this transformation that are well known in the art such as HATU in the presence of a base such as DIEA in organic solvents such as DMF at room or elevated temperatures, wherein Q is a leaving group (including, but not limiting to, Br, Cl, I, triflate, and the like).

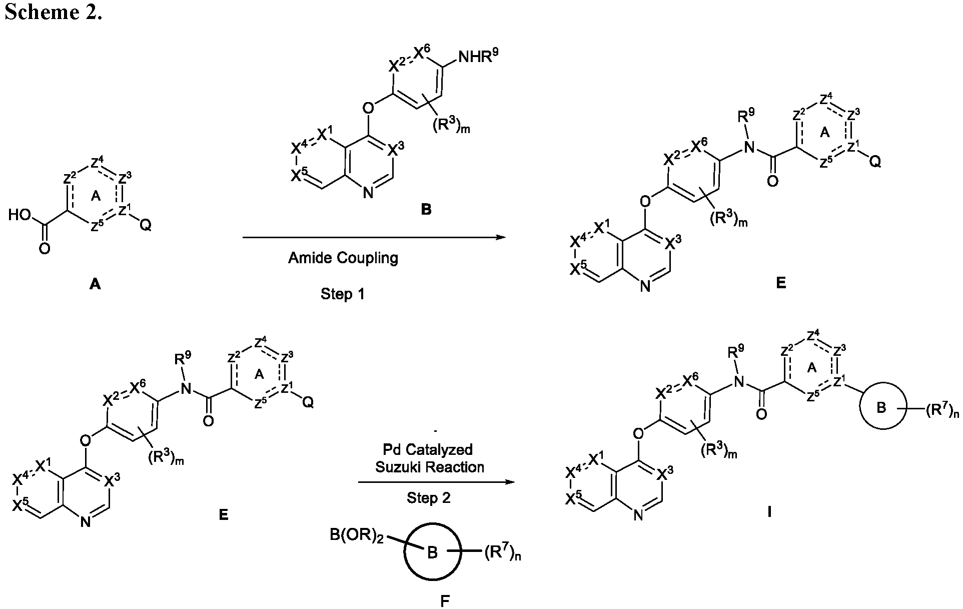

As shown in Scheme 2, a compound of formula **I** can be made from a two-step process starting from bromocarboxylic acid **D**, where Q is a leaving group, including Cl, Br, I or triflate, and aniline **B** which are coupled together by standard methods to form amide bonds using coupling agents appropriate for this transformation that are well known in the art such as HATU in the presence of a base such as DIEA in organic solvents such as DMF at room or elevated temperatures to form a compound of formula **E**. In a second step, compounds of formula **E** can be converted to compounds of formula **I** by coupling with boron acid or ester compounds of the formula **F** using coupling chemistry known to those skilled in the art. Typical procedures to accomplish this type of coupling involve the use palladium-containing complexes as a catalyst in the presence of an inorganic base such as tripotassium phosphate in a mixture of water and a water-miscible solvent such as dioxane.

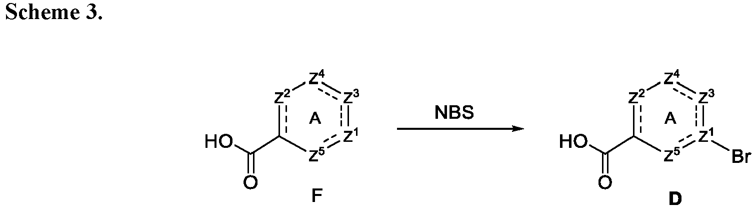

As shown in Scheme 3, a compound of formula **D** (Q = Br) can be prepared from carboxylic acid **F** through treatment with NBS in an appropriate solvent typically at room temperature.

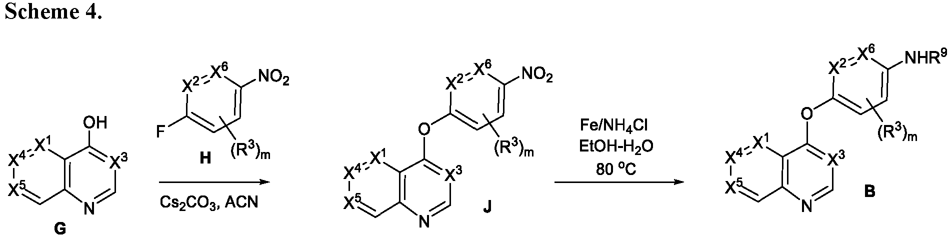

As shown in Scheme 4, a compound of formula J can be prepared by reacting a compound of formula **G** with a compound of formula **H** in the presence of a base such as cesium carbonate in an appropriate organic solvent, typically at room temperature. A compound of formula **B** can be made from a compound of formula **J** by reducing the nitro group with a mixture of ammonium chloride and iron typically in a solvent mixture of water and an alcohol such as methanol or ethanol at elevated temperatures. 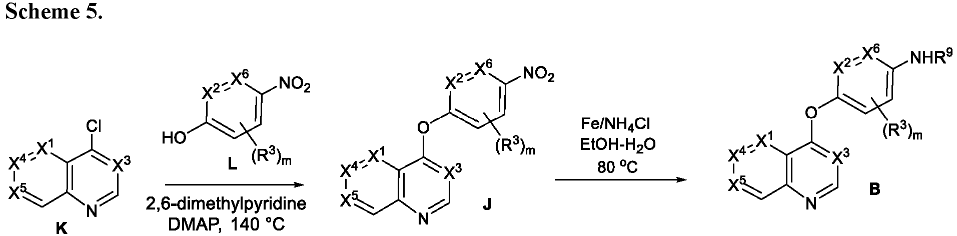 As shown in Scheme 5, a compound of formula **J** can also be synthesized by reacting a compound of formula **K** with a compound of formula **L** in an appropriate solvent such as 2,6-dimethylpyridine in the presence of a catalytic amount of dimethylaminopyridine at elevated temperatures. A compound of formula **B** can be prepared from a compound of formula **J** by reducing the nitro group with a mixture of ammonium chloride and iron typically in a solvent mixture of water and an alcohol such as methanol or ethanol at elevated temperatures.

### EXAMPLES

The following examples are included to demonstrate specific embodiments of the disclosure. It should be appreciated by those of skill in the art that the techniques disclosed in the examples which follow represent techniques to function well in the practice of the disclosure, and thus can be considered to constitute specific modes for its practice. However, those of skill in the art should, in light of the present disclosure, appreciate that many changes can be made in the specific embodiments which are disclosed and still obtain a like or similar result without departing from the scope of the disclosure. Compounds **49, 54, 54A, 223, 258** and **259** are not according to the claimed invention and are present for reference purposes only.

### Synthetic Examples

The following examples are provided for the purpose of further illustration and are not intended to limit the scope of the claimed invention. The following examples are included to demonstrate specific embodiments of the disclosure. It should be appreciated by those of skill in the art that the techniques disclosed in the examples which follow represent techniques to function well in the practice of the disclosure, and thus can be considered to constitute specific modes for its practice. However, those of skill in the art should, in light of the present disclosure, appreciate that many changes can be made in the specific embodiments which are disclosed and still obtain a like or similar result without departing from the scope of the disclosure.

### General Procedure A: 4-((1,5-Naphthyridin-4-yl)oxy)anilines (6)

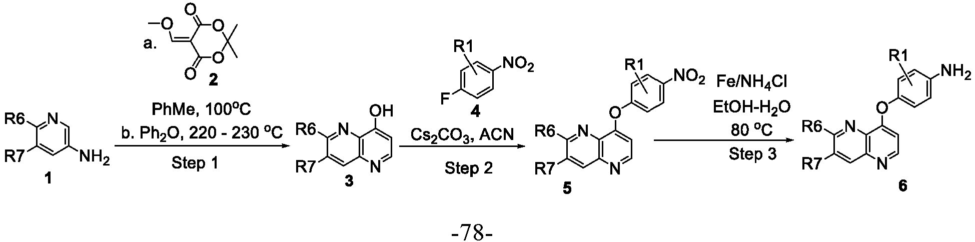

**Step 1:** A mixture of Compound **1** (32 mmol) and Compound **2** (5.92 g, 32 mmol) in toluene (50 mL) was stirred at 105 °C for 1.5 h and cooled to room temperature. Hexane (50 mL) was added and the suspension filtered to give a brown solid. This solid was mixed with Ph₂O (50 mL) and the resulting mixture was stirred at 220-230 °C for 1 h, cooled to room temperature and poured into Et₂O (100 mL). The resulting suspension was filtered, washed with Et₂O and dried to give Compound **3** typically as a brown solid (25-85% yield).

**Step 2:** A mixture of Compound **3** (4.8 mmol), Compound **4** (6.8 mmol), and Cs₂CO₃ (6.6 g, 20 mmol) in acetonitrile (20 mL) was stirred at room temperature overnight. EtOAc (80 mL) was added and the resulting mixture filtered. The filtrate was evaporated and residue purified by silica gel column chromatography to give Compound **5** (20-50%).

**Step 3:** A mixture of Compound **5** (1.8 mmol), NH₄Cl (500 mg, 9.3 mmol), and Fe (260 mg, 4.6 mmol) in MeOH / water (20 / 5 mL) was refluxed for 1 h and then cooled to room temperature. The resulting mixture was filtered through Celite and filtrate concentrated to remove MeOH. To residue was added aq saturated NaHCO₃ (6 mL) and the resulting aqueous mixture was extracted with EtOAc. The organic extract was dried over anhyd. Na₂SO₄ and evaporated give Compound **6** typically as a brown solid (50-100% yield).

The following intermediates were made following **General Procedure A** for the synthesis of 4-((1,5-Naphthyridin-4-yl)oxy)anilines **6:**

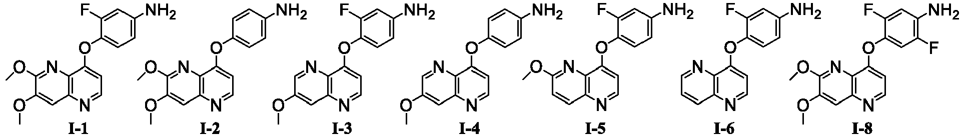

**4-((6,7-Dimethoxy-1,5-naphthyridin-4-yl)oxy)-3-fluoroaniline (I-1):** MS for C₁₆H₁₄FN₃O₃: *m*/*z* 316 (MH+).

**4-((6,7-Dimethoxy-1,5-naphthyridin-4-yl)oxy)aniline (I-2):** MS for C₁₆H₁₅N₃O₃: *m*/*z* 298 (MH+).

**3-Fluoro-4-((7-methoxy-1,5-naphthyridin-4-yl)oxy)aniline (I-3):** MS for C₁₅H₁₂FN₃O₂: *m*/*z* 286 (MH+).

**4-((7-Methoxy-1,5-naphthyridin-4-yl)oxy)aniline (I-4):** MS for C₁₅H₁₃N₃O₂: *m*/*z* 268 (MH+).

**3-Fluoro-4-((6-methoxy-1,5-naphthyridin-4-yl)oxy)aniline (I-5):** MS for C₁₅H₁₂FN₃O₂: *m*/*z* 286.0 (MH+).

**4-((1,5-Naphthyridin-4-yl)oxy)-3-fluoroaniline (I-6):** MS for C₁₄H₁₀FN₃O: *m*/*z* 256 (MH+).

**4-((6,7-Dimethoxy-1,5-naphthyridin-4-yl)oxy)-2,5-difluoroaniline (I-8):** MS for C₁₆H₁₃F₂N₃O₃: *m*/*z* 334.0 (MH⁺).

### General Procedure B: 4-(Quinolin-4-yloxy)aniline (10)

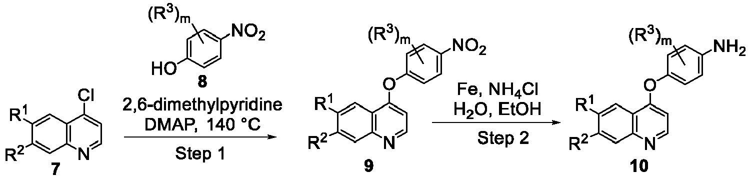

**Step 1:** To a mixture of Compound **7** (44.7 mmol, 1 eq) and Compound **8** (62.5 mmol, 1.4 eq) in 2,6-dimethylpyridine (50 mL) was added DMAP (1.10 g, 9.0 mmol, 0.2 eq). The mixture was stirred at 140 °C for 36 h. The reaction was cooled to room temperature, MeOH (32 g) was added, followed by aq K₂CO₃ (4 g in water (62 g)). The resulting mixture was stirred at 0 °C for 2 h. The resulting precipitate was filtered and washed with water (200 mL) to give Compound 9 as a yellow solid (50-60% yield).

**Step 2:** To a mixture of Compound 9 (6.1 mmol, 1 eq) in EtOH (40 mL) and water (8 mL) was added Fe (1.71 g, 30.6 mmol, 5.0 eq) and NH₄Cl (2.62 g, 49.0 mmol, 8.0 eq). The mixture was stirred at 85 °C for 3 h. The reaction was filtered, and the filtrate was dried over anhyd. Na₂SO₄ and concentrated to give crude product. To this crude product was added EtOAc (150mL) and DCM (150 mL). The resulting mixture was filtered, and the filtrate was concentrated to give Compound **10** as a yellow solid (50-70% yield).

The following intermediate was made following **General Procedure B** for the synthesis of 4-(quinolin-4-yloxy)anilines **10:**

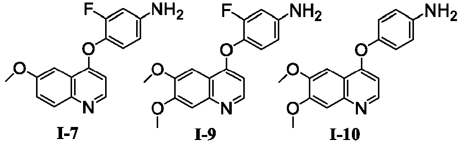

**3-Fluoro-4-((6-methoxyquinolin-4-yl)oxy)aniline (I-7):** MS for C₁₆H₁₃FN₂O₂: *m*/*z* 285.0 (MH+).

**4-((6,7-Dimethoxyquinolin-4-yl)oxy)-3-fluoroaniline (I-9):** MS for C₁₇H₁₅FN₂O₃: *m*/*z* 315 (MH⁺).

**4-((6,7-Dimethoxyquinolin-4-yl)oxy)aniline (1-10):** MS for C₁₇H₁₆N₂O₃: *m*/*z* 297.2 (MH⁺).

### General Procedure C: Suzuki Reaction

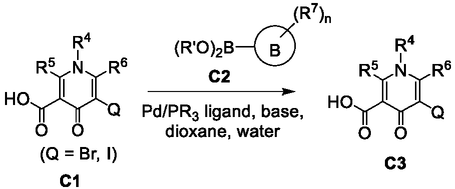

A mixture of the Compound **C1** (1 eq), boronic acid/ester **C2** (1-5 eq), catalytic Pd/PR₃ complex such as, but not limited to, Pd(PPh₃)₄ (5-10 mol%), Pd(dppf)Cl₂ (mol10-20%), Pd(Amphos)₂Cl₂ (10-20 mol%)/SPhos (1 eq), and a base (2-5 eq) such as, but not limited to, Cs₂CO₃, K₂CO₃, Na₂CO₃, K₃PO₄, KF, in dioxane/water (1/1 to 5/1) (1.5-5 mL/mmol of **C1**) was degassed and purged with nitrogen 3 times. The resulting mixture was stirred at 80 - 160 °C, with or without microwave irradiation, under an atmosphere of nitrogen until the starting material **C1** was consumed (0.5-20 hr) as monitored by LC-MS and/or TLC. The reaction mixture was then concentrated under reduced pressure. To the resulting residue was added water and resulting mixture was washed with EtOAc, followed by DCM. The aqueous phase was acidified with aq 2 N HCl to pH 2 - 5. If a suspension resulted, the mixture was filtered, the solid washed with water and dried under reduced pressure to give crude Compound **C3.** If a filterable solid did not result, the acidic aqueous phase was extracted with an organic solvent such as, but not limited to, EtOAc or DCM. The combined organic extracts were dried over anhyd Na₂SO₄ or MgSO₄ and concentrated to provide crude Compound **C3.** Crude Compound **C3** was either purified by silica gel chromatography or used directly in subsequent steps without further purification (9 - 97% yield).

### General Procedure D: HATU Coupling

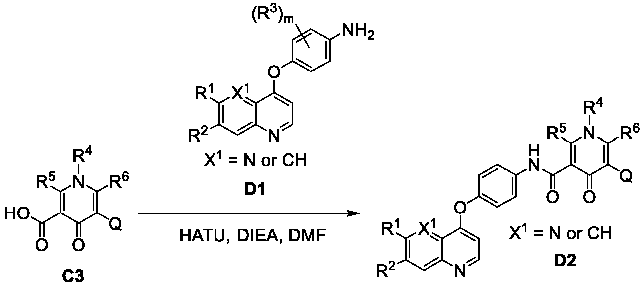

To a solution of Compound **C3** (1 eq) in DMF (2-5 mL/mmol of **C3**) was added the aniline **D1** (0.7-1.1 eq), HATU (1.1-2 eq), and DIEA (3-5 eq). The mixture was stirred (room temperature to 40 °C) until Compound **C3** was consumed based on LC-MS and/or TLC (4-15 h). The reaction was quenched with water and extracted with EtOAc twice. The combined organic extracts were washed with aq saturated NaCl (3 - 5 times), dried over anhyd Na₂SO₄ and concentrated under reduced pressure. The resulting residue was purified by flash silica gel chromatography to give the Compound **D2** (6 - 63% yield).

### Example 1: 6-Methoxy-5-(2-methoxyethoxy)pyridin-3-amine (13)

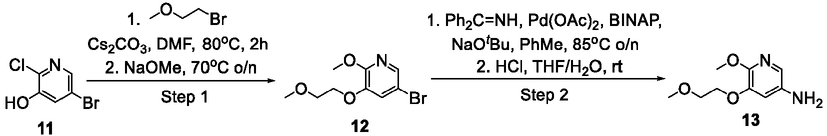

**Step 1: 2,3-Dimethoxy-8-(4-nitrophenoxy)-1,5-naphthyridine (12):** A mixture of Compound **11** (2.10 g, 10.0 mmol), 1-bromo-2-methoxyethane (1.50 g, 10.8 mmol), and Cs₂CO₃, (6.6 g, 20.2 mmol) in DMF was stirred at 80 °C for 2 h, quenched with water and extracted with EtOAc (2x), The combined extracts were washed with aq saturated NaCl, dried over Na₂SO₄ and evaporated to give the crude intermediate product as an off-white solid (2.68 g, MS for C₈H₉BrClNO₂, found 268 (MH+)). This intermediate product (2.68 g, 10.0 mmol) was mixed with NaOMe (3.0 g, 55.5 mmol) in MeOH (40 mL) and heated at 70 °C overnight. The reaction mixture was concentrated to remove MeOH and the resulting residue was partitioned between water and EtOAc. The EtOAc phase was washed with aq saturated NaCl, dried over Na₂SO₄ and evaporated to give crude Compound **12** as an oil (3.0 g) which was used in the next step without further purification. MS for C₉H₁₂BrNO₃: *m*/*z* 262/264 (MH+).

**Step 2: 6-Methoxy-5-(2-methoxyethoxy)pyridin-3-amine (13):** Compound **12** (3.0 g, crude) was mixed with diphenylmethanimine (3.6 g, 20 mmol), Pd(OAc)2 (360 mg, 1.61 mmol), BINAP (1.3 g, 2.08 mmol) and NaO*^{t}*Bu (1.6 g, 16.7 mmol) in toluene (60 mL). The resulting mixture was degassed with argon and stirred at 85 °C overnight. The reaction mixture was partitioned between water and EtOAc. The organic phase was separated and evaporated to dryness under reduced pressure. To the residue was added THF (40 mL) and aqueous 2M HCl (40 mL) and resulting mixture was stirred at room temperature overnight. The pH of the reaction mixture was adjusted to pH 10 with NaHCO₃ and extracted with EtOAc. The extract was concentrated and the resulting residue subjected to chromatography on silica gel(0-90% EtOAc in hexanes) to afford Compound **13** as a brown oil (1.4 g, 71% yield from Compound 11). MS for C₉H₁₄N₂O₃: *m*/*z* 199 (MH+).

The following compounds can be made by adapting the procedure used to make Compound **13** in **Example 1:**

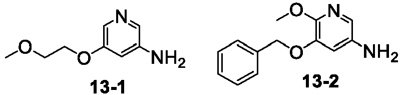

**5-(2-Methoxyethoxy)pyridin-3-amine (13-1):** Step 1 was replaced with the following method: To a mixture of 5-bromopyridin-3-ol (2 g, 11.5 mmol, 1 eq) and 1-bromo-2-methoxy-ethane (2.4 g, 17 mmol, 11.5 eq) in DMF (20 mL) was added Cs₂CO₃ (4.9 g, 14.9 mmol, 1.3 eq). The mixture was stirred at 80 °C for 12 h. The reaction mixture was diluted with EtOAc (10 mL) and washed with water (5 x 30 mL) washed with aq saturated NaCl (30 mL), dried over anhyd Na₂SO₄ and concentrated under reduced pressure to give 3-bromo-5-(2-methoxyethoxy)pyridine as a yellow solid (4.2 g, crude) which was used to replace Compound 12 in Step 2. ¹H NMR (400 MHz, DMSO-*d₆*) δ 7.55 (d, 1H), 7.47 (d, 1H), 6.50 (t, 1H), 5.30 (s, 2H), 4.06 - 4.00 (m, 2H), 3.65 - 3.59 (m, 2H), 3.33 - 3.23 (m, 3H).

**5-(Benzyloxy)-6-methoxypyridin-3-amine (13-2):** 1-Bromo-2-methoxyethane was replaced by 4-methoxybenzyl chloride. MS for C₁₃H₁₄N₂O₂: *m*/*z* 231.0 (MH+).

### Example 2: 3-Fluoro-4-((6-methoxy-7-(2-methoxyethoxy)-1,5-naphthyridin-4-yl)oxy)aniline (17)

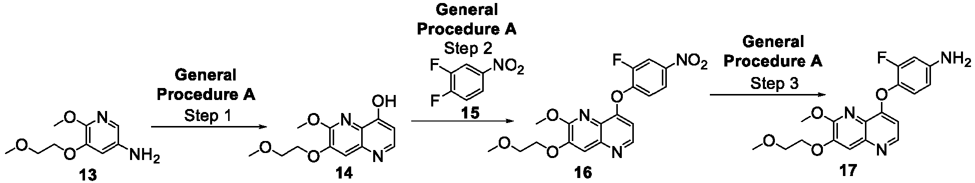

**3-Fluoro-4-((6-methoxy-7-(2-methoxyethoxy)-1,5-naphthyridin-4-yl)oxy)aniline (17):** Compound **17** was made from Compound **13** following the three step procedure outlined in **General Procedure A for** the synthesis of 4-((1,5-naphthyridin-4-yl)oxy)anilines **6.** ¹H NMR (400 MHz, DMSO-d6) δ 8.47 (d, 1H), 7.64 (s, 1H), 7.05 (t, 1H), 6.61 - 6.50 (m, 2H), 6.46 (dd, 1H), 5.47 (s, 2H), 4.31 (t, 2H), 4.04 (s, 3H), 3.76 (t, 2H), 3.34 (s, 3H); MS for C₁₈H₁₈FN₃O₄: *m*/*z* 360.2 (MH+).

The following compounds can be made by adapting the procedure used to make Compound **17** in **Example 2:**

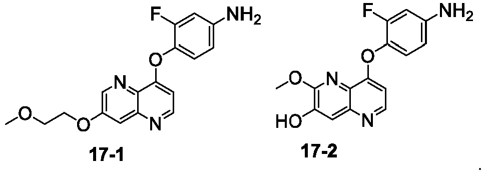

### Example 3: 4-((6-Chloro-1,7-naphthyridin-4-yl)oxy)-3-fluoroaniline (24)

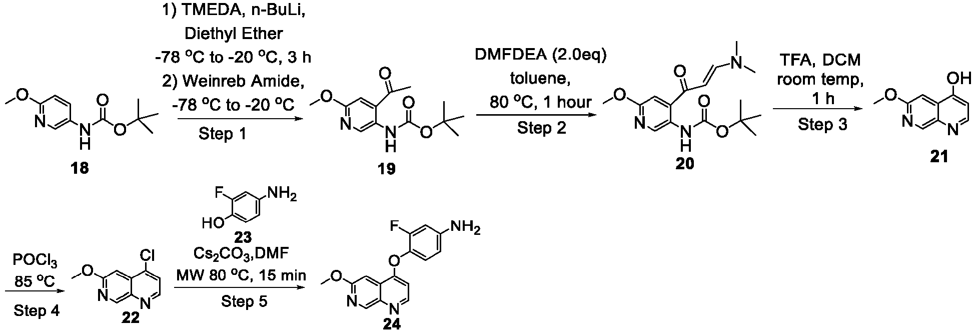

**Step 1: tert-Butyl (4-acetyl-6-methoxypyridin-3-yl)carbamate (19):** Compound **18** (2.5g, 11 mmol) was added to an oven dried round bottom flask equipped with a magnetic stir bar under nitrogen. Anhydrous diethyl ether (50 mL) was added to the flask under nitrogen followed by TMEDA (5.0 mL, 3.0 eq). The homogenous mixture was cooled to -78 °C and stirred for 15 min under nitrogen. N-Butyl lithium (10 mL, 2.5M in hexanes) was added to the mixture dropwise. After addition was complete, the mixture was warmed up to -20 °C and stirred at that temperature for 2 h. After 2 h, the mixture was cooled to -78 °C, Weinreb amide was added to the mixture, after which the mixture was allowed to warm up to -20 °C and stirred under nitrogen for 2 h. The mixture was quenched at low temperature with water. EtOAc was used to extract the aqueous layer. The combined organic extracts were dried over anhyd. Na₂SO₄ and concentrated under reduced pressure. The residue was absorbed onto silica gel and purified by flash chromatography (EtOAc:hexanes) to give Compound **19** as an off white solid (745 mg, 25% yield). ¹H NMR (400 MHz, CDCl₃) δ 9.61 (bs, 1H), 9.20 (s, 1H), 7.10 (s, 1H), 3.97 (s, 3H), 2.64 (s, 3H), 1.54 (s, 9H).

**Step 2: tert-Butyl (E)-(4-(3-(dimethylamino)acryloyl)-6-methoxypyridin-3-yl)carbamate (20):** Compound 19 (745 mg, 2.79 mmol) was dissolved in toluene and DMFDEA (1.46 mL, 2.0 eq) was added to the resulting solution. The reaction mixture was heated at 80 °C. Upon completion of the reaction, the solution was concentrated under reduced pressure to remove toluene. EtOAc was added to the residue and the mixture was absorbed onto silica gel under reduced pressure and purified by flash chromatography (EtOAc:hexanes) to give Compound **20** as an orange solid (800 mg, 89% yield). ¹H NMR (400 MHz, CDCl₃) δ 9.50 (bs, 1H), 9.03 (s, 1H), 7.79 (d, 1H), 6.95 (s, 1H), 5.56 (d, 1H), 3.97 (s, 3H), 3.21 (s, 3H), 2.96 (s, 3H), 1.51 (s, 9H); MS for C₁₆H₂₃N₃O₄: *m*/*z* 322 (MH+).

**Step 3: 6-Methoxy-1,7-naphthyridin-4-ol (21):** Compound **20** (800 mg, 2.49 mmol) was dissolved in DCM (12.45 mL, 0.2M) and trifluoroacetic acid (3.81 mL, 20 eq) was added dropwise. The resulting mixture was stirred at room temperature for 1 h. Upon completion of the reaction, the reaction mixture was concentrated under reduced pressure to remove trifluoroacetic acid. The residual solid was suspended in EtOAc and a small amount of aq saturated NaHCO₃ solution was added dropwise until the solid dissolved into the organic layer. The phases were separated and solid NaCl was added to the aq layer. The resulting mixture was extracted with DCM. The combined organic extracts were dried over anhyd. Na₂SO₄ and concentrated under reduced pressure to give crude Compound **21** (435 mg, 99.2% yield) which used for the next step without further purification. ¹H NMR (400 MHz, DMSO-*d₆*) δ 8.76 (s, 1H), 7.97 (d, 1H), 7.24 (s, 1H), 6.02 (d, 1H), 3.91 (s, 3H); MS for C₉H₈N₂O₂: *m*/*z* 177 (MH+).

**Step 4: 4-Chloro-6-methoxy-1,7-naphthyridine (22):** Compound **21** (435 mg, 2.47 mmol) was suspended in POCl₃ (6.5mL) and the mixture was heated at 80 °C for 2 h. Upon completion of reaction, excess POCl₃ was mostly removed by concentrating under reduced pressure. EtOAc was added to the residue, and the resulting mixture was cooled in an ice bath. Aqueous saturated NaHCO₃ was added dropwise until all the residual POCl₃ was consumed. The organic phase was separated from the aqueous phase and concentrated under reduced pressure. DCM was added to the resulting residue. The resulting solution was dried over anhyd. Na₂SO₄ and concentrated under reduced pressure to give Compound **22** as an off while solid (353 mg, 73% yield) which used in the next step without further purification. ¹H NMR (400 MHz, DMSO-*d₆*) δ 9.24 (s, 1H), 8.79 (d, 1H), 7.92 (d, 1H), 7.28 (s, 1H), 4.03 (s, 3H); MS for C₉H₇ClN₂O: *m*/*z* 195 (MH+).

**Step 5: 3-Fluoro-4-((6-methoxy-1,7-naphthyridin-4-yl)oxy)aniline (23):** Compound **22** (353 mg, 1.81 mmol), was dissolved in anhydrous DMF (9 mL, 0.2 M) in a 20 mL microwave tube. Cs₂CO₃ (1.77 g, 3.0 eq) was added to the mixture followed by Compound **23** (461 mg, 2.0 eq). The mixture was degassed with nitrogen for 5 min, then sealed and heated at 85 °C under microwave irradiation for 15 min. Upon completion of the reaction, the mixture was diluted with DCM and filtered. The filtrate was washed with water and concentrated under reduced pressure. The resulting residue was diluted with DCM again and washed with water again. The phases were separated and the organic phase was dried over anhyd. Na₂SO₄ and concentrated under reduced pressure. The resulting residue was absorbed onto silica gel and purified by flash column chromatography to give Compound **24** as a light brown solid (402 mg, 77.7% yield). ¹H NMR (400 MHz, DMSO-*d₆*) δ 9.15 (s, 1H), 8.62 (d, 1H), 7.39 (s, 1H), 7.10 (t, 1H), 6.66 (d, 1H), 6.56 (d, 1H), 6.48 (d, 1H), 5.54 (s, 2H), 4.01 (s, 3H); MS for C₁₃H₁₂FN₃O₂: *m*/*z* 286 (MH+).

The following compound was made using the same 5 step synthesis used to make Compound **24** in Example 3:

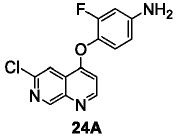

**4-((6-Chloro-1,7-naphthyridin-4-yl)oxy)-3-fluoroaniline (24A):** Compound **18** was replaced with tert-butyl (6-chloropyridin-3-yl)carbamate. ¹H NMR (400 MHz, DMSO-*d₆*) δ 9.29 (s, 1H), 8.85 (d, 1H), 8.25 (s, 1H), 7.13 (t, 1H), 6.85 (d, 1H), 6.57 (d, 1H), 6.48 (d, 1H), 5.71 (s, 2H); MS for C₁₄H₉ClFN₃O: *m*/*z* 290 (MH+).

### Example 4: Ethyl 3-((5-fluoropyridin-2-yl)amino)-3-oxopropanoate (27)

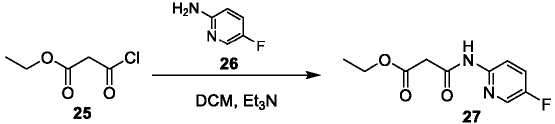

A solution of Compound **25** (1 g, 6.64 mmol, 0.83 mL, 1 eq) in DCM (5 mL) was added dropwise to a stirred solution of Compound **26** (745 mg, 6.64 mmol, 1 eq) and Et₃N (0.92 mL, 6.64 mmol) in DCM (20 mL) at -70 °C under nitrogen. Once addition of the Compound **25** solution was complete, the resulting reaction mixture was allowed to warm up to 15 °C for 12 h. The reaction mixture was concentrated in vacuo and the resulting residue was dissolved in EtOAc (30 mL), washed successively with water (2 x 15 mL) and aq saturated NaCl (2 x 15 mL), dried over anhyd. Na₂SO₄ and concentrated under vacuum to give Compound **27** as a yellow solid (1.0 g, 66.6% yield). ¹H NMR (400 MHz, CDCl₃) δ 9.64 (br s, 1H), 8.22 (br dd, 1H), 8.15 (d, 1H), 7.40-7.50 (m, 1H), 4.27 (q, 2H), 3.50 (s, 2H), 1.35-1.30 (m, 3H).

The following compounds were made in a manner similar to Compound **27** in **Example 4:**

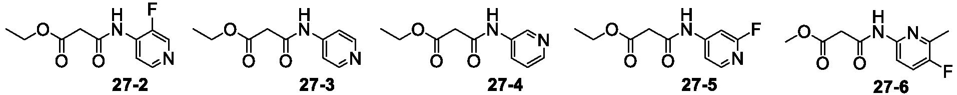

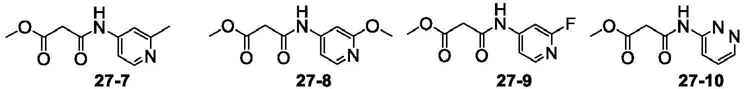

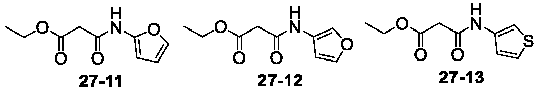

**Ethyl 3-((3-fluoropyridin-4-yl)amino)-3-oxopropanoate (27-2):** Compound **26** was replaced with 3-**fluoropyridin**-4-amine. ¹H NMR (400 MHz, CDCl₃) δ 9.99 (s, 1H), 8.51 - 8.40 (m, 1H), 8.41 - 8.29 (m, 2H), 4.31 (q, 2H), 3.56 (s, 2H), 1.35 (t, 3H); MS for C₁₀H₁₁FN₂O₃: *m*/*z* 227.0 (MH+).

**Ethyl 3-oxo-3-(pyridin-4-ylamino)propanoate (27-3):** Compound **26** was replaced with pyridin-4-amine. ¹H NMR (400 MHz, DMSO-*d*₆) δ 10.57 (s, 1H), 8.44 (d, 2H), 7.54 (d, 2H), 4.13 (q, 2H), 3.51 (s, 2H), 1.20 (t, 3H); MS for C₁₀H₁₂N₂O₃: *m*/*z* 209.0 (MH+).

**Ethyl 3-oxo-3-(pyridin-3-ylamino)propanoate (27-4):** Compound **26** was replaced with pyridin-3-amine. ¹H NMR (400 MHz, DMSO-*d*₆) δ 10.40 (s, 1H), 8.71 (d, 1H), 8.29 (dd, 1H), 8.05 - 7.96 (m, 1H), 7.36 (dd, 1H), 4.13 (q, 2H), 3.49 (s, 2H), 1.21 (t, 3H); MS for C₁₀H₁₂N₂O₃: *m*/*z* 209.0 (MH+).

**Ethyl 3-((2-fluoropyridin-4-yl)amino)-3-oxopropanoate (27-5):** Compound **26** was replaced with 2-fluoropyridin-4-amine. ¹H NMR (400 MHz, CDCl₃) δ 9.88 (s, 1H), 8.13 (d, 1H), 7.41 (d, 1H), 7.32 - 7.21 (m, 1H), 4.36 - 4.24 (m, 2H), 3.53 (s, 2H), 1.36 (t, 3H).

**Methyl 3-((5-fluoro-6-methylpyridin-2-yl)amino)-3-oxopropanoate (27-6):** Compound **26** was replaced with 5-fluoro-6-methylpyridin-2-amine and Compound **25** was replaced by methyl 3-chloro-3-oxopropanoate. MS for C₁₀H₁₁FN₂O₃: *m*/*z* 227 (MH+).Methyl **3-((2-methylpyridin-4-yl)amino)-3-oxopropanoate (27-7):** Compound **26** was replaced with 2-methylpyridin-4-amine and Compound **25** was replaced by methyl 3-chloro-3-oxopropanoate. ¹H NMR (400 MHz, DMSO-*d₆*) δ 10.58 (s, 1H), 8.31 (d, 1H), 7.44 (d, 1H), 7.35 (dd, 1H), 3.65 (s, 3H), 3.53 (s, 2H), 2.41 (s, 3H); MS for C₁₀H₁₂N₂O₃: *m*/*z* 208.9 (MH+).

**Methyl 3-((2-methoxypyridin-4-yl)amino)-3-oxopropanoate (27-8):** Compound **26** was replaced with 2-methoxypyridin-4-amine and Compound **25** was replaced by methyl 3-chloro-3-oxopropanoate. ¹H NMR (400 MHz, CDCl₃) δ 9.45 (br s, 1H), 8.08 (d, 1H), 7.11 (d, 1H), 7.03 (dd, 1H), 3.94 (s, 3H), 3.82 (s, 3H), 3.51 (s, 2H); MS for C₁₀H₁₂N₂O₄: *m*/*z* 224.9 (MH+).

**Methyl 3-((2-fluoropyridin-4-yl)amino)-3-oxopropanoate (27-9):** Compound **26** was replaced with 2-fluoropyridin-4-amine and Compound **25** was replaced by methyl 3-chloro-3-oxopropanoate. MS for C₉H₉FN₂O₃: *m*/*z* 212.9 (MH+).

**Methyl 3-oxo-3-(pyridazin-3-ylamino)propanoate (27-10):** Compound **26** was replaced with pyridazin-3-amine and Compound **25** was replaced by methyl 3-chloro-3-oxopropanoate. MS for C₈H₉N₃O₃: *m*/*z* 195.9 (MH+).

**Ethyl 3-(furan-2-ylamino)-3-oxopropanoate (27-11):** Compound **26** was replaced with furan-2-amine. MS for C₉H₁₁NO₄: *m*/*z* 198 (MH+).

**Ethyl 3-(furan-3-ylamino)-3-oxopropanoate (27-12):** Compound **26** was replaced with furan-3-amine. MS for C₉H₁₁NO₄: *m*/*z* 198 (MH+).

**Ethyl 3-oxo-3-(thiophen-3-ylamino)propanoate (27-13):** Compound **26** was replaced with thiophen-3-amine. MS for C₉H₁₁NO₃S: *m*/*z* 214 (MH+).

### Example 5: Ethyl 3-((5-fluoro-3-methylpyridin-2-yl)amino)-3-oxopropanoate (29)

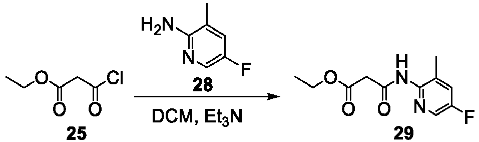

Compound **29** was synthesized using an analogous method to the synthesis of Compound **27** in Example 4, replacing Compound **26** with Compound **28.** ¹H NMR (CDCl₃) δ: 9.49 (s, 1H), 8.10 (d, 1H), 7.30 (dd, 1H), 4.22 (q, 2H), 3.53 (s, 2H), 2.28 (s, 3H), 1.28 (t, 3H). MS for C₁₁H₁₃FN₂O₃: *m*/*z* 241 (MH+).

### Example 6: Ethyl 3-((5-fluoropyridin-2-yl)amino)-3-oxopropanoate 5'-fluoro-6-methyl-2-oxo-2H-[1,2'-bipyridine]-3-carboxylic acid (31)

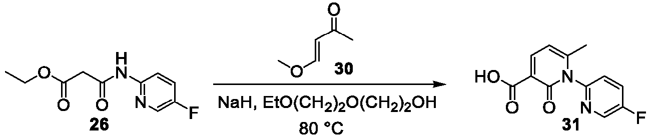

To a flask containing 2-(2-ethoxyethoxy)ethan-1-ol (6 mL) at room temperature was added NaH (60% in oil, 380 mg, 9.94 mmol). The mixture was stirred at room temperature for 10 min until all NaH was dissolved. To this mixture were added Compound **26** (1.41 g, 6.63 mmol) and Compound **30** (995 mg, 9.95 mmol). The resulting mixture was stirred at 80 °C for 1 h. After the mixture was cooled to room temperature, 1 N HCl was added until pH 3. The mixture was filtered and the solid was dried under vacuum to give Compound **31** (600 mg, 36%). ¹H NMR (400 MHz, DMSO-*d*₆) δ 13.87 (s, 1H), 8.72 (d, 1H), 8.43 (d, 1H), 8.10 (td, 1H), 7.80 (dd, 1H), 6.79 (d, 1H), 2.08 (s, 3H); MS for C₁₂H₉FN₂O₃: *m*/*z* 249 (MH+).

The following compounds were made in a manner similar to Compound **31** in **Example 6:**

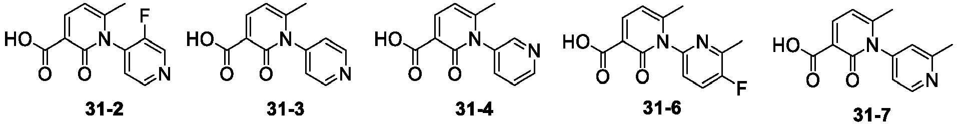

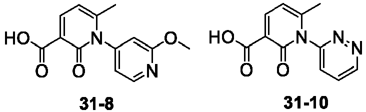

**3'-Fluoro-6-methyl-2-oxo-2H-[1,4'-bipyridine]-3-carboxylic acid (31-2):** Compound **27** was replaced with Compound **27-2.** ¹H NMR (400 MHz, CDCl₃) δ 13.34 (s, 1H), 8.82 (s, 1H), 8.72 (d, 1H), 8.55 (dd, 1H), 7.35 (t, 1H), 6.61 (d, 1H), 2.21 (s, 3H); MS for C₁₂H₉FN₂O₃: *m*/*z* 249.0 (MH+).

**6-Methyl-2-oxo-2H-[1,4'-bipyridine]-3-carboxylic acid (31-3):** Compound **27** was replaced with Compound **27-3.** ¹H NMR (400 MHz, DMSO-*d*₆) δ 13.92 (s, 1H), 8.85 (d, 2H), 8.43 (d, 1H), 7.59 (d, 2H), 6.82 (d, 1H), 2.11 (s, 3H); MS for C₁₂H₁₀N₂O₃: *m*/*z* 231.0 (MH+).

**6-Methyl-2-oxo-2H-[1,3'-bipyridine]-3-carboxylic acid (31-4):** Compound **27** was replaced with Compound **27-4.** ¹H NMR (400 MHz, DMSO-*d*₆) δ 14.00 (s, 1H), 8.75 (d, 1H), 8.74 - 8.63 (m, 1H), 8.43 (dd, 1H), 8.02 - 7.92 (m, 1H), 7.67 (dd, 1H), 6.83 (d, 1H), 2.11 (s, 3H); MS for C₁₂H₁₀N₂O₃: *m*/*z* 231.0 (MH+).

**5'-Fluoro-6,6'-dimethyl-2-oxo-2H-[1,2'-bipyridine]-3-carboxylic acid (31-6):** Compound 27 was replaced with Compound **27-6.** MS for C₁₃H₁₁FN₂O₃: *m*/*z* 263 (MH+).

**2',6-Dimethyl-2-oxo-2H-[1,4'-bipyridine]-3-carboxylic acid (31-7):** Compound **27** was replaced with Compound **27-7.** ¹H NMR (400 MHz, DMSO-*d₆*) δ 13.97 (br s, 1H), 8.69 (d, 1H), 8.42 (d, 1H), 7.42 (s, 1H), 7.37 (br d, 1H), 6.81 (d, 1H), 2.56 (s, 3H), 2.11 (s, 3H); MS for C₁₃H₁₂N₂O₃: *m*/*z* 245 (MH+).

**2'-Methoxy-6-methyl-2-oxo-2H-[1,4'-bipyridine]-3-carboxylic acid (31-8):** Compound **27** was replaced with Compound **27-8.** ¹H NMR: (400 MHz, DMSO-*d₆*) δ 8.41 (s, 1H), 8.39 (d, 1H), 7.13 (dd, 1H), 7.07 (d, 1H), 6.80 (d, 1H), 3.93 (s, 3H), 2.13 (s, 3H); MS for C₁₃H₁₂N₂O₄: *m*/*z* 260.9 (MH+).

**6-Methyl-2-oxo-1-(pyridazin-3-yl)-1,2-dihydropyridine-3-carboxylic acid (31-10):** Compound **27** was replaced with Compound **27-10.** ¹H NMR: (400 MHz, DMSO-*d₆*) δ 9.45-9.49 (m, 1H), 8.47 (d, 1H), 8.08-8.12 (m, 2H), 6.83 (d, 1H), 2.08 (s, 3H); MS for C₁₁H₉N₃O₃: *m*/*z* 231.9 (MH+).

### Example 6a: 5'-Ethoxy-6-methyl-2-oxo-2H-[1,2'-bipyridine]-3-carboxylic acid (31a)

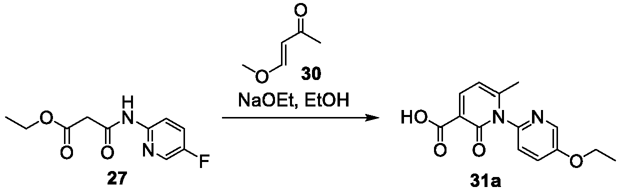

**5'-Ethoxy-6-methyl-2-oxo-2H-[1,2'-bipyridine]-3-carboxylic acid (31a):** To a mixture of Compound **27** (4.4 mmol, 1 eq) and Compound **30** (6.6 mmol, 1.5 eq) in EtOH (15 mL) was added NaOEt (210 mg, 3.0 mmol, 3 eq). The mixture was stirred at 80 °C for 10 h. The reaction was cooled to room temperature, concentrated, and extracted with DCM. The organic phase was washed with aq 1 N HCl and concentrated. To the resulting residue was added aq saturated NaHCO₃. The resulting mixture was stirred and filtered to give crude Compound **31a** (520 mg, 43% yield) which was used in the next step without further purification. MS for C₁₄H₁₄N₂O₄: *m*/*z* 275 (MH+).

### Example 6b: 5-Acetyl-1-(furan-3-yl)-6-methyl-2-oxo-1,2-dihydropyridine-3-carboxylic acid (6b-3)

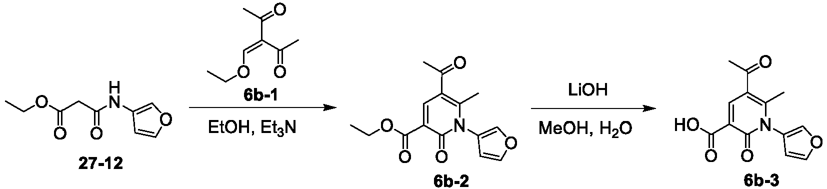

**Step 1: Ethyl 5-acetyl-1-(furan-3-yl)-6-methyl-2-oxo-1,2-dihydropyridine-3-carboxylate (6b-2):** A mixture of Compound **27-12** (590 mg, 3.0 mmol), Compound **6b-1** (0.47 mL, 3.0 mmol), triethylamine (1.30 mL, 9.0 mmol) and EtOH (10 mL) was stirred at room temperature. After 6 d, the resulting mixture was concentrated in vacuo, dissolved in EtOAc (10 mL), and washed with aq saturated NaHCO₃ (6 mL). The aqueous layer was extracted with EtOAc (2 x 5 mL) and the combined organic layers were dried over anhyd. Na₂SO₄ and concentrated under vacuum to give Compound **6b-2** which was used in the next step without further purification. MS for C₁₅H₁₅NO₅: *m*/*z* 290 (MH+).

**Step 2: 5-Acetyl-1-(furan-3-yl)-6-methyl-2-oxo-1,2-dihydropyridine-3-carboxylic acid (6b-3):** A mixture of crude Compound **6b-2** from the previous step (~ 3.0 mmol, 1 eq), LiOH monohydrate (190 mg, 4.5 mmol), MeOH (6 mL) and water (2 mL) was stirred at room temperature for 1 h. After concentrating the reaction mixture in vacuo, water (1 mL) was added and the resulting solution was washed with EtOAc (2 x 3 mL). The aqueous layer was acidified with 1N HCl and extracted with EtOAc (2 x 3 mL). The combined organic layers were concentrated in vacuo and purified by prep HPLC to give Compound **6b-3** as beige solid (22 mg). MS for C₁₃H₁₁NO₅: *m*/*z* 262 (MH+).

The following compounds were made using the same 2 step synthesis used to make Compound **6b-3 in Example 6b:**

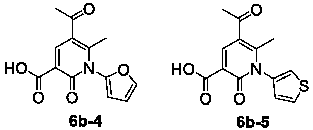

**5-Acetyl-1-(furan-2-yl)-6-methyl-2-oxo-1,2-dihydropyridine-3-carboxylic acid (6b-4):** Compound **27-12** was replaced with Compound **27-11.** MS for C₁₃H₁₁NO₅: *m*/*z* 262 (MH+).

**5-Acetyl-6-methyl-2-oxo-1-(thiophen-3-yl)-1,2-dihydropyridine-3-carboxylic acid (6b-5):** Compound **27-12** was replaced with Compound **27-13.** MS for C₁₃H₁₁NO₄S: *m*/*z* 278 (MH+).

### Example 7: 5'-Fluoro-3',6-dimethyl-2-oxo-2H-[1,2'-bipyridine]-3-carboxylic acid (32)

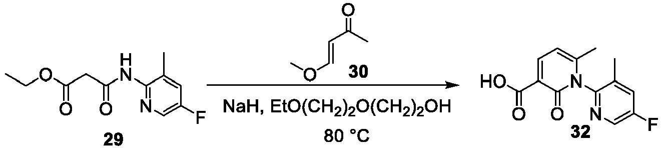

Compound **32** was synthesized using an analogous method to the synthesis of Compound **31** in Example 6, replacing Compound **26** with Compound **29.** ¹H NMR (DMSO-*d*₆) δ: 13.76 (s, 1H), 8.56 (d, 1H), 8.47 (d, 1H), 8.04 (dd, 1H), 6.83 (d, 1H), 2.11 (s, 3H), 2.05 (s, 3H). MS for C₁₃H₁₁FN₂O₃: *m*/*z* 263 (MH+).

### Example 8: 5'-Fluoro-4-methyl-2-oxo-2H-[1,2'-bipyridine]-3-carboxylic acid (35)

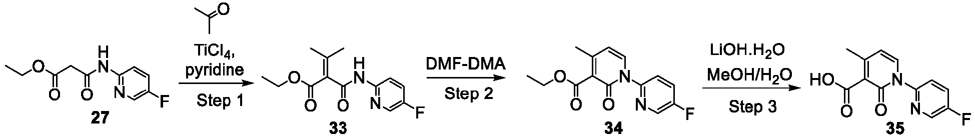

**Step 1: Ethyl 2-((5-fluoropyridin-2-yl)carbamoyl)-3-methylbut-2-enoate (33):** A solution of Compound 27 (500 mg, 2.21 mmol, 1 eq) in THF (6 mL) in a round-bottom flask was cooled to 0 °C in an ice bath. To the cooled solution was added TiCl₄ (461.19 mg, 2.43 mmol, 1.1 eq) by syringe (1M solution in THF, 2.4mL, 2.43 mmol) dropwise over 30 min. The resulting mixture was stirred with cooling for 30 min at 0 °C, after which acetone (0.33 mL, 4.42 mmol, 2 eq) was added. The reaction mixture was stirred for 30 min at 0 °C before dropwise addition of anhydrous pyridine (0.71 mL, 8.84 mmol, 4 eq). The reaction was allowed to warm gradually to 25 °C for 24 h, then poured into ice water and extracted with EtOAc (3 x 30 mL). The combined organic extracts were washed with aq saturated NaCl, dried over anhyd. MgSO₄ and concentrated to dryness. The crude residue was purified by silica gel chromatography (petroleum ether/EtOAc = 5/1) to give Compound **33** as a colorless oil (110 mg, 16.82% yield, 90% purity). ¹H NMR (400 MHz, CDCl₃) δ 8.51 (br s, 1H), 8.31 (br dd, 1H), 8.14 (d, 1H), 7.52-7.42 (m, 1H), 4.25 (q, 2H), 2.19 (s, 3H), 2.06 (s, 3H), 1.29 (t, 3H).

**Step 2: Ethyl 5'-fluoro-4-methyl-2-oxo-2H-[1,2'-bipyridine]-3-carboxylate (34):** A solution of Compound **33** (80 mg, 0.3 mmol, 1 eq) in DMF-DMA (3 mL) was heated at 80 °C for 12 h. The mixture was concentrated under vacuum to give crude Compound **34** as a yellow solid (80 mg) which was used without further purification.

**Step 3: 5'-Fluoro-4-methyl-2-oxo-2H-[1,2'-bipyridine]-3-carboxylic acid (35):** To a solution of Compound 34 (80 mg, 0.29 mmol, 1 eq) in MeOH (5 mL) was added LiOH monohydrate (61 mg, 1.45 mmol, 5 eq) in H₂O (1 mL). The mixture was stirred at 40 °C for 2 h, followed by stirring at 45 °C for 12 h. Water (20 mL) was added to the reaction mixture which was then washed with EtOAc (25 mL). The aqueous layer was acidified with 2N HCl to pH 5 and extracted with EtOAc (3 x 20 mL). The combined organic extracts were dried over anhyd. Na₂SO₄ and concentrated in vacuo to give crude Compound **35** as a yellow solid (40 mg). ¹H NMR (400 MHz, CDCl₃) δ 8.46 (d, 1H), 7.97 (d, 1H), 7.92 (dd, 1H), 7.70-7.60 (m, 1H), 6.55 (d, 1H), 2.85 (s, 3H).

### Example 9: 5-Bromo-5'-fluoro-6-methyl-2-oxo-2H-[1,2'-bipyridine]-3-carboxylic acid (36)

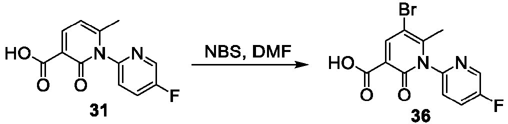

To a mixture of Compound 31 (660 mg, 2.66 mmol, 1 eq) in DMF (7 mL) was added NBS (568 mg, 3.19 mmol, 1.2 eq) under nitrogen. The mixture was stirred at 25 °C for 12 h. The reaction mixture was concentrated in vacuo, water (30 mL) was added and the mixture extracted with DCM (2 x 20 mL). The combined organic extracts were concentrated in vacuo to give crude Compound **36** as a yellow solid (940 mg,). ¹H NMR (400 MHz, CDCl₃) δ 13.44 (s, 1H), 8.71 (s, 1H), 8.57 (d, 1H), 7.74 (m, 1H), 7.41 (dd, 1H), 2.28 (s, 3H); MS for C₁₂H₈BrFN₂O₃: *m*/*z* 328.9 (MH+).

### Example 10: 5'-Fluoro-2-oxo-2H-[1,2'-bipyridine]-3-carboxylic acid (40)

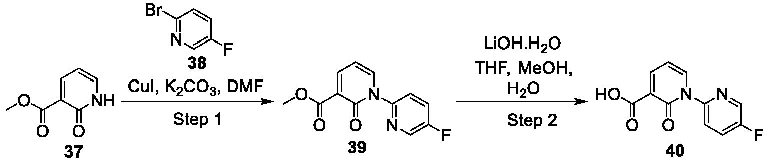

**Step 1: Methyl 5'-fluoro-2-oxo-2H-[1,2'-bipyridine]-3-carboxylate (39):** To a mixture of Compound **37** (1 g, 6.53 mmol, 1 eq), CuI (125 mg, 0.66 mmol, 0.1 eq) and K₂CO₃ (903 mg, 6.53 mmol, 1 eq) in DMF (10 mL) was added Compound **38** (2.30 g, 13.07 mmol, 2 eq) and the resulting mixture was stirred at 150 °C for 6 h under nitrogen. The resulting gray suspension of Compound **39** in DMF was used in the next step without isolation. MS for C₁₂H₉FN₂O₃: *m*/*z* 248.9 (MH+).

**Step 2: 5'-Fluoro-2-oxo-2H-[1,2'-bipyridine]-3-carboxylic acid (40):** To the suspension of Compound **39** in DMF from the previous step (6.53 mmol) was added THF (7.5 mL), MeOH (15 mL) and water (1.5 mL) followed by the addition of LiOH monohydrate (548 mg, 13.06 mmol, 2 eq). The mixture was stirred at 15 °C for 1 h. The reaction mixture was then concentrated in vacuo. The resulting residue was diluted with water (10 mL) and washed with EtOAc (2 x 10 mL). The aqueous phase was acidified with HCl solution (2M) to pH ~5. The resulting solid was filtered and dried under reduced pressure to give Compound **40** as a yellow solid (620 mg, 41% yield) which was used in subsequent steps without further purification. ¹H NMR (400 MHz, DMSO-*d₆*) δ 13.92 (br s, 1H), 8.69 (br s, 1H), 8.50 (br d, 1H), 8.32 (br d, 1H), 8.06 (br s, 1H), 7.94 (br s, 1H), 6.82 (br s, 1H); MS for C₁₁H₇FN₂O₃: *m*/*z* 234.9 (MH+).

### Example 11: N-[4-[(6,7-Dimethoxy-1,5-naphthyridin-4-yl)oxy]-3-fluorophenyl]-1-(5-fluoropyridin-2-yl)-4-methyl-2-oxopyridine-3-carboxamide (41)

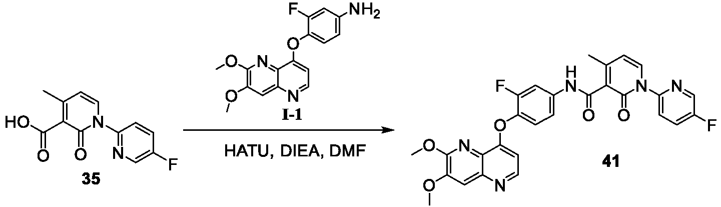

Using a method similar to **General Procedure D,** to a solution of Compound **35** (40 mg, 0.16 mmol, 1 eq) in DMF (3 mL) was added Intermediate **I-1** (45.73 mg, 0.145mmol, 0.9 eq), HATU (73.53 mg, 0.19 mmol, 1.2 eq) and DIEA (0.084 mL, 0.48 mmol, 3 eq). The mixture was heated at 40 °C for 3 h. Water (20 mL) was added and the resulting mixture was extracted with DCM (3 x 25 mL). The combined organic extracts were dried over anhyd. Na₂SO₄ and concentrated in vacuo. The resulting crude product was purified by silica gel chromatography (DCM/MeOH = 10/1) to give Compound **41** as a yellow solid (35.7 mg, 38.58% yield). ¹H NMR (400 MHz, CDCl₃) δ 11.84 (s, 1H), 8.52 (d, 1H), 8.47 (d, 1H), 7.92 (d, 1H), 7.86 (dd, 1H), 7.83 (d, 1H), 7.66 (dd, 1H), 7.52 (s, 1H), 7.33 (br d, 1H), 7.18 (t, 1H), 6.74 (d, 1H), 6.45 (d, 1H), 4.13 (s, 3H), 4.03 (s, 3H), 2.85 (s, 3H); MS for C₂₈H₂₁F₂N₅O₅: *m*/*z* 568 [M+Na]⁺.

The following compounds were made following methods analogous to that used to synthesize Compound **41** in Example 11 from Compound **35** and Intermediate **I-1**. The temperatures of the reactions can vary from room temperature to 40 °C. Reaction times can vary from 2-4 h. EtOAc can replace DCM as the extraction solvent.

**N-[4-[(6,7-Dimethoxy-1,5-naphthyridin-4-yl)oxy]-3-fluorophenyl]-1-(5-fluoropyridin-2-yl)-2-oxopyridine-3-carboxamide (42):** Compound **35** was replaced with Compound **40.** ¹H NMR (400 MHz, DMSO-*d₆*) δ 11.91 (br s, 1H), 8.69 (br s, 1H), 8.57 (br d, 2H), 8.24 (br s, 1H), 8.10 - 7.92 (m, 3H), 7.65 (br s, 1H), 7.46 (br d, 1H), 7.34 (br d, 1H), 6.88 - 6.74 (m, 2H), 3.95 (br d, 6H); MS for C₂₇H₁₉F₂N₅O₅: *m*/*z* 532.1 (MH+).

**N-[4-[(6,7-Dimethoxy-1,5-naphthyridin-4-yl)oxy]-3-fluorophenyl]-1-(5-fluoropyridin-2-yl)-6-methyl-2-oxopyridine-3-carboxamide (43):** Compound **35** was replaced with Compound **31.** ¹H NMR (400 MHz, DMSO-*d*₆) δ 11.86 (s, 1H), 8.73 (d, 1H), 8.54 (t, 2H), 8.11 (td, 1H), 8.00 (dd, 1H), 7.80 (dd, 1H), 7.65 (s, 1H), 7.45 (d, 1H), 7.31 (t, 1H), 6.83 (d, 1H), 6.73 (d, 1H), 3.96 (s, 3H), 3.93 (s, 3H), 2.07 (d, 3H); MS for C₂₈H₂₁F₂N₅O₅: *m*/*z* 546.2 (MH+).

**N-[4-[(6,7-Dimethoxy-1,5-naphthyridin-4-yl)oxy]phenyl]-1-(5-fluoropyridin-2-yl)-6-methyl-2-oxopyridine-3-carboxamide (44):** Intermediate **I-1** was replaced with Intermediate **1-2,** and Compound **35** was replaced with Compound **31.** ¹H NMR (400 MHz, DMSO-*d*₆) δ 11.68 (s, 1H), 8.66 (d, 1H), 8.46 (dd, 2H), 8.04 (td, 1H), 7.76 - 7.67 (m, 3H), 7.57 (s, 1H), 7.15 - 7.06 (m, 2H), 6.74 (d, 1H), 6.65 (d, 1H), 3.89 (s, 3H), 3.85 (s, 3H), 1.99 (s, 3H); MS for C₂₈H₂₂FN₅O₅: *m*/*z* 528.2 (MH+).

**N-[3-Fluoro-4-[(7-methoxy-1,5-naphthyridin-4-yl)oxy]phenyl]-1-(5-fluoropyridin-2-yl)-6-methyl-2-oxopyridine-3-carboxamide (45):** Intermediate **I-1** was replaced with Intermediate **1-3,** and Compound **35** was replaced with Compound **31.** ¹H NMR (400 MHz, DMSO-*d*₆) δ 11.90 (s, 1H), 8.77 - 8.73 (m, 2H), 8.71 (d, 1H), 8.54 (d, 1H), 8.11 (td, 1H), 8.05 (dd, 1H), 7.84 - 7.75 (m, 2H), 7.55 - 7.47 (m, 1H), 7.40 (t, 1H), 6.78 - 6.70 (m, 2H), 4.00 (s, 3H), 2.07 (s, 3H); MS for C₂₇H₁₉F₂N₅O₄: *m*/*z* 516.2 (MH+).

**1-(5-Fluoropyridin-2-yl)-N-[4-[(7-methoxy-1,5-naphthyridin-4-yl)oxy]phenyl]-6-methyl-2-oxopyridine-3-carboxamide (46):** Intermediate **I-1** was replaced with Intermediate 1-4, and Compound **35** was replaced with Compound **31.** ¹H NMR (400 MHz, DMSO-*d*₆) δ 11.78 (s, 1H), 8.76 - 8.71 (m, 2H), 8.70 (d, 1H), 8.54 (d, 1H), 8.11 (td, 1H), 7.86 - 7.76 (m, 4H), 7.28 - 7.20 (m, 2H), 6.77 - 6.69 (m, 2H), 4.00 (s, 3H), 2.07 (s, 3H); MS for C₂₇H₂₀FN₅O₄: *m*/*z* 498.2 (MH+).

**N-[3-Fluoro-4-[(6-methoxy-1,5-naphthyridin-4-yl)oxy]phenyl]-1-(5-fluoropyridin-2-yl)-6-methyl-2-oxopyridine-3-carboxamide (47):** Intermediate **I-1** was replaced with Intermediate **1-5,** and Compound **35** was replaced with Compound **31.** ¹H NMR (400 MHz, DMSO-*d*₆) δ 11.87 (s, 1H), 8.73 (d, 1H), 8.62 (d, 1H), 8.53 (d, 1H), 8.29 (d, 1H), 8.11 (td, 1H), 8.02 (dd, 1H), 7.80 (dd, 1H), 7.51 - 7.43 (m, 1H), 7.33 (dd, 2H), 6.97 (d, 1H), 6.76 - 6.70 (m, 1H), 3.92 (s, 3H), 2.07 (s, 3H); MS for C₂₇H₁₉F₂N₅O₄: *m*/*z* 516.2 (MH+).

**N-[3-Fluoro-4-(1,5-naphthyridin-4-yloxy)phenyl]-1-(5-fluoropyridin-2-yl)-6-methyl-2-oxopyridine-3-carboxamide (48):** Intermediate **I-1** was replaced with Intermediate **1-6,** and Compound **35** was replaced with Compound **31.** ¹H NMR (400 MHz, DMSO-*d*₆) δ 11.91 (s, 1H), 9.02 (dd, 1H), 8.79 (d, 1H), 8.74 (d, 1H), 8.55 (d, 1H), 8.45 (dd, 1H), 8.12 (td, 1H), 8.06 (dd, 1H), 7.87 (dd, 1H), 7.81 (dd, 1H), 7.56 - 7.49 (m, 1H), 7.43 (t, 1H), 6.91 (dd, 1H), 6.74 (d, 1H), 2.07 (s, 3H); MS for C₂₆H₁₇F₂N₅O₃: *m*/*z* 486.2 (MH+).

**N-[3-Fluoro-4-(6-methoxyquinolin-4-yl)oxyphenyl]-1-(5-fluoropyridin-2-yl)-6-methyl-2-oxopyridine-3-carboxamide (49):** Intermediate **I-1** was replaced with Intermediate **I-7,** and Compound **35** was replaced with Compound **31.** ¹H NMR (400 MHz, DMSO-*d*₆) δ 11.92 (s, 1H), 8.74 (d, 1H), 8.54 (d, 2H), 8.17 - 8.02 (m, 2H), 7.96 (d, 1H), 7.81 (dd, 1H), 7.59 (d, 1H), 7.54 (dd, 1H), 7.51 - 7.41 (m, 2H), 6.74 (d, 1H), 6.61 (d, 1H), 3.94 (s, 3H), 2.08 (s, 3H).); MS for C₂₈H₂₀F₂N₄O₄: *m*/*z* 515.2 (MH+).

**N-[3-Fluoro-4-[[6-methoxy-7-(2-methoxyethoxy)-1,5-naphthyridin-4-yl]oxy]phenyl]-1-(5-fluoropyridin-2-yl)-6-methyl-2-oxopyridine-3-carboxamide (50):** Intermediate **I-1** was replaced with Compound **17,** and Compound **35** was replaced with Compound **31.** ¹H NMR (400 MHz, DMSO-d6) δ 11.80 (s, 1H), 8.66 (d, 1H), 8.49 - 8.39 (m, 2H), 8.04 (td, 1H), 7.94 (dd, 1H), 7.73 (dd, 1H), 7.60 (s, 1H), 7.38 (d, 1H), 7.24 (t, 1H), 6.75 (d, 1H), 6.66 (d,1H), 4.24 (t, 2H), 3.87 (s, 3H), 3.68 (t, 2H), 3.27 (s, 3H), 2.00 (s, 3H); MS for C₃₀H₂₅F₂N₅O₆: *m*/*z* 590.2 (MH+).

**N-[4-[(6,7-Dimethoxy-1,5-naphthyridin-4-yl)oxy]-3-fluorophenyl]-1-(5-fluoro-3-methylpyridin-2-yl)-6-methyl-2-oxopyridine-3-carboxamide (51):** Compound **35** was replaced with Compound **32.** ¹H NMR (CDCl₃) δ: 11.81 (s, 1H), 8.68 (d, 1H), 8.52 (d, 1H), 8.42 (d, 1H), 7.94 (dd, 1H), 7.57 (dd, 1H), 7.53 (s, 1H), 7.33 (ddd, 1H), 7.16 (t, 1H), 6.75 (dd, 1H), 6.54 (d, 1H), 4.11 (s, 3H), 4.03 (s, 3H), 2.22 (s, 3H), 2.07 (s, 3H). MS for C₂₉H₂₃F₂N₅O₅: *m*/*z* 560 (MH+).

**N-[3-Fluoro-4-[(7-methoxy-1,5-naphthyridin-4-yl)oxy]phenyl]-1-(5-fluoro-3-methylpyridin-2-yl)-6-methyl-2-oxopyridine-3-carboxamide (52):** Compound 35 was replaced with Compound **32** and Intermediate **I-1** was replaced with Intermediate **I-3.** ¹H NMR (CDCl₃ δ: 11.86 (s, 1H), 8.78 (d, 1H), 8.68 (d, 1H), 8.66 (d, 1H), 8.42 (d, 1H), 7.97 (dd, 1H), 7.67 (d, 1H), 7.57 (dd, 1H), 7.38 (ddd, 1H), 7.25 (t, 1H), 6.63 (dd, 1H), 6.54 (d, 1H), 4.01 (s, 3H), 2.21 (s, 3H), 2.07 (s, 3H). MS for C₂₈H₂₁F₂N₅O₄: *m*/*z* 530 [MH]⁺.

**N-[3-Fluoro-4-[(6-methoxy-1,5-naphthyridin-4-yl)oxy]phenyl]-1-(5-fluoro-3-methylpyridin-2-yl)-6-methyl-2-oxopyridine-3-carboxamide (53):** Compound **35** was replaced with Compound 32 and Intermediate **I-1** was replaced with Intermediate **I-5.** ¹H NMR (Chloroform-*d*) δ: 11.82 (s, 1H), 8.67 (d, 1H), 8.57 (d, 1H), 8.42 (d, 1H), 8.20 (d, 1H), 7.94 (dd, 1H), 7.56 (dd, 1H), 7.34 (ddd, 1H), 7.21 - 7.11 (m, 2H), 6.84 (dd, 1H), 6.54 (d, 1H), 4.02 (s, 3H), 2.21 (s, 3H), 2.06 (s, 3H). MS for C₂₈H₂₁F₂N₅O₄: *m*/*z* 530 [MH]⁺.

**N-[3-Fluoro-4-[(6-methoxy-1,7-naphthyridin-4-yl)oxy]phenyl]-1-(5-fluoropyridin-2-yl)-6-methyl-2-oxopyridine-3-carboxamide (54):** Compound **35** was replaced with Compound **31** and Intermediate **I-1** was replaced with Compound **24.** ¹H NMR (400 MHz, DMSO-*d₆*) δ 11.86 (s, 1H), 9.11 (s, 1H), 8.67 (d, 1H), 8.58 (d, 1H), 8.47 (d, 1H), 8.14 - 7.91 (m, 2H), 7.74 (dd, 1H), 7.58 - 7.30 (m, 3H), 6.68 (t, 2H), 3.95 (s, 3H), 2.01 (s, 3H); MS for C₂₇H₁₉F₂N₅O₄: *m*/*z* 516.0 (MH+).

**N-[4-[(6-Chloro-1,7-naphthyridin-4-yl)oxy]-3-fluorophenyl]-1-(5-fluoropyridin-2-yl)-6-methyl-2-oxopyridine-3-carboxamide (54A):** Compound **35** was replaced with Compound **31** and Intermediate **I-1** was replaced with Compound **24A.** ¹H NMR (400 MHz, DMSO-*d₆*) δ 11.87 (s, 1H), 9.25 (s, 1H), 8.80 (d, 1H), 8.67 (d, 1H), 8.48 (d, 1H), 8.22 (s, 1H), 8.03 (ddd, 2H), 7.74 (dd, 1H), 7.59 - 7.32 (m, 2H), 6.87 (d, 1H), 6.67 (d, 1H), 2.01 (s, 3H); MS for C₂₆H₁₆ClF₂N₅O₃: *m*/*z* 520 (MH+).

### Example 12: 5-Bromo-N-[4-[(6,7-dimethoxy-1,5-naphthyridin-4-yl)oxy]-3-fluorophenyl]-1-(5-fluoropyridin-2-yl)-6-methyl-2-oxopyridine-3-carboxamide (55):

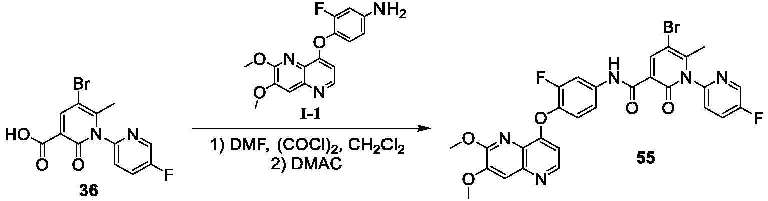

To a solution of Compound **36** (250 mg, 0.76 mmol, 1 eq) in DCM (5 mL) was added (COCl)₂ (1.47 mL, 17 mmol, 22 eq) and DMF (0.006 mL, 0.076 mmol, 0.1 eq). The reaction mixture was stirred at 20 °C for 0.5 h. The mixture was concentrated in vacuo to give the acid chloride of Compound 36 as a brown solid (260 mg, 98.45% yield). To a solution of this acid chloride (210 mg, 0.61 mmol, 1.2 eq) in DMAC (2 mL) was added Intermediate **I-1** (160 mg, 0.51 mmol, 1 eq). The mixture was stirred at 25 °C for 3 h. The reaction mixture was poured into water (20 mL) and extracted with DCM (3 x 20 mL). The combined organic extracts were washed with aq saturated NaCl (10 mL) and concentrated in vacuo. The resulting residue was purified by flash silica gel chromatography (ISCO^{®}; 4 g SepaFlash^{®} Silica Flash Column, Eluent of 0~50% EtOAc/Petroleum ether gradient @ 30 mL/min) followed by further purification by prep-TLC (dichloromethane: methanol = 20:1, R_{f} = 0.4) to give Compound **55** as a white solid (28.0 mg, 8.8% yield). ¹H NMR (400 MHz, DMSO-*d₆*) δ 11.66 (s, 1H), 8.75 (br s, 1H), 8.58 (s, 1H), 8.54 (d, 1H), 8.14 (m, 1H), 7.99 (br d, 1H), 7.85 - 7.76 (m, 1H), 7.65 (s, 1H), 7.48 (br d, 1H), 7.31 (t, 1H), 6.83 (d, 1H), 3.96 (s, 3H), 3.91 (s, 3H), 2.17 (s, 3H); MS for C₂₈H₂₀BrF₂N₅O₅: *m*/*z* 624.0 (MH+).

### Example 13: 5-Cyano-N-[4-[(6,7-dimethoxy-1,5-naphthyridin-4-yl)oxy]-3-fluorophenyl]-1-(5-fluoropyridin-2-yl)-6-methyl-2-oxopyridine-3-carboxamide (56):

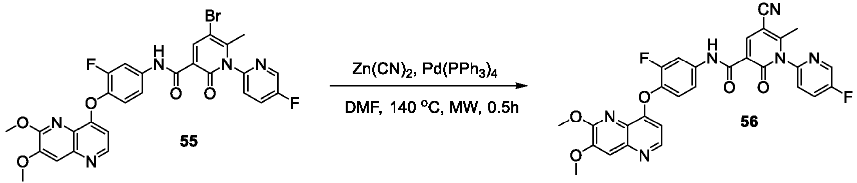

A mixture of Compound **55** (150 mg, 0.24 mmol, 1 eq), Zn(CN)₂ (90 mg, 0.77 mmol, 0.049 mL, 3.2 eq) and Pd(PPh₃)₄ (28 mg, 0.024 mmol, 0.1 eq) in DMF (4 mL) under nitrogen was stirred at 140 °C under microwave irradiation for 0.5 h. The reaction mixture was cooled to room temperature, followed by the addition of saturated FeSO₄ solution. The resulting mixture was extracted with EtOAc (3 x 50 mL). The combined organic extracts were washed with water, aq saturated NaCl, dried over anhyd. Na₂SO₄ and concentrated. The resulting residue was purified by column chromatography (SiO₂, DCM:MeOH = 20:1, R_{f} = 0.4) followed by further purification by prep-HPLC (column: Venusil ASB Phenyl 150 * 30 mm * 5um; mobile phase: [water (0.05% HCl)-ACN]; B%: 35% - 65%, 9 min) to give Compound **56** as a yellow solid (17.5 mg, 17.5% yield). ¹H NMR (400 MHz, DMSO-*d₆*) δ 11.31 (br s, 1H), 8.77 (m, 2H), 8.67 (br s, 1H), 8.17 (br s, 1H), 8.05 (br d, 1H), 7.88 - 7.72 (m, 2H), 7.57 (m, 1H), 7.46 (m, 1H), 7.09 (m, 1H), 4.02 (br d, 6H), 2.28 (br s, 3H); MS for C₂₀H₂₀F₂N₆O₅: *m*/*z* 571.2 (MH+).

### Example 14: 5-Acetyl-N-[4-[(6,7-dimethoxy-1,5-naphthyridin-4-yl)oxy]-3-fluorophenyl]-1-(5-fluoropyridin-2-yl)-6-methyl-2-oxopyridine-3-carboxamide (59):

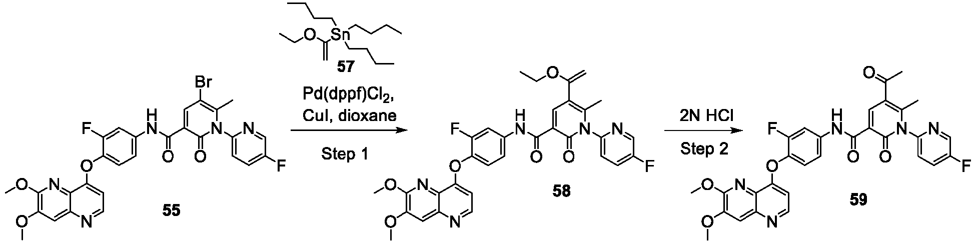

**Step 1: N-(4-((6,7-Dimethoxy-1,5-naphthyridin-4-yl)oxy)-3-fluorophenyl)-5-(1-ethoxyvinyl)-5'-fluoro-6-methyl-2-oxo-2H-[1,2'-bipyridine]-3-carboxamide (58):** To a solution of Compound **55** (220 mg, 0.35 mmol, 1 eq) in dioxane (4 mL) was added Pd(dppf)Cl₂ (26 mg, 0.035 mmol, 0.1 eq), CuI (13 mg, 0.070 mmol, 0.2 eq) and Compound **57** (445 mg, 1.23 mmol, 0.42 mL, 3.5 eq). The reaction mixture was allowed to stir at 100 °C for 12 h under nitrogen. Aq saturated KF (20mL) was added and the mixture was stirred at 25 °C for 1 h. Ammonium hydroxide (1 mL) was added and the resulting mixture was extracted with EtOAc (3 x 30 mL). The combined organic extracts were washed with aq saturated NaCl (10 mL), dried over anhyd. Na₂SO₄ and concentrated under reduced pressure. The resulting residue was purified by flash silica gel chromatography (ISCO^{®}; 12 g SepaFlash^{®} Silica Flash Column, Eluent of 0~5% MeOH/DCM gradient @ 30 mL/min) to give Compound **58** as a yellow solid (200 mg, 49.42% yield). MS for C₃₂H₂₇F₂N₅O₆: *m*/*z* 616.2 (MH+).

**Step 2: 5-Acetyl-N-[4-[(6,7-dimethoxy-1,5-naphthyridin-4-yl)oxy]-3-fluorophenyl]-1-(5-fluoropyridin-2-yl)-6-methyl-2-oxopyridine-3-carboxamide (59):** Compound **58** (100 mg, 0.16 mmol, 1 eq) in HCl (2 M, 0.081 mL, 1 eq) was stirred at 25 °C for 2 h. The reaction mixture was extracted with DCM (3 x 20 mL). The combined organic extracts were dried over anhyd. Na₂SO₄ and concentrated in vacuo. The resulting residue was purified by prep-TLC (SiO₂, DCM:MeOH = 20:1, Rf = 0.2) followed by further purification by prep-HPLC (column: Boston Green ODS 150 * 30 mm * 5um; mobile phase: [water (0.075% TFA) - ACN]; B%: 25% - 55%, 8 min) to give Compound 59 as a white solid (38.3 mg, 41% yield). ¹H NMR (400 MHz, CDCl₃) δ 11.46 (s, 1H), 9.13 (s, 1H), 8.61 (d, 1H), 8.53 (d, 1H), 7.91 (m, 1H), 7.76 (m, 1H), 7.52 (s, 1H), 7.39 (dd, 1H), 7.31 (m, 1H), 7.16 (br t, 1H), 6.76 (d, 1H), 4.10 (s, 3H), 4.03 (s, 3H), 2.68 (s, 3H), 2.43 (s, 3H); MS for C₃₀H₂₃F₂N₅O₆: *m*/*z* 588.1 (MH+).

### Example 15A: 5-(4-Fluorophenyl)-2-methyl-4-oxo-1,4-dihydropyridine-3-carboxylic acid (15A-3)

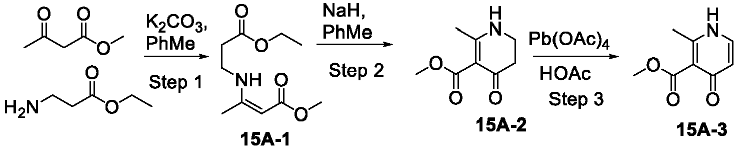

**Step 1: Methyl 3-((3-ethoxy-3-oxopropyl)amino)but-2-enoate (15A-1):** A mixture of 3-aminopropionic acid ethyl ester hydrochloride (15.6 g, 101 mmol), methyl 3-oxobutanoate (10.8mL, 101 mmol) and anhyd. K₂CO₃ (28.0 g, 203 mmol) in toluene (200 mL) was refluxed with a Dean-Stark trap overnight. The reaction mixture was cooled and diluted with EtOAc (150 mL), filtered and the filtrate was concentrated under reduced pressure to give crude Compound 15A-1 (21 g), which was used in the next step without further purification.

**Step 2: Methyl 2-methyl-4-oxo-1,4,5,6-tetrahydropyridine-3-carboxylate (15A-2):** To a solution of crude Compound **15A-1** (21 g, 95 mmol) in 200 mL of toluene was added sodium hydride (6.0 g, 60 % dispersion in oil, 150 mmol) and the resulting yellow suspension was refluxed overnight. The mixture was concentrated to remove solvent and the residue was treated carefully with water (100 mL), acidified to pH 2 with 6 M HCl and then washed with Et₂O (3x). The aqueous phase was basified with NaHCO₃ and extracted with EtOAc (5x). The combined EtOAc extracts were dried over anhyd. Na₂SO₄ and concentrated to give Compound **15A-2** (7.6 g, 46% yield) which was used without further purification. MS for C₈H₁₁NO₃: *m*/*z* 170 (MH+).

**Step 3: Methyl 2-methyl-4-oxo-1,4-dihydropyridine-3-carboxylate (15A-3):** A mixture of Compound **15A-2** (3.6 g, 21 mmol) and lead tetraacetate (20 g, 58 mmol) in 30 mL of acetic acid was stirred at 100 °C overnight and concentrated under reduced pressure to remove acetic acid. The resulting residue was purified by silica gel chromatography ( 5 - 15% MeOH in DCM) to give Compound **15A-3** as a red oil (2.0 g, 56%). MS for C₈H₉NO₃: *m*/*z* 168 (MH+).

### Example 15: Ethyl 5-bromo-4-hydroxy-2-methylnicotinate (61)

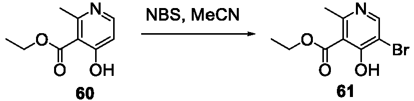

Compound **60** can be made using the same method used to make Compound **15A-3** in **Example 15A.** To a solution of Compound **60** (13.7 g, 80.7 mmol) in acetonitrile (250 mL) was added NBS (14.4 g, 80.7 mmol) in portions over a period of 10 min at room temperature. The reaction was allowed to proceed for 1 hour, and the product precipitated from the reaction mixture. The precipitate was collected by vacuum filtration to yield Compound **61** as a white solid (10.9 g, 52% yield). ¹H NMR (400 MHz, CDCl₃) δ 8.14 (s, 1H), 4.37 (q, 2H), 2.52 (s, 3H), 1.38 (t, 3H); MS for C₉H₁₀BrNO₃: *m*/*z* 260.0 (MH+).

The following compounds were made in a manner similar to Compound **61** using NBS as a brominating agent in an appropriate solvent. If the desired product did not precipitate from the reaction mixture, water was added to aid the precipitation. If no filterable solid resulted, the reaction mixture was diluted with DCM, washed with water, washed with aq saturated NaCl, dried over anhyd Na₂SO₄ and concentrated to give the desired product:

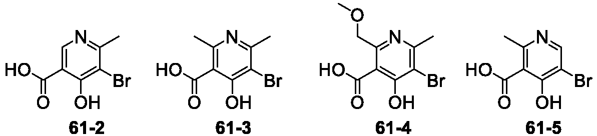

**5-Bromo-4-hydroxy-6-methylnicotinic acid (61-2):** Compound **60** was replaced with 4-hydroxy-6-methylnicotinic acid. MS for C₇H₆BrNO₃: *m*/*z* 232/234 (MH+).

**5-Bromo-4-hydroxy-2,6-dimethylnicotinic acid (61-3):** Compound **60** was replaced with 4-hydroxy-2,6-dimethylnicotinic acid. MS for C₈H₈BrNO₃: *m*/*z* 246 (MH+).

**5-Bromo-4-hydroxy-2-(methoxymethyl)-6-methylnicotinic acid (61-4):** MS for C₉H₁₀BrNO₄: *m*/*z* 276 (MH+). See **Example 26** for the synthesis of 4-hydroxy-2-(methoxymethyl)-6-methylnicotinic acid (Compound **132**).

**5-Bromo-4-hydroxy-2-methylnicotinic acid (61-5):** MS for C₇H₆BrNO₃: *m*/*z 232.0* (MH+). In addition to the direct bromination of 4-hydroxy-2-methylnicotinic acid, Compound **61-5** can also be made through the ester hydrolysis of Compound **61** using standard LiOH hydrate ester hydrolysis conditions in MeOH and water with heating at 65 °C.

### Example 16: 5-Fluoro-4'-hydroxy-6'-methyl-[2,3'-bipyridine]-5'-carboxylic acid (63)

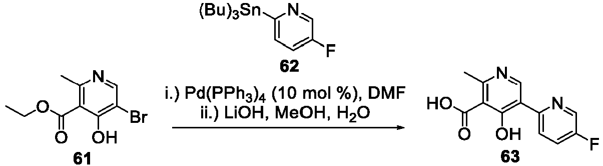

Compound **62** is commercially available or can be made by the method shown in Step 1 of **Example 21.** To a solution of Compound **61** (100 mg, 0.76 mmol) in DMF (5 mL) was added Compound **62** (356 mg, 0.92 mmol) and Pd(PPh₃)₄ (22 mg, 0.09 mmol). The solution was brought to 90 °C and the reaction was allowed to proceed overnight. The solution was then filtered through Celite and the filter cake was washed with DCM (25 mL). The resulting filtrate was then transferred to a separatory funnel and partitioned with aq 10 % NaOH solution (25 mL). The phases were separated and the organic phase was concentrated in vacuo. The resulting residue was taken up in MeOH (5 mL) and water (1 mL) and LiOH monohydrate (200 mg, 4.87 mmol) was added in a single portion. The resulting solution was heated to 60 °C for 16 h. The reaction was cooled to room temperature and was acidified to pH 4 with aq 6 M HCl. The resulting mixture was concentrated to near dryness, and the precipitate was collected by vacuum filtration to yield Compound **63** as a white solid (40 mg, 21% yield). ¹H NMR (400 MHz, CD₃OD) δ 8.86 (s, 1H), 8.75 (dd, 1H), 8.56 (dd, 1H), 8.12 (td, 1H), 2.86 (s, 3H); MS for C₁₂H₉FN₂O₃: *m*/*z* 249.0 (MH+).

### Example 17: 4'-Hydroxy-6'-methyl-[2,3'-bipyridine]-5'-carboxylic acid (65)

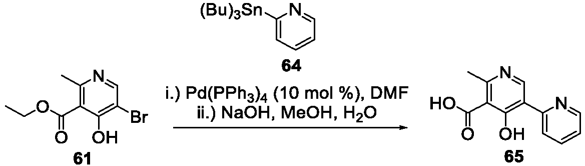

Compound **65** was synthesized using an analogous method to the synthesis of Compound **63** in Example 16, replacing Compound **62** with Compound **64.** ¹H NMR (400 MHz, DMSO-*d₆*) δ 13.28 (s, 1H), 8.67 (d, 1H), 8.61 (s, 1H), 8.40 (d, 1H), 7.88 (td, 1H), 7.39 (dd, 1H), 2.79 (s, 3H); MS for C₁₂H₁₀N₂O₃: *m*/*z* 231.0 (MH+).

### Example 18: Ethyl 5-bromo-1,2-dimethyl-4-oxo-1,4-dihydropyridine-3-carboxylate (66)

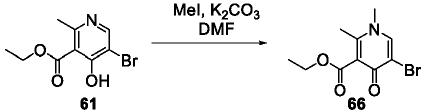

To a solution of Compound 61 (1.0 g, 3.84 mmol) and potassium carbonate (636 mg, 4.61 mmol) in DMF (15 mL) at 0 °C was added MeI (0.26 mL, 4.22 mmol) dropwise over a period of 5 min. The reaction was allowed to warm to room temperature over 1 h. The solution was then partitioned between water (40 mL) and 10% MeOH in DCM (40 mL). The organics were collected, dried over anhyd. Na₂SO₄, and concentrated under reduced pressure to yield a yellow oil which was triturated with EtOAc to yield Compound 66 as a yellow solid (260 mg, 24% yield). ¹H NMR (400 MHz, CDCl₃) δ 7.71 (s, 1H), 4.48 - 4.20 (m, 2H), 3.58 (d, 3H), 2.44 - 2.19 (m, 3H), 1.48 - 1.23 (m, 3H); MS for C₁₀H₁₂BrNO₃: *m*/*z* 274.0 (MH+).

### Example 19: 5-Fluoro-1',6'-dimethyl-4'-oxo-1',4'-dihydro-[2,3'-bipyridine]-5'-carboxylic acid (67)

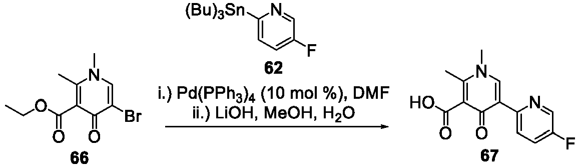

Compound 67 was synthesized using an analogous method to the synthesis of Compound 63 in Example 16, replacing Compound 61 with Compound 66. ¹H NMR (400 MHz, CDCl₃) δ 8.65 (dd, 1H), 8.61 (s, 1H), 8.48 (d, 1H), 7.55 - 7.46 (m, 1H), 3.94 (s, 3H), 3.08 (s, 3H); MS for C₁₃H₁₁FN₂O₃: *m*/*z* 263.0 (MH+).

### Example 20: N-[4-[(6,7-Dimethoxy-1,5-naphthyridin-4-yl)oxy]-3-fluorophenyl]-5-(5-fluoropyridin-2-yl)-4-hydroxy-2-methylpyridine-3-carboxamide (68):

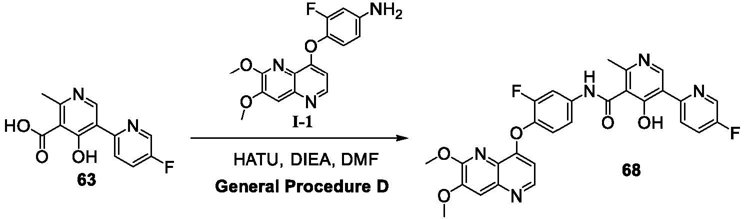

Compound 68 and the following compounds were made by General Procedure D, similar to the synthesis of Compound 41 from Compound 35 and Intermediate **I-1** in Example 11. The temperatures of the reactions can vary from room temperature to 40 °C. Reaction times can vary from 2-4 h. EtOAc can replace DCM as the extraction solvent.

N-[4-[(6,7-Dimethoxy-1,5-naphthyridin-4-yl)oxy]-3-fluorophenyl]-5-(5-fluoropyridin-2-yl)-4-hydroxy-2-methylpyridine-3-carboxamide (68): Compound 35 in Example 11 was replaced with Compound 63. ¹H NMR (400 MHz, DMSO-*d₆*) δ 12.34 (s, 2H), 8.63 - 8.51 (m, 2H), 8.47 (d, 1H), 8.36 (s, 1H), 7.94 (dd, 1H), 7.71 (td, 1H), 7.59 (s, 1H), 7.42 - 7.33 (m, 1H), 7.27 (t, 1H), 6.74 (d, 1H), 3.91 (s, 6H), 2.53 (s, 3H); MS for C₂₈H₂₁F₂N₅O₅: *m*/*z* 546.2 (MH+).

**N-[4-[(6,7-Dimethoxy-1,5-naphthyridin-4-yl)oxy]-3-fluorophenyl]-4-hydroxy-2-methyl-5-pyridin-2-ylpyridine-3-carboxamide (69): Compound 35 in Example 11** was replaced with Compound **65.** ¹H NMR (400 MHz, DMSO-*d₆*) δ 12.77 (s, 1H), 8.58 - 8.38 (m, 4H), 7.95 (dd, 1H), 7.75 (t, 1H), 7.59 (s, 1H), 7.37 (d, 1H), 7.32 - 7.19 (m, 2H), 6.73 (d, 1H), 3.91 (s, 6H), 2.55 (s, 3H); MS for C₂₈H₂₂FN₅O₅: *m*/*z* 528.2 (MH+).

**N-[3-Fluoro-4-[(7-methoxy-1,5-naphthyridin-4-yl)oxy]phenyl]-4-hydroxy-2-methyl-5-pyridin-2-ylpyridine-3-carboxamide (70):** Compound **35** in **Example 11** was replaced with Compound **65** and Intermediate **I-1** was replaced with Intermediate **1-3.** ¹H NMR (400 MHz, DMSO-*d₆*) *δ* 12.39 (s, 2H), 8.76 (d, 1H), 8.71 (d, 1H), 8.63 (d, 1H), 8.56 (d, 1H), 8.48 (s, 1H), 8.04 (dd, 1H), 7.84 (t, 1H), 7.81 (d, 1H), 7.51 (dd, 1H), 7.42 (t, 1H), 7.34 (t, 1H), 6.76 (d, 1H), 4.01 (s, 3H), 2.61 (s, 3H); MS for C₂₇H₂₀FN₅O₄: *m*/*z* 498.2 (MH+).

**N-[4-[(6,7-Dimethoxy-1,5-naphthyridin-4-yl)oxy]-3-fluorophenyl]-5-(5-fluoropyridin-2-yl)-1,2-dimethyl-4-oxopyridine-3-carboxamide (71):** Compound **35** in **Example 11** was replaced with Compound **67.** ¹H NMR (400 MHz, DMSO-*d₆*) δ 10.82 (s, 1H), 8.66 (dd, 1H), 8.61 - 8.51 (m, 2H), 8.46 (d, 1H), 7.90 (dd, 1H), 7.70 (td, 1H), 7.59 (s, 1H), 7.42 (d, 1H), 7.29 (t, 1H), 6.71 (d, 1H), 3.92 (s, 3H), 3.91 (s, 3H), 3.75 (s, 3H), 2.36 (s, 3H); MS for C₂₉H₂₃F₂N₅O₅: *m*/*z* 560.2 (MH+).

**N-[4-[(6,7-Dimethoxy-1,5-naphthyridin-4-yl)oxy]phenyl]-5-(5-fluoropyridin-2-yl)-1,2-dimethyl-4-oxopyridine-3-carboxamide (72): Compound 35 in Example 11** was replaced with Compound **67** and Intermediate **I-1** was replaced with Intermediate **1-2.** ¹H NMR (400 MHz, DMSO-*d₆)* δ 10.70 (s, 1H), 8.74 (dd, 1H), 8.66 - 8.57 (m, 2H), 8.52 (d, 1H), 7.85 - 7.69 (m, 3H), 7.64 (s, 1H), 7.20 (d, 2H), 6.75 (d, 1H), 3.98 (s, 3H), 3.97 (s, 3H), 3.82 (s, 3H), 2.43 (s, 3H); MS for C₂₉H₂₄FN₅O₅: *m*/*z* 542.2 (MH+).

**N-[3-Fluoro-4-[(7-methoxy-1,5-naphthyridin-4-yl)oxy]phenyl]-5-(5-fluoropyridin-2-yl)-1,2-dimethyl-4-oxopyridine-3-carboxamide (73): Compound 35** in **Example 11** was replaced with Compound **67** and Intermediate **I-1** was replaced with Intermediate **I-3.** ¹H NMR (400 MHz, DMSO-*d₆*) δ 10.85 (s, 1H), 8.69 - 8.61 (m, 3H), 8.59 - 8.46 (m, 2H), 7.93 (dd, 1H), 7.78 - 7.63 (m, 2H), 7.46 (d, 1H), 7.35 (t, 1H), 6.69 (d, 1H), 3.94 (s, 3H), 3.75 (s, 3H), 2.36 (s, 3H); MS for C₂₈H₂₁F₂N₅O₄: *m*/*z* 530.0 (MH+).

### Example 21: 5-Fluoro-4'-hydroxy-2'-methyl-[2,3'-bipyridine]-5'-carboxylic acid (78)

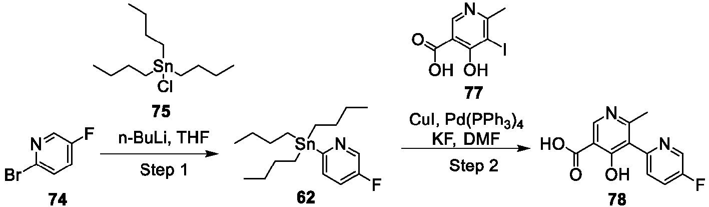

**Step 1: 5-Fluoro-2-(tributylstannyl)pyridine (62):** To a solution of Compound **74** (1.21 g, 6.88 mmol, **1** eq) in THF (15 mL) was added n-BuLi (2.5 M, 2.75 mL, 1eq) and the mixture was stirred at -78 °C for 30 min under nitrogen. Compound 75 (12.35 g, 7.22 mmol, 1.94 mL, 1.05 eq) was added and the mixture was stirred at the same temperature for another 2 h. Aq saturated NH₄Cl solution (150 mL) was added to the reaction solution and the resulting mixture was extracted with EtOAc (3 x 150 mL). The combined organic extracts were dried over anhyd. Na₂SO₄ and concentrated in vacuum to give crude Compound 62 as a colorless oil (3 g). MS for C₁₇H₃₀FNSn: *m*/*z* 387.0 (MH+).

**Step 2: 5-Fluoro-4'-hydroxy-2'-methyl-[2,3'-bipyridine]-5'-carboxylic acid (78):** A mixture of Compound **77** (200 mg, 0.72 mmol, 1 eq), Compound **62** (1.94 g, 5.02 mmol, 7 eq), Pd(PPh₃)₄ (166 mg, 0.14 mmol, 0.2 eq), CuI (27.3 mg, 0.14 mmol, 0.2eq) and KF (125 mg, 2.15 mmol, 3 eq) in DMF (20 mL) was degassed and purged with nitrogen 3 times and then the mixture was stirred at 120 °C for 16 h under nitrogen. The reaction mixture was concentrated under reduced pressure and the resulting residue was purified by column chromatography (SiO₂, DCM:MeOH = 100/1 to 1/100) followed by further purification by HCl prep-HPLC (Column: Venusil ASB Phenyl 150*30mm*5um; mobile phase:[water (0.05% HCl)-ACN]; B%: 10%-40%, 9 min) to give Compound **78** as a yellow solid (50 mg, 12.50% yield). ¹H NMR (400 MHz, DMSO-*d₆*) δ 13.45 (br s, 1H), 8.90 - 8.38 (m, 3H), 7.49 (br s, 1H), 2.24 (s, 3H); MS for C₁₂H₉FN₂O₃: *m*/*z* 248.8 (MH+).

The following compounds were made in a manner similar to Compounds **62** and **78** in **Example 21:**

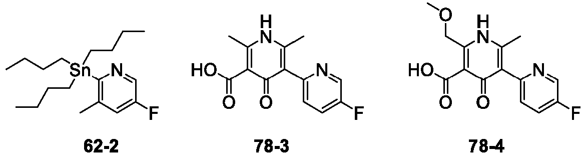

**5-Fluoro-3-methyl-2-(tributylstannyl)pyridine (62-2):** Compound **74** in Step 1 was replaced with 2-bromo-5-fluoro-3-methylpyridine. ¹H NMR (400 MHz, CDCl₃) δ 8.47 (d, 1H), 7.11 (dd, 1H), 2.41 (s, 3H), 1.64 - 1.48 (m, 6H), 1.34 (h, 6H), 1.24 - 1.06 (m, 6H), 0.93 - 0.85 (m, 9H)

**5-Fluoro-2',6'-dimethyl-4'-oxo-1',4'-dihydro-[2,3'-bipyridine]-5'-carboxylic acid (78-3):** Compound **77** in Step 2 was replaced with Compound **61-3.** MS for C₁₃H₁₁FN₂O₃: *m*/*z* 263 (MH+).

**5-Fluoro-6'-(methoxymethyl)-2'-methyl-4'-oxo-1',4'-dihydro-[2,3'-bipyridine]-5'-carboxylic acid (78-4):** Compound 77 in Step 2 was replaced with Compound 61-4. ¹H NMR (400 MHz, DMSO-*d₆*) δ 12.05 (s, 1H), 8.60 (d, 1H), 7.76 (tdd, 1H), 7.48 (dd, 1H), 4.99 (s, 2H), 3.42 (s, 3H), 2.24 (s, 3H); MS for C₁₄H₁₃FN₂O₄: *m*/*z* 293.0 (MH+).

### Example 22: 5-Fluoro-1',2'-dimethyl-4'-oxo-1',4'-dihydro-[2,3'-bipyridine]-5'-carboxylic acid (84)

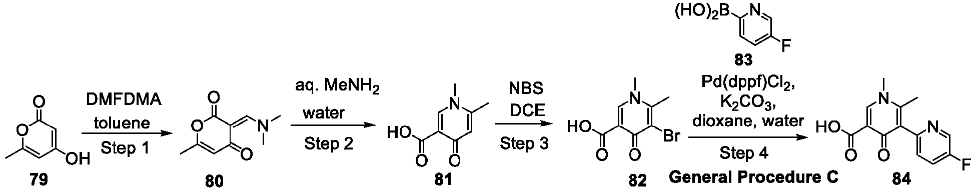

**Step 1: (E)-3-((Dimethylamino)methylene)-6-methyl-2H-pyran-2,4(3H)-dione (80):** To a white suspension of Compound **79** (15 g, 119 mmol, 1 eq) in toluene (40 mL) was added DMF-DMA (15 g, 127 mmol, 17 mL, 1.1 eq). The orange mixture was stirred at 15 °C for 2 h. The reaction mixture was concentrated under reduced pressure and co-evaporated with toluene (50 mL) 3 times and with DCM (50 mL) 2 times to give Compound **80** as a red-brown solid (21 g, 97% yield) which was used in subsequent reactions without further purification. ¹H NMR (400 MHz, CDCl₃) δ 8.23 (s, 1H), 5.66 (s, 1H), 3.46 (s, 3H), 3.37 (s, 3H), 2.13 (s, 3H); MS for C₉H₁₁NO₃: *m*/*z* 181.8 (MH+).

**Step 2: 1,6-Dimethyl-4-oxo-1,4-dihydropyridine-3-carboxylic acid (81):** To a red-brown solution of Compound **80** (5 g, 27.6 mmol, 1 eq) in water (50 mL) was added MeNH₂ (15 mL). The red-brown solution was stirred at 100 °C for 1 h. The reaction solution was acidified with AcOH to pH 3 and concentrated under reduced pressure. The residue was triturated with DCM: EtOH (10: 1) for 1 min. The mixture was filtered and the filter cake was washed with DCM (2 x 20 mL). The filtrate was concentrated under reduced pressure. Water (50 mL) was added to the resulting residue and the mixture extracted with 10:1 DCM:MeOH (3 x 50 mL). The combined organic extracts were concentrated under reduced pressure to give crude Compound **81** as a red-brown solid (2.5 g) which was used without further purification in subsequent reactions. ¹H NMR (400 MHz, DMSO-*d₆*) δ 8.71 (s, 1H), 6.71 (s, 1H), 3.82 (s, 3H), 2.42 (s, 3H).

**Step 3: 5-Bromo-1,6-dimethyl-4-oxo-1,4-dihydropyridine-3-carboxylic acid (82):** To a mixture of Compound **81** (2 g, 12 mmol, 1 eq) in DCE (50 mL) was added NBS (3.19 g, 17.95 mmol, 1.5 eq). The red-brown solution was stirred at 20 °C for 4 h. The reaction mixture was diluted with water and extracted with DCM (3 x 50 mL). The combined organic extracts were dried over anhyd. Na₂SO₄ and concentrated to give crude Compound **82** with was used in subsequent reactions without further purification. ¹H NMR (400 MHz, DMSO-*d₆*) δ 11.05 (br s, 1H), 8.79 (s, 1H), 3.94 (s, 3H), 2.66 (s, 3H).

**Step 4: 5-Fluoro-1',2'-dimethyl-4'-oxo-1',4'-dihydro-[2,3'-bipyridine]-5'-carboxylic acid (84):** Using **General Procedure C,** to a mixture of Compound **82** (0.2 g, 0.81 mmol, 1 eq) and Compound 83 (172 mg, 1.22 mmol, 1.5 eq) in dioxane (5 mL) and water (2 mL) was added K₂CO₃ (337 mg, 2.44 mmol, 3 eq) and Pd(dppf)Cl₂ (178 mg, 0.24 mmol, 0.3 eq). The mixture was stirred at 105 °C for 15 h under nitrogen. Water (50 mL) and aq 1N NaOH (50 mL) was added to the reaction mixture to give a pH of 13. The resulting mixture was washed with DCM (2 x 80 mL). The aqueous layer was acidified with aq 2N HCl to pH 1-2 and extracted with 9:1 DCM:MeOH (5 x 50 mL). The combined organic extracts were dried over anhyd. Na₂SO₄ and concentrated under reduced pressure Compound **84** as a brown oil (150 mg, 70.37% yield) which was used directly without further purification in subsequent steps. MS for C₁₃H₁₁FN₂O₃: *m*/*z* 263.1 (MH+).

### Example 23: N-[4-[(6,7-Dimethoxy-1,5-naphthyridin-4-yl)oxy]-3-fluorophenyl]-5-(5-fluoropyridin-2-yl)-4-hydroxy-6-methylpyridine-3-carboxamide (85):

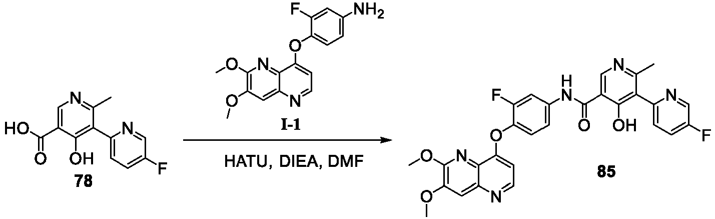

Compound **85** and the following compounds were made by **General Procedure D,** similar to the synthesis of Compound **41** in **Example 11** from Compound **35** and Intermediate **I-1**. The temperatures of the reactions can vary from room temperature to 40 °C. Reaction times can vary from 2-4 h. EtOAc can replace DCM as the extraction solvent.

**N-[4-[(6,7-Dimethoxy-1,5-naphthyridin-4-yl)oxy]-3-fluorophenyl]-5-(5-fluoropyridin-2-yl)-4-hydroxy-6-methylpyridine-3-carboxamide (85):** Compound **35** in **Example 11** was replaced with Compound **78.** ¹H NMR (400 MHz, DMSO-*d₆*) δ 12.92 (s, 1H), 8.66 (s, 1H), 8.61 (s, 1H), 8.54 (d, 1H), 8.51 (d, 1H), 8.00 (dd, 1H), 7.64 (s, 1H), 7.48 - 7.37 (m, 2H), 7.37 - 7.25 (m, 1H), 6.82 (d, 1H), 3.95 (d, 6H), 2.19 (s, 3H); MS for C₂₈H₂₁F₂N₅O₅: *m*/*z* 546.1 (MH+).

**N-[4-[(6,7-Dimethoxy-1,5-naphthyridin-4-yl)oxy]phenyl]-5-(5-fluoropyridin-2-yl)-1,6-dimethyl-4-oxopyridine-3-carboxamide (86):** Compound **35** in **Example 11** was replaced with Compound **84** and Intermediate **I-1** was replaced with Intermediate **I-2**. ¹H NMR (400 MHz, CD₃OD) δ 8.92 - 8.82 (m, 2H), 8.68 (br d, 1H), 8.21 - 8.12 (m, 1H), 7.89 (br d, 2H), 7.85 - 7.78 (m, 1H), 7.65 - 7.58 (m, 1H), 7.33 (br d, 2H), 7.12 (br d, 1H), 4.19 (s, 3H), 4.14 (s, 3H), 3.97 (s, 3H), 2.34 (s, 3H); MS for C₂₉H₂₄FN₅O₅: *m*/*z* 542.2 (MH+).

### Example 24: N-[4-[(6,7-Dimethoxy-1,5-naphthyridin-4-yl)oxy]-3-fluorophenyl]-5-(furan-2-yl)-4-hydroxy-6-methylpyridine-3-carboxamide (89):

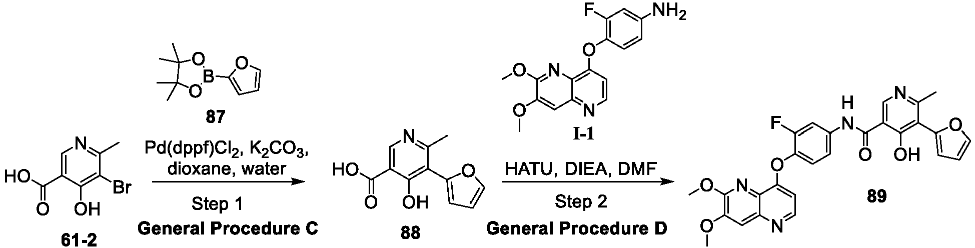

**Step 1: 5-(Furan-2-yl)-4-hydroxy-6-methylnicotinic acid (88):** Compound **88** was made following **General Procedure C.** Specifically in this case, a mixture of Compound **61-2** (717 mg, 3.1 mmol, 1 eq), Compound **87** (1.5 g, 7.7 mmol, 2.5 eq), Pd(dppf)Cl₂ (226 mg, 0.31 mmol, 0.1 eq) and K₂CO₃ (855 mg, 6.2 mmol, 2 eq) in water (2.5 mL) and dioxane (10 mL) was degassed and purged with nitrogen 3 times, and then the mixture was stirred at 110 °C for 12 h under an atmosphere of nitrogen. The reaction mixture was concentrated under reduced pressure. The resulting residue was dissolved in water (200 mL) and washed with EtOAc (200 mL) followed with DCM (200 mL). The aqueous phase was acidified with aq 2 N HCl to pH 2 and the resulting solid was filtered. The solid was then washed with water (2 x 50 mL) and dried under reduced pressure to give Compound **88** as a yellow solid (250 mg, 37% yield) which was used without further purification. MS for C₁₁H₉NO₄: *m*/*z* 219.9 (MH+).

**Step 2: N-[4-[(6,7-Dimethoxy-1,5-naphthyridin-4-yl)oxy]-3-fluorophenyl]-5-(furan-2-yl)-4-hydroxy-6-methylpyridine-3-carboxamide (89):** Compound **89** was made following **General Procedure D.** Specifically in this case, to a solution of Compound **88** (150 mg, 0.68 mmol, 1 eq) in DMF (2 mL) was added Intermediate **I-1** (173 mg, 0.55 mmol, 0.8 eq), HATU (312 mg, 0.82 mmol, 1.2 eq) and DIEA (265 mg, 2.1 mmol, 3 eq). The mixture was stirred at 25 °C for 15 hr. The reaction mixture was diluted with water (50 mL) and extracted with EtOAc (2 x 50 mL). The combined organic extracts were washed with aq saturated NaCl (5 x 150 mL), dried over anhyd Na₂SO₄ and concentrated under reduced pressure. The resulting residue was purified by flash silica gel chromatography (0-2% MeOH in DCM) to give Compound **89** as a white solid (77.4 mg, 22% yield). ¹H NMR (400MHz, DMSO-*d₆*) δ 13.08 (s, 1H), 12.66 (br s, 1H), 8.54 (d, 1H), 8.49 (s, 1H), 8.02 (dd, 1H), 7.77 (d, 1H), 7.64 (s, 1H), 7.46 - 7.41 (m, 1H), 7.36 - 7.31 (m, 1H), 6.94 (d, 1H), 6.82 (d, 1H), 6.61 (dd, 1H), 3.95 (d, 6H), 2.45 (s, 3H); MS for C₂₇H₂₁FN₄O₆: *m*/*z* 517.1 (MH+).

The following compounds were made using **General Procedure C** followed by **General Procedure D** as exemplified by the synthesis of Compound **89** in **Example 24:**

**N-[4-[(6,7-Dimethoxy-1,5-naphthyridin-4-yl)oxy]-3-fluoro-phenyl]-4-hydroxy-6-methyl-5-(2-thienyl)pyridine-3-carboxamide (90)):** ¹H NMR (400MHz, DMSO-*d₆*) δ 13.10 (s, 1H), 8.57 - 8.51 (m, 2H), 8.02 (dd, 1H), 7.67 - 7.64 (m, 2H), 7.42 (br d, 1H), 7.35 - 7.29 (m, 1H), 7.16 - 7.11 (m, 2H), 6.82 (d, 1H), 3.95 (d, 6H), 2.37 (s, 3H); MS for C₂₇H₂₁FN₄O₅S: *m*/*z* 533.1 (MH+).

**N-[4-[(6,7-Dimethoxy-1,5-naphthyridin-4-yl)oxy]-3-fluoro-phenyl]-4-hydroxy-6-methyl-5-(4-methyl-2-thienyl) pyridine-3-carboxamide (91):** ¹H NMR (400MHz, DMSO-*d₆*) δ 13.15 (s, 1H), 12.92 (br d, 1H), 8.81 (d, 1H), 8.49 (d, 1H), 8.13 - 8.06 (m, 1H), 7.83 (s, 1H), 7.54 - 7.47 (m, 2H), 7.22 (s, 1H), 7.17 (d, 1H), 6.93 (d, 1H), 4.05 (d, 6H), 2.39 (s, 3H), 2.26 (s, 3H); MS for C₂₈H₂₃FN₄O₅S: *m*/*z* 547.2 (MH+).

**N-[4-[(6,7-Dimethoxy-1,5-naphthyridin-4-yl)oxy]-3-fluoro-phenyl]-4-hydroxy-6-methyl-5-(5-methyl-2-furyl)pyridine-3-carboxamide (92):** ¹H NMR (400MHz, DMSO-*d₆*) δ 13.14 (s, 1H), 12.72 (br d, 1H), 8.60 (d, 1H), 8.47 (d, 1H), 8.04 (dd, 1H), 7.67 (s, 1H), 7.46 (br d, 1H), 7.40 - 7.33 (m, 1H), 6.89 (d, 1H), 6.84 (d, 1H), 6.21 (d, 1H), 3.97 (d, 6H), 2.48 (s, 3H), 2.33 (s, 3H). MS for C₂₈H₂₃FN₄O₆: *m*/*z* 531.2 (MH+).

**N-[4-[(6,7-Dimethoxy-1,5-naphthyridin-4-yl)oxy]-3-fluoro-phenyl]-4-hydroxy-6-methyl-5-(3-thienyl)pyridine-3-carboxamide (93):** MS for C₂₇H₂₁FN₄O₅S: *m*/*z* 533.4 (MH+).

**N-[4-[(6, 7-Dimethoxy-1, 5-naphthyridin-4-yl) oxy]-3-fluoro-phenyl]-4-hydroxy-6-methyl-5-(5-methyl-2-thienyl) pyridine-3-carboxamide (94):** ¹H NMR (400MHz, CD₃OD) δ 9.22 (s, 1H), 8.78 (d, 1H), 8.12 - 8.03 (m, 1H), 7.78 (s, 1H), 7.70 (br d, 1H), 7.52 (t, 1H), 7.26 (d, 1H), 7.02 - 6.99 (m, 1H), 7.00 (d, 1H), 6.90 (br s, 1H), 4.15 (d, 6H), 2.59 (s, 3H), 2.56 (s, 3H); MS for C₂₈H₂₃FN₄O₅S: *m*/*z* 547.2 (MH+).

**N-[4-[(6,7-Dimethoxy-1,5-naphthyridin-4-yl)oxy]-3-fluoro-phenyl]-5-(3-furyl)-4-hydroxy-6-methyl-pyridine-3-carboxamide (95):** ¹H NMR (400MHz, DMSO-*d₆*) δ 13.24 (s, 1H), 12.58 (s, 1H), 8.54 (d, 1H), 8.49 (s, 1H), 8.02 (dd, 1H), 7.90 (d, 1H), 7.74 (t, 1H), 7.65 (s, 1H), 7.43 - 7.39 (m, 1H), 7.36-7.30 (m, 1H), 6.82 (d, 1H), 6.69 (d, 1H), 3.97 (s, 3H), 3.95 (s, 3H), 2.38 (s, 3H); MS for C₂₇H₂₁FN₄O₆: *m*/*z* 517.0 (MH+).

**N-[3-Fluoro-4-[(7-methoxy-1,5-naphthyridin-4-yl)oxy]phenyl]-5-(2-furyl)-4-hydroxy-6-methyl-pyridine-3-carboxamide (96):** ¹H NMR (400MHz, DMSO-*d₆*) δ 13.17 (s, 1H), 12.80 - 12.76 (m, 1H), 8.89 - 8.85 (m, 2H), 8.51 (d, 1H), 8.10 (dd, 1H), 7.85 (d, 1H), 7.80 - 7.77 (m, 1H), 7.56 - 7.46 (m, 2H), 7.00 - 6.93 (m, 2H), 6.62 (dd, 1H), 4.05 (s, 3H), 2.46 (s, 3H); MS for C₂₆H₁₉FN₄O₅: *m*/*z* 487.1 (MH+).

**N-[3-Fluoro-4-[(7-methoxy-1,5-naphthyridin-4-yl)oxy]phenyl]-4-hydroxy-6-methyl-5-(5-methyl-2-furyl)pyridine-3-carboxamide (97):** ¹H NMR (400MHz, DMSO-*d₆*) δ 13.20 (s, 1H), 12.78 - 12.73 (m, 1H), 8.91 - 8.85 (m, 2H), 8.48 (d, 1H), 8.11 (dd, 1H), 7.86 (d, 1H), 7.56 - 7.46 (m, 2H), 6.98 (d, 1H), 6.84 (d, 1H), 6.23 - 6.20 (m, 1H), 4.05 - 4.02 (m, 3H), 2.48 (br s, 3H), 2.33 (s, 3H). MS for C₂₇H₂₁FN₄O₅: *m*/*z* 501.1 (MH+).

**N-[3-Fluoro-4-[(7-methoxy-1,5-naphthyridin-4-yl)oxy]phenyl]-4-hydroxy-6-methyl-5-(3-thienyl)pyridine-3-carboxamide (98):** ¹H NMR (400MHz, DMSO-*d₆*) δ 13.28 (s, 1H), 12.69 - 12.64 (m, 1H), 8.88 - 8.81 (m, 2H), 8.52 (d, 1H), 8.08 (dd, 1H), 7.83 (d, 1H), 7.59 (dd, 1H), 7.53 - 7.43 (m, 3H), 7.15 (dd, 1H), 6.91 (br d, 1H), 4.03 (s, 3H), 2.28 (s, 3H); MS for C₂₆H₁₉FN₄O₄S: m/z 503.1 (MH+).

**N-[3-Fluoro-4-[(7-methoxy-1,5-naphthyridin-4-yl)oxy]phenyl]-5-(3-furyl)-4-hydroxy-6-methyl-pyridine-3-carboxamide (99):** ¹H NMR (400MHz, DMSO-*d₆*) δ 13.30 (s, 1H), 12.76 (d, 1H), 8.93-8.84 (m, 2H), 8.49 (d, 1H), 8.14-8.06 (m, 1H), 7.93-7.86 (m, 2H), 7.74 (t, 1H), 7.53-7.46 (m, 2H), 7.01 (d, 1H), 6.69 (d, 1H), 4.05 (s, 3H), 2.39 (s, 3H); MS for C₂₆H₁₉FN₄O₅: *m*/*z* 487.1 (MH+).

**N-[3-Fluoro-4-[(7-methoxy-1,5-naphthyridin-4-yl)oxy]phenyl]-4-hydroxy-6-methyl-5-(2-thienyl)pyridine-3-carboxamide (100):** MS for C₂₆H₁₉FN₄O₄S: *m*/*z* 503.1 (MH+).

**N-[3-Fluoro-4-[(7-methoxy-1, 5-naphthyridin-4-yl)** oxy] phenyl]-4-hydroxy-6-methyl-**5-(5-methyl-2-thienyl) pyridine-3-carboxamide (101):** ¹H NMR (400MHz, CD₃OD) δ 8.99 - 8.94 (m, 2H), 8.60 (s, 1H), 8.12 (br d, 1H), 7.81 (br s, 1H), 7.56 - 7.46 (m, 2H), 7.25 (br d, 1H), 6.88 - 6.82 (m, 2H), 4.17 (s, 3H), 2.53 (s, 3H), 2.43 (s, 3H); MS for C₂₇H₂₁FN₄O₄S: *m*/*z* 517.1 (MH+).

**N-[3-Fluoro-4-[(7-methoxy-1,5-naphthyridin-4-yl)oxy]phenyl]-4-hydroxy-6-methyl-5-(4-methyl-2-thienyl) pyridine-3-carboxamide (102):** ¹H NMR (400 MHz, DMSO-*d₆*) δ 13.16 (s, 1H), 12.90 (br d, 1H), 8.97 - 8.92 (m, 2H), 8.51 (d, 1H), 8.15 - 8.08 (m, 1H), 7.93 (d, 1H), 7.57 - 7.48 (m, 2H), 7.23 (s, 1H), 7.09 (d, 1H), 6.94 (d, 1H), 4.07 (s, 3H), 2.38 (s, 3H), 2.26 (s, 3H); MS for C₂₇H₂₁FN₄O₄S: *m*/*z* 517.1 (MH+).

**N-[4-[(6, 7-Dimethoxy-1, 5-naphthyridin-4-yl) oxy]-3-fluoro-phenyl]-4-hydroxy-6-methyl-5-(3-methyl-2-thienyl) pyridine-3-carboxamide (103):** MS for C₂₈H₂₃FN₄O₅S: m/z 547.2 (MH+).

**N-[4-[(6,7-Dimethoxy-1,5-naphthyridin-4-yl)oxy]-3-fluoro-phenyl]-4-hydroxy-6-methyl-5-(2-methylpyrazol-3-yl)pyridine-3-carboxamide (104):** MS for C₂₇H₂₃FN₆O₅: *m*/*z 531.1* (MH+).

**N-[4-[(6,7-Dimethoxy-1,5-naphthyridin-4-yl)oxy]-3-fluoro-phenyl]-4-hydroxy-6-methyl-5-[2-methyl-5-(trifluoromethyl)pyrazol-3-yl]pyridine-3-carboxamide (105):** ¹H NMR (400MHz, DMSO-*d₆*) δ 13.12 (br d, 1H), 12.93 (s, 1H), 8.77 (d, 1H), 8.62 (d, 1H), 8.07 (dd, 1H), 7.77 (s, 1H), 7.56 - 7.43 (m, 2H), 7.11 (d, 1H), 6.78 (s, 1H), 4.04 (s, 6H), 3.72 (s, 3H), 2.25 (s, 3H); MS for C₂₈H₂₂F₄N₆O₅: *m*/*z* 599.1 (MH+).

**N-[4-[(6,7-Dimethoxy-1,5-naphthyridin-4-yl)oxy]-3-fluoro-phenyl]-4-hydroxy-2,6-dimethyl-5-(3-thienyl)pyridine-3-carboxamide (106):** MS for C₂₈H₂₃FN₄O₅S: *m*/*z* 547.1 (MH+).

**N-[4-[(6,7-Dimethoxy-1,5-naphthyridin-4-yl)oxy]-3-fluoro-phenyl]-4-hydroxy-2,6-dimethyl-5-(2-thienyl)pyridine-3-carboxamide (107):** ¹H NMR (400MHz, DMSO-*d₆*) δ 13.05 - 12.85 (m, 1H), 12.07 - 11.87 (m, 1H), 8.61 - 8.45 (m, 1H), 8.06 - 7.91 (m, 1H), 7.64 (br s, 2H), 7.42 - 7.26 (m, 2H), 7.18 - 7.06 (m, 2H), 6.86 - 6.67 (m, 1H), 3.96 (br s, 6H), 2.83 - 2.65 (s, 3H), 2.43 - 2.27 (s, 3H); MS for C₂₈H₂₃FN₄O₅S *m*/*z* 547.1 (MH+).

**N-[4-[(6,7-Dimethoxy-1,5-naphthyridin-4-yl)oxy]-3-fluoro-phenyl]-4-hydroxy-2, 6-dimethyl-5-(2-methylpyrazol-3-yl) pyridine-3-carboxamide (108):** ¹H NMR (400MHz, DMSO-*d₆*) δ 13.06 (br s, 1H), 12.41 - 11.66 (m, 1H), 8.53 (d, 1H), 7.98 (dd, 1H), 7.64 (s, 1H), 7.45 (d, 1H), 7.41 - 7.37 (m, 1H), 7.33 - 7.28 (m, 1H), 6.78 (d, 1H), 6.19 (d, 1H), 3.96 (s, 3H), 3.95 (s, 3H), 3.60 (s, 3H), 2.73 (s, 3H), 2.17 (s, 3H); MS for C₂₈H₂₅FN₆O₅: *m*/*z* 545.2 (MH+).

**N-[4-[(6,7-Dimethoxy-1,5-naphthyridin-4-yl)oxy]-3-fluoro-phenyl]-5-(2,5-dimethylpyrazol-3-yl)-4-hydroxy-2,6-dimethyl-pyridine-3-carboxamide (109):** ¹H NMR (DMSO-*d₆* 400 MHz) δ 13.10 (s, 1H), 12.01 (s, 1H), 8.53 (d, 1H), 7.98 (dd, 1H), 7.64 (s, 1H), 7.40 - 7.35 (m, 1H), 7.33 - 7.28 (m, 1H), 6.78 (d, 1H), 5.95 (s, 1H), 3.96 (s, 3H), 3.95 (s, 3H), 3.50 (s, 3H), 2.73 (s, 3H), 2.17 (d, 6H); MS for C₂₉H₂₇FN₆O₅: *m*/*z* 559.3 (MH+).

**N-[4-[(6,7-Dimethoxy-1,5-naphthyridin-4-yl)oxy]-3-fluoro-phenyl]-4-hydroxy-2,6-dimethyl-5-(5-methyl-2-furyl)pyridine-3-carboxamide** (110): ¹H NMR (400MHz, DMSO-d₆) δ 12.98 (s, 1H), 11.92 - 11.80 (m, 1H), 8.54 (d, 1H), 7.99 (dd, 1H), 7.64 (s, 1H), 7.42 - 7.37 (m, 1H), 7.34 - 7.28 (m, 1H), 6.79 (d, 1H), 6.75 (d, 1H), 6.18 (d, 1H), 3.96 (d, 6H), 2.68 (s, 3H), 2.43 (s, 3H), 2.31 (s, 3H); MS for C₂₉H₂₅FN₄O₆: *m*/*z* 545.2 (MH+).

**N-[4-[(6,7-Dimethoxy-1,5-naphthyridin-4-yl)oxy]-3-fluoro-phenyl]-4-hydroxy-2,6-dimethyl-5-(5-methyl-2-thienyl)pyridine-3-carboxamide (111):** ¹H NMR (400MHz, DMSO-*d₆*) δ 13.09 (s, 1H), 12.08 (br s, 1H), 8.74 (d, 1H), 8.05 (br d, 1H), 7.75 (s, 1H), 7.49 - 7.37 (m, 2H), 7.07 (br d, 1H), 6.87 (d, 1H), 6.80 (d, 1H), 4.04 (d, 6H), 2.70 (s, 3H), 2.47 (s, 3H), 2.37 (s, 3H); MS for C₂₉H₂₅FN₄O₅S: *m*/*z* 561.2 (MH+).

**N-[4-[(6,7-Dimethoxy-1,5-naphthyridin-4-yl)oxy]-3-fluoro-phenyl]-5-(3-furyl)-4-hydroxy-2,6-dimethyl-pyridine-3-carboxamide (112):** ¹H NMR (400MHz, DMSO-*d₆*) δ 13.28 (s, 1H), 11.94 - 11.79 (m, 1H), 8.54 (d, 1H), 8.05 - 7.96 (m, 1H), 7.87 (d, 1H), 7.73 (t, 1H), 7.65 (s, 1H), 7.37 (br d, 1H), 7.35 - 7.27 (m, 1H), 6.80 (d, 1H), 6.66 (d, 1H), 3.97 (d, 6H), 2.72 (s, 3H), 2.36 (s, 3H); MS for C₂₈H₂₃FN₄O₆: *m*/*z* 531.1 (MH+).

### Example 25: N-[4-[(6,7-Dimethoxy-1,5-naphthyridin-4-yl)oxy]-3-fluorophenyl]-4-hydroxy-2-(methoxymethyl)-6-methyl-5-thiophen-2-ylpyridine-3-carboxamide (115):

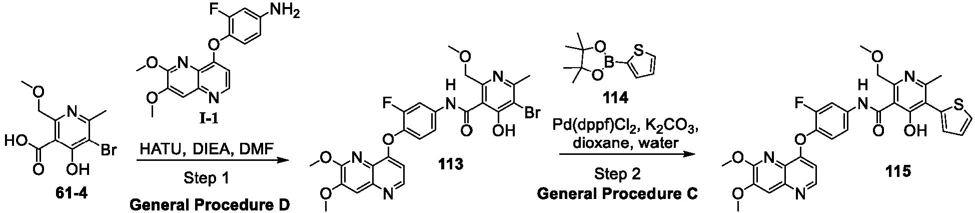

**Step 1: 5-Bromo-N-(4-((6,7-dimethoxy-1,5-naphthyridin-4-yl)oxy)-3-fluorophenyl)-4-hydroxy-2-(methoxymethyl)-6-methylnicotinamide (113):** Compound **113** was made following **General Procedure D.** Specifically in this case, a mixture of Intermediate **I-1** (94.5 mg, 0.30 mmol), Compound **61-4** (124 mg, 0.45 mmol), HATU (230 mg, 0.61 mmol) in DMF (1.5 mL) was chilled to 0 °C in an ice bath and DIEA (0.15 mL, 0.86 mmol) was added dropwise. The resulting reaction mixture was stirred at room temperature overnight. The reaction mixture was diluted with DCM and was washed with aq saturated K₂CO₃ solution followed by water twice. The organic phase was then dried over anhyd Na₂SO₄ and concentrated under reduced pressure. The resulting residue was absorbed into silica gel and purified by flash chromatography (95% DCM:5% MeOH) to give Compound **113** as an off-white solid (140 mg, 81% yield). MS for C₂₅H₂₂BrFN₄O₆: *m*/*z* 573/575 (MH+).

**Step 2: N-[4-[(6,7-Dimethoxy-1,5-naphthyridin-4-yl)oxy]-3-fluorophenyl]-4-hydroxy-2-(methoxymethyl)-6-methyl-5-thiophen-2-ylpyridine-3-carboxamide (115):** Compound **115** was made following **General Procedure C.** Specifically in this case, a mixture of Compound **113** (37.6 mg, 0.066 mmol), Compound **114** (12.6mg, 0.10 mmol) and K₂CO₃ (21.0 mg, 0.152 mmol) in 1,4-dioxane (0.69 mL) and water (0.2 mL) in a 5 mL microwave tube was degassed with nitrogen followed by addition of Pd(PPh₃)₄ (3.6 mg, 0.0031 mmol). The resulting mixture was heated at 160 °C under microwave irradiation for 40 min. The mixture was then diluted with MeOH and filtered. The filtrate was concentrated, and the resulting residue was purified using flash chromatography (95% EtOAc:5% MeOH) to give Compound **115** (11.6 mg, 31% yield). ¹H NMR (400MHz, DMSO-*d₆*) δ 12.93 (s, 1H), 11.54 (s, 1H), 8.55 (d, 1H), 7.99 (dd, 1H), 7.77 - 7.57 (m, 2H), 7.47 - 7.35 (m, 1H), 7.32 (t, 1H), 7.19 - 7.06 (m, 2H), 6.81 (dd, 1H), 4.98 (s, 2H), 3.97 (d, 6H), 3.47 (s, 3H), 2.45 (s, 3H). MS for C₂₉H₂₅FN₄O₆S: *m*/*z* 577.0 (MH+).

The following compounds were made using **General Procedure D** followed by **General Procedure C** as exemplified by the syntheses of **115** in **Example 25:**

**N-[4-[(6,7-Dimethoxy-1,5-naphthyridin-4-yl)oxy]-3-fluorophenyl]-4-hydroxy-2-(methoxymethyl)-6-methyl-5-thiophen-2-ylpyridine-3-carboxamide (116):** MS for C₂₉H₂₅FN₄O₇: *m*/*z* 561.0 (MH+).

**N-[4-[(6,7-Dimethoxy-1,5-naphthyridin-4-yl)oxy]-3-fluorophenyl]-4-hydroxy-2-(methoxymethyl)-6-methyl-5-(5-methylfuran-2-yl)pyridine-3-carboxamide (117):** MS for C₃₀H₂₇FN₄O₇: *m*/*z* 575.0 (MH+).

**N-[3-Fluoro-4-[(6-methoxy-1,5-naphthyridin-4-yl)oxy]phenyl]-5-(furan-2-yl)-4-hydroxy-6-methylpyridine-3-carboxamide (118):** ¹H NMR (400MHz, DMSO-*d₆*) δ 13.11 (s, 1H), 12.69 (d, 1H), 8.63 (d, 1H), 8.51 (d, 1H), 8.30 (d, 1H), 8.05 (dd, 1H), 7.79 (d, 1H), 7.55 - 7.22 (m, 3H), 6.97 (dd, 2H), 6.62 (dd, 1H), 3.94 (s, 3H), 2.46 (s, 3H); MS for C₂₆H₁₉FN₄O₅: *m*/*z* 487.0 (MH+).

**N-[4-[(6,7-Dimethoxy-1,5-naphthyridin-4-yl)oxy]-3-fluorophenyl]-5-(furan-2-yl)-4-hydroxy-2-methylpyridine-3-carboxamide (119):** ¹H NMR (400MHz, DMSO-*d₆*) δ 12.43 (s, 1H), 12.32 (s, 1H), 8.48 (d, 1H), 8.06 (s, 1H), 7.94 (dd, 1H), 7.69 - 7.62 (m, 1H), 7.59 (s, 1H), 7.41 - 7.33 (m, 1H), 7.33 - 7.15 (m, 2H), 6.82 - 6.64 (m, 1H), 6.53 (dd, 1H), 3.91 (s, 6H), 2.55 (s, 3H); MS for C₂₇H₂₁FN₄O₆: *m*/*z* 517.1 (MH+).

**N-[4-[(6,7-Dimethoxy-1,5-naphthyridin-4-yl)oxy]-3-fluorophenyl]-4-hydroxy-2-methyl-5-(5-methylfuran-2-yl)pyridine-3-carboxamide (120):** ¹H NMR (400MHz, DMSO-*d₆*) δ 12.50 (s, 1H), 12.27 (s, 1H), 8.48 (d, 1H), 7.94 (dd, 2H), 7.59 (s, 1H), 7.41 - 7.33 (m, 1H), 7.27 (t, 1H), 7.15 (d, 1H), 6.74 (d, 1H), 6.13 (dd, 1H), 3.90 (s, 6H), 2.55 (s, 3H), 2.28 (s, 3H); MS for C₂₈H₂₃FN₄O₆: *m*/*z* 531.1 (MH+).

**N-[4-[(6,7-Dimethoxy-1,5-naphthyridin-4-yl)oxy]-3-fluorophenyl]-5-(5-formylfuran-2-yl)-4-hydroxy-2-methylpyridine-3-carboxamide (121):** ¹H NMR (400MHz, DMSO-*d₆*) δ 12.47 (s, 1H), 12.01 (s, 1H), 9.53 (s, 1H), 8.47 (d, 1H), 8.24 (s, 1H), 7.93 (dd, 1H), 7.66 - 7.50 (m, 2H), 7.47 (d, 1H), 7.38 (d, 1H), 7.29 (d, 1H), 6.73 (d, 1H), 3.91 (s, 6H), 2.50 (s, 3H); MS for C₂₈H₂₁FN₄O₇: *m*/*z* 545 (MH+).

**N-[4-[(6,7-Dimethoxy-1,5-naphthyridin-4-yl)oxy]-3-fluorophenyl]-5-(furan-3-yl)-4-hydroxy-2-methylpyridine-3-carboxamide (122):** ¹H NMR (400MHz, DMSO-*d₆*) δ 12.77 (s, 1H), 12.36 (d, 1H), 8.67 - 8.50 (m, 2H), 8.19 (d, 1H), 8.02 (dd, 1H), 7.73 (t, 1H), 7.66 (s, 1H), 7.49 - 7.39 (m, 1H), 7.35 (t, 1H), 7.11 (d, 1H), 6.81 (dd, 1H), 3.98 (s, 6H), 2.65 (s, 3H); MS for C₂₇H₂₁FN₄O₆: *m*/*z* 517.1 (MH+).

**N-[4-[(6,7-Dimethoxy-1,5-naphthyridin-4-yl)oxy]-3-fluorophenyl]-4-hydroxy-2-methyl-5-thiophen-2-ylpyridine-3-carboxamide (123):** ¹H NMR (400MHz, DMSO-*d₆*) δ 12.46 (s, 1H), 12.40 (s, 1H), 8.55 (d, 1H), 8.43 (s, 1H), 8.02 (dd, 1H), 7.75 (dd, 1H), 7.66 (s, 1H), 7.54 (dd, 1H), 7.47 - 7.31 (m, 2H), 7.13 (dd, 1H), 6.81 (dd, 1H), 3.98 (s, 6H), 2.63 (s, 3H); MS for C₂₇H₂₁FN₄O₅S: *m*/*z* 533.0 (MH+).

**N-[4-[(6,7-Dimethoxy-1,5-naphthyridin-4-yl)oxy]-3-fluorophenyl]-4-hydroxy-2-methyl-5-(5-methylthiophen-2-yl)pyridine-3-carboxamide (124):** ¹H NMR (400MHz, DMSO-*d₆*) δ 12.55 (s, 1H), 12.43 (s, 1H), 8.55 (d, 1H), 8.33 (s, 1H), 8.02 (dd, 1H), 7.66 (s, 1H), 7.53 (d, 1H), 7.44 (ddd, 1H), 7.35 (t, 1H), 6.99 - 6.70 (m, 2H), 3.97 (s, 6H), 2.63 (s, 3H), 2.48 (d, 3H); MS for C₂₈H₂₃FN₄O₅S: *m*/*z* 547 (MH+).

**N-[4-[(6,7-Dimethoxy-1,5-naphthyridin-4-yl)oxy]-3-fluorophenyl]-4-hydroxy-2-methyl-5-(4-methylthiophen-2-yl)pyridine-3-carboxamide (125):** ¹H NMR (400MHz, DMSO-*d₆*) δ 12.36 (d, 2H), 8.48 (d, 1H), 8.31 (s, 1H), 7.95 (dd, 1H), 7.59 (s, 1H), 7.50 (d, 1H), 7.38 (dd, 1H), 7.28 (t, 1H), 7.04 (d, 1H), 6.74 (d, 1H), 3.90 (s, 6H), 2.55 (s, 3H), 2.19 (s, 3H); MS for C₂₈H₂₃FN₄O₅S: m/z 547.1 (MH+).

**N-[4-[(6,7-Dimethoxy-1,5-naphthyridin-4-yl)oxy]-3-fluorophenyl]-4-hydroxy-2-methyl-5-(3-methylthiophen-2-yl)pyridine-3-carboxamide (126):** ¹H NMR (400MHz, DMSO-*d₆*) δ 12.61 (s, 1H), 12.34 (d, 1H), 8.54 (d, 1H), 8.01 (dd, 1H), 7.91 (d, 1H), 7.66 (s, 1H), 7.49 (d, 1H), 7.42 (d, 1H), 7.34 (t, 1H), 6.98 (d, 1H), 6.80 (d, 1H), 3.97 (s, 6H), 2.65 (s, 3H), 2.23 (s, 3H); MS for C₂₈H₂₃FN₄O₅S: *m*/*z* 547.1 (MH+).

**N-[4-[(6,7-Dimethoxy-1,5-naphthyridin-4-yl)oxy]-3-fluorophenyl]-4-hydroxy-2-methyl-5-thiophen-3-ylpyridine-3-carboxamide (127):** ¹H NMR (400MHz, DMSO-*d₆*) δ 12.67 (s, 1H), 12.24 (s, 1H), 8.47 (d, 1H), 8.27 (dd, 1H), 8.12 (s, 1H), 7.95 (dd, 1H), 7.63 - 7.55 (m, 2H), 7.51 (dd, 1H), 7.43 - 7.30 (m, 1H), 7.27 (t, 1H), 6.74 (d, 1H), 3.90 (s, 6H), 2.57 (s, 3H); MS for C₂₇H₂₁FN₄O₅S: *m*/*z* 533.0 (MH+).

**N-[3-Fluoro-4-[(6-methoxy-1,5-naphthyridin-4-yl)oxy]phenyl]-4-hydroxy-6-methyl-5-thiophen-2-ylpyridine-3-carboxamide (128):** MS (EI) for C₂₆H₁₉FN₄O₄S: *m*/*z* 503.1 (MH+).

**5-(Furan-2-yl)-4-hydroxy-N-[4-[(7-methoxy-1,5-naphthyridin-4-yl)oxy]phenyl]-6-methylpyridine-3-carboxamide (129):** ¹H NMR (400MHz, DMSO-*d₆*) δ 12.92 (s, 1H), 12.59 (s, 1H), 8.65 (dd, 2H), 8.44 (s, 1H), 7.85 - 7.73 (m, 2H), 7.72 (d, 2H), 7.26 - 7.08 (m, 2H), 6.88 (d, 1H), 6.68 (d, 1H), 6.55 (dd, 1H), 3.93 (s, 3H), 2.38 (s, 3H); MS for C₂₆H₂₀N₄O₅: *m*/*z* 469.2 (MH+).

### Example 26: 4-Hydroxy-2-(methoxymethyl)-6-methylnicotinic acid (132)

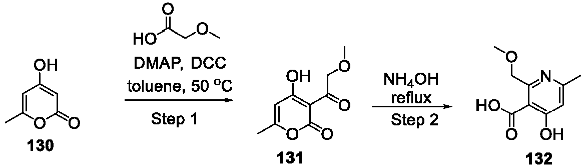

**Step 1: 4-Hydroxy-3-(2-methoxyacetyl)-6-methyl-2H-pyran-2-one (131):** To a solution of Compound **130** (10 g, 79 mmol, 1 eq) in toluene (100 mL) was added 2-methoxyacetic acid (7.1 g, 79 mmol, 1 eq), DCC (16.3 g, 79 mmol, 1 eq) and DMAP (9.6 g, 79 mmol, 1 eq). The resulting mixture was heated to 50 °C. The resulting solid was filtered and then purified by flash silica gel column chromatography (0-100% EtOAc/hexanes) to give Compound 131. MS for C₉H₁₀O₅: m/z 199 (MH+).

**Step 2: 4-Hydroxy-2-(methoxymethyl)-6-methylnicotinic acid (132):** To a solution of Compound **131** (12.5 g, 63 mmol, 1eq) in water (200 mL) was added ammonium hydroxide (40% in water, 60 mL). The resulting mixture was heated to reflux overnight. After allowing the reaction mixture to cool to room temperature, the solvent was partially removed under vacuum. The resulting mixture was cooled to 0 °C and acidified to pH 2 using aq 6 N HCl. The resulting solid was filtered and allowed to dry in the open air to give Compound **132.** MS for C₉H₁₁NO₄: *m*/*z* 198 (MH+).

### Example 27: N-[4-[(6,7-Dimethoxy-1,5-naphthyridin-4-yl)oxy]-3-fluorophenyl]-5-(furan-2-yl)-4-hydroxy-2,6-dimethylpyridine-3-carboxamide (135):

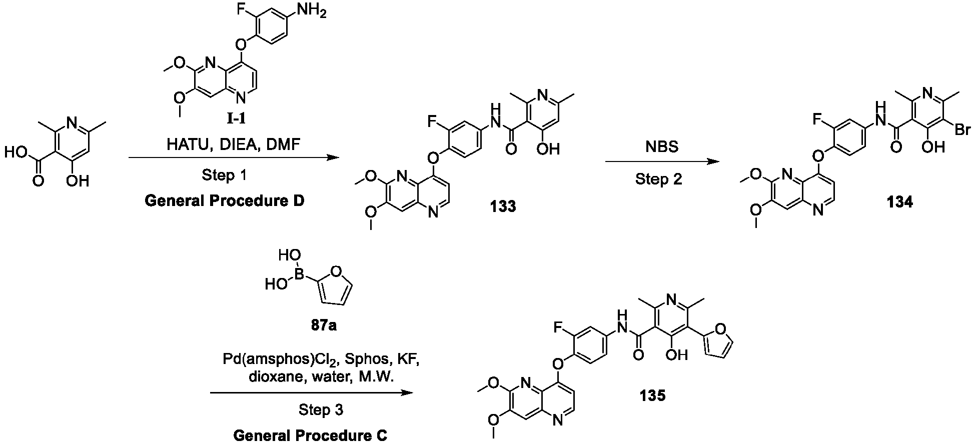

**Step 1: N-(4-((6,7-Dimethoxy-1,5-naphthyridin-4-yl)oxy)-3-fluorophenyl)-4-hydroxy-2,6-dimethylnicotinamide (133):** Compound 133 was synthesized using **General Procedure D.** Specifically in this case, to a mixture of 4-hydroxy-2,6-dimethyl-pyridine-3-carboxylic acid (150 mg, 0.9 mmol, 1 eq) and Intermediate **I-1** (226 mg, 0.72 mmol, 0.8 eq) in DMF (1 mL) was added HATU (512 mg, 1.35 mmol, 1.5 eq) and DIEA (116 mg, 0.9 mmol, 1 eq). The mixture was stirred at 25 °C for 24 h. The reaction mixture was diluted with EtOAc (50 mL) and washed with water (10 x 30 mL). The combined organic layers were washed with aq saturated NaCl (10 mL), dried over anhyd Na₂SO₄ and concentrated under reduced pressure to give Compound 133 as a yellow solid (270 mg, 80% yield). MS for C₂₄H₂₁FN₄O₅: *m*/*z* 465.1 (MH+).

**Step 2: 5-Bromo-N-(4-((6,7-dimethoxy-1,5-naphthyridin-4-yl)oxy)-3-fluorophenyl)-4-hydroxy-2,6-dimethylnicotinamide (134):** Compound **134** was synthesized in a manner similar to Compound **61** in **Example 15.** Specifically, in this case, to a solution of Compound **133** (270 mg, 0.58 mmol, 1 eq) in DMF (5 mL) was added NBS (109 mg, 0.61 mmol, 1.05 eq). The mixture was stirred at 25 °C for 1 h. The reaction mixture was diluted with water (10 mL) and extracted with EtOAc (3 x 20 mL). The combined organic layers were washed with aq saturated NaCl (10 mL), dried over anhyd Na₂SO₄ and concentrated under reduced pressure to give Compound 134 as a yellow solid (304 mg, 96% yield). MS for C₂₄H₂₀BrFN₄O₅: *m*/*z* 544.7 (MH+).

**Step 3: N-[4-[(6,7-Dimethoxy-1,5-naphthyridin-4-yl)oxy]-3-fluorophenyl]-5-(furan-2-yl)-4-hydroxy-2,6-dimethylpyridine-3-carboxamide (135):** Compound **135** was synthesized using **General Procedure C.** Specifically in this case, Compound **134** (30.0 mg, 0.06 mmol, 1 eq), Compound **87a** (18.5 mg, 0.17 mmol, 3 eq), Sphos (22.7 mg, 0.06 mmol, 1 eq), KF (9.6 mg, 0.17 mmol, 3 eq) and 4-ditert-butylphosphanyl-N,N-dimethyl-aniline dichloropalladium (3.9 mg, 0.006 mmol, 0.1 eq) were combined in a microwave tube in dioxane (1 mL) and water (1 mL). The sealed tube was heated under microwave irradiation at 120 °C for 35 min, cooled to room temperature, diluted with water (15 mL), and extracted with EtOAc (3 x 15 mL). The combined extracts were washed with aq saturated NaCl (10 mL), dried over anhyd Na₂SO₄ and concentrated under reduced pressure. The resulting residue was purified by prep HPLC to give Compound **135** as yellow solid (23.6 mg, 81% yield). ¹H NMR (400MHz, DMSO-*d₆*) δ 12.99 (s, 1H), 12.20 (br s, 1H), 8.83 (d, 1H), 8.15 - 8.01 (m, 1H), 7.85 (s, 1H), 7.75 (s, 1H), 7.53 - 7.41 (m, 2H), 7.17 (d, 1H), 6.91 - 6.80 (m, 1H), 6.59 (dd, 1H), 4.07 (d, 6H), 2.69 (s, 3H), 2.43 (s, 3H); MS for C₂₈H₂₃FN₄O₆: *m*/*z* 530.9 (MH+).

The following compounds were made using the same three step procedure as exemplified by the synthesis of Compound **135** in **Example 27:**

**N-[4-[(6,7-Dimethoxy-1,5-naphthyridin-4-yl)oxy]-3-fluorophenyl]-5-(2,5-dimethylpyrazol-3-yl)-4-hydroxy-6-methylpyridine-3-carboxamide (136):** ¹H NMR (400MHz, DMSO-*d₆*) δ 13.14 (br d, 1H), 13.09 (s, 1H), 8.84 (br d, 1H), 8.56 (br d, 1H), 8.09 (br d, 1H), 7.87 (s, 1H), 7.51 (br s, 2H), 7.20 (br d, 1H), 6.05 (s, 1H), 4.07 (br s, 3H), 4.06 (br s, 3H), 3.54 (s, 3H), 2.23 (br s, 3H), 2.19 (s, 3H); MS for C₂₈H₂₅FN₆O₅: *m*/*z* 545.2 (MH+).

**N-[4-[(6,7-Dimethoxy-1,5-naphthyridin-4-yl)oxy]-3-fluorophenyl]-4-hydroxy-2,6-dimethyl-5-(4-methylthiophen-2-yl)pyridine-3-carboxamide (137):** ¹H NMR (400MHz, DMSO-*d₆*) δ 13.04 (s, 1H), 12.31 (br s, 1H), 8.87 - 8.77 (m, 1H), 8.08 (br d, 1H), 7.87 (s, 1H), 7.52 - 7.42 (m, 2H), 7.21 - 7.15 (m, 2H), 6.90 (d, 1H), 4.07 (d, 6H), 2.71 (s, 3H), 2.38 (s, 3H), 2.29 - 2.18 (m, 3H); MS for C₂₉H₂₅FN₄O₅S: *m*/*z* 560.9 (MH+).

**N-[4-[(6,7-Dimethoxy-1,5-naphthyridin-4-yl)oxy]-3-fluorophenyl]-4-hydroxy-2,6-dimethyl-5-[2-methyl-5-(trifluoromethyl)pyrazol-3-yl]pyridine-3-carboxamide (138):** ¹H NMR (400MHz, DMSO-*d₆*) δ 12.92 (s, 1H), 12.60 (br s, 1H), 8.84 (d, 1H), 8.11 - 7.97 (m, 1H), 7.92 - 7.83 (m, 1H), 7.54 - 7.44 (m, 2H), 7.18 (d, 1H), 6.74 (s, 1H), 4.06 (d, 6H), 3.70 (s, 3H), 2.75 (s, 3H), 2.24 (s, 3H); MS for C₂₉H₂₄F₄N₆O₅: *m*/*z* 613.1 (MH+).

**N-[4-[(6,7-Dimethoxy-1,5-naphthyridin-4-yl)oxy]-3-fluorophenyl]-4-hydroxy-2,6-dimethyl-5-(3-methylthiophen-2-yl)pyridine-3-carboxamide (139):** ¹H NMR (400MHz, DMSO-*d₆*) *δ 13.27* (s, 1H), 12.25 (br s, 1H), 8.67 (d, 1H), 8.02 (dd, 1H), 7.73 (s, 1H), 7.51 (d, 1H), 7.46 - 7.30 (m, 2H), 7.01 - 6.93 (m, 2H), 4.00 (s, 6H), 2.75 (s, 3H), 2.15 (s, 3H), 1.96 (s, 3H); MS for C₂₉H₂₅FN₄O₅S: *m*/*z* 561.2 (MH+).

**N-[4-[(6,7-Dimethoxy-1,5-naphthyridin-4-yl)oxy]-3-fluorophenyl]-4-hydroxy-6-methyl-5-(1,2-oxazol-4-yl)pyridine-3-carboxamide (140):** ¹H NMR (400MHz, DMSO-*d₆*) δ 9.28 (s, 1H), 9.16 (s, 1H), 8.99 (s, 1H), 8.84 (s, 1H), 8.81 (br s, 1H), 8.52 (s, 1H), 8.09 (brd, 1H), 7.70 (s, 1H), 7.51 (brs, 2H), 7.19 (brs, 1H), 4.05 (br s, 6H), 2.41 (br s, 3H); MS for C₂₆H₂₀FN₅O₆: *m*/*z* 518.0 (MH+).

**N-[4-[(6,7-Dimethoxy-1,5-naphthyridin-4-yl)oxy]-3-fluorophenyl]-4-hydroxy-6-methyl-5-(1,3-thiazol-5-yl)pyridine-3-carboxamide (141):** ¹H NMR (400MHz, DMSO-*d₆*) δ 13.01 - 12.87 (m, 2H), 9.21 (s, 1H), 8.67 (br s, 1H), 8.56 (d, 1H), 8.06 (br d, 1H), 8.02 (s, 1H), 7.72 (br s, 1H), 7.51 - 7.45 (m, 1H), 7.44 - 7.37 (m, 1H), 6.99 (br s, 1H), 4.00 (br d, 6H), 2.46 (s, 3H); MS for C₂₆H₂₀FN₅O₅S: *m*/*z* 534.1 (MH+).

**N-[4-[(6,7-Dimethoxy-1,5-naphthyridin-4-yl)oxy]-3-fluorophenyl]-4-hydroxy-6-methyl-5-(1,3-thiazol-4-yl)pyridine-3-carboxamide (142):** MS for C₂₆H₂₀FN₅O₅S: *m*/*z* 534.1 (MH+).

**N-[4-[(6,7-Dimethoxy-1,5-naphthyridin-4-yl)oxy]-3-fluorophenyl]-4-hydroxy-6-methyl-5-(1,3-thiazol-2-yl)pyridine-3-carboxamide (143):** MS for C₂₆H₂₀FN₅O₅S: *m*/*z* 534.1 (MH+).

### Example 28: N-[4-[(6,7-Dimethoxy-1,5-naphthyridin-4-yl)oxy]-3-fluorophenyl]-5-(5-ethylfuran-2-yl)-4-hydroxy-2,6-dimethylpyridine-3-carboxamide (147):

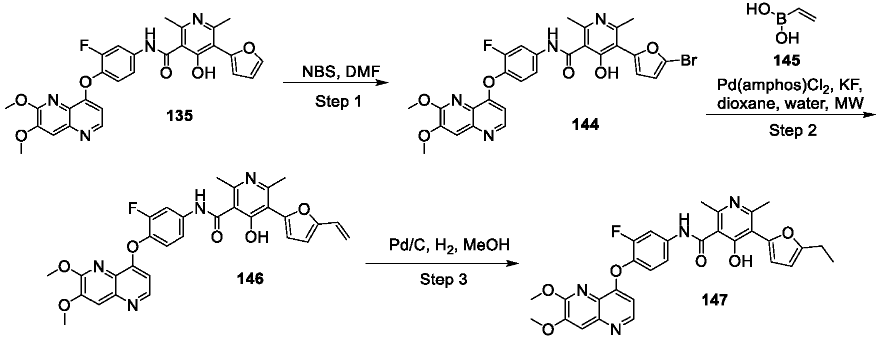

**Step 1: 5-(5-Bromofuran-2-yl)-N-(4-((6,7-dimethoxy-1,5-naphthyridin-4-yl)oxy)-3-fluorophenyl)-4-hydroxy-2,6-dimethylnicotinamide (144):** To a mixture of Compound 135 (0.12 g, 0.22 mmol, 1 eq) in DMF (3 mL) was added NBS (40.7 mg, 0.23 mmol, 1.1 eq). The mixture was stirred at 25 °C for 40 min, diluted with EtOAc (40 mL), and washed with water several times (40 mL each) followed by aq saturated NaCl (10 mL). The organic phase was dried over anhyd Na₂SO₄ and concentrated under reduced pressure to Compound 144 as a black solid (120 mg, 90% yield). MS for C₂₈H₂₂BrFN₄O₆: *m*/*z* 609/611 (MH+).

**Step 2: N-(4-((6,7-Dimethoxy-1,5-naphthyridin-4-yl)oxy)-3-fluorophenyl)-4-hydroxy-2,6-dimethyl-5-(5-vinylfuran-2-yl)nicotinamide (146):** Compound **144** (200 mg, 0.33 mmol, 1 eq), Compound **145** (283 mg, 3.9 mmol, 12 eq), KF (114 mg, 2.0 mmol, 6 eq) and 4-ditert-butylphosphanyl-N,N-dimethyl-aniline dichloropalladium (46.5 mg, 0.66 mmol, 0.2 eq) were combined in a microwave tube with dioxane (2 mL) and water (1 mL). The sealed tube was heated under microwave irradiation at 110 °C for 30 min, cooled to room temperature, diluted with water (20 mL), and extracted with EtOAc (30 mL x 3). The combined extracts were washed with aq saturated NaCl (10 mL), dried over anhyd Na₂SO₄ and concentrated under reduced pressure to give Compound **146** as a black solid (150 mg, 82% yield). MS for C₃₀H₂₅FN₄O₆: *m*/*z* 557.3 (MH+).

**Step 3: N-[4-[(6,7-Dimethoxy-1,5-naphthyridin-4-yl)oxy]-3-fluorophenyl]-5-(5-ethylfuran-2-yl)-4-hydroxy-2,6-dimethylpyridine-3-carboxamide (147):** To a mixture of Compound **146** (150 mg, 0.27 mmol, 1 eq) in MeOH (40 mL) was added 10% Pd/C (30 mg) in one portion at 25 °C under an atmosphere of hydrogen (15 psi). The mixture was stirred at 25 °C for 60 min. The reaction mixture was filtered, and the filter cake washed with MeOH (60 mL). The filtrate was dried over anhyd Na₂SO₄ and concentrated under reduced pressure. The resulting residue was purified by prep-HPLC to give Compound **147** as a brown solid (27.6 mg, 18% yield). MS for C₃₀H₂₇FN₄O₆: *m*/*z* 559.2 (MH+).

The following compound was made using the same three step procedure as exemplified by the synthesis of Compound **147** in **Example 28:**

**N-[4-[(6,7-Dimethoxy-1,5-naphthyridin-4-yl)oxy]-3-fluorophenyl]-4-hydroxy-2,6-dimethyl-5-(5-propan-2-ylfuran-2-yl)pyridine-3-carboxamide (148):** MS for C₃₀H₂₇FN₄O₆: *m*/*z* 573.2 (MH+).

### Example 29: N-[4-[(6,7-Dimethoxy-1,5-naphthyridin-4-yl)oxy]-3-fluorophenyl]-5-(5-ethenylfuran-2-yl)-4-hydroxy-6-methylpyridine-3-carboxamide (151):

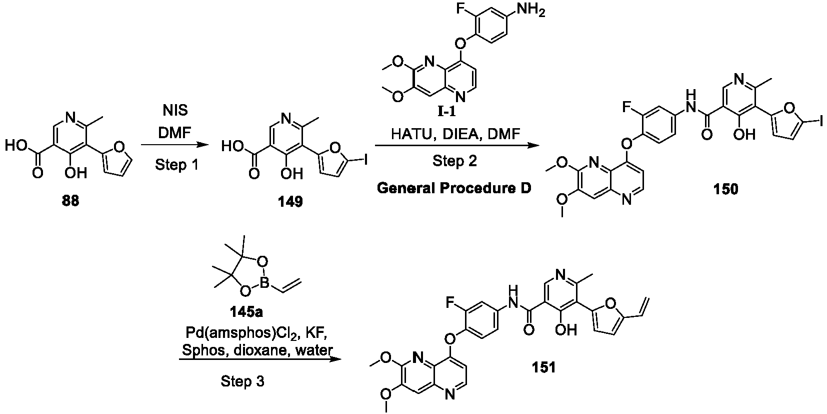

**Step 1: 4-Hydroxy-5-(5-iodofuran-2-yl)-6-methylnicotinic acid (149):** To a mixture of Compound **88** (1.15 g, 5.3 mmol, 1 eq) in DMF (10 mL) was added NIS (1.30 g, 5.8 mmol, 1.1 eq). The mixture was stirred at 25 °C for 15 h. The reaction mixture was diluted with water (30 mL) and extracted with EtOAc (30 mL x 3). The combined extracts were washed with aq saturated NaCl (10 mL), dried over anhyd Na₂SO₄ and concentrated under reduced pressure to give Compound **149** as a yellow solid (1.2 g, 66 % yield). MS for C₁₁H₈INO₄: *m*/*z* 345.9 (MH+).

**Step 2: N-(4-((6,7-Dimethoxy-1,5-naphthyridin-4-yl)oxy)-3-fluorophenyl)-4-hydroxy-5-(5-iodofuran-2-yl)-6-methylnicotinamide (150):** Compound **149** was synthesized in the manner of General Procedure **D.** Specifically in this case, to a mixture of Compound **149** (160 mg, 0.46 mmol, 1.1 eq) and intermediate **I-1** (120 mg, 0.38 mmol, 0.9 eq) in DMF (2 mL) was added HATU (321 mg, 0.84 mmol, 2 eq) and DIEA (163 mg, 1.26 mmol, 3 eq). The mixture was stirred at 25 °C for 15 h. The reaction mixture was diluted with water (40 mL) and extracted with EtOAc (30 mL x 5). The combined extracts were washed with aq saturated NaCl (20 mL), dried over anhyd Na₂SO₄ and concentrated under reduced pressure to give Compound **150** as a yellow solid (260 mg, 96.0% yield). MS for C₂₇H₂₀FIN₄O₆: *m*/*z* 643.0 (MH+).

**Step 3: N-[4-[(6,7-Dimethoxy-1,5-naphthyridin-4-yl)oxy]-3-fluorophenyl]-5-(5-ethenylfuran-2-yl)-4-hydroxy-6-methylpyridine-3-carboxamide (151):** Compound **150** (160 mg, 0.25 mmol, 1 eq), Compound **145a** (230 mg, 1.5 mmol, 6 eq), 4-ditert-butylphosphanyl-N,N-dimethyl-aniline dichloropalladium (17.6 mg, 0.025 mmol, 0.1 eq), KF (14.5 mg, 0.25 mmol, 1 eq) and SPhos (102 mg, 0.25 mmol, 1 eq) were combined in a microwave tube with dioxane (1 mL) and water (1 mL). The sealed tube was heated under microwave irradiation at 140 °C for 35 min, cooled to room temperature, diluted with water (10 mL) and extracted with EtOAc (15 mL x 3). The combined extracts were washed with aq saturated NaCl (10 mL), dried over anhyd Na₂SO₄ and concentrated under reduced pressure. The resulting residue was purified prep HPLC to give Compound **151** as a yellow solid (65 mg, 48% yield). ¹H NMR (400MHz, DMSO-*d₆*) δ 13.14 (s, 1H), 12.84 (br d, 1H), 8.76 (d, 1H), 8.49 (d, 1H), 8.10 (dd, 1H), 7.77 - 7.70 (m, 1H), 7.56 - 7.42 (m, 2H), 7.16 - 7.06 (m, 2H), 6.69 - 6.57 (m, 2H), 5.63 (d, 1H), 5.23 (d, 1H), 4.04 (s, 6H), 2.57 (s, 3H); MS for C₂₉H₂₃FN₄O₆: *m*/*z* 543.1 (MH+).

The following compound was made using the same three step procedure as exemplified by the synthesis of Compound **151** in **Example 29:**

**N-[4-[(6,7-Dimethoxy-1,5-naphthyridin-4-yl)oxy]-3-fluorophenyl]-4-hydroxy-6-methyl-5-(5-prop-1-en-2-ylfuran-2-yl)pyridine-3-carboxamide (152):** MS for C₃₀H₂₅FN₄O₆: *m*/*z 557.2* (MH+).

### Example 30: N-[4-[(6,7-Dimethoxy-1,5-naphthyridin-4-yl)oxy]-3-fluorophenyl]-5-(5-ethylfuran-2-yl)-4-hydroxy-6-methylpyridine-3-carboxamide (153):

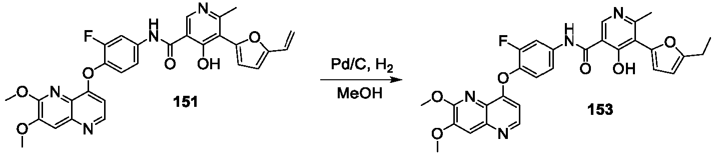

**N-[4-[(6,7-Dimethoxy-1,5-naphthyridin-4-yl)oxy]-3-fluorophenyl]-5-(5-ethylfuran-2-yl)-4-hydroxy-6-methylpyridine-3-carboxamide (153): To** a mixture of Compound 151 (72 mg, 0.13 mmol, 1 eq) in MeOH (10 mL) was added 10% Pd/C (20 mg) in one portion at 25 °C under an atmosphere of hydrogen. The mixture was stirred at 25 °C for 120 min under hydrogen (15 psi). The reaction mixture was filtered, and the filtrate concentrated under reduced pressure. The resulting residue was purified by silica gel column chromatography (DCM:MeOH = 20: 1) to give Compound 153 as a light-yellow solid (20 mg, 28% yield). MS for C₂₉H₂₅FN₄O₆: *m*/*z* 545.2 (MH+).

The following compound was made using the same procedure as exemplified by the synthesis of Compound **153** in **Example 30:**

**N-[4-[(6,7-Dimethoxy-1,5-naphthyridin-4-yl)oxy]-3-fluorophenyl]-4-hydroxy-6-methyl-5-(5-propan-2-ylfuran-2-yl)pyridine-3-carboxamide (154):** ¹H NMR 400MHz, DMSO-*d₆*) δ 13.23 (s, 1H), 13.02 (br d, 1H), 8.79 (d, 1H), 8.44 (d, 1H), 8.18 - 8.05 (m, 1H), 7.87 (s, 1H), 7.56 - 7.45 (m, 2H), 7.14 (d, 1H), 6.96 (d, 1H), 6.20 (d, 1H), 4.05 (d, 6H), 3.02 - 2.86 (m, 1H), 2.53 (s, 3H), 1.24 (d, 6H); MS for C₃₀H₂₇FN₄O₆: *m*/*z* 559.2 (MH+).

### Example 31: N-[4-[(6,7-Dimethoxy-1,5-naphthyridin-4-yl)oxy]-3-fluorophenyl]-4-hydroxy-6-methyl-5-(5-prop-2-enylfuran-2-yl)pyridine-3-carboxamide (155):

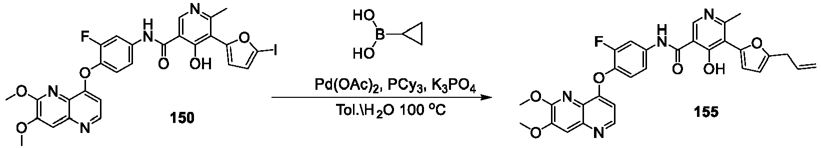

**N-[4-[(6,7-Dimethoxy-1,5-naphthyridin-4-yl)oxy]-3-fluorophenyl]-4-hydroxy-6-methyl-5-(5-prop-2-enylfuran-2-yl)pyridine-3-carboxamide (155):** To a mixture of Compound 150 (100 mg, 0.16 mmol, 1 eq), cyclopropylboronic acid (66.9 mg, 0.78 mmol, 5 eq) and P(Cy)₃ (8.73 mg, 0.031 mmol, 0.2 eq) in toluene (5 mL) and water (0.1 mL) was added K₃PO₄ (99.1 mg, 0.47 mmol, 3 eq) followed by Pd(OAc)₂ (3.49 mg, 0.016 mmol, 0.1 eq). The resulting mixture was flushed with nitrogen, and then stirred at 100 °C for 12 h under an atmosphere of nitrogen. The reaction mixture was then cooled to room temperature, diluted with water (20 mL) and extracted with DCM (20 mL x 3). The combined extracts were washed with aq saturated NaCl (10 mL), dried over anhyd Na₂SO₄ and concentrated under reduced pressure. The resulting residue was purified by flash silica gel chromatography (0-5% of MeOH in DCM) followed additional purification by prep HPLC to give Compound 155 as a yellow solid (8.2 mg, 9.5% yield). ¹H NMR (400 MHz, DMSO-*d₆*) *δ* 12.96 (s, 1H), 8.72 (s, 1H), 8.54 (d, 1H), 8.00 (br dd, 1H), 7.78 (s, 1H), 7.64 (s, 1H), 7.43 (br d, 1H), 7.38 - 7.29 (m, 1H), 6.83 (d, 1H), 6.68 - 6.58 (m, 2H), 6.19 - 6.02 (m, 1H), 5.32 (br d, 1H), 5.07 (br d, 1H), 4.95 (br s, 2H), 3.96 (s, 3H), 3.93(s, 3H), 2.34 (s, 3H); MS for C₃₀H₂₅FN₄O₆: *m*/*z* 557.3 (MH+).

### Example 31A: 5-(5-Cyclopropylfuran-2-yl)-N-[4-[(6,7-dimethoxy-1,5-naphthyridin-4-yl)oxy]-3-fluorophenyl]-4-hydroxy-6-methylpyridine-3-carboxamide (155A):

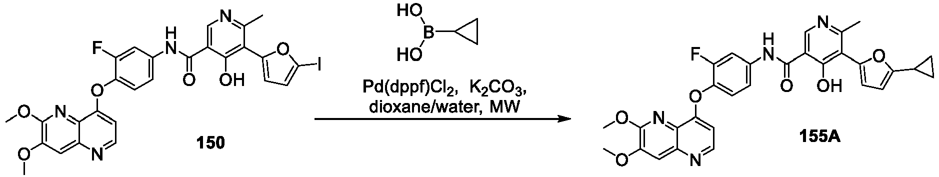

5-(5-Cyclopropylfuran-2-yl)-N-[4-[(6,7-dimethoxy-1,5-naphthyridin-4-yl)oxy]-3-fluorophenyl]-4-hydroxy-6-methylpyridine-3-carboxamide hydrochloride (155A): Compound 150 (50 mg, 0.078 mmol, 1 eq), cyclopropylboronic acid (33 mg, 0.39 mmol, 5 eq), K₂CO₃ (54 mg, 0.39 mmol, 5 eq) and Pd(dppf)Cl₂ (5.7 mg, 0.0078 mmol, 0.1 eq) were combined in a microwave tube in dioxane (0.5 mL) and water (0.5 mL). The sealed tube was heated at 100 °C for 30 min under microwave irradiation. The reaction mixture was diluted with water (10 mL) and extracted with EtOAc (3 x 15 mL). The combined organic layers were washed with aq saturated NaCl (10 mL), dried over with anhyd. Na₂SO₄ and concentrated under reduced pressure. The resulting residue was purified by prep-HPLC to give Compound 155A as a yellow solid (2.9 mg, 6% yield). ¹H NMR (400 MHz, DMSO-*d₆*) *δ* 13.21 (s, 1H), 12.94 (br d, 1H), 8.75 (d, 1H), 8.45 (d, 1H), 8.06-8.17 (m, 1H), 7.81 (s, 1H), 7.41-7.55 (m, 2H), 7.08 (d, 1H), 6.94 (d, 1H), 6.23 (d, 1H), 4.04 (s, 6H), 2.49-2.50 (m, 3H), 1.93-2.07 (m, 1H), 0.89-0.99 (m, 2H), 0.72-0.84 (m, 2H); MS for C₃₀H₂₅FN₄O₆: *m*/*z* 557.1 (MH+).

The following compound was made in the same manner as Compound **155A in Example 31A:**

5-(5-Cyclopropylfuran-2-yl)-N-[4-[(6,7-dimethoxy-1,5-naphthyridin-4-yl)oxy]-3-fluorophenyl]-4-hydroxy-2,6-dimethylpyridine-3-carboxamide (155B): Compound 150 was replaced with Compound 144. MS for C₃₁H₂₇FN₄O₆: *m*/*z* 571.2 (MH+).

### Example 32: N-[4-[(6,7-Dimethoxy-1,5-naphthyridin-4-yl)oxy]-3-fluorophenyl]-5-(furan-2-yl)-1,2,6-trimethyl-4-oxopyridine-3-carboxamide (160):

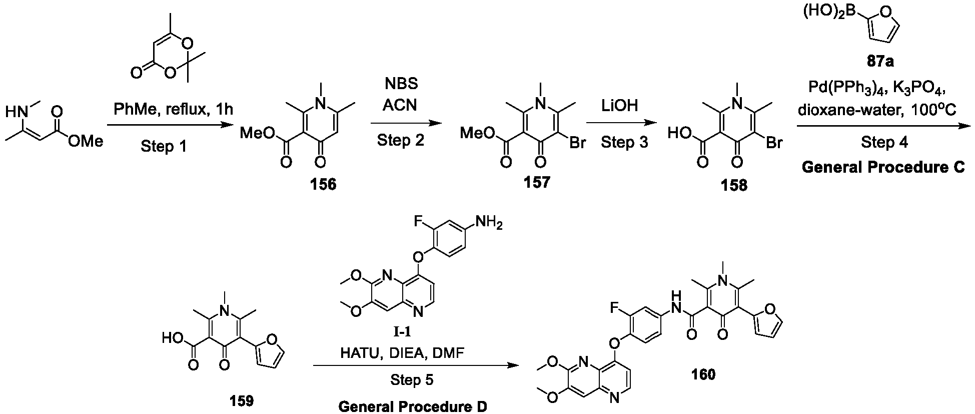

**Step 1: Methyl 1,2,6-trimethyl-4-oxo-1,4-dihydropyridine-3-carboxylate (156):** Methyl 3-(methylamino)but-2-enoate (1.7 g, 13 mmol), 2,2,6-trimethyl-4H-1,3-dioxin-4-one (4 g, 28 mmol) and toluene (20 mL) were combined in a round bottom flask equipped with a with a Dean-Stark trap. The resulting mixture was stirred at 130 °C until reflux ceased. The mixture was allowed to cool to room temperature, followed by the addition of acetone (5 mL). The resulting suspension was filtered, washed with cold acetone and dried to give crude Compound 156 (2.11 g, 82%). MS for C₁₀H₁₃NO₃: *m*/*z* 196 (MH+).

**Step 2: Methyl 5-bromo-1,2,6-trimethyl-4-oxo-1,4-dihydropyridine-3-carboxylate (157):** Compound **157** was synthesized from Compound **156** using the bromination procedure exemplified by the synthesis of Compound **61** in **Example 15.** MS for C₁₀H₁₂BrNO₃: *m*/*z* 274/276 (MH+).

**Step 3: 5-Bromo-1,2,6-trimethyl-4-oxo-1,4-dihydropyridine-3-carboxylic acid (158):** Compound **158** was synthesized from Compound **157** using standard lithium hydroxide ester hydrolysis conditions much like those used to convert Compound **34** to Compound **35** in Step 3 of **Example 8.** MS for C₉H₁₀BrNO₃: *m*/*z* 260/262 (MH+).

**Step 4: 5-(Furan-2-yl)-1,2,6-trimethyl-4-oxo-1,4-dihydropyridine-3-carboxylic acid** (159): Compound **159** was synthesized from Compound **158** and Compound **87a** using **General Procedure C.** MS for C₁₃H₁₃NO₄: *m*/*z* 248 (MH+).

**Step 5: N-[4-[(6,7-dimethoxy-1,5-naphthyridin-4-yl)oxy]-3-fluorophenyl]-5-(furan-2-yl)-1,2,6-trimethyl-4-oxopyridine-3-carboxamide (160):** Compound **160** was made from Compound **159** and Intermediate **I-1** using **General Procedure D.** ¹H NMR (400MHz, DMSO-*d₆*) δ 10.96 (s, 1H), 8.45 (d, 1H), 7.88 (dd, 1H), 7.65 (d, 1H), 7.58 (s, 1H), 7.43 - 7.36 (m, 1H), 7.28 (t, 1H), 6.70 (d, 1H), 6.55 - 6.43 (m, 2H), 3.91 (d, 6H), 3.56 (s, 3H), 2.41 (s, 3H), 2.27 (s, 3H): MS for C₂₉H₂₅FN₄O₆: *m*/*z 545* (MH+).

The following compounds were made using the same procedure as exemplified by the synthesis of Compound **160** in **Example 32:**

**5-(Furan-2-yl)-N-[4-[(7-methoxy-1,5-naphthyridin-4-yl)oxy]phenyl]-1,2,6-trimethyl-4-oxopyridine-3-carboxamide** (161): In Step 5 of **Example 32,** Compound **I-1** was replaced with Compound **I-4**. ¹H NMR (400MHz, DMSO-*d₆*) δ 10.85 (s, 1H), 8.73 (d, 1H), 8.68 (d, 1H), 7.86 - 7.76 (m, 3H), 7.73 (d, 1H), 7.27 - 7.20 (m, 2H), 6.72 (d, 1H), 6.60 - 6.55 (m, 1H), 6.52 (d, 1H), 4.01 (s, 3H), 1693.63 (s, 3H), 2.49 (s, 3H), 2.34 (s, 3H); MS for C₂₈H₂₄N₄O₅: *m*/*z* 497 (MH+).

**N-[3-Fluoro-4-[(7-methoxy-1,5-naphthyridin-4-yl)oxy]phenyl]-5-(furan-2-yl)-1,2,6-trimethyl-4-oxopyridine-3-carboxamide (162):** In Step 5 of **Example 32,** Compound **I-1** was replaced with Compound **I-3**. ¹H NMR (400MHz, DMSO-*d₆*) δ 10.99 (s, 1H), 8.68 (d, 1H), 8.62 (d, 1H), 7.91 (dd, 1H), 7.73 (d, 1H), 7.65 (dd, 1H), 7.44 (dd, 1H), 7.34 (t, 1H), 6.68 (d, 1H), 6.53 - 6.43 (m, 2H), 3.94 (s, 3H), 3.56 (s, 3H), 2.42 (s, 3H), 2.27 (s, 3H); MS for C₂₈H₂₃FN₄O₅: *m*/*z* 515 (MH+).

**N-[4-[(6,7-Dimethoxy-1,5-naphthyridin-4-yl)oxy]-2,5-difluorophenyl]-5-(furan-2-yl)-1,2,6-trimethyl-4-oxopyridine-3-carboxamide (163):** In Step 5 of **Example 32,** Compound **I-1** was replaced with Compound **I-8.** ¹H NMR (400MHz, DMSO-*d₆*) δ 12.90 (s, 1H), 8.57 (d, 1H), 8.41 (dd, 1H), 7.76 (d, 1H), 7.67 (s, 1H), 7.52 (dd, 1H), 6.93 (d, 1H), 6.62 - 6.56 (m, 1H), 6.53 (d, 1H), 3.95 (d, 6H), 3.69 (s, 3H), 2.80 (s, 3H), 2.35 (s, 3H); MS for C₂₉H₂₄FN₄O₆: *m*/*z* 563 (MH+).

**N-[4-[(6,7-Dimethoxy-1,5-naphthyridin-4-yl)oxy]phenyl]-5-(furan-2-yl)-1,2,6-trimethyl-4-oxopyridine-3-carboxamide (164):** In Step 5 of **Example 32,** Compound **I-1** was replaced with Compound **I-2**. ¹H NMR (400MHz, DMSO-*d₆*) δ 10.84 (s, 1H), 8.52 (d, 1H), 7.82 - 7.75 (m, 2H), 7.72 (dd, 1H), 7.64 (s, 1H), 7.22 - 7.15 (m, 2H), 6.75 (d, 1H), 6.57 (dd, 1H), 6.52 (dd, 1H), 3.97 (d, 6H), 3.63 (s, 3H), 2.51 (s, 3H), 2.34 (s, 3H); MS for C₂₉H₂₆N₄O₆: *m*/*z* 527 (MH+).

**N-[4-[(7-Methoxy-1,5-naphthyridin-4-yl)oxy]phenyl]-1,2,6-trimethyl-4-oxo-5-thiophen-2-ylpyridine-3-carboxamide (165):** In Step 4 of **Example 32,** Compound **87a** was replaced with thiophen-2-ylboronic acid and in Step 5, Compound **I-1** was replaced with Compound **I-4.** ¹H NMR (400MHz, DMSO-*d₆*) δ 10.89 (s, 1H), 8.73 (d, 1H), 8.68 (d, 1H), 7.86 - 7.76 (m, 3H), 7.61 (d, 1H), 7.23 (d, 2H), 7.11 (dd, 1H), 6.93 (d, 1H), 6.72 (d, 1H), 4.00 (s, 3H), 3.64 (s, 3H), 2.51 (s, 3H), 2.39 (s, 3H); MS for C₂₈H₂₄N₄O₄S: *m*/*z* 513 (MH+).

**N-[3-Fluoro-4-[(7-methoxy-1,5-naphthyridin-4-yl)oxy]phenyl]-1,2,6-trimethyl-4-oxo-5-thiophen-2-ylpyridine-3-carboxamide (166):** In Step 4 of **Example 32,** Compound **87a** was replaced with thiophen-2-ylboronic acid and in Step 5, Compound **I-1** was replaced with Compound **I-3.** ¹H NMR (400MHz, DMSO-*d₆*) δ 11.10 (s, 1H), 8.75 (d, 1H), 8.70 (d, 1H), 7.98 (dd, 1H), 7.80 (d, 1H), 7.64 - 7.58 (m, 1H), 7.51 (d, 1H), 7.41 (t, 1H), 7.12 (dd, 1H), 6.94 (dd, 1H), 6.75 (d, 1H), 4.01 (s, 3H), 3.65 (s, 3H), 2.51 (s, 3H), 2.39 (s, 3H); MS for C₂₈H₂₃FN₄O₄S: *m*/*z* 531 (MH+).

**N-[4-[(6,7-Dimethoxy-1,5-naphthyridin-4-yl)oxy]phenyl]-1,2,6-trimethyl-4-oxo-5-thiophen-2-ylpyridine-3-carboxamide (167): In** Step 4 of **Example 32,** Compound **87a** was replaced with thiophen-2-ylboronic acid and in Step 5, Compound **I-1** was replaced with Compound 1-2. ¹H NMR (400MHz, DMSO-*d₆*) δ 10.80 (s, 1H), 8.44 (d, 1H), 7.75 - 7.69 (m, 2H), 7.59 - 7.50 (m, 2H), 7.15 - 7.08 (m, 2H), 7.04 (dd, 1H), 6.85 (dd, 1H), 6.67 (d, 1H), 3.90 (d, 6H), 3.57 (s, 3H), 2.51 (s, 3H), 2.31 (s, 3H); MS for C₂₉H₂₆N₄O₅S: *m*/*z* 543 (MH+).

**N-[4-[(6,7-Dimethoxy-1,5-naphthyridin-4-yl)oxy]-3-fluorophenyl]-1,2,6-trimethyl-4-oxo-5-thiophen-2-ylpyridine-3-carboxamide (168):** In Step 4 of **Example 32,** Compound 87a was replaced with thiophen-2-ylboronic acid. ¹H NMR (400MHz, DMSO-*d₆*) δ 11.08 (s, 1H), 8.53 (d, 1H), 7.95 (dd, 1H), 7.68 - 7.58 (m, 2H), 7.47 (d, 1H), 7.35 (t, 1H), 7.11 (dd, 1H), 6.93 (dd, 1H), 6.78 (d, 1H), 3.98 (d, 6H), 3.64 (s, 3H), 2.39 (s, 3H), 2.08 (d, 3H); MS for C₂₉H₂₅FN₄O₅S: *m*/*z* 561 (MH+).

### Example 33: 4-Ethoxy-5'-fluoro-2-oxo-2H-[1,2'-bipyridine]-3-carboxylic acid (174):

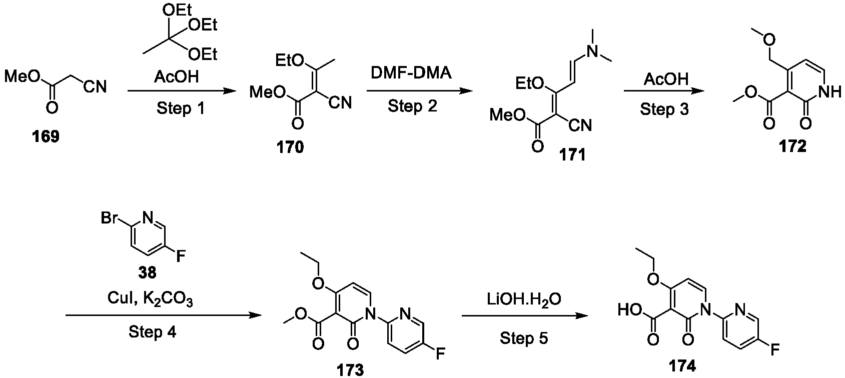

**Step 1: Methyl (Z)-2-cyano-3-ethoxybut-2-enoate (170):** To a solution of Compound 169 (10 g, 101 mmol, 1 eq) in triethyl orthoacetate (30 g, 185 mmol, 1.8 eq) was added AcOH (3.03 g, 50.5 mmol, 0.5 eq). The mixture was stirred at 120 °C for 12 h. The reaction mixture was concentrated under reduced pressure to give crude Compound 170 as a brown oil (17 g) which was used without further purification. MS for C₈H₁₁NO₃: *m*/*z* 169.8 (MH+).

**Step 2: Methyl (2Z,4E)-2-cyano-5-(dimethylamino)-3-ethoxypenta-2,4-dienoate (171):** A solution of crude Compound **170** (17 g, 100 mmol, 1 eq) in DMF-DMA (15.6 g, 131 mmol, 1.3 eq) was stirred at 70 °C for 12 h. The reaction mixture was concentrated under reduced pressure to give crude Compound **171** as a brown solid (20 g, 89% yield) which was used without further purification. MS for C₁₁H₁₆N₂O₃: *m*/*z* 224.8 (MH+).

**Step 3: Methyl 4-(methoxymethyl)-2-oxo-1,2-dihydropyridine-3-carboxylate (172):** A solution of Compound 171 (20 g, 89mmol, 1 eq) in AcOH (50 mL) was stirred at 130 °C for 12 h. The reaction mixture was concentrated under reduced pressure and the pH was adjusted to 8 with aq 20% NaOH. The resulting mixture was extracted with EtOAc (3 x 50 mL) and DCM (5 x 50 mL). The combined organic extracts were washed with aq saturated NaCl (50 mL), dried over anhyd. Na₂SO₄ and concentrated under reduced pressure to give crude Compound **172** as a red solid (5 g, 28% yield). MS for C₉H₁₁NO₄: *m*/*z* 197.9 (MH+).

**Step 4: Methyl 4-ethoxy-5'-fluoro-2-oxo-2H-[1,2'-bipyridine]-3-carboxylate (173):** Compound **173** was synthesized from Compound **172** and Compound **38** in a similar manner to the method used to synthesize Compound **39** from Compound **37** and Compound **38** in step 1 of **Example 10.** MS for C₁₄H₁₃FN₂O₄: *m*/*z* 293 (MH⁺).

**Step 5: 4-Ethoxy-5'-fluoro-2-oxo-2H-[1,2'-bipyridine]-3-carboxylic acid (174):** Compound **174** was made from Compound **173** using the same method that was used to convert Compound **39** to Compound **40** in Step 2 of **Example 10.** MS for C₁₃H₁₁FN₂O₄: *m*/*z* 279 (MH⁺).

### Example 34: 5-(5-Fluoropyridin-2-yl)-4-methoxy-6-methylpyridazine-3-carboxylic acid (179)

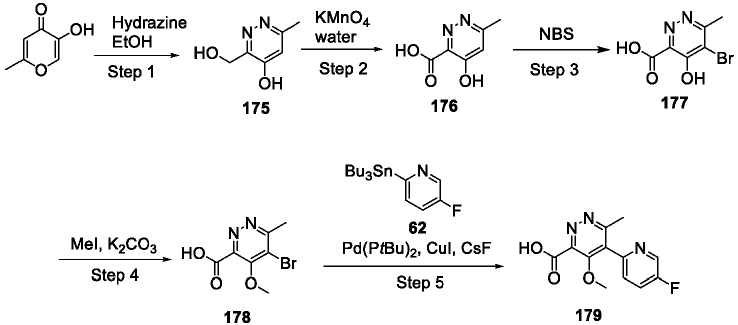

**Step 1: 3-(Hydroxymethyl)-6-methylpyridazin-4-ol (175):** To a solution of 5-hydroxy-2-methyl-4H-pyran-4-one (2.5 g, 19.8 mmol) in EtOH (80 mL) was added hydrazine (4 mL, 60% in water). The resulting mixture was heated to reflux for 90 min. The reaction mixture was allowed to cool to room temperature. The resulting precipitate was filtered and allowed to dry in the open air to afford Compound **175** as a white powder (1.4 g, 51% yield). MS for C₆H₈N₂O₂: *m*/*z* 141 (MH+).

**Step 2: 4-Hydroxy-6-methylpyridazine-3-carboxylic acid (176):** To a solution of Compound **175** (1.4 g, 10 mmol) in water (56 mL) at 75 °C was added KMnO₄ (17.2 mmol, 1.8 eq) in water (84 mL) dropwise over 20 min. The mixture was allowed to cool to room temperature and filtered through a Celite pad. The solvent was partially removed, and the resulting mixture was acidified using 6 M HCl to pH 2. The solution was chilled to 0 °C along with scraping the side of the flask to facilitate precipitation. The resulting solid was filtered and allowed to dry in the open air to afford Compound **176** as a white powder (912 mg, 59% yield). MS for C₆H₆N₂O₃: *m*/*z* 155 (MH+).

**Step 3: 5-Bromo-4-hydroxy-6-methylpyridazine-3-carboxylic acid (177):** Compound **177** was synthesized from Compound **176** using a similar method to that used to convert Compound **60** to Compound **61** in **Example 15.** MS for C₆H₅BrN₂O₃: *m*/*z* 233 (MH+).

**Step 4: 5-Bromo-4-methoxy-6-methylpyridazine-3-carboxylic acid (178):** To a 40 mL vial equipped with a magnetic stir bar and a pressure relief septum was added Compound **177** (1.31 g, 5.6 mmol, 1.0 eq) in DMF (10 mL) and water (10 mL). Cesium carbonate (4.0 g, 12 mmol, 2.2 eq) was added portionwise at room temperature, followed by iodomethane (2 mL, 32.1 mmol, 5.7 eq). The reaction was then heated to 60 °C for 3 h. Aq 10% NaOH (10 mL) was then added, and the reaction heated to 60 °C for 2 h. The reaction was then acidified to pH = 4 with aq 6 M HCl, and the resulting precipitate was collected by vacuum filtration to yield a mixture of Compound **178** and 5-bromo-1,6-dimethyl-4-oxo-pyridazine-3-carboxylic acid, which was carried forward without further purification.

**Step 5: 5-(5-Fluoropyridin-2-yl)-4-methoxy-6-methylpyridazine-3-carboxylic acid (179):** To a 20-mL vial equipped with a magnetic stir bar and a pressure relief septum was added the crude mixture from Step 4 (300 mg, 1.2 mmol), Compound **62** (1.50 g, 3.9 mmol, 3.2 eq), and cesium fluoride (500 mg, 3.29 mmol, 2.7 eq) in DMF (5 mL). Copper iodide (30 mg, 0.16 mmol, 0.13 eq) and palladium tri-tert-butylphosphane (80 mg, 0.16 mmol, 0.13 eq) were then added, and the reaction vial was purged with N₂ and sealed. The resulting solution was heated to 85 °C overnight. The resulting mixture was concentrated, adsorbed onto Celite and purified by silica gel column chromatography (0 to 10% MeOH in DCM), followed by further purification by prep HPLC to give Compound 179 as an off-white, foamy solid (25 mg, 7.8% yield over two steps). ¹H NMR (400 MHz, CDCl₃) δ 8.60 (d, 1H), 7.68 (dd, 1H), 7.59 (td, 1H), 4.23 (s, 3H), 2.52 (s, 3H).

### Example 35: 5'-Fluoro-4,6-dimethyl-2-oxo-2H-[1,2'-bipyridine]-3-carboxylic acid (182)

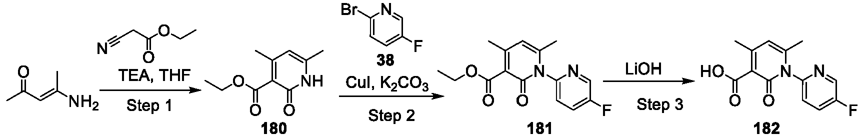

**Step 1: Ethyl 4,6-dimethyl-2-oxo-1,2-dihydropyridine-3-carboxylate (180):** A mixture of ethyl 2-cyanoacetate (1.71 g, 15.1 mmol) and TEA (1.53 g, 15.1 mmol) in THF (15 mL) at 25 °C was added dropwise to a solution of 4-aminopent-3-en-2-one (1.5 g, 15.1 mmol) in THF (15 mL). The mixture was stirred at 65 °C for 36 h. After cooling to room temperature, water (50 mL) and DCM (50 mL) were added. The phases were separated, and the aqueous phase further extracted with DCM (3 x 20 mL). The combined organic extracts were concentrated under reduced pressure and the residue was purified by column chromatography to give Compound 180 as a yellow solid (900 mg, 30.5%). ¹H NMR (400 MHz, CDCl₃) δ 12.57 (br s, 1H), 5.94 (s, 1H), 4.39 (q, 2H), 2.31 (s, 3H), 2.24 (s, 3H), 1.38 (t, 3H).

**Step 2: Ethyl 5'-fluoro-4,6-dimethyl-2-oxo-2H-[1,2'-bipyridine]-3-carboxylate (181):** Compound **181** was made from Compound **180** in a similar manner to the way Compound **39** was made from Compound **37** in Step 1 of **Example 10.** ¹H NMR: (400MHz, CDCl₃) δ 8.48 (d, 1H), 7.65-7.55 (m, 1H), 7.38 (dd, 1H), 6.01 (s, 1H), 4.36 (q, 2H), 2.27 (s, 3H), 1.99 (s, 3H), 1.36 (t, 3H); MS for C₁₅H₁₅FN₂O₃: m/z 291.1 (MH⁺).

**Step 3: 5'-Fluoro-4,6-dimethyl-2-oxo-2H-[1,2'-bipyridine]-3-carboxylic acid (182):** Compound **182** was made from Compound **181** in a similar manner to the way Compound **40** was made from Compound **39** in Step 2 of **Example 10.** MS for C₁₃H₁₁FN₂O₃: m/z 262.1 (MH⁺).

### Example 36: 4-(5-Fluoropyridin-2-yl)-5-methyl-3-oxo-3,4-dihydropyrazine-2-carboxylic acid (189)

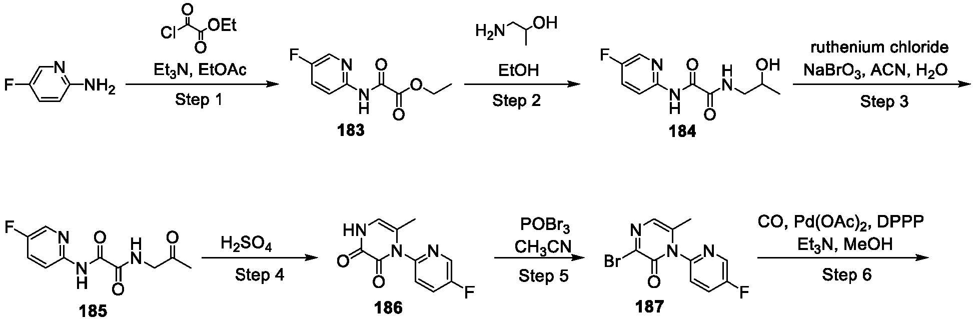

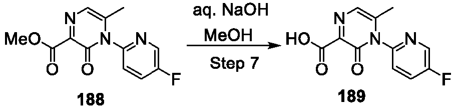

**Step 1: Ethyl 2-((5-fluoropyridin-2-yl)amino)-2-oxoacetate (183):** To a solution of 5-fluoropyridin-2-amine (3 g, 27 mmol) and Et₃N (4.06 g, 40 mmol) in EtOAc (40 mL) was added ethyl 2-chloro-2-oxo-acetate (4.38 g, 32 mmol) at 0 °C. The yellow suspension was stirred at room temperature for 15 h. The reaction mixture was quenched with water (100 mL) and extracted with EtOAc (3 x 50 mL). The combined organic extracts were dried over anhyd. Na₂SO₄ and concentrated under reduced pressure. The residue was purified by flash silica gel chromatography to give Compound **183** as a white solid (2.2 g, 39% yield). ¹H NMR: (400MHz, CDCl₃) δ 9.41 (br s, 1H), 8.32 - 8.25 (m, 1H), 8.22 (d, 1H), 7.55 - 7.47 (m, 1H), 4.45 (q, 2H), 1.45 (t, 3H); MS for C₉H₉FN₂O₃: m/z 213 (MH⁺).

**Step 2: N1-(5-Fluoropyridin-2-yl)-N2-(2-hydroxypropyl)oxalamide (184):** To a mixture of Compound **183** (0.6 g, 2.8 mmol) in EtOH (10 mL) was added 1-aminopropan-2-ol (234 mg, 3.1 mmol). The mixture was stirred at 80 °C for 1 h. The reaction mixture was concentrated under reduced pressure. To the resulting residue was added with EtOH (5 mL) and petroleum ether (100 mL). The mixture was stirred for 0.5 h and filtered. The filter cake was washed with petroleum ether (2 x 15 mL) and dried to give Compound **184** as a white solid (660 mg, 97% yield) which was used without further purification. ¹H NMR: (400MHz, CDCl₃) δ 9.79 (br s, 1H), 8.27 - 8.21 (m, 2H), 7.97 - 7.80 (m, 1H), 7.54-7.44 (m, 1H), 4.10 - 3.97 (m, 1H), 3.63-3.53 (m, 1H), 3.33-3.23 (m 1H), 2.11 (br s, 1H), 1.27 (d, 3H); MS for C₁₀H₁₂FN₃O₃: m/z 242 (MH⁺).

**Step 3: N1-(5-Fluoropyridin-2-yl)-N2-(2-oxopropyl)oxalamide (185):** To a mixture of Compound **184** (660 mg, 2.7 mmol) in ACN (10 mL) was added a solution of RuCl₃ (8.5 mg, 0.041 mmol) in water (1 mL) followed by a solution of sodium bromate (454 mg, 3.0 mmol) in water (2 mL). The resulting mixture was stirred at room temperature for 1 h. The reaction mixture was concentrated under reduced pressure. To the residue was added water (80 mL) and the resulting mixture stirred for 0.5 h. The resulting solid was filtered, and the filter cake was washed with water (2 x 20 mL) and dried under vacuum to give Compound **185** as an off-white solid (0.55 g, 84% yield) which was used without further purification.

**Step 4: 1-(5-Fluoropyridin-2-yl)-6-methyl-1,4-dihydropyrazine-2,3-dione (186):** To H₂SO₄ (3 mL) was added Compound **185** (0.3 g, 1.25 mmol) at 55 °C. The resulting solution was stirred at 55 °C for 2 h. The reaction was slowly added to ice-water. The mixture was neutralized with aq 3 N LiOH to pH = 6 and concentrated under reduced pressure. To the residue was added 10:1 DCM:MeOH (100 mL) and the resulting mixture stirred for 5 h. The mixture was filtered, and the filter cake was washed with DCM (50 mL). The filtrate was concentrated under reduced pressure. The resulting residue was purified by flash silica gel chromatography to give Compound 186 as a yellow solid (250 mg, 90% yield) as yellow solid. MS for C₁₀H₈FN₃O₂: m/z 221.9 (MH⁺).

**Step 5: 3-Bromo-1-(5-fluoropyridin-2-yl)-6-methylpyrazin-2(1H)-one (187):** To a mixture of Compound **186** (250 mg, 1.13 mmol) in ACN (3 mL) was added POBr₃ (356 mg, 1.2 mmol). The resulting suspension was stirred at 65 °C for 6 h. The reaction mixture was added to aq saturated NaHCO₃ (100 mL). The resulting mixture was stirred for 10 min and then extracted with EtOAc (3 x 50 mL). The combined organic extracts were dried over anhyd. Na₂SO₄ and concentrated under reduced pressure. The resulting residue was purified by flash silica gel chromatography to give Compound **187** as a white solid (150 mg, 47% yield). MS for C₁₀H₇BrFN₃O: m/z 285.9 (MH⁺).

**Step 6: Methyl 4-(5-fluoropyridin-2-yl)-5-methyl-3-oxo-3,4-dihydropyrazine-2-carboxylate (188):** To a mixture of Compound **187** (150 mg, 0.53 mmol) in MeOH (10 mL) was added DPPP (43.6 mg, 0.106 mmol), Et₃N (107 mg, 1.1 mmol) and Pd(OAc)₂ (11.9 mg, 0.053 mmol). The mixture was stirred at 70 °C for 30 h under CO (50 PSI). The reaction mixture was concentrated under reduced pressure. The resulting residue was purified by flash chromatography over silica gel to give Compound **188** as a yellow solid (110 mg, 79% yield). MS for C₁₂H₁₀FN₃O₃: m/z 264 (MH⁺).

**Step 7: 4-(5-Fluoropyridin-2-yl)-5-methyl-3-oxo-3,4-dihydropyrazine-2-carboxylic acid (189):** To a solution of Compound **188** (110 mg, 0.42 mmol, 1 eq) in MeOH (2 mL) was added water (0.5 mL) and NaOH (50 mg, 1.25 mmol). The mixture was stirred at room temperature for 1 h. The reaction mixture was diluted with water (10 mL) and then concentrated under reduced pressure to remove MeOH. The resulting aqueous mixture was acidified with aq 1 N HCl to pH = 2-3 and then extracted with EtOAc (3 x 30 mL) and DCM (3 x 30 mL). The combined organic extracts were dried over anhyd. Na₂SO₄ and concentrated under reduced pressure to Compound **189** as a yellow solid (100 mg, 96% yield) which was used without further purification.

### Example 37: 4-(5-Fluoropyridin-2-yl)-3-oxo-3,4-dihydropyrazine-2-carboxylic acid (194)

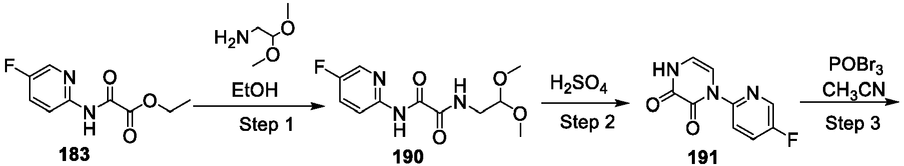

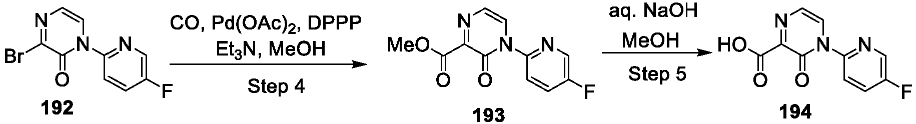

**Step 1: N1-(2,2-Dimethoxyethyl)-N2-(5-fluoropyridin-2-yl)oxalamide (190):** Compound **190** was synthesized from Compound **183** in a similar way to the method used to synthesize Compound **184** from Compound **183** in Step 2 of **Example 36,** replacing 1-amino-2-propanol with 2,2-dimethoxyethanamine. MS for C₁₁H₁₄FN₃O₄: *m*/*z* 271.8 (MH⁺).

**Step 2: 1-(5-Fluoropyridin-2-yl)-1,4-dihydropyrazine-2,3-dione (191):** Compound **191** was synthesized from Compound **190** in a similar way to the method used to synthesize Compound 186 from Compound **185** in Step 4 of **Example 36** MS for C₉H₆FN₃O₂: *m*/*z* 207.9 (MH⁺).

**Step 3: 3-Bromo-1-(5-fluoropyridin-2-yl)pyrazin-2(1H)-one (192):** Compound **192** was synthesized from Compound **191** in a similar way to the method used to synthesize Compound **187** from Compound **186** in Step 5 of **Example 36.** ¹H NMR: (400 MHz, DMSO-*d₆*) δ 8.66 (d, 1H), 8.07-8.00 (m, 1H), 7.99-7.95 (m, 1H), 7.94 (d, 1H), 7.30 (d, 1H); MS for C₉H₅BrFN₃O: m/z 271.8 (MH⁺).

**Step 4: Methyl 4-(5-fluoropyridin-2-yl)-3-oxo-3,4-dihydropyrazine-2-carboxylate (193):** Compound **193** was synthesized from Compound **192** in a similar way to the method used to synthesize Compound **188** from Compound **187** in Step 6 of **Example 36.** MS for C₁₁H₈FN₃O₃: m/z 249.9 (MH⁺).

**Step 5: 4-(5-Fluoropyridin-2-yl)-3-oxo-3,4-dihydropyrazine-2-carboxylic acid (194):** Compound **194** was synthesized from Compound **193** in a similar way to the method used to synthesize Compound **189** from Compound **188** in Step 7 of **Example 36.** MS for C₁₀H₆FN₃O₃: *m*/*z* 235.9 (MH+).

### Example 38: N-[4-[(6,7-Dimethoxy-1,5-naphthyridin-4-yl)oxy]-3-fluorophenyl]-4-hydroxy-2-(methoxymethyl)-6-methyl-5-pyridin-2-ylpyridine-3-carboxamide (195)

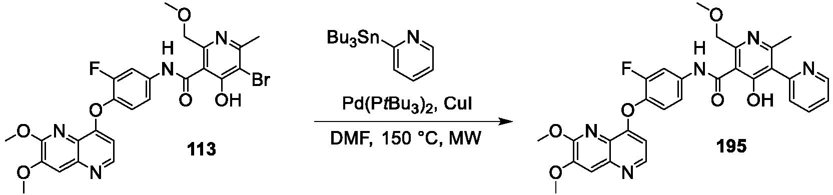

**N-[4-[(6,7-Dimethoxy-1,5-naphthyridin-4-yl)oxy]-3-fluorophenyl]-4-hydroxy-2-(methoxymethyl)-6-methyl-5-pyridin-2-ylpyridine-3-carboxamide (195):** To a 2 mL microwave vial equipped with a magnetic stir bar was added Compound 113 (100 mg, 0.17 mmol, 1 eq), tributyl(2-pyridyl)stannane (150 mg, 0.41 mmol, 2.34 eq) and DMF (1.5 mL). The solution was degassed with Argon for 30 min, followed by the addition of Pd(P*t*Bu₃)₂ (20 mg, 0.04 mmol, 0.22 eq) and copper iodide (8 mg, 0.04 mmol, 0.24 eq). The reaction vial was sealed, and the resulting heterogeneous mixture was heated to 150 °C for two h. The reaction mixture was filtered over Celite, concentrated, and purified by prep HPLC to yield a white solid (17.3 mg, 17% yield). ¹H NMR (400 MHz, DMSO-*d₆*) δ 13.23 (s, 1H), 11.49 (s, 1H), 8.65 (d, 1H), 8.54 (dd, 1H), 8.02 - 7.93 (m, 1H), 7.86 (t, 1H), 7.65 (s, 1H), 7.44 (d, 1H), 7.40 - 7.25 (m, 3H), 6.81 (d, 1H), 5.05 (s, 2H), 3.97 (s, 3H), 3.95 (s, 3H), 3.50 (s, 3H), 2.24 (s, 3H); MS for C₃₀H₂₆FN₅O₆: *m*/*z* 572.2 (MH+).

The following compounds were made in the same manner as Compound 195 in **Example 38:**

**N-[4-[(6,7-Dimethoxy-1,5-naphthyridin-4-yl)oxy]-3-fluorophenyl]-4-hydroxy-2-(methoxymethyl)-6-methyl-5-(3-methylpyridin-2-yl)pyridine-3-carboxamide (196):** ¹H NMR (400 MHz, CD₃OD) δ 8.53 - 8.45 (m, 2H), 7.95 (dd, 1H), 7.87 - 7.80 (m, 1H), 7.52 (s, 1H), 7.43 (dd, 1H), 7.32 (ddd, 1H), 7.25 (t, 1H), 6.82 (dd, 1H), 5.20 (d, 2H), 4.05 (s, 3H), 4.03 (s, 3H), 3.65 (s, 3H), 2.24 (s, 3H), 2.18 (s, 3H); MS for C₃₁H₂₈FN₅O₆: *m*/*z* 586.1 (MH+).

**N-[4-[(6,7-Dimethoxy-1,5-naphthyridin-4-yl)oxy]-3-fluorophenyl]-4-hydroxy-2-(methoxymethyl)-6-methyl-5-(6-methylpyridin-2-yl)pyridine-3-carboxamide (197):** ¹H NMR (400 MHz, DMSO-*d₆*) δ 8.47 (d, 1H), 8.34 (s, 1H), 7.92 (dd, 1H), 7.64 (t, 1H), 7.58 (s, 1H), 7.32 - 7.17 (m, 2H), 7.11 (dd, 2H), 6.72 (d, 1H), 6.63 (s, 1H), 4.94 (s, 2H), 3.90 (s, 3H), 3.88 (s, 3H), 3.39 (s, 3H), 2.42 (s, 3H), 2.10 (s, 3H); MS for C₃₁H₂₈FN₅O₆: *m*/*z* 586.1 (MH+).

**N-[4-[(6,7-Dimethoxy-1,5-naphthyridin-4-yl)oxy]-3-fluorophenyl]-4-hydroxy-2-(methoxymethyl)-6-methyl-5-(2-methylpyridin-4-yl)pyridine-3-carboxamide (198):** ¹H NMR (400 MHz, DMSO-*d₆*) δ 13.09 (s, 1H), 11.48 (s, 1H), 8.47 (d, 1H), 8.42 (d, 1H), 7.90 (dd, 1H), 7.58 (s, 1H), 7.34 - 7.27 (m, 1H), 7.23 (t, 1H), 7.08 (d, 1H), 7.01 (dd, 1H), 6.73 (d, 1H), 4.96 (s, 2H), 3.90 (s, 3H), 3.87 (s, 3H), 3.42 (s, 3H), 2.43 (s, 3H), 2.17 (s, 3H); MS for C₃₁H₂₈FN₅O₆: *m*/*z* 586.1 (MH+).

### Example 39: N-[4-[(6,7-Dimethoxy-1,5-naphthyridin-4-yl)oxy]-3-fluorophenyl]-5-(5-fluoro-3-methylpyridin-2-yl)-4-hydroxy-2-methylpyridine-3-carboxamide (200)

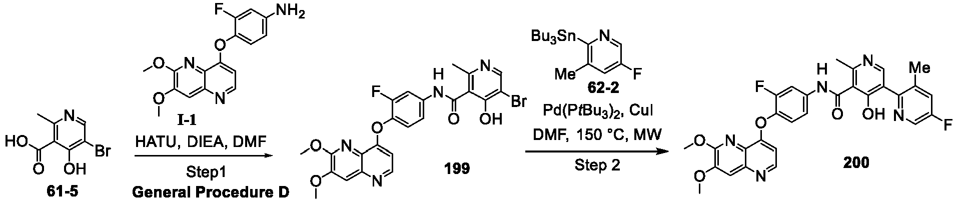

**Step 1: 5-Bromo-N-(4-((6,7-dimethoxy-1,5-naphthyridin-4-yl)oxy)-3-fluorophenyl)-4-hydroxy-2-methylnicotinamide (199):** Compound **199** was synthesized from Compound 61-5 and Intermediate **I-1** using **General Procedure D.** ¹H NMR (400 MHz, DMSO- *d₆*) δ 14.80 (s, 1H), 8.46 (d, 1H), 7.97 (d, 1H), 7.89 (s, 1H), 7.58 (s, 1H), 7.22 (d, 2H), 6.71 (d, 1H), 3.92 (s, 3H), 3.90 (s, 3H), 2.52 (s, 3H); MS for C₂₃H₁₈BrFN₄O₅: *m*/*z* 531.0 (MH+).

**Step 2: N-[4-[(6,7-Dimethoxy-1,5-naphthyridin-4-yl)oxy]-3-fluorophenyl]-5-(5-fluoro-3-methylpyridin-2-yl)-4-hydroxy-2-methylpyridine-3-carboxamide** (200): Compound **200** was made from Compound 199 and Compound **62-2** in the same manner that Compound 195 was made from Compound **113** and tributyl(2-pyridyl)stannane in **Example 38.** ¹H NMR (400 MHz, DMSO-*d₆*) *δ* 13.35 (s, 1H), 8.46 (d, 1H), 8.35 (d, 1H), 7.94 (dd, 1H), 7.77 (s, 1H), 7.58 (q, 2H), 7.34 - 7.27 (m, 1H), 7.23 (t, 1H), 6.72 (dd, 1H), 3.90 (s, 6H), 2.64 (s, 3H), 2.16 (s, 3H); MS for C₂₉H₂₃F₂N₅O₅: *m*/*z* 560.2 (MH+).

### Example 40: 2'-(2-(2-Ethoxyethoxy)ethoxy)-6-methyl-2-oxo-2H-[1,4'-bipyridine]-3-carboxylic acid (201)

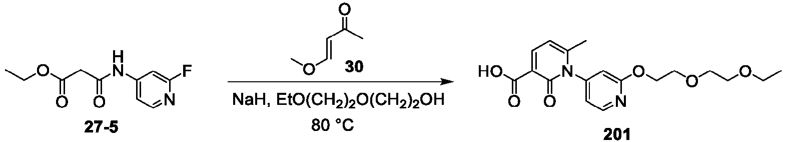

**2'-(2-(2-Ethoxyethoxy)ethoxy)-6-methyl-2-oxo-2H-[1,4'-bipyridine]-3-carboxylic acid (201):** Compound **201** was synthesized from Compound **27-5** using a method similar to that of **Example 6.** ¹H NMR (400 MHz, DMSO-*d₆*) δ 13.99 (s, 1H), 8.50 - 8.32 (m, 2H), 7.15 (dd, 1H), 7.09 (dd, 1H), 6.81 (dd, 1H), 4.53 - 4.35 (m, 2H), 3.77 (t, 2H), 3.61 - 3.55 (m, 2H), 3.52 - 3.46 (m, 2H), 3.43 (q, 2H), 2.17 - 2.11 (m, 3H), 1.09 (t, 3H).

### Example 41: 2'-Isopropoxy-6-methyl-2-oxo-2H-[1,4'-bipyridine]-3-carboxylic acid (202)

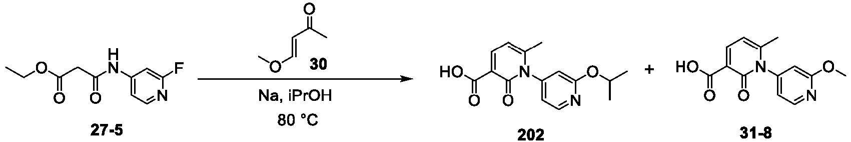

**2'-Isopropoxy-6-methyl-2-oxo-2H-[1,4'-bipyridine]-3-carboxylic acid (202) and 2'-Methoxy-6-methyl-2-oxo-2H-[1,4'-bipyridine]-3-carboxylic acid (31-8):** To a 100 mL round bottom flask equipped with a magnetic stir bar and a pressure relief septum was added isopropanol (10 mL), and the flask was cooled to 0 °C. Sodium (200 mg, 8.7 mmol) was added in small portions until completely dissolved with vigorous stirring. Compound **27-5** (520 mg, 2.3 mmol, 1.0 eq) was added in a single portion, followed by Compound **30** (300 mg, 3.0 mmol, 1.3 eq). The resulting mixture was then heated to 80 °C for 3 h. After cooling to room temperature, the reaction mixture was acidified to pH = 3 with aq 6 M HCl. The resulting suspension was filtered to yield a yellow solid, which was collected by vacuum filtration and purified by chromatography over silica gel to give Compound **202** and Compound 31-8 (72% yield total). Compound **202:** ¹H NMR (400 MHz, CDCl₃) δ 13.67 (s, 1H), 8.54 (dd, 1H), 8.42 (dt, 1H), 6.77 (dt, 1H), 6.68 - 6.40 (m, 2H), 5.43 - 5.28 (m, 1H), 2.23 (d, 3H), 1.42 (ddd, 6H); MS for C₁₅H₁₆N₂O₄: *m*/*z* 289.0 (MH+). Compound **31-8:** ¹H NMR (400 MHz, CDCl₃) δ 13.67 (s, 1H), 8.54 (d, 1H), 8.45 (dd, 1H), 6.80 (dd, 1H), 6.68 (dd, 1H), 6.57 (dd, 1H), 4.05 (s, 3H), 2.22 (s, 3H).

### Example 42: 2-Oxo-2H-[1,4'-bipyridine]-3-carboxylic acid (204)

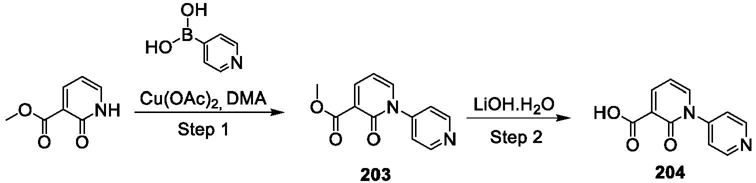

**Step 1: Methyl 2-oxo-2H-[1,4'-bipyridine]-3-carboxylate (203):** A mixture of methyl 2-oxo-1H-pyridine-3-carboxylate (1.00 g, 6.53 mmol), 4-pyridylboronic acid (2.41 g, 19.6 mmol) and Cu(OAc)₂ (3.56 g, 19.6 mmol) in DMA (10 mL) was stirred at 90 °C under an atmosphere of O₂ for 0.5 h, and then cooled to room temperature. Ammonium hydroxide (50 mL) was added and the resulting mixture was extracted with EtOAc (3 x 20 mL). The combined organic extracts were washed with water (20 mL), aq saturated NaCl (3 x 20 mL) and dried over anhyd. Na₂SO₄ and concentrated under reduced pressure. The resulting residue was purified by column chromatography to Compound **203** as a white solid (150 mg, 9.9% yield). ¹H NMR (400 MHz, DMSO-*d*₆) δ 8.77 - 8.74 (m, 2H), 8.15 (dd, 1H), 8.00 (dd, 1H), 7.57 - 7.54 (m, 2H), 6.48 (t, 1H), 3.76 (s, 3H); MS for C₁₂H₁₀N₂O₃: *m*/*z* 231 (MH+).

**Step 2: 2-Oxo-2H-[1,4'-bipyridine]-3-carboxylic acid (204):** Compound **204** was synthesized from Compound **203** in the same manner Compound **40** was made from Compound **39** in Step 2 of **Example 10.** ¹H NMR (400 MHz, DMSO-*d₆*) δ 13.94 (br s, 1H), 8.84 - 8.79 (m, 2H), 8.49 (dd, 1H), 8.23 (dd, 1H), 7.68 - 7.64 (m, 2H), 6.82 (t, 1H); MS for C₁₁H₈N₂O₃: *m*/*z* 216.8 (MH+).

The following compound was made in the same manner as Compound **204** in **Example 42:**

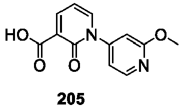

**2'-Methoxy-2-oxo-2H-[1,4'-bipyridine]-3-carboxylic acid (205):** In Step 1, the 4-pyridylboronic acid was replaced with (2-methoxypyridin-4-yl)boronic acid. MS for C₁₂H₁₀N₂O₄: *m*/*z* 246.9 (MH+).

### Example 43: 3-Carboxy-6-methyl-2-oxo-2H-[1,4'-bipyridine]1'-oxide (206)

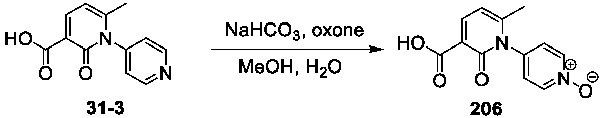

**3-Carboxy-6-methyl-2-oxo-2H-[1,4'-bipyridine] 1'-oxide (206):** A mixture of Compound 31-3 (200 mg, 0.87 mmol) in MeOH (3 mL), NaHCO₃ (146 mg, 1.74 mmol), oxone (401 mg, 0.65 mmol) and water (3 mL) was stirred at 25 °C for 12 h. Aq Na₂SO₃ was added until starch potassium iodide paper tested negative. The mixture was concentrated and the pH adjusted to 1 with aq 2 M HCl. The mixture was extracted with 5:1 DCM:MeOH (2 x 15 mL). The combined organic extracts were dried over anhyd. Na₂SO₄ and concentrated under vacuum. The resulting residue was purified by prep-TLC to give Compound **206** as a white solid (94 mg, 44% yield). ¹H NMR: (400MHz, DMSO-*d₆*) δ 13.98 (s, 1H), 8.54 - 8.44 (m, 3H), 7.70 - 7.63 (m, 2H), 6.86 (d, 1H), 2.24 (s, 3H); MS for C₁₂H₁₀N₂O₄: *m*/*z* 247 (MH+).

The following compound was made in the same manner as Compound **206** in **Example 43:**

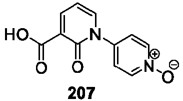

**3-Carboxy-2-oxo-2H-[1,4'-bipyridine] 1'-oxide (207):** Compound 31-3 was replaced with Compound **204.** ¹H NMR (400 MHz, DMSO-*d₆*) δ 13.91 (br s, 1H), 8.47 (dd, 1H), 8.44 - 8.37 (m, 2H), 8.22 (dd, 1H), 7.68 (d, 2H), 6.80 (t, 1H); MS for C₁₁H₈N₂O₄: *m*/*z* 232.9 (MH+).

### Example 44: 2'-Hydroxy-6-methyl-2-oxo-2H-[1,4'-bipyridine]-3-carboxylic acid (208)

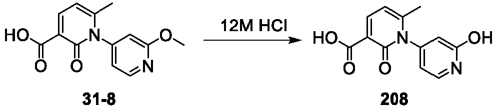

**2'-Hydroxy-6-methyl-2-oxo-2H-[1,4'-bipyridine]-3-carboxylic acid (208):** A mixture of Compound **31-8** (50 mg, 0.19 mmol) and HCl (12 M, 2.33 mL) was stirred at 110 °C for 4 h. The mixture was concentrated under vacuum to give Compound **208** as a yellow solid (47 mg, 99% yield). ¹H NMR: (400 MHz, DMSO-*d₆*) δ 7.88 (d, 1H), 7.69 (d, 1H), 6.20 (d, 1H), 5.77 - 5.71 (m, 1H), 5.71 - 5.63 (m, 1H), 2.06 (s, 3H); MS for C₁₂H₁₀N₂O₄: *m*/*z* 246.9 (MH+).

The following compound was made in the same manner as Compound **208** in **Example 44:**

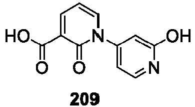

**2'-Hydroxy-2-oxo-2H-[1,4'-bipyridine]-3-carboxylic acid (209):** Compound **31-8** was replaced with Compound 205. ¹H NMR (400 MHz, DMSO-*d₆*) δ 8.45 (br, d, 1H), 8.17 (br, d, 1H), 7.57 (br, d, 1H), 6.77 (br, t, 1H), 6.55 (s, 1H), 6.40 (br, d, 1H); MS for C₁₁H₈N₂O₄: *m*/*z* 232.9 (MH+).

### Example 45: 2'-Fluoro-6-methyl-2-oxo-2H-[1,4'-bipyridine]-3-carboxylic acid (211)

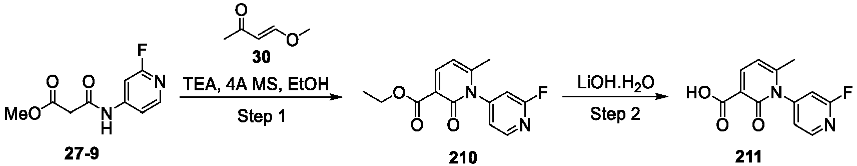

**Step 1: Ethyl 2'-fluoro-6-methyl-2-oxo-2H-[1,4'-bipyridine]-3-carboxylate (210):** To a solution of Compound **30** (354 mg, 3.5 mmol) and Compound 27-9 (500 mg, 2.4 mmol) in EtOH (5 mL) was added TEA (954 mg, 9.4 mmol) and 4A molecular sieves (50 mg, 2.4 mmol) under N₂. The resulting mixture was stirred at 90 °C for 36 h. The mixture was filtered, and the filtrate was concentrated under vacuum. The resulting residue was purified by column chromatography to give Compound **210** as a yellow solid (200 mg, 31% yield). ¹H NMR: (400 MHz, DMSO-*d₆*) δ 8.46 (d, 1H), 8.10 (d, 1H), 7.45 (d, 1H), 7.43 (s, 1H), 6.43 (d, 1H), 4.20 (q, 2H), 2.02 (s, 3H), 1.25 - 1.22 (m, 3H); MS for C₁₄H₁₃FN₂O₃: *m*/*z* 276.9 (MH+).

**Step 2: 2'-Fluoro-6-methyl-2-oxo-2H-[1,4'-bipyridine]-3-carboxylic acid (211):** Compound **211** was synthesized from Compound **210** in the same manner Compound **40** was made from Compound **39** in Step 2 of **Example 10.** ¹H NMR: (400 MHz, DMSO-*d₆*) δ 13.77 (br s, 1H), 8.53 (d, 1H), 8.43 (d, 1H), 7.59 (d, 1H), 7.55 (s, 1H), 6.81 (d, 1H), 2.14 (s, 3H); MS for C₁₂H₉FN₂O₃: *m*/*z* 249 (MH+).

### Example 46: N-[4-[(6,7-Dimethoxy-1,5-naphthyridin-4-yl)oxy]-3-fluorophenyl]-1-(5-ethoxypyridin-2-yl)-6-methyl-2-oxopyridine-3-carboxamide (212)

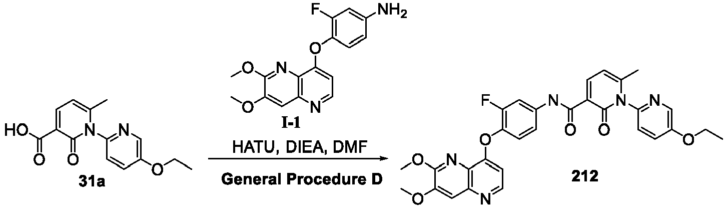

**N-[4-[(6,7-Dimethoxy-1,5-naphthyridin-4-yl)oxy]-3-fluorophenyl]-1-(5-ethoxypyridin-2-yl)-6-methyl-2-oxopyridine-3-carboxamide (212):** Compound **212** was synthesized from Compound 31a and Intermediate **I-1** using **General Procedure D.** ¹H NMR (400MHz, DMSO-*d₆*) δ 11.97 (s, 1H), 8.63 - 8.41 (m, 2H), 8.35 (d, 1H), 8.00 (dt, 1H), 7.76 - 7.61 (m, 2H), 7.57 (dd, 1H), 7.44 (d, 1H), 7.35 - 7.21 (m, 1H), 6.82 (d, 1H), 6.70 (d, 1H), 4.33 - 4.13 (q, 2H), 3.97 (s, 3H), 3.93 (s, 3H), 2.06 (s, 3H), 1.40 (t, 3H); MS for C₃₀H₂₆FN₅O₆: *m*/*z* 572 (MH+).

The following compounds were also made using **General Procedure D** in the same fashion that Compound **212** was made from Compound **31a** and Intermediate **I-1** in **Example 46:**

**1-(5-Ethoxypyridin-2-yl)-N-[3-fluoro-4-[(7-methoxy-1,5-naphthyridin-4-yl)oxy]phenyl]-6-methyl-2-oxopyridine-3-carboxamide (213):** Intermediate **I-1** was replaced with Intermediate **I-3**. ¹H NMR (400MHz, DMSO-*d₆*) δ 12.01 (s, 1H), 8.80 - 8.63 (m, 2H), 8.52 (dd, 1H), 8.35 (d, 1H), 8.04 (dt, 1H), 7.80 (dd, 1H), 7.68 (dd, 1H), 7.58 (dd, 1H), 7.50 (d, 1H), 7.43 - 7.31 (m, 1H), 6.72 (dd, 2H), 4.22 (q, 3H), 4.00 (s, 3H), 2.06 (s, 3H), 1.40 (t, 3H); MS for C₂₉H₂₄FN₅O₅: m/z 542 (MH+).

**N-[4-[(6,7-Dimethoxy-1,5-naphthyridin-4-yl)oxy]-3-fluorophenyl]-5-(5-fluoropyridin-2-yl)-4-hydroxy-2-(methoxymethyl)-6-methylpyridine-3-carboxamid (214):** Compound **31a** was replaced with Compound **78-4.** ¹H NMR (400MHz, DMSO-*d₆*) δ 13.28 (s, 1H), 8.57 (d, 1H), 8.47 (d, 1H), 7.91 (dd, 1H), 7.72 (td, 1H), 7.58 (s, 1H), 7.46 (dd, J = 8.8, 4.6 Hz, 1H), 7.35 - 7.27 (m, 1H), 7.22 (t, 1H), 6.73 (d, 1H), 4.95 (s, 2H), 3.90 (s, 3H), 3.88 (s, 3H), 3.41 (s, 3H), 2.16 (s, 3H); MS for C₃₀H₂₅F₂N₅O₆: *m*/*z* 590.2 (MH+).

**N-[4-[(6,7-Dimethoxy-1,5-naphthyridin-4-yl)oxy]-3-fluorophenyl]-5-(5-fluoropyridin-2-yl)-4-methoxy-6-methylpyridazine-3-carboxamide (215):** Compound **31a** was replaced with Compound **180.** ¹H NMR (400MHz, DMSO-*d₆*) δ 12.49 (s, 1H), 8.65 (d, 1H), 8.48 (d, 2H), 7.92 (dd, 1H), 7.81 (td, 1H), 7.57 (d, 1H), 7.55 - 7.49 (m, 1H), 7.41 (d, 1H), 7.28 (t, 1H), 6.77 (d, 1H), 4.05 (s, 3H), 3.90 (s, 3H), 3.87 (s, 3H), 2.28 (s, 3H); MS for C₂₈H₂₂F₂N₆O₅: *m*/*z* 561.1 (MH+).

**N-[4-[(6,7-Dimethoxy-1,5-naphthyridin-4-yl)oxy]phenyl]-5-(5-fluoropyridin-2-yl)-4-hydroxy-2,6-dimethylpyridine-3-carboxamide (216):** Compound **31a** was replaced with Compound **78-3** and Intermediate **I-1** was replaced with Intermediate **I-2**. ¹H NMR (400 MHz, DMSO-*d6*) δ 12.92 (s, 1H), 11.92 (s, 1H), 8.63 (s, 1H), 8.53 (d, 1H), 7.81 - 7.71 (m, 3H), 7.63 (s, 1H), 7.57 - 7.52 (m, 1H), 7.15 (d, 2H), 6.77 (d, 1H), 3.96 (d, 6H), 2.74 (s, 3H), 2.17 (s, 3H); MS for C₂₉H₂₄FN₅O₅: m/z 542 (MH+).

**N-[3-Fluoro-4-[(7-methoxy-1,5-naphthyridin-4-yl)oxy]phenyl]-5-(5-fluoropyridin-2-yl)-4-hydroxy-2,6-dimethylpyridine-3-carboxamide (217):** Compound **31a** was replaced with Compound **78-3** and Intermediate **I-1** was replaced with Intermediate **I-3**. ¹H NMR (400MHz, DMSO-*d₆*) δ 13.21 (s, 1H), 12.07 (s, 1H),8.72 (dd, 2H), 8.63 (s, 1H), 8.03 (d, 1H), 7.79 (s, 2H), 7.53 (s, 1H), 7.42 - 7.34 (m, 2H), 6.73 (d, 1H), 4.00 (s, 3H), 2.74 (s, 3H), 2.16 (s, 3H); MS for C₂₈H₂₁F₂N₅O₄: *m*/*z* 530.2 (MH+).

**N-[4-[(6,7-Dimethoxy-1,5-naphthyridin-4-yl)oxy]-3-fluorophenyl]-5-(5-fluoropyridin-2-yl)-4-hydroxy-2,6-dimethylpyridine-3-carboxamide (218):** Compound **31a** was replaced with Compound **78-4.** ¹H NMR (400MHz, DMSO-*d₆*) δ 13.08 (s, 1H), 8.57 (d, 1H), 8.47 (d, 1H), 8.15 (s, 1H), 7.91 (d, 1H), 7.72 (td, 1H), 7.58 (s, 1H), 7.46 (dd, 1H), 7.30 (d, 1H), 7.22 (t, 1H), 6.72 (d, 1H), 3.89 (d, 6H), 2.67 (s, 3H), 2.10 (s, 3H); MS for C₂₉H₂₃F₂N₅O₅: *m*/*z* 560 (MH+).

**N-[4-[(6,7-Dimethoxy-1,5-naphthyridin-4-yl)oxy]-3-fluorophenyl]-4-(5-fluoropyridin-2-yl)-5-methyl-3-oxopyrazine-2-carboxamide (219):** Compound **31a** was replaced with Compound 189. ¹H NMR (400MHz, CDCl₃) δ 11.60 (s, 1H), 8.61 (d, 1H), 8.53 (d, 1H), 7.95 (dd, 1H), 7.84 (s, 1H), 7.82-7.72 (m, 1H), 7.52 (s, 1H), 7.49 - 7.44 (m, 1H), 7.37 (d, 1H), 7.17 (t, 1H), 6.77 (d, 1H), 4.10 (s, 3H), 4.03 (s, 3H), 2.17 (s, 3H); MS for C₂₇H₂₀F₂N₆O₅: 547.1 (MH+).

**N-[4-[(6,7-Dimethoxy-1,5-naphthyridin-4-yl)oxy]-3-fluorophenyl]-1-(3-fluoropyridin-4-yl)-6-methyl-2-oxopyridine-3-carboxamide (220):** Compound **31a** was replaced with Compound **31-2.** ¹H NMR (400MHz, DMSO-*d₆*) δ 11.60 (s, 1H), 8.88 (s, 1H), 8.66 (d, 1H), 8.53 - 8.42 (m, 2H), 7.93 (dd, 1H), 7.75 (t, 1H), 7.58 (s, 1H), 7.41 (dd, 1H), 7.24 (t, 1H), 6.84 - 6.65 (m, 2H), 3.89 (s, 3H), 3.85 (s, 3H), 2.10 (s, 3H); MS for C₂₈H₂₁F₂N₅O₅: *m*/*z* 546.2 (MH+).

**N-[4-[(6,7-Dimethoxy-1,5-naphthyridin-4-yl)oxy]-3-fluorophenyl]-6-methyl-2-oxo-1-pyridin-4-ylpyridine-3-carboxamid (221):** Compound **31a** was replaced with Compound **31-3.** ¹H NMR (400MHz, DMSO-*d₆*) δ 11.82 (s, 1H), 8.78 (d, 2H), 8.46 (dd, 2H), 7.99 - 7.86 (m, 1H), 7.58 (s, 1H), 7.51 (d, 2H), 7.39 (d, 1H), 7.23 (t, 1H), 6.75 (d, 1H), 6.69 (d, 1H), 3.89 (s, 3H), 3.85 (s, 3H), 2.02 (s, 3H); MS for C₂₈H₂₂FN₅O₅: *m*/*z* 528.2 (MH+).

**N-[4-[(6,7-Dimethoxy-1,5-naphthyridin-4-yl)oxy]-3-fluorophenyl]-6-methyl-2-oxo-1-pyridin-3-ylpyridine-3-carboxamide (222):** Compound **31a** was replaced with Compound 65. . ¹H NMR (400MHz, DMSO-*d₆*) δ 11.86 (s, 1H), 8.69 - 8.62 (m, 1H), 8.59 (d, 1H), 8.53 - 8.40 (m, 2H), 7.97 - 7.83 (m, 2H), 7.67 - 7.53 (m, 2H), 7.39 (d, 1H), 7.23 (t, 1H), 6.75 (d, 1H), 6.69 (d, 1H), 3.89 (d, 3H), 3.85 (d, 3H), 2.02 (s, 3H); MS for C₂₈H₂₂FN₅O₅: m/z 528.1 (MH+).

**N-[3-Fluoro-4-[(6-methoxy-1,7-naphthyridin-4-yl)oxy]phenyl]-4-hydroxy-2-methyl-5-pyridin-2-ylpyridine-3-carboxamide (223):** Compound **31a** was replaced with Compound **65** and Intermediate **I-1** was replaced with Compound **24.** ¹H NMR (400MHz, DMSO-*d₆*) δ 12.41 (s, 2H), 9.19 (d, 1H), 8.67 - 8.61 (m, 2H), 8.56 (dt, 1H), 8.50 (s, 1H), 8.07 (dd, 1H), 7.85 (td, 1H), 7.56 - 7.45 (m, 2H), 7.44 (d, 1H), 7.34 (ddd, 1H), 6.78 (dd, 1H), 4.03 (s, 3H), 2.61 (s, 3H); MS for C₂₇H₂₀FN₅O₄: *m*/*z* 498.0 (MH+).

**N-[4-[(6,7-Dimethoxy-1,5-naphthyridin-4-yl)oxy]-3-fluorophenyl]-1-[2-[2-(2-ethoxyethoxy)ethoxy]pyridin-4-yl]-6-methyl-2-oxopyridine-3-carboxamide (224):** Compound **31a** was replaced with Compound 201. ¹H NMR (400 MHz, DMSO- *d₆*) δ 11.87 (s, 1H), 8.48 (d, 1H), 8.43 (d, 1H), 8.32 (d, 1H), 7.94 (dd, 1H), 7.58 (s, 1H), 7.39 (ddd, 1H), 7.24 (t, 1H), 7.06 (dd, 1H), 7.00 (d, 1H), 6.75 (d, 1H), 6.67 (d, 1H), 4.39 (qt, 2H), 3.89 (s, 3H), 3.86 (s, 3H), 3.71 (t, 2H), 3.51 (dd, 2H), 3.42 (dd, 2H), 3.36 (q, 2H), 2.07 (s, 3H), 1.02 (t, 3H); MS for C₃₄H₃₄FN₅O₈: *m*/*z* 660.2 (MH+).

**N-[4-[(6,7-Dimethoxy-1,5-naphthyridin-4-yl)oxy]-3-fluorophenyl]-6-methyl-2-oxo-1-(2-propan-2-yloxypyridin-4-yl)pyridine-3-carboxamide (225):** Compound **31a** was replaced with Compound 202. ¹H NMR(400 MHz, DMSO- *d₆*) δ 11.86 (s, 1H), 8.48 (d, 1H), 8.43 (d, 1H), 8.31 (d, 1H), 7.94 (dd, 1H), 7.58 (s, 1H), 7.40 (dd, 1H), 7.24 (t, 1H), 7.01 (dd, 1H), 6.89 (d, 1H), 6.75 (d, 1H), 6.66 (d, 1H), 5.28 - 5.11 (m, 1H), 3.89 (s, 3H), 3.86 (s, 3H), 2.07 (s, 3H), 1.27 (t, 6H); MS for C₃₁H₂₈FN₅O₆: *m*/*z* 586.2 (MH+).

**N-[4-[(6,7-Dimethoxy-1,5-naphthyridin-4-yl)oxy]-3-fluorophenyl]-1-(2-methoxypyridin-4-yl)-6-methyl-2-oxopyridine-3-carboxamide (226):** Compound **31a** was replaced with Compound **31-8.** ¹H NMR (400 MHz, DMSO- *d₆*) δ 11.86 (s, 1H), 8.48 (d, 1H), 8.43 (d, 1H), 8.34 (d, 1H), 7.94 (dd, 1H), 7.58 (s, 1H), 7.39 (dt, 1H), 7.24 (t, 1H), 7.06 (dd, 1H), 7.00 (d, 1H), 6.75 (d, 1H), 6.66 (d, 1H), 3.89 (s, 3H), 3.87 (s, 3H), 3.86 (s, 3H), 2.06 (s, 3H); MS for C₂₉H₂₄FN₅O₆: *m*/*z* 558.1 (MH+).

**N-[4-[(6,7-Dimethoxy-1,5-naphthyridin-4-yl)oxy]-3-fluorophenyl]-1-(5-fluoro-6-methylpyridin-2-yl)-6-methyl-2-oxopyridine-3-carboxamide hydrochloride (227):** Compound **31a** was replaced with Compound 31-6. ¹H NMR: (400 MHz, DMSO-*d₆*) δ 11.89 (s, 1H), 8.49-8.56 (m, 2H), 7.95-8.03 (m, 2H), 7.64 (s, 1H), 7.58 (m, 1H), 7.44 (br d, 1H), 7.27-7.33 (m, 1H), 6.82 (d, 1H), 6.71 (d, 1H), 3.94 (d, 6H), 3.43 (br s, 3H), 2.07 (s, 3H); MS for C₂₉H₂₃F₂N₅O₅: *m*/*z* 560.1 (MH+).

**N-[4-[(6,7-Dimethoxy-1,5-naphthyridin-4-yl)oxy]-3-fluorophenyl]-4-hydroxy-2-methyl-5-pyridin-3-ylpyridine-3-carboxamide (228):** Compound **31a** was replaced with Compound **31-4.** ¹H NMR: (400 MHz, DMSO-*d₆*) δ 11.86 (s, 1H), 8.69 - 8.62 (m, 1H), 8.59 (d, 1H), 8.53 - 8.40 (m, 2H), 7.97 - 7.83 (m, 2H), 7.67 - 7.53 (m, 2H), 7.39 (d, 1H), 7.23 (t, 1H), 6.75 (d, 1H), 6.69 (d, 1H), 3.89 (d, 3H), 3.85 (d, 3H), 2.02 (s, 3H); MS for C₂₈H₂₂FN₅O₅: m/z 528.1 (MH+).

**N-[4-[(6,7-Dimethoxy-1,5-naphthyridin-4-yl)oxy]-3-fluorophenyl]-2-oxo-1-pyridin-4-ylpyridine-3-carboxamide (229):** Compound 31a was replaced with Compound **204.** ¹H NMR (400 MHz, DMSO-*d₆*) δ 11.92 (s, 1H), 8.83 - 8.80 (m, 2H), 8.60 (dd, 1H), 8.55 (d, 1H), 8.17 (dd, 1H), 8.01 (dd, 1H), 7.68 - 7.65 (m, 2H), 7.65 (s, 1H), 7.47 (dd, 1H), 7.32 (t, 1H), 6.84 (d, 1H), 6.78 (t, 1H), 3.96 (s, 3H), 3.92 (s, 3H); MS for C₂₇H₂₀FN₅O₅: *m*/*z* 514.1 (MH+).

**N-[4-[(6,7-Dimethoxy-1,5-naphthyridin-4-yl)oxy]-3-fluorophenyl]-6-methyl-1-(2-methylpyridin-4-yl)-2-oxopyridine-3-carboxamide (230):** Compound **31a** was replaced with Compound **31-7.** ¹H NMR (400 MHz, DMSO-*d₆*) δ 11.91 (s, 1H), 8.69 (d, 1H), 8.54 (d, 1H), 8.51 (d, 1H), 8.00 (dd, 1H), 7.64 (s, 1H), 7.45 (br d, 1H), 7.41 (s, 1H), 7.35 (dd, 1H), 7.30 (t, 1H), 6.82 (d, 1H), 6.74 (d, 1H), 3.96 (s, 3H), 3.92 (s, 3H), 2.57 (s, 3H), 2.10 (s, 3H); MS for C₂₉H₂₄FN₅O₅: m/z 542.1 (MH+).

**N-[4-[(6,7-Dimethoxy-1,5-naphthyridin-4-yl)oxy]-3-fluorophenyl]-6-methyl-1-(1-oxidopyridin-1-ium-4-yl)-2-oxopyridine-3-carboxamide (231):** Compound **31a** was replaced with Compound **206.** ¹H NMR: (400 MHz, CD₃OD) δ 8.78-8.69 (m, 3H), 8.63 (d, 1H), 8.13-8.06 (m, 1H), 7.82 (d, 2H), 7.64 (s, 1H), 7.51-7.42 (m, 2H), 7.23 (dd, 1H), 6.76 (d, 1H), 4.18 (d, 6H), 2.28 (s, 3H); MS for C₂₈H₂₂FN₅O₆: *m*/*z* 544 (MH+).

**N-[4-[(6,7-Dimethoxy-1,5-naphthyridin-4-yl)oxy]-3-fluorophenyl]-1-(2-hydroxypyridin-4-yl)-6-methyl-2-oxopyridine-3-carboxamide (232):** Compound **31a** was replaced with Compound **208.** ¹H NMR: (400 MHz, CDCl₃) δ 11.62 (s, 1H), 8.57 (d, 1H), 8.45 (d, 1H), 7.84 (s, 1H), 7.51 (d, 1H), 7.44 (s, 1H), 7.25 (s, 1H), 7.09 (s, 1H), 6.67 (d, 1H), 6.50 - 6.44 (m, 2H), 6.14 (dd, 1H), 4.03 (s, 3H), 3.95 (s, 3H), 2.24 (s, 3H); MS for C₂₈H₂₂FN₅O₆: *m*/*z* 544.1 (MH+).

**N-[4-[(6,7-Dimethoxy-1,5-naphthyridin-4-yl)oxy]-3-fluorophenyl]-1-(2-fluoropyridin-4-yl)-6-methyl-2-oxopyridine-3-carboxamide (233):** Compound **31a** was replaced with Compound 211. ¹H NMR: (400 MHz, DMSO-*d₆*) δ 11.81 (s, 1H), 8.55 - 8.51 (m, 3H), 8.00 (dd, 1H), 7.64 (s, 1H), 7.59 (br d, 1H), 7.56 (s, 1H), 7.46 (br d, 1H), 7.30 (t, 1H), 6.82 (d, 1H), 6.76 (d, 1H), 3.96 (s, 3H), 3.92 (s, 3H), 2.13 (s, 3H); MS for C₂₈H₂₁F₂N₅O₅: *m*/*z* 546.2 (MH+).

**N-[4-[(6,7-Dimethoxy-1,5-naphthyridin-4-yl)oxy]-3-fluorophenyl]-6-methyl-2-oxo-1-pyridazin-3-ylpyridine-3-carboxamide (234):** Compound **31a** was replaced with Compound **31-10.** ¹H NMR: (400 MHz, CDCl₃) δ 11.61 (s, 1H), 9.41 (br d, 1H), 8.70 (d, 1H), 8.52 (d, 1H), 7.91 (br d, 1H), 7.83 (m, 1H), 7.69 (br d, 1H), 7.53 (s, 1H), 7.31 (br d, 1H), 7.16 (br m, 1H), 6.74 (d, 1H), 6.58 (br d, 1H), 4.11 (s, 3H), 4.03 (s, 3H), 2.17 (s, 3H); MS for C₂₇H₂₁FN₆O₅: *m*/*z* 529.1 (MH+).

**5-Acetyl-N-[4-[(6,7-dimethoxy-1,5-naphthyridin-4-yl)oxy]-3-fluorophenyl]-1-(furan-3-yl)-6-methyl-2-oxopyridine-3-carboxamide (235):** Compound **31a** was replaced with Compound **6b-3.** ¹H NMR (400MHz, DMSO-*d₆*) δ 11.61 (s, 1H), 8.83 (s, 1H), 8.49 (d, 1H), 7.96 (m, 2H), 7.86 (t, 1H), 7.58 (s, 1H), 7.48 - 7.41 (m, 1H), 7.27 (t, 1H), 6.78 (d, 1H), 6.64 (d, 1H), 3.88 (d, 6H), 2.55 (s, 3H), 2.42 (d, 3H): MS for C₂₉H₂₃FN₄O₇: *m*/*z* 559 (MH+).

**5-Acetyl-N-[4-[(6,7-dimethoxy-1,5-naphthyridin-4-yl)oxy]-3-fluorophenyl]-1-(furan-2-yl)-6-methyl-2-oxopyridine-3-carboxamide (236):** Compound **31a** was replaced with Compound **6b-4.** ¹H NMR (400MHz, DMSO-*d₆*) δ 11.31 (s, 1H), 8.83 (s, 1H), 8.49 (d, 1H), 7.95 (dd, 1H), 7.81 (dd, 1H), 7.58 (s, 1H), 7.46 (dd, 1H), 7.27 (t, 1H), 6.78 (d, 1H), 6.71 - 6.62 (m, 2H), 3.88 (d, 6H), 2.54 (s, 3H), 2.30 (s, 3H): MS for C₂₉H₂₃FN₄O₇: *m*/*z* 559 (MH+).

**5-Acetyl-N-[4-[(6,7-dimethoxy-1,5-naphthyridin-4-yl)oxy]phenyl]-1-(furan-2-yl)-6-methyl-2-oxopyridine-3-carboxamide (237):** Intermediate **I-1** was replaced with Intermediate **I-2** and Compound **31a** was replaced with Compound **6b-4.** ¹H NMR (400MHz, DMSO-*d₆*) δ 11.38 (s, 1H), 8.99 (s, 1H), 8.64 (d, 1H), 7.97 (d, 1H), 7.94 - 7.86 (m, 2H), 7.73 (s, 1H), 7.33 - 7.25 (m, 2H), 6.92 (d, 1H), 6.87 - 6.78 (m, 2H), 4.03 (d, 6H), 2.70 (s, 3H), 2.45 (s, 3H): MS for C₂₉H₂₄N₄O₇: m/z 541 (MH+).

**5-Acetyl-N-[3-fluoro-4-[(7-methoxy-1,5-naphthyridin-4-yl)oxy]phenyl]-1-(furan-2-yl)-6-methyl-2-oxopyridine-3-carboxamide (238):** Intermediate **I-1** was replaced with Intermediate **I-3** and Compound **31a** was replaced with Compound 6b-4. ¹H NMR (400MHz, DMSO-*d₆*) δ 11.35 (s, 1H), 8.84 (s, 1H), 8.71 - 8.61 (m, 2H), 7.99 (dd, 1H), 7.82 (t, 1H), 7.73 (d, 1H), 7.56 - 7.48 (m, 1H), 7.36 (t, 1H), 6.72 - 6.63 (m, 3H), 3.94 (s, 3H), 2.55 (s, 3H), 2.30 (s, 3H): MS for C₂₈H₂₁FN₄O₆: *m*/*z* 529 (MH+).

**5-Acetyl-N-[4-[(6,7-dimethoxy-1,5-naphthyridin-4-yl)oxy]phenyl]-1-(furan-3-yl)-6-methyl-2-oxopyridine-3-carboxamide (239):** Intermediate **I-1** was replaced with Intermediate **I-2** and Compound **31a** was replaced with Compound **6b-3.** ¹H NMR (400MHz, DMSO-*d*₆) 11.17 (s, 1H), 8.83 (s, 1H), 8.61 (d, 1H), 8.10 (d, 1H), 7.62 (s, 1H), 7.52 (t, 1H), 7.38 (d, 2H), 7.25 (d, 2H), 7.03 (d, 1H), 6.64 (d, 1H), 3.91 (s, 3H), 3.79 (s, 3H), 2.55 (s, 3H), 2.29 (s, 3H): MS for C₂₉H₂₄N₄O₇: *m*/*z* 541 (MH+).

**5-Acetyl-N-[3-fluoro-4-[(7-methoxy-1,5-naphthyridin-4-yl)oxy]phenyl]-1-(furan-3-yl)-6-methyl-2-oxopyridine-3-carboxamide (240):** Intermediate **I-1** was replaced with Intermediate **I-3** and Compound **31a** was replaced with Compound **6b-3.** ¹H NMR (400MHz, DMSO-*d*₆) δ 11.66 (s, 1H), 8.85 (s, 1H), 8.68 (d, 1H), 8.65 (d, 1H), 8.01 (dd, 1H), 7.96 (dd, 1H), 7.87 (t, 1H), 7.74 (d, 1H), 7.51 (d, 1H), 7.36 (t, 1H), 6.71 - 6.67 (m, 1H), 6.66 (dd, 1H), 3.94 (s, 3H), 2.55 (s, 3H), 2.41 (s, 3H): MS for C₂₉H₂₄N₄O₇: *m*/*z* 529 (MH+).

**5-acetyl-N-[4-[(6,*7*-dimethoxy-1,5-naphthyridin-4-yl)oxy]-3-fluorophenyl]-6-methyl-2-oxo-1-thiophen-3-ylpyridine-3-carboxamide (241):** Compound **31a** was replaced with Compound 6b-5. MS for C₂₉H₂₃FN₄O₆S: *m*/*z* 575 (MH+).

**5-acetyl-N-[4-[(6,7-dimethoxy-1,5-naphthyridin-4-yl)oxy]phenyl]-6-methyl-2-oxo-1-thiophen-3-ylpyridine-3-carboxamide (242):** Intermediate **I-1** was replaced with Intermediate **I-2** and Compound **31a** was replaced with Compound **6b-5.** MS for C₂₉H₂₄N₄O₆S: *m*/*z* 557 (MH+).

### Example 47: N-[4-[(6,7-dimethoxy-1,5-naphthyridin-4-yl)oxy]-3-fluorophenyl]-4-ethoxy-1-(5-fluoropyridin-2-yl)-2-oxopyridine-3-carboxamide (243)

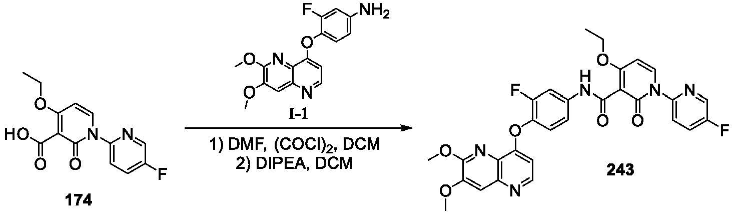

DMF (0.01 mL, 0.13 mmol, 0.48 eq) was added to a stirring suspension of oxalyl dichloride (0.06 mL, 0.7 mmol, 3 eq) and Compound 174 (75 mg, 0.27 mmol, 1eq) in DCM (2 mL) at 0 °C. The ice bath was removed, and the solution was stirred for another 30 minutes, then concentrated to dryness. Intermediate **I-1** (85 mg, 0.27 mmol, 1 eq) was added and the resulting mixture was suspended in DCM (2 mL) and cooled to 0 °C. DIEA (0.4 mL, 2 mmol, 9 eq) was added and the resulting mixture stirred overnight while the mixture was allowed to warm to room temperature. The reaction mixture was concentrated, and the resulting residue was purified by prep HPLC (0.1% formic acid in water and acetonitrile) to give Compound **243** as a white solid (36 mg, 23.2%). ¹H NMR (400MHz, DMSO-*d*₆) δ 10.53 (s, 1H), 8.63 (d, 1H), 8.53 (d, 1H), 8.05 (d, 1H), 7.98 (m, 1H), 7.91 (m, 1H), 7.85 (m, 1H), 7.65 (s, 1H), 7.46 (m, 1H), 7.34 (t, 1H), 6.79 (d, 1H), 6.60 (d, 1H), 4.29 (q, 2H), 3.98 (s, 6H), 1.32 (t, 3H); MS for C₂₉H₂₃F₂N₅O₆: *m*/*z* 576 (MH+).

The following compound was made using the same method used to make Compound **243** from Compound **174** in **Example 47:**

**N-[4-[(6,7-Dimethoxy-1,5-naphthyridin-4-yl)oxy]phenyl]-4-ethoxy-1-(5-fluoropyridin-2-yl)-2-oxopyridine-3-carboxamide (244):** Intermediate **I-1** was replaced with Intermediate **1-2.** ¹H NMR (400MHz, DMSO-*d*₆) δ 10.33 (s, 1H), 8.63 (d, 1H), 8.51 (d, 1H), 8.02 (d, 1H), 7.96 (m, 1H), 7.85 (m, 1H), 7.78 (m, 2H), 7.63 (s, 1H), 7.18 (m, 2H), 6.74 (d, 1H), 6.59 (d, 1H), 4.28 (q, 2H), 3.97 (s, 6H), 1.31 (t, 3H); MS for C₂₉H₂₄FN₅O₆: *m*/*z* 558 (MH+).

**N-[4-[(6,7-Dimethoxy-1,5-naphthyridin-4-yl)oxy]phenyl]-1-(5-fluoropyridin-2-yl)-4,6-dimethyl-2-oxopyridine-3-carboxamide (245):** Compound **174** was replaced with Compound **182** and Intermediate **I-1** was replaced with Intermediate **1-2.** ¹H NMR (400MHz, CD₃OD) δ 8.68 (d, 1H), 8.61 (d, 1H), 7.95 - 7.90 (m, 1H), 7.90 - 7.86 (m, 2H), 7.63 - 7.56 (m, 2H), 7.35 - 7.28 (m, 2H), 7.11 (d, 1H), 6.46 (s, 1H), 4.20 (s, 3H), 4.15 (s, 3H), 2.54 (s, 3H), 2.08 (s, 3H); MS for C₂₉H₂₄FN₅O₅: m/z 542.2 (MH+).

### Example 48: N-[4-[(6,7Dimethoxy-1,5-naphthyridin-4-yl)oxy]-3-fluorophenyl]-4-(5-fluoropyridin-2-yl)-3-oxopyrazine-2-carboxamide (246)

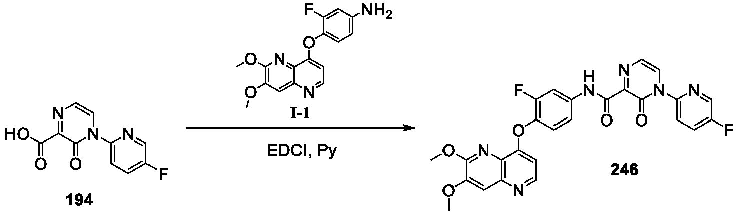

EDCI (96.0 mg, 0.50 mmol) was added to a solution of Compound **194** (40 mg, 170 µmol) and Intermediate **I-1** (56 mg, 178 µmol) in pyridine (1 mL). The mixture was stirred at 20-30 °C for 4 h. The mixture was concentrated under vacuum and the resulting residue was purified by column chromatography on silica gel to give Compound **246** as a yellow solid (61 mg, 67% yield). ¹H NMR: (400 MHz, DMSO-*d₆*) δ 11.12 (s, 1H), 8.72 (d, 1H), 8.55 (d, 1H), 8.13 (d, 1H), 8.10-8.00 (m, 2H), 7.94 (dd, 1H), 7.69-7.61 (m, 2H), 7.51 (br d, 1H), 7.42-7.31 (m, 1H), 6.84 (d, 1H), 3.97 (s, 3H), 3.95 (s, 3H); MS for C₂₆H₁₈F₂N₆O₅: *m*/*z* 533.1 (MH+).

### Example 49: N-[4-[(6,7-Dimethoxy-1,5-naphthyridin-4-yl)oxy]-3-fluorophenyl]-1-(1-oxidopyridin-1-ium-4-yl)-2-oxopyridine-3-carboxamide hydrochloride (247):

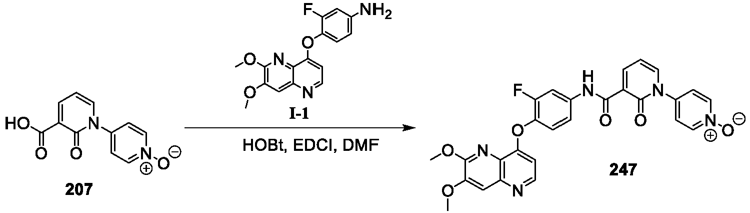

To a solution of Compound **207** (50 mg, 0.215 mmol) and Intermediate **I-1** (56.6 mg, 0.18 mmol) in DMF (0.5 mL) was added HOBt (36.4 mg, 0.27 mmol) and EDCI (51.6 mg, 0.27 mmol). The resulting solution was stirred at 25 °C for 20 h. The mixture was filtered, and the filtrate was purified by prep-HPLC to give Compound **247** as a brown solid (16.2 mg, 16% yield). ¹H NMR: (400 MHz, DMSO-*d₆*) δ 11.97 (s, 1H), 8.83 (d, 1H), 8.58 (dd, 1H), 8.45 (d, 2H), 8.17 (dd, 1H), 8.11 (dd, 1H), 7.86 (s, 1H), 7.71 (d, 2H), 7.61 - 7.55 (m, 1H), 7.55 - 7.49 (m, 1H), 7.20 (d, 1H), 6.78 (t, 1H), 4.05 (s, 6H); MS for C₂₇H₂₀FN₅O₆: *m*/*z* 530.2 (MH+).

The following compound was made using the same method used to make Compound **247** from Compound **207** in **Example 49:**

**N-[4-[(6,7-Dimethoxy-1,5-naphthyridin-4-yl)oxy]-3-fluorophenyl]-1-(2-hydroxypyridin-4-yl)-2-oxopyridine-3-carboxamide hydrochloride (248):** Compound **207** was replaced with Compound **209.** ¹H NMR (400 MHz, DMSO-*d₆*) δ 12.01 (s, 1H), 8.83 (d, 1H), 8.56 (dd, 1H), 8.15 - 8.08 (m, 2H), 7.85 (s, 1H), 7.58 (d, 2H), 7.54 - 7.49 (m, 1H), 7.19 (d, 1H), 6.74 (t, 1H), 6.56 (d, 1H), 6.42 (dd, 1H), 4.05 (s, 6H); MS for C₂₇H₂₀FN₅O₆: *m*/*z* 530.1 (MH+).

### Example 50: 5-Bromo-N-(3-fluoro-4-((6-methoxy-7-(2-methoxyethoxy)-1,5-naphthyridin-4-yl)oxy)phenyl)-1,2,6-trimethyl-4-oxo-1,4-dihydropyridine-3-carboxamide (249).

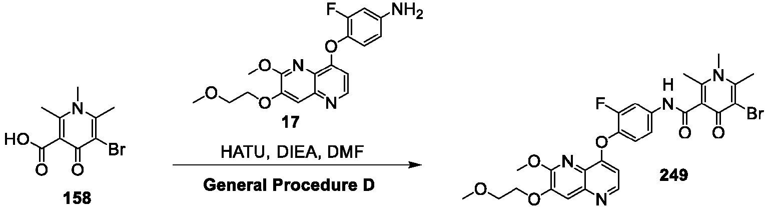

Compound **249** was made using **General Procedure D.** ¹H NMR (400MHz, DMSO-*d*₆) δ 10.79 (s, 1H), 8.53 (d, 1H), 7.95 (dd, 1H), 7.68 (s, 1H), 7.60 - 7.43 (m, 1H), 7.37 (t, 1H), 6.78 (dd, 1H), 4.38 - 4.25 (m, 2H), 4.00 (s, 3H), 3.86 - 3.74 (m, 2H), 3.67 (s, 3H), 2.70 (s, 3H), 2.42 (s, 3H); MS for C₂₇H₂₆BrFN₄O₆: *m*/*z* 601 (MH+).

The following compounds were made using the same method used to make Compound **249** from Compound **158** in Example **50:**

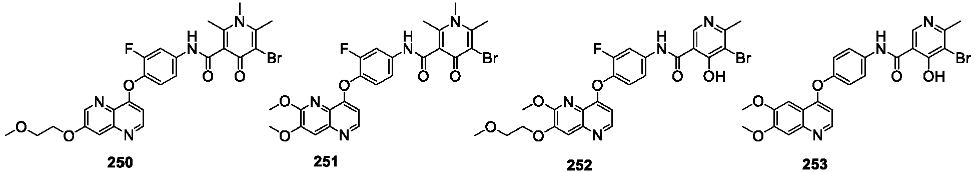

**5-Bromo-N-(3-fluoro-4-((7-(2-methoxyethoxy)-1,5-naphthyridin-4-yl)oxy)phenyl)-1,2,6-trimethyl-4-oxo-1,4-dihydropyridine-3-carboxamide (250):** ¹H NMR (400 MHz, DMSO-*d₆*) δ 10.74 (s, 1H), 8.69 (d, 1H), 8.62 (d, 1H), 7.90 (dd, 1H), 7.76 (d, 1H), 7.44 (ddd, 1H), 7.36 (t, 1H), 6.68 (dd, 1H), 4.36 - 4.23 (m, 2H), 3.76 - 3.63 (m, 2H), 3.59 (s, 3H), 3.28 (s, 3H), 2.62 (s, 3H), 2.35 (s, 3H); MS for C₂₆H₂₄BrFN₄O₅: *m*/*z* 573 (MH+).

**5-Bromo-N-(4-((6,7-dimethoxy-1,5-naphthyridin-4-yl)oxy)-3-fluorophenyl)-1,2,6-trimethyl-4-oxo-1,4-dihydropyridine-3-carboxamide (251):** ¹H NMR (400 MHz, DMSO-*d₆*) δ 10.82 (s, 1H), 8.64 (dd, 1H), 7.98 (dd, 1H), 7.68 (s, 1H), 7.51 (dd, 1H), 7.42 (t, 1H), 6.93 (d, 1H), 4.02 (d, 6H), 3.67 (s, 3H), 2.70 (s, 3H), 2.43 (s, 3H); MS for C₂₅H₂₂BrFN₄O₅: *m*/*z* 559 (MH+).

**5-Bromo-N-(3-fluoro-4-((6-methoxy-7-(2-methoxyethoxy)-1,5-naphthyridin-4-yl)oxy)phenyl)-4-hydroxy-6-methylnicotinamide (252):** ¹H NMR (400 MHz, DMSO-*d₆*) δ 13.02 (d, 1H), 12.83 (s, 1H), 8.62 (d, 1H), 8.54 (d, 1H), 8.05 (dd, 1H), 7.70 (s, 1H), 7.50 (dd, 1H), 7.41 (t, 1H), 6.93 (d, 1H), 4.35 (t, 2H), 3.99 (s, 3H), 3.77 (t, 2H), 3.35 (s, 3H), 2.4 (s, 3H).

**5-Bromo-N-(4-((6,7-dimethoxyquinolin-4-yl)oxy)phenyl)-4-hydroxy-6-methylnicotinamide (253):** ¹H NMR (400 MHz, DMSO-*d₆*) δ 13.79 (s, 1H), 8.49 (d, 2H), 8.13 - 8.03 (m, 1H), 7.55 (s, 1H), 7.47 - 7.32 (m, 4H), 6.50 (dd, 1H), 3.96 (s, 6H), 2.44 (s, 3H).

### Example 51: N-[3-Fluoro-4-[[6-methoxy-7-(2-methoxyethoxy)-1,5-naphthyridin-4-yl]oxy]phenyl]-5-(furan-2-yl)-1,2,6-trimethyl-4-oxopyridine-3-carboxamide (254).

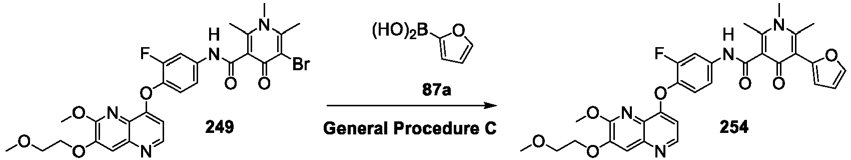

Compound **254** was made using **General Procedure** C. ¹H NMR (400MHz, DMSO-*d*₆) δ 10.98 (s, 1H), 8.45 (d, 1H), 7.88 (dd, 1H), 7.65 (dd, 1H), 7.61 (s, 1H), 7.40 (ddd, 1H), 7.28 (t, 1H), 6.70 (dd, 1H), 6.53 - 6.40 (m, 2H), 4.35 - 4.18 (m, 2H), 3.92 (s, 3H), 3.75 - 3.64 (m, 2H), 3.56 (s, 3H), 3.27 (s, 3H), 2.44 (s, 3H), 2.27 (s, 3H); MS for C₃₁H₂₉FN₄O₇: *m*/*z* 589 (MH+).

The following compounds were made using the same method used to make Compound **254** from Compound **249** in **Example 51:**

**N-[4-[(6,7-Dimethoxy-1,5-naphthyridin-4-yl)oxy]phenyl]-5-(furan-2-yl)-4-hydroxy-6-methylpyridine-3-carboxamide (255):** ¹H NMR (400MHz, DMSO-*d*₆) δ 12.87 (s, 1H), 12.57 (s, 1H), 8.47 (d, 1H), 8.43 (s, 1H), 7.74 (d, 1H), 7.72 (d, 2H), 7.58 (s, 1H), 7.14 (d, 1H), 7.12 (d, 1H), 6.88 (d, 1H), 6.74 (d, 1H), 6.55 (dd, 1H), 3.90 (s, 3H), 3.87 (s, 3H), 2.39 (s, 3H); MS for C₂₇H₂₂N₄O₆: *m*/*z* 499.15 (MH+).

**N-[4-[(6,7-Dimethoxy-1,5-naphthyridin-4-yl)oxy]phenyl]-4-hydroxy-6-methyl-5-thiophen-2-ylpyridine-3-carboxamide (256):** ¹H NMR (400MHz, DMSO-*d*₆) δ 12.83 (s, 1H), 12.61 (s, 1H), 8.47 (d, 1H), 8.45 (s, 1H), 7.73 (s, 1H), 7.71 (s, 1H), 7.59 (dd, 1H), 7.57 (s, 1H), 7.13 (s, 1H), 7.11 (s, 1H), 7.10 - 7.02 (m, 2H), 6.74 (s, 1H), 3.90 (s, 3H), 3.87 (s, 3H), 2.31 (s, 3H); MS for C₂₇H₂₂N₄O₅S: *m*/*z* 515.15 (MH+).

**4-Hydroxy-N-[4-[(7-methoxy-1,5-naphthyridin-4-yl)oxy]phenyl]-6-methyl-5-thiophen-2-ylpyridine-3-carboxamide (257):** ¹H NMR (400MHz, DMSO-*d*₆) δ 12.86 (s, 1H), 12.62 (s, 1H), 8.65 (dd, 2H), 8.46 (s, 1H), 7.84 - 7.66 (m, 3H), 7.60 (dd, 1H), 7.27 - 7.13 (m, 2H), 7.13 - 7.00 (m, 2H), 6.67 (d, 1H), 3.93 (s, 3H), 2.31 (s, 3H); MS for C₂₆H₂₀N₄O₄S: *m*/*z* 485.15 (MH+).

**N-[4-(6,7-Dimethoxyquinolin-4-yl)oxyphenyl]-5-(furan-2-yl)-4-hydroxy-6-methylpyridine-3-carboxamide (258):** ¹H NMR (400MHz, DMSO-*d*₆) δ 12.91 (s, 1H), 12.58 (s, 1H), 8.50 - 8.29 (m, 2H), 7.78 (d, 2H), 7.72 (dd, 1H), 7.45 (s, 1H), 7.33 (s, 1H), 7.20 (d, 2H), 6.88 (dd, 1H), 6.55 (dd, 1H), 6.43 (d, 1H), 3.88 (d, 6H), 2.39 (s, 3H); MS C₂₈H₂₃N₃O₆: *m*/*z* 498.0 (MH+).

**N-[4-(6,7-Dimethoxyquinolin-4-yl)oxy-3-fluorophenyl]-5-(furan-2-yl)-4-hydroxy-6-methylpyridine-3-carboxamide (259).** ¹H NMR (400MHz, DMSO-*d*₆) δ 13.14 (s, 1H), 12.64 (s, 1H), 8.45 (s, 1H), 8.42 (d, 1H), 8.08 (s, 1H), 8.00 (dd, 1H), 7.71 (dd, 1H), 7.47 (s, 1H), 7.45 - 7.36 (m, 1H), 7.35 (s, 1H), 6.87 (dd, 1H), 6.55 (dd, 1H), 6.43 (dd, 1H), 3.89 (s, 6H), 2.38 (s, 4H); MS for C₂₈H₂₂FN₃O₆: *m*/*z* 516.2 (MH+).

**N-[3-Fluoro-4-[[6-methoxy-7-(2-methoxyethoxy)-1,5-naphthyridin-4-yl]oxy]phenyl]-5-(furan-2-yl)-4-hydroxy-6-methylpyridine-3-carboxamide (260):** ¹H NMR (400MHz, DMSO-*d*₆) δ 13.06 (s, 1H), 12.62 (s, 1H), 8.47 (d, 1H), 8.44 (s, 1H), 7.96 (dd, 1H), 7.71 (dd, 1H), 7.61 (s, 1H), 7.37 (ddd, 1H), 7.27 (t, 1H), 6.87 (dd, 1H), 6.76 (dd, 1H), 6.54 (dd, 1H), 4.28 - 4.20 (m, 2H), 3.89 (s, 3H), 3.73 - 3.65 (m, 2H), 3.27 (s, 3H), 2.38 (s, 3H); MS for C₂₉H₂₅FN₄O₇: *m*/*z* 561.0 (MH+).

**5-(1-Benzofuran-2-yl)-N-[4-[(6,7-dimethoxy-1,5-naphthyridin-4-yl)oxy]-3-fluorophenyl]-1,2,6-trimethyl-4-oxopyridine-3-carboxamide (261):** ¹H NMR (400MHz, DMSO-*d*₆) δ 10.94 (s, 1H), 8.45 (d, 1H), 7.88 (dd, 1H), 7.69 - 7.56 (m, 2H), 7.56 - 7.45 (m, 1H), 7.45 - 7.36 (m, 1H), 7.36 - 7.16 (m, 3H), 6.90 (d, 1H), 6.71 (dd, 1H), 3.90 (d, 6H), 3.60 (s, 3H), 2.44 (s, 3H), 2.35 (s, 3H); MS for C₃₃H₂₇FN₄O₆: *m*/*z* 595.2 (MH+).

**5-(1-Benzofuran-3-yl)-N-[4-[(6,7-dimethoxy-1,5-naphthyridin-4-yl)oxy]-3-fluorophenyl]-1,2,6-trimethyl-4-oxopyridine-3-carboxamide (262):** ¹H NMR (400MHz, DMSO-*d*₆) δ 11.22 (s, 1H), 8.45 (d, 1H), 7.92 - 7.78 (m, 2H), 7.57 (d, 2H), 7.38 (ddd, 1H), 7.35 - 7.23 (m, 3H), 7.18 (td, 1H), 6.70 (dd, 1H), 3.90 (d, 6H), 3.61 (s, 3H), 2.51 (s, 3H), 2.27 (s, 3H); MS for C₃₃H₂₇FN₄O₆: *m*/*z* 595.2 (MH+).

**5-(1-Benzofuran-2-yl)-N-[3-fluoro-4-[[6-methoxy-7-(2-methoxyethoxy)-1,5-naphthyridin-4-yl]oxy]phenyl]-1,2,6-trimethyl-4-oxopyridine-3-carboxamide (263):** ¹H NMR (400MHz, DMSO-*d*₆) δ 10.94 (s, 1H), 8.45 (d, 1H), 7.88 (dd, 1H), 7.66 - 7.58 (m, 2H), 7.55 - 7.46 (m, 1H), 7.46 - 7.37 (m, 1H), 7.35 - 7.10 (m, 3H), 6.90 (d, 1H), 6.71 (dd, 1H), 4.34 - 4.13 (m, 2H), 3.91 (s, 3H), 3.81 - 3.64 (m, 2H), 3.60 (s, 3H), 3.27 (s, 3H), 2.44(s, 3H), 2.35 (s, 3H); MS for C₃₅H₃₁FN₄O₇: *m*/*z* 639.2 (MH+).

**5-(1-Benzofuran-3-yl)-N-[3-fluoro-4-[[6-methoxy-7-(2-methoxyethoxy)-1,5-naphthyridin-4-yl]oxy]phenyl]-1,2,6-trimethyl-4-oxopyridine-3-carboxamide (264):** ¹H NMR (400MHz, DMSO-*d*₆) δ 11.22 (s, 1H), 8.44 (d, 1H), 7.95 - 7.79 (m, 2H), 7.65 - 7.53 (m, 2H), 7.38 (ddd, 1H), 7.32 - 7.12 (m, 4H), 6.69 (dd, 1H), 4.30 - 4.17 (m, 2H), 3.91 (s, 3H), 3.76 - 3.66 (m, 2H), 3.61 (s, 3H), 3.27 (s, 3H), 2.51 (s, 3H), 2.27 (s, 3H); MS for C₃₅H₃₁FN₄O₇: *m*/*z* 639.25 (MH+).

**5-(1-Benzofuran-3-yl)-N-[3-fluoro-4-[[7-(2-methoxyethoxy)-1,5-naphthyridin-4-yl]oxy]phenyl]-1,2,6-trimethyl-4-oxopyridine-3-carboxamide (265):** ¹H NMR (400MHz, DMSO-*d*₆) δ 11.25 (s, 1H), 8.70 (d, 1H), 8.63 (d, 1H), 7.98 - 7.80 (m, 2H), 7.75 (d, 1H), 7.57 (dd, 1H), 7.46 - 7.38 (m, 1H), 7.38 - 7.24 (m, 3H), 7.18 (td, 1H), 6.69 (dd, 1H), 4.41 - 4.26 (m, 2H), 3.77 - 3.67 (m, 2H), 3.62 (s, 3H), 3.28 (s, 3H), 2.51 (s, 3H), 2.28 (s, 3H); MS for C₃₄H₂₉FN₄O₆: *m*/*z* 609.2 (MH+).

**N-[3-Fluoro-4-[[7-(2-methoxyethoxy)-1,5-naphthyridin-4-yl]oxy]phenyl]-5-(furan-2-yl)-1,2,6-trimethyl-4-oxopyridine-3-carboxamide (266):** ¹H NMR (400MHz, DMSO-*d*₆) δ 11.00 (s, 1H), 8.69 (d, 1H), 8.62 (d, 1H), 7.91 (dd, 1H), 7.75 (d, 1H), 7.65 (dd, 1H), 7.42 (dd, 1H), 7.34 (t, 1H), 6.68 (dd, 1H), 6.53 - 6.41 (m, 2H), 4.38 - 4.19 (m, 2H), 3.80 - 3.65 (m, 2H), 3.56 (s, 3H), 3.29 (s, 3H), 2.44 (s, 3H), 2.27 (s, 3H); MS for C₃₀H₂₇FN₄O₆: *m*/*z* 559.15 (MH+).

### Example 52: N-[4-[(6,7-Dimethoxy-1,5-naphthyridin-4-yl)oxy]-3-fluorophenyl]-6-(furan-2-yl)-5-methylpyrazine-2-carboxamide (269):

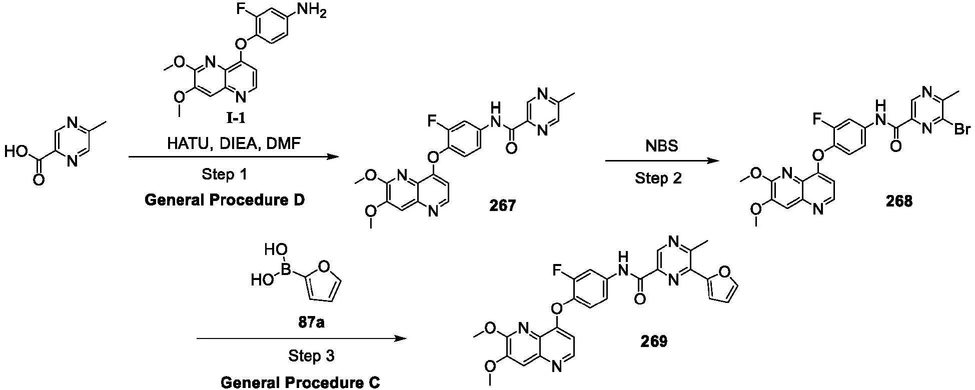

**Step 1: N-(4-((6,7-Dimethoxy-1,5-naphthyridin-4-yl)oxy)-3-fluorophenyl)-5-methylpyrazine-2-carboxamide (267):** Compound **267** was synthesized using **General Procedure** D. ¹H NMR (400MHz, DMSO-*d*₆) δ 11.02 (s, 1H), 9.20 (d, 1H), 8.73 (d, 1H), 8.56 (d, 1H), 8.12 (dd, 1H), 7.85 (dt, 1H), 7.66 (s, 1H), 7.39 (t, 1H), 6.82 (d, 1H), 3.96 (d, 6H), 2.65 (s, 3H); MS C₂₂H₁₈FN₅O₄: *m*/*z* 436.1 (MH+).

**Step 2: 6-Bromo-N-(4-((6,7-dimethoxy-1,5-naphthyridin-4-yl)oxy)-3-fluorophenyl)-5-methylpyrazine-2-carboxamide (268):** Compound **268** was synthesized in a manner similar to Compound **36** in **Example 9.** In this particular case the reaction was heated at 60 °C overnight. ¹H NMR (400MHz, DMSO-*d*₆) δ 10.44 (s, 1H), 9.23 (d, 1H), 8.75 (s, 1H), 8.62 (d, 1H), 8.34 (d, 1H), 7.85 (d, 1H), 7.68 (s, 1H), 7.04 (d, 1H), 3.97 (s, 3H), 3.88 (s, 3H), 2.66 (s, 3H); MS for C₂₂H₁₇BrFN₅O₄: *m*/*z* 516.0 (MH+).

**Step** 3: **N-[4-[(6,7-Dimethoxy-1,5-naphthyridin-4-yl)oxy]-3-fluorophenyl]-6-(furan-2-yl)-5-methylpyrazine-2-carboxamide (269):** Compound **135** was synthesized using **General Procedure C.** ¹H NMR (400MHz, DMSO-*d*₆) δ 11.06 (s, 1H), 9.15 (d, 1H), 8.69 (d, 1H), 8.53 (d, 1H), 8.29 (d, 1H), 7.82 (dd, 1H), 7.66 (d, 1H), 7.60 (s, 1H), 7.02 - 6.86 (m, 2H), 6.60 (dd, 1H), 3.90 (s, 3H), 3.85 (s, 3H), 2.59 (s, 3H); MS for C₂₆H₂₀FN₅O₅: *m*/*z* 502.1 (MH+).

### Example 53: 1-(2,2-Difluoroethyl)-N-[4-[(6,7-dimethoxy-1,5-naphthyridin-4-yl)oxy]-3-fluorophenyl]-5-(furan-2-yl)-2-methyl-4-oxopyridine-3-carboxamide (275)

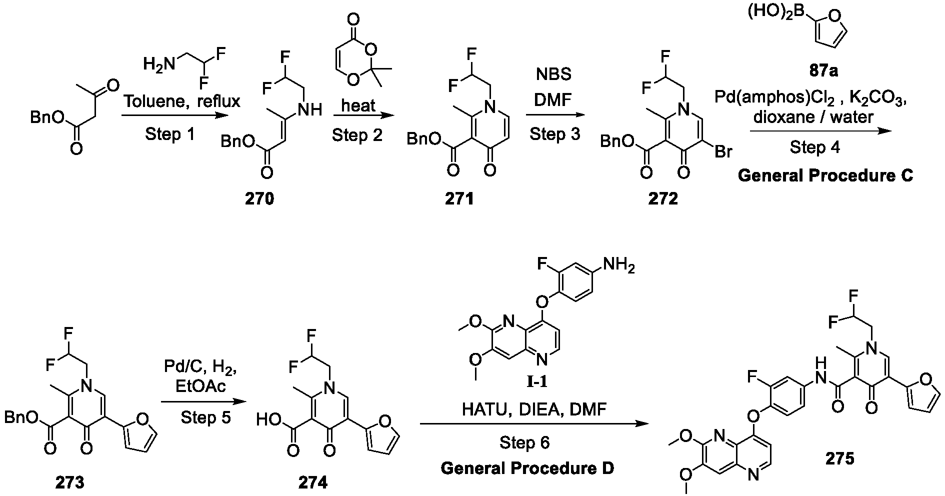

**Step 1: Benzyl (E)-3-((2,2-difluoroethyl)amino)but-2-enoate (270):** To a 100 mL round bottom flask equipped with a magnetic stir bar, a Dean Stark trap, and a pressure relief septum was added benzyl 3-oxobutanoate (6 g, 31.2 mmol, 1.0 eq), 2,2-difluoroethanamine (2.5 g, 31 mmol, 1.0 eq), and toluene (20 mL). The solution was heated to reflux for 3 hours, until no precipitation of water was observed. The solution was then concentrated under reduced pressure to yield **270** as a yellow oil (7.8 g), which was used without further purification. ¹H NMR (400 MHz, DMSO-*d₆*) δ 8.63 (t, 1H), 7.42 - 7.27 (m, 5H), 6.15 (tt, 1H), 5.04 (s, 2H), 4.56 (s, 1H), 3.69 (tdd, 2H), 1.94 (s, 3H).

**Step 2: Benzyl 1-(2,2-difluoroethyl)-2-methyl-4-oxo-1,4-dihydropyridine-3-carboxylate (271):** Compound **271** can be synthesized from Compound **270** and 2,2-dimethyl-4H-1,3-dioxin-4-one in a manner similar to the way Compound **156** was made from methyl 3-(methylamino)but-2-enoate and 2,2,6-trimethyl-4H-1,3-dioxin-4-one in the first step of **Example 32.** 1H NMR (400 MHz, DMSO-d6) δ 7.48 - 7.42 (m, 2H), 7.42 - 7.29 (m, 3H), 6.47 (tt, 1H), 6.15 (s, 1H), 5.26 (s, 2H), 4.51 (td, 2H), 2.35 (s, 3H), 2.26 (s, 3H); MS for C₁₇H₁₈F₂NO₃: m/z 322.1 (MH+).

**Step 3: Benzyl 5-bromo-1-(2,2-difluoroethyl)-2-methyl-4-oxo-1,4-dihydropyridine-3-carboxylate (272):** Compound **272** can be synthesized from Compound **271** by established conditions using NBS such as those exemplified by the synthesis of Compound **36** in **Example 9.**

**Step 4: Benzyl 1-(2,2-difluoroethyl)-5-(furan-2-yl)-2-methyl-4-oxo-1,4-dihydropyridine-3-carboxylate (273):** Compound **273** can be synthesized from Compound **272** using **General Procedure C.**

**Step 5: 1-(2,2-Difluoroethyl)-5-(furan-2-yl)-2-methyl-4-oxo-1,4-dihydropyridine-3-carboxylic acid (274):** Compound **274** can be synthesized from Compound **273** using standard hydrogenation conditions.

**Step 6: 1-(2,2-Difluoroethyl)-N-[4-[(6,7-dimethoxy-1,5-naphthyridin-4-yl)oxy]-3-fluorophenyl]-5-(furan-2-yl)-2-methyl-4-oxopyridine-3-carboxamide (275):** Compound **275** can be synthesized from Compound 274 using General Procedure D. MS for C₂₉H₂₃F₃N₄O₆: *m*/*z* 581.2 (MH+).

### Example 54: Methyl 7-bromo-8-oxo-1,3,4,8-tetrahydropyrido[2,1-c][1,4]oxazine-9-carboxylate (282)

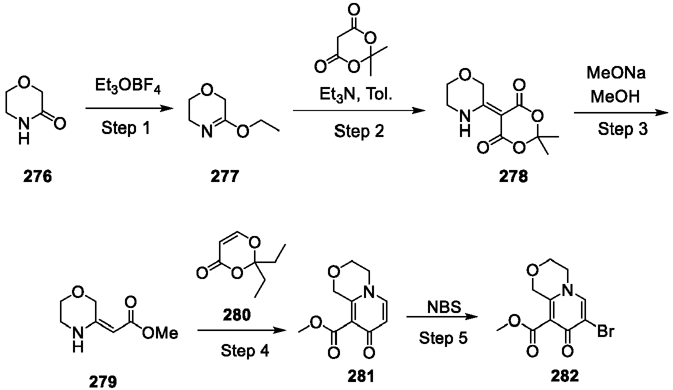

**Step** 1: **5-Ethoxy-3,6-dihydro-2H-1,4-oxazine (277):** To a solution of Compound **276** (5 g, 49.4 mmol, 1 eq) in DCM (80 mL) was added Et₃OBF₄ (10.3 g, 54.4 mmol, 1.1 eq) at 20 °C and the resulting reaction mixture was stirred at 20 °C for 12 h . The reaction mixture was quenched by the addition of aq saturated Na₂CO₃ to pH=8. The organic layer was separated, dried over anhyd Na₂SO₄ and concentrated *in vacuo* to give crude Compound **277** as a yellow oil in DCM (6.3 g) which was used without further purification. ¹H NMR (400 MHz, CDCl₃) δ 4.09 (m, 2H), 4.03 (s, 2H), 3.62-3.68 (m, 2H), 3.49-3.55 (m, 2H), 1.26 (m, 3H).

**Step 2: 2,2-Dimethyl-5-(morpholin-3-ylidene)-1,3-dioxane-4,6-dione (278):** A solution of Compound **277** (5 g, 39 mmol, 1 eq), 2,2-dimethyl-1,3-dioxane-4,6-dione (5.6 g, 39 mmol, 1 eq) and Et₃N (1.08 mL, 7.74 mmol, 0.2 eq) in toluene (50 mL) was stirred at 105 °C for 3 h. The mixture was cooled to room temperature and the solvent evaporated *in vacuo.* The resulting residue was purified by flash silica gel chromatography (0~50% EtOAc /petroleum ether) to give Compound **278** as a yellow solid (1.4 g, 16% yield). ¹H NMR (400 MHz, CDCl₃) δ 11.48 (br s, 1H), 5.05 (s, 2H), 3.94 (m, 2H), 3.55-3.63 (m, 2H), 1.68 (s, 6H); MS for C₁₀H₁₃NO₅: *m*/*z* 169.8 (MH+).

**Step 3: Methyl (E)-2-(morpholin-3-ylidene)acetate (279):** A solution of Compound **278** (1.3 g, 5.7 mmol, 1 eq) and NaOMe (371 mg, 6.9 mmol, 1.2 eq) in MeOH (30 mL) was stirred at 80 °C for 12 h. The mixture was cooled to room temperature and then concentrated *in vacuo.* The resulting residue was dissolved in aq saturated NH₄Cl (100 mL) and extracted with EtOAc (3 x 50 mL). The combined organic extracts were dried over anhyd Na₂SO₄ and concentrated *in vacuo* to give Compound **279** as a yellow solid (617 mg, 69% yield) which was used without further purification. ¹H NMR (400 MHz, CDCl₃) δ 8.56 (br s, 1H), 4.34 (s, 1H), 4.28 (s, 2H), 3.88-3.94 (m, 2H), 3.64 (s, 3H), 3.36 (m, 2H); MS for C₇H₁₁NO₃: *m*/*z* 157.9 (MH+).

**Step 4: 5 Methyl 8-oxo-1,3,4,8-tetrahydropyrido[2,1-c] [1,4] oxazine-9-carboxylate (281):** A mixture of Compound **279** (300 mg, 1.9 mmol, 1 eq) and Compound **280** (596 mg, 3.8 mmol, 2 eq) was stirred at 130 °C for 1.5 h with Dean-Stark trap removal of water. The reaction mixture was concentrated under vacuum. The resulting residue was purified by flash silica gel chromatography (0~10% MeOH in DCM) to give Compound **281** as a yellow solid (220 mg, 55% yield). ¹H NMR (400 MHz, CDCl₃) δ 7.17 (d, 1H), 6.48 (d, 1H), 4.83 (s, 2H), 4.08 - 4.05 (t, 2H), 3.95 - 3.92 (t, 2H), 3.90 (s, 3H); MS for C₁₀H₁₁NO₄: *m*/*z* 209.9 (MH+).

**Methyl 7-bromo-8-oxo-1,3,4,8-tetrahydropyrido[2,1-c][1,4]oxazine-9-carboxylate (282):** To a solution of Compound **281** (210 mg, 1.00 mmol, 1 eq) in DMF (2 mL) was added NBS (214 mg, 1.20 mmol, 1.2 eq) and the resulting mixture was stirred at 25 °C for 2 h. The resulting mixture was diluted with aq saturated NaHCO₃ (50 mL) and extracted with DCM (3 x 50 mL). The combined DCM extracts were washed with aq saturated NaCl (20 mL), dried over anhyd Na₂SO₄ and concentrated to give Compound **282** as a yellow solid (260 mg, 90% yield). MS for C₁₀H₁₀BrNO₄: *m*/*z* 289.7 (MH+).

### Example 55: Methyl 7-bromo-6-methyl-8-oxo-1,3,4,8-tetrahydropyrido[2,1-c][1,4]oxazine-9-carboxylate (284)

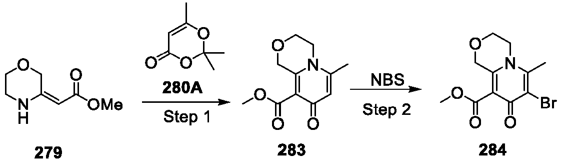

**Step** 1: **Methyl 6-methyl-8-oxo-1,3,4,8-tetrahydropyrido[2,1-c][1,4]oxazine-9-carboxylate (283):** A mixture of Compound 279 (500 mg, 3.2 mmol, 1 eq) and Compound **280A** (1.08 g, 7.6 mmol, 2.4 eq) was stirred at 130 °C for 1 h with a Dean-Stark trap. The reaction mixture was concentrated under vacuum. The resulting residue was purified by column chromatography to give Compound **283** as a brown solid (330 mg, 41% yield). MS for C₁₁H₁₃NO₄: *m*/*z* 224.1 (MH+).

**Step 2: Methyl 7-bromo-6-methyl-8-oxo-1,3,4,8-tetrahydropyrido[2,1-c][1,4]oxazine-9-carboxylate (284):** NBS (300 mg, 1.7 mmol, 1.25 eq) was added to a solution of Compound **283** (300 mg, 1.34 mmol, 1 eq) in DCM (10 mL). The mixture was stirred at 25-30°C for 12 h. The mixture was concentrated, and the resulting residue was purified by column chromatography to give Compound **284** as a yellow solid (373 mg, 90% yield). MS for C₁₁H₁₂BrNO₄: *m*/*z* 304.0 (MH+).

### Example 56: 7-(Furan-2-yl)-8-oxo-1,3,4,8-tetrahydropyrido[2,1-c][1,4]oxazine-9-carboxylic acid (286)

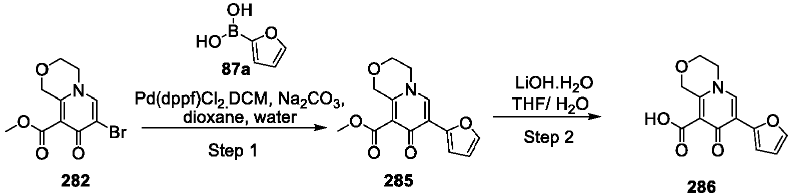

**Step 1: Methyl 7-(furan-2-yl)-8-oxo-1,3,4,8-tetrahydropyrido[2,1-c][1,4]oxazine-9-carboxylate (285):** To a solution of Compound **282** (50 mg, 0.17 mmol, 1 eq) and Compound **87a** (58 mg, 0.52 mmol, 3 eq) in dioxane (3 mL) and water (0.3 mL) was added Pd(dppf)Cl₂.CH₂Cl₂ (14.2 mg, 0.017 mmol, 0.1 eq) and Na₂CO₃ (55 mg, 0.52 mmol, 3 eq). The mixture was stirred at 100 °C for 12 h. The reaction mixture was concentrated under reduced pressure and the resulting residue was purified by flash chromatography over silica gel to give Compound **285** as a yellow solid (32 mg, 67% yield). MS for C₁₄H₁₃NO₅: *m*/*z* 276.0 (MH+).

**Step** 2: **7-(Furan-2-yl)-8-oxo-1,3,4,8-tetrahydropyrido[2,1-c][1,4]oxazine-9-carboxylic acid (286):** To a solution of Compound **285** (32 mg, 0.12 mmol, 1 eq) in THF (1 mL) and water (1 mL) was added LiOH·H₂O (14.6 mg, 0.35 mmol, 3 eq). The mixture was stirred at 25 °C for 2 h. The reaction mixture was concentrated under reduced pressure to remove THF. The resulting residue was diluted with water (5 mL) and extracted with EtOAc (30 mL). The organic layer was washed with aq saturated NaCl (10 mL), dried over anhyd. Na₂SO₄ and concentrated under reduced pressure to give Compound **286** as a yellow solid (16 mg, 53% yield). MS for C₁₃H₁₁NO₅: *m*/*z* 262.1 (MH+).

The following compounds were made using the same method used to make Compound 286 in two steps from Compound 282 and Compound **87a** in **Example 56:**

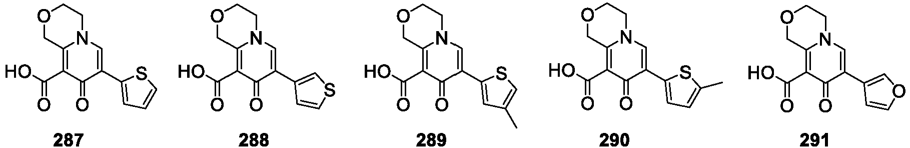

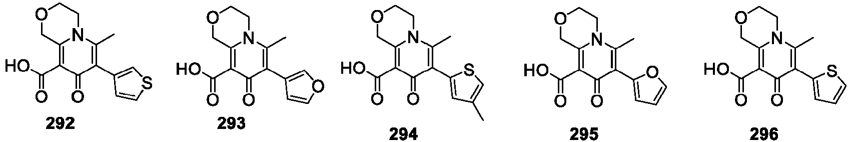

**8-oxo-7-(Thiophen-2-yl)-1,3,4,8-tetrahydropyrido[2,1-c][1,4]oxazine-9-carboxylic acid (287):** MS for C₁₃H₁₁NO₄S: *m*/*z* 278.0 (MH+).

**8-oxo-7-(Thiophen-3-yl)-1,3,4,8-tetrahydropyrido[2,1-c][1,4]oxazine-9-carboxylic acid (288):** MS for C₁₃H₁₁NO₄S: *m*/*z* 277.8 (MH+).

**7-(4-Methylthiophen-2-yl)-8-oxo-1,3,4,8-tetrahydropyrido[2,1-c][1,4]oxazine-9-carboxylic acid (289):** MS for C₁₄H₁₃NO₄S: *m*/*z* 291.8 (MH+).

**7-(5-Methylthiophen-2-yl)-8-oxo-1,3,4,8-tetrahydropyrido[2,1-c][1,4]oxazine-9-carboxylic acid (290):** MS for C₁₄H₁₃NO₄S: *m*/*z* 291.9 (MH+).

**7-(Furan-3-yl)-8-oxo-1,3,4,8-tetrahydropyrido[2,1-c][1,4]oxazine-9-carboxylic acid (291):** MS for C₁₃H₁₁NO₅: *m*/*z* 262.1 (MH+).

**6-Methyl-8-oxo-7-(thiophen-3-yl)-1,3,4,8-tetrahydropyrido[2,1-c][1,4]oxazine-9-carboxylic acid (292):** MS for C₁₄H₁₃NO₄S: *m*/*z* 292.0 (MH+).

**7-(Furan-3-yl)-6-methyl-8-oxo-1,3,4,8-tetrahydropyrido[2,1-c][1,4]oxazine-9-carboxylic acid (293).**

**6-Methyl-7-(4-methylthiophen-2-yl)-8-oxo-1,3,4,8-tetrahydropyrido[2,1-c][1,4]oxazine-9-carboxylic acid (294).**

**7-(Furan-2-yl)-6-methyl-8-oxo-1,3,4,8-tetrahydropyrido[2,1-c][1,4]oxazine-9-carboxylic acid (295).**

**6-Methyl-8-oxo-7-(thiophen-2-yl)-1,3,4,8-tetrahydropyrido[2,1-c][1,4]oxazine-9-carboxylic acid (296).**

### Example 57: N-[4-[(6,7-Dimethoxy-1,5-naphthyridin-4-yl)oxy]-3-fluorophenyl]-7-(furan-2-yl)-8-oxo-3,4-dihydro-1H-pyrido[2,1-c][1,4]oxazine-9-carboxamide (297)

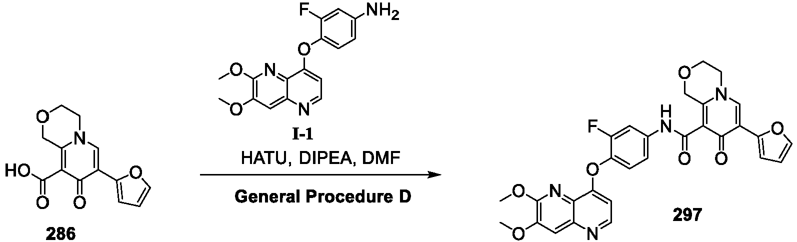

**N-[4-[(6,7-Dimethoxy-1,5-naphthyridin-4-yl)oxy]-3-fluorophenyl]-7-(furan-2-yl)-8-oxo-3,4-dihydro-1H-pyrido[2,1-c][1,4]oxazine-9-carboxamide (297).** Compound **297** was made using **General Procedure D.** ¹H NMR (400MHz, DMSO-*d*₆) δ 12.92 (s, 1H), 8.54 (d, 1H), 8.30 (s, 1H), 7.98 (d, 1H), 7.79 (s, 1H), 7.66 (s, 1H), 7.43 (m, 2H), 7.34 (t, 1H), 6.81 (d, 1H), 6.64 (d, 1H), 5.20 (s, 2H), 4.30 (t, 2H), 4.07 (t, 2H), 3.97 (s, 3H), 3.96 (s, 3H), MS for C₂₉H₂₃FN₄O₇: *m*/*z* 559.1 (MH+).

The following compounds were made using the same method used to make Compound **297** from Compound **286** and Intermediate **I-1** using **General Procedure D** as in Example 57:

**N-[4-[(6,7-Dimethoxy-1,5-naphthyridin-4-yl)oxy]phenyl]-7-(furan-2-yl)-8-oxo-3,4-dihydro-1H-pyrido[2,1-c][1,4]oxazine-9-carboxamide (298):** Intermediate **I-1** was replaced with Intermediate **1-2.** ¹H NMR (400MHz, DMSO-*d*₆) δ 12.76 (s, 1H), 8.54 (d, 1H), 8.29 (s, 1H), 7.78 (dd, 3H), 7.64 (s, 1H), 7.41 (d, 1H), 7.19 (dd, 2H), 6.79 (d, 1H), 6.64 (s, 1H), 5.21 (s, 2H), 4.29 (t, 2H), 4.07 (t, 2H), 3.97 (s, 3H), 3.95 (s, 3H); MS for C₂₉H₂₄N₄O₇: *m*/*z* 541.0 (MH+).

**N-[4-[(6,7-Dimethoxy-1,5-naphthyridin-4-yl)oxy]phenyl]-8-oxo-7-thiophen-2-yl-3,4-dihydro-1H-pyrido[2,1-c][1,4]oxazine-9-carboxamide (299):** Intermediate **I-1** was replaced with Intermediate **I-2** and Compound **286** was replaced with Compound **287.** ¹H NMR (400MHz, DMSO-*d*₆) δ 12.7 (s, 1H), 8.59 (s, 1H), 8.54 (d, 1H), 7.79 - 7.75 (m, 3H), 7.64 - 7.59 (m, 2H), 7.2 - 7.16 (m, 3H), 6.79 (d, 1H), 5.22 (s, 2H), 4.27 (t, 2H), 4.09 (t, 2H), 3.96 (s, 3H), 3.95 (s, 3H); MS for C₂₉H₂₄N₄O₆S: *m*/*z* 556.9 (MH+).

**N-[4-[(6,7-Dimethoxy-1,5-naphthyridin-4-yl)oxy]-3-fluorophenyl]-8-oxo-7-thiophen-3-yl-3,4-dihydro-1H-pyrido[2,1-c][1,4]oxazine-9-carboxamide (300):** Compound **286** was replaced with Compound **288.** ¹H NMR (400MHz, DMSO-*d*₆) δ 13.17 (s, 1H), 8.54 (d, 1H), 8.37 (t, 2H), 7.97 (s, 1H), 7.67 - 7.64 (m, 3H), 7.41 (d, 1H), 7.34 (t, 1H), 6.81 (d, 1H), 5.22 (s, 2H), 4.24 (t, 2H), 4.08 (t, 2H), 3.97 (s, 3H), 3.96 (s, 3H); MS for C₂₉H₂₃FN₄O₆S: *m*/*z* 575.2 (MH+).

**N-[4-[(6,7-Dimethoxy-1,5-naphthyridin-4-yl)oxy]phenyl]-8-oxo-7-thiophen-3-yl-3,4-dihydro-1H-pyrido[2,1-c][1,4]oxazine-9-carboxamide (301):** Intermediate **I-1** was replaced with Intermediate **I-2** and Compound **286** was replaced with Compound **288.** ¹H NMR (400MHz, DMSO-*d*₆) δ 12.99 (s, 1H), 8.54 (d, 1H), 8.38 (d, 1H), 8.35 (s, 1H), 7.77 (d, 2H), 7.65 - 7.62 (m, 3H), 7.19 (dd, 2H), 6.78 (d, 1H), 5.23 (s, 2H), 4.24 (t, 2H), 4.08 (t, 2H), 3.97 (s, 3H), 3.95 (s, 3H); MS for C₂₉H₂₄N₄O₆S: *m*/*z* 557.2 (MH+).

**N-[4-[(6,7-Dimethoxy-1,5-naphthyridin-4-yl)oxy]-3-fluorophenyl]-7-(4-methylthiophen-2-yl)-8-oxo-3,4-dihydro-1H-pyrido[2,1-c][1,4]oxazine-9-carboxamide (302):** Compound **286** was replaced with Compound **289.** ¹H NMR (400MHz, DMSO-*d*₆) δ 12.87 (s, 1H), 8.55 (d, 2H), 7.98 (dd, 1H), 7.65 (s, 1H), 7.58 (s, 1H), 7.41 (m, 1H), 7.34 (t, 1H), 7.18 (s, 1H), 6.82 (d, 1H), 5.21 (s, 2H), 4.26 (t, 2H), 4.08 (t, 2H), 3.97 (s, 3H), 3.96 (s, 3H), 2.26 (s, 3H); MS for C₃₀H₂₅FN₄O₆S: *m*/*z* 589.0 (MH+).

**N-[4-[(6,7-Dimethoxy-1,5-naphthyridin-4-yl)oxy]phenyl]-7-(4-methylthiophen-2-yl)-8-oxo-3,4-dihydro-1H-pyrido[2,1-c][1,4]oxazine-9-carboxamide (303):** Intermediate **I-1** was replaced with Intermediate **I-2** and Compound **286** was replaced with Compound **289.** ¹H NMR (400MHz, DMSO-*d*₆) δ 12.72 (s, 1H), 8.54 (m, 2H), 7.77 (d, 2H), 7.64 (s, 1H), 7.58 (s, 1H), 7.20 - 7.18 (m, 3H), 6.79 (d, 1H), 5.22 (s, 2H), 4.25 (t, 2H), 4.09 (t, 2H), 3.97 (s, 3H), 3.95 (s, 3H), 2.27 (s, 3H); MS for C₃₀H₂₆N₄O₆S*: m*/*z* 571.2 (MH+).

**N-[4-[(6,7-Dimethoxy-1,5-naphthyridin-4-yl)oxy]-3-fluorophenyl]-7-(5-methylthiophen-2-yl)-8-oxo-3,4-dihydro-1H-pyrido[2,1-c][1,4]oxazine-9-carboxamide (304):** Compound **286** was replaced with Compound **290.** ¹H NMR (400MHz, DMSO-*d*₆) δ 12.98 (s, 1H), 8.56 (d, 1H), 8.51 (s, 1H), 7.99 (d, 1H), 7.66 (s, 1H), 7.55 (d, 1H), 7.43 (d, 1H), 7.36 (t, 1H), 6.84 - 6.83 (m, 2H), 5.22 (s, 2H), 4.26 (t, 2H), 4.08 (t, 2H), 3.98 (s, 3H), 3.96 (s, 3H), 2.48 (s, 3H); MS for C₃₀H₂₅FN₄O₆S: *m*/*z* 589.1 (MH+).

**N-[4-[(6,7-Dimethoxy-1,5-naphthyridin-4-yl)oxy]phenyl]-7-(5-methylthiophen-2-yl)-8-oxo-3,4-dihydro-1H-pyrido[2,1-c][1,4]oxazine-9-carboxamide (305):** Intermediate **I-1** was replaced with Intermediate **I-2** and Compound **286** was replaced with Compound **290.** ¹H NMR (400MHz, DMSO-*d*₆) δ 12.82 (s, 1H), 8.54 (d, 1H), 8.50 (s, 1H), 7.77 (d, 2H), 7.64 (s, 1H), 7.55 (d, 1H), 7.19 (d, 2H), 6.85 (s, 1H), 6.80 (d, 1H), 5.23 (s, 2H), 4.26 (t, 2H), 4.08 (t, 2H), 3.97 (s, 3H), 3.95 (s, 3H), 2.48 (s, 3H); MS for C₃₀H₂₆N₄O₆S: *m*/*z* 571.0 (MH+).

**N-[4-[(6,7-Dimethoxy-1,5-naphthyridin-4-yl)oxy]-3-fluorophenyl]-7-(furan-3-yl)-8-oxo-3,4-dihydro-1H-pyrido[2,1-c][1,4]oxazine-9-carboxamide (306):** Compound **286** was replaced with Compound **291.** ¹H NMR (400MHz, DMSO-*d*₆) δ 13.2 (s, 1H), 8.66 - 8.63 (m, 2H), 8.37 (s, 1H), 8.01 (d, 1H), 7.76 (d, 1H), 7.67 (s, 1H), 7.45 - 7.38 (m, 2H), 7.07 (d, 1H), 6.95 (d, 1H), 5.32 (s, 2H), 4.23 (t, 2H), 4.08 (t, 2H), 4.00 (s, 6H); MS for C₂₉H₂₃FN₄O₇: *m*/*z* 559.2 (MH+).

**N-[4-[(6,7-Dimethoxy-1,5-naphthyridin-4-yl)oxy]-3-fluorophenyl]-6-methyl-8-oxo-7-thiophen-3-yl-3,4-dihydro-1H-pyrido[2,1-c][1,4]oxazine-9-carboxamide (307):** Compound **286** was replaced with Compound **292.** ¹H NMR (400MHz, DMSO-*d*₆) δ 13.48 (s, 1H), 8.51 (d, 1H), 7.91 (d, 1H), 7.56 (s, 1H), 7.5 (m, 1H), 7.48 (d, 1H), 7.24 (bs, 1H), 7.15 (t, 1H), 7.07 (d, 1H), 6.73 (d, 1H), 5.58 (s, 2H), 4.15 - 4.04 (m, 7H), 3.5 (d, 3H), 2.37 (s, 3H); MS for C₃₀H₂₅FN₄O₆S*: m*/*z* 589.1 (MH+).

**N-[4-[(6,7-Dimethoxy-1,5-naphthyridin-4-yl)oxy]-3-fluorophenyl]-7-(furan-3-yl)-6-methyl-8-oxo-3,4-dihydro-1H-pyrido[2,1-c][1,4]oxazine-9-carboxamide (308):** Compound **286** was replaced with Compound **293.** ¹H NMR (400MHz, DMSO-*d*₆) δ 13.27 (s, 1H), 8.53 (d, 1H), 7.95 (br d, 1H), 7.75 (br s, 2H), 7.64 (s, 1H), 7.41 - 7.35 (m, 1H), 7.33 - 7.26 (m, 1H), 6.79 (br d, 1H), 6.53 (s, 1H), 5.27 (s, 2H), 4.14 (br d, 2H), 4.06 (br d, 2H), 3.95 (br d, 6H), 2.43 (s, 3H); MS for C₃₀H₂₅FN₄O₇*: m*/*z* 573.3 (MH+).

**N-[4-[(6,7-Dimethoxy-1,5-naphthyridin-4-yl)oxy]-3-fluorophenyl]-8-oxo-7-thiophen-2-yl-3,4-dihydro-1H-pyrido[2,1-c][1,4]oxazine-9-carboxamide (309):** Compound **286** was replaced with Compound **287.** ¹H NMR (400MHz, DMSO-*d*₆) δ 12.85 (s, 1H), 8.59 (s, 1H), 8.55 (d, 1H), 8.00 (s, 1H), 7.76 (d, 1H), 7.65 (s, 1H), 7.60 (d, 1H), 7.42 (s, 1H), 7.34 (s, 1H), 7.17 (t, 1H), 6.82 (d, 1H), 5.22 (s, 2H), 4.28 (t, 2H), 4.09 (t, 2H), 3.97 (s, 3H), 3.96 (s, 3H); MS for C₂₉H₂₃FN₄O₆S: *m*/*z* 575.0 (MH+).

**N-[4-[(6,7-Dimethoxy-1,5-naphthyridin-4-yl)oxy]phenyl]-7-(furan-3-yl)-8-oxo-3,4-dihydro-1H-pyrido[2,1-c][1,4]oxazine-9-carboxamide (310):** Intermediate **I-1** was replaced with Intermediate **I-2** and Compound **286** was replaced with Compound **291.** ¹H NMR (400MHz, DMSO-*d*₆) δ 13.00 (s, 1H), 8.63 (s, 1H), 8.54 (d, 1H), 8.35 (s, 1H), 7.78 - 7.76 (m, 3H), 7.64 (s, 1H), 7.19 (d, 2H), 7.06 (d, 1H), 6.79 (d, 1H), 5.24 (s, 2H), 4.25 (t, 2H), 4.08 (t, 2H), 3.97 (s, 3H), 3.95 (s, 3H); MS for C₂₉H₂₄N₄O₇*: m*/*z* 541.2 (MH+).

**N-[4-[(6,7-Dimethoxy-1,5-naphthyridin-4-yl)oxy]phenyl]-7-(furan-3-yl)-6-methyl-8-oxo-3,4-dihydro-1H-pyrido[2,1-c][1,4]oxazine-9-carboxamide (311):** Intermediate **I-1** was replaced with Intermediate **I-2** and Compound **286** was replaced with Compound **293.** MS for C₃₀H₂₆N₄O₇*: m*/*z* 555.2 (MH+).

**N-[4-[(6,7-Dimethoxy-1,5-naphthyridin-4-yl)oxy]phenyl]-6-methyl-8-oxo-7-thiophen-3-yl-3,4-dihydro-1H-pyrido[2,1-c][1,4]oxazine-9-carboxamide (312):** Intermediate **I-1** was replaced with Intermediate **I-2** and Compound **286** was replaced with Compound **292.** MS for C₃₀H₂₆N₄O₆S: *m*/*z* 571.2 (MH+).

**N-[4-[(6,7-Dimethoxy-1,5-naphthyridin-4-yl)oxy]phenyl]-6-methyl-7-(4-methylthiophen-2-yl)-8-oxo-3,4-dihydro-1H-pyrido[2,1-c][1,4]oxazine-9-carboxamide (313):** Intermediate **I-1** was replaced with Intermediate **I-2** and Compound **286** was replaced with Compound **294.** MS for C₃₁H₂₈N₄O₆S: *m*/*z* 585.2 (MH+).

**N-[4-[(6,7-Dimethoxy-1,5-naphthyridin-4-yl)oxy]-3-fluorophenyl]-7-(furan-2-yl)-6-methyl-8-oxo-3,4-dihydro-1H-pyrido[2,1-c][1,4]oxazine-9-carboxamide (314):** Compound **286** was replaced with Compound 295. MS for C₃₀H₂₅FN₄O₇*: m*/*z* 573.2 (MH+).

**N-[4-[(6,7-Dimethoxy-1,5-naphthyridin-4-yl)oxy]phenyl]-7-(furan-2-yl)-6-methyl-8-oxo-3,4-dihydro-1H-pyrido[2,1-c][1,4]oxazine-9-carboxamide (315):** Intermediate **I-1** was replaced with Intermediate **I-2** and Compound **286** was replaced with Compound **295.** MS for C₃₀H₂₆N₄O₇*: m*/*z* 555.2 (MH+).

**N-[4-[(6,7-Dimethoxy-1,5-naphthyridin-4-yl)oxy]phenyl]-6-methyl-8-oxo-7-thiophen-2-yl-3,4-dihydro-1H-pyrido[2,1-c][1,4]oxazine-9-carboxamide (316):** Intermediate **I-1** was replaced with Intermediate **I-2** and Compound **286** was replaced with Compound **296.** MS for C₃₀H₂₆N₄O₆S: *m*/*z* 571.2 (MH+).

**N-[4-[(6,7-Dimethoxy-1,5-naphthyridin-4-yl)oxy]-3-fluorophenyl]-6-methyl-8-oxo-7-thiophen-2-yl-3,4-dihydro-1H-pyrido[2,1-c][1,4]oxazine-9-carboxamide (317):** Compound **286** was replaced with Compound **296.** MS for C₃₀H₂₅FN₄O₆S*: m*/*z* 589.4 (MH+).

**N-[4-[(6,7-Dimethoxy-1,5-naphthyridin-4-yl)oxy]-3-fluorophenyl]-6-methyl-7-(4-methylthiophen-2-yl)-8-oxo-3,4-dihydro-1H-pyrido[2,1-c][1,4]oxazine-9-carboxamide (318):** Compound 286 was replaced with Compound 294. MS for C₃₁H₂₇FN₄O₆S*: m*/*z* 603.3 (MH+).

### Example 58: N-[4-[(6,7-Dimethoxy-1,5-naphthyridin-4-yl)oxy]-3-fluorophenyl]-2-ethyl-5-(furan-2-yl)-1,6-dimethyl-4-oxopyridine-3-carboxamide (324)

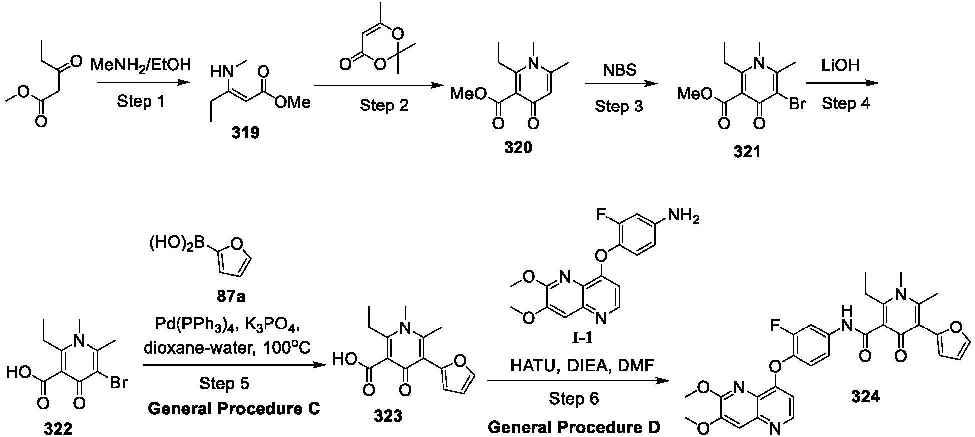

**Step 1: Methyl 3-(methylamino)pent-2-enoate (319):** A mixture of methyl 3-oxopentanoate (20 g, 154 mmol, 1 eq) and 33% methylamine in EtOH (50 mL, 154 mmol) was stirred at 25°C for 30 min. The reaction mixture was concentrated under reduced pressure to give crude Compound 319 as a yellow oil (20 g, 91% yield) which was used without further purification.

**Steps 2-6: N-[4-[(6,7-Dimethoxy-1,5-naphthyridin-4-yl)oxy]-3-fluorophenyl]-2-ethyl-5-(furan-2-yl)-1,6-dimethyl-4-oxopyridine-3-carboxamide (324):** Compound 324 was synthesized in 5 steps from Compound 319 using the same 5 step sequence exemplified by the synthesis of Compound **160** in **Example 32.** ¹H NMR (400MHz, DMSO-*d*₆) δ 10.83 (s, 1H), 8.45 (d, 1H), 7.87 (dd, 1H), 7.65 (dd, 1H), 7.58 (s, 1H), 7.41 (ddd, 1H), 7.28 (t, 1H), 6.70 (dd, 1H), 6.49 (dd, 1H), 6.45 (dd, 1H), 3.91 (s, 3H), 3.90 (s, 3H), 3.60 (s, 3H), 2.75 (q, 2H), 2.28 (s, 3H), 1.18 (t, 3H); MS for C₃₀H₂₇FN₄O₆: *m*/*z* 559.2 (MH+).

The following compounds were made using the same procedure as exemplified by the synthesis of Compound **160** in **Example 32:**

**N-[4-[(6,7-Dimethoxy-1,5-naphthyridin-4-yl)oxy]phenyl]-2-ethyl-5-(furan-2-yl)-1,6-dimethyl-4-oxopyridine-3-carboxamide (325):** Intermediate **I-1** was replaced with Intermediate **I-2** in Step 6. ¹H NMR (400MHz, DMSO-*d*₆) δ 10.61 (s, 1H), 8.44 (d, 1H), 7.77 - 7.68 (m, 2H), 7.67 - 7.62 (m, 1H), 7.57 (s, 1H), 7.16 - 7.07 (m, 2H), 6.67 (d, 1H), 6.49 (dd, 1H), 6.45 (dd, 1H), 3.90 (s, 3H), 3.90 (s, 3H), 3.59 (s, 3H), 2.75 (q, 2H), 2.27 (s, 3H), 1.18 (t, 3H); MS for C₃₀H₂₈N₄O₆: *m*/*z* 541.1 (MH+).

**2-Ethyl-N-[3-fluoro-4-[[6-methoxy-7-(2-methoxyethoxy)-1,5-naphthyridin-4-yl]oxy]phenyl]-5-(furan-2-yl)-1,6-dimethyl-4-oxopyridine-3-carboxamide (326):** Intermediate **I-1** was replaced with Compound **17** in Step 6. ¹H NMR (400MHz, DMSO-*d*₆) δ 10.83 (s, 1H), 8.45 (d, 1H), 7.87 (dd, 1H), 7.65 (dd, 1H), 7.61 (s, 1H), 7.41 (ddd, 1H), 7.28 (t, 1H), 6.70 (dd, 1H), 6.49 (dd, 1H), 6.45 (dd, 1H), 4.28 - 4.21 (m, 2H), 3.92 (s, 3H), 3.72 - 3.65 (m, 2H), 3.59 (s, 3H), 3.27 (s, 3H), 2.74 (q, 2H), 2.28 (s, 3H), 1.18 (t, 3H); MS for C₃₂H₃₁FN₄O₇: *m*/*z* 603.2 (MH+).

### Example 59: N-[4-[(6,7-Dimethoxy-1,5-naphthyridin-4-yl)oxy]-3-fluorophenyl]-1-(2-fluoroethyl)-6-methyl-4-oxo-5-thiophen-2-ylpyridine-3-carboxamide (327)

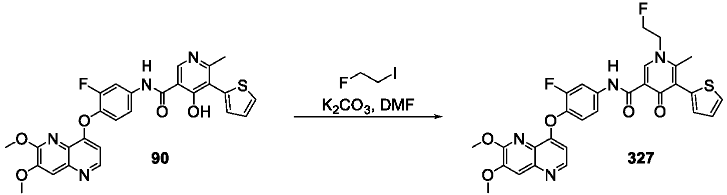

**N-[4-[(6,7-Dimethoxy-1,5-naphthyridin-4-yl)oxy]-3-fluorophenyl]-1-(2-fluoroethyl)-6-methyl-4-oxo-5-thiophen-2-ylpyridine-3-carboxamide (327):** Compound **327** was synthesized from Compound **90** using the same method as that used to make Compound **66** from Compound **61** in **Example 18.** ¹H NMR (400MHz, DMSO-*d*₆) δ 12.88 (s, 1H), 8.67 (s, 1H), 8.50 (s, 1H), 7.94 (dd, 1H), 7.62 (dd, 1H), 7.59 (s, 1H), 7.37 (dd, 1H), 7.27 (dd, 1H), 7.09 (dd, 1H), 6.95 (dd, 1H), 6.76 (d, 1H), 4.83 (t, 1H), 4.72 (t, 1H), 4.62 (t, 1H), 4.55 (t, 1H), 3.90 (s, 3H), 3.87 (s, 3H), 2.31 (s, 3H); MS for C₂₉H₂₄F₂N₄O₅S: *m*/*z* 579.1 (MH+).

The following compounds were made using the same procedure as exemplified by the synthesis of Compound **327** in **Example 59:**

**1-(2-Fluoroethyl)-N-[3-fluoro-4-[(7-methoxy-1,5-naphthyridin-4-yl)oxy]phenyl]-6-methyl-5-(4-methylthiophen-2-yl)-4-oxopyridine-3-carboxamide (328):** Compound **90** was replaced with Compound **102.** ¹H NMR (400MHz, DMSO-*d*₆) δ 13.01 (s, 1H), 8.75 (d, 1H), 8.74 (s, 1H), 8.71 (d, 1H), 8.05 (dd, 1H), 7.80 (d, 1H), 7.49 (dd, 1H), 7.43 (t, 1H), 7.25 (t, 1H), 6.83 (d, 1H), 6.76 (dd, 1H), 4.90 (t, 1H), 4.78 (t, 1H), 4.68 (t, 1H), 4.62 (t, 1H), 4.01 (s, 3H), 2.39 (s, 3H), 2.26 (d, 3H); MS for C₂₉H₂₄F₂N₄O₄S: *m*/*z* 563.1 (MH+).

**1-(2-Fluoroethyl)-N-[3-fluoro-4-[(7-methoxy-1,5-naphthyridin-4-yl)oxy]phenyl]-6-methyl-5-(5-methylthiophen-2-yl)-4-oxopyridine-3-carboxamide (329):** Compound **90** was replaced with Compound **101.** MS for C₂₉H₂₄F₂N₄O₄S: *m*/*z* 563.1 (MH+).

**1-(2-Fluoroethyl)-N-[3-fluoro-4-[(7-methoxy-1,5-naphthyridin-4-yl)oxy]phenyl]-6-methyl-4-oxo-5-thiophen-3-ylpyridine-3-carboxamide (330):** Compound **90** was replaced with Compound 98.. ¹H NMR(400MHz, DMSO-*d*₆) δ 13.08 (s, 1H), 8.68 (d, 1H), 8.66 (s, 1H), 8.64 (d, 1H), 7.98 (dd, 1H), 7.73 (d, 1H), 7.55 (dd, 1H), 7.46 - 7.37 (m, 2H), 7.35 (t, 1H), 7.02 (dd, 1H), 6.68 (d, 1H), 4.86 - 4.79 (m, 1H), 4.74 - 4.67 (m, 1H), 4.65 - 4.59 (m, 1H), 4.58 - 4.52 (m, 1H), 3.94 (s, 3H), 2.25 (s, 3H); MS for C₂₈H₂₂F₂N₄O₄S: *m*/*z* 549.1 (MH+).

**1-(2-Fluoroethyl)-N-[3-fluoro-4-[(7-methoxy-1,5-naphthyridin-4-yl)oxy]phenyl]-6-methyl-4-oxo-5-thiophen-2-ylpyridine-3-carboxamide (331):** Compound **90** was replaced with Compound **100.** ¹H NMR (400MHz, DMSO-*d*₆) δ 12.92 (s, 1H), 8.71 - 8.66 (m, 2H), 8.64 (d, 1H), 7.98 (dd, 1H), 7.73 (d, 1H), 7.63 (dd, 1H), 7.43 (dd, 1H), 7.35 (dd, 1H), 7.10 (dd, 1H), 6.96 (dd, 1H), 6.69 (dd, 1H), 4.84 (t, 1H), 4.72 (t, 1H), 4.63 (t, 1H), 4.56 (t, 1H), 3.94 (s, 3H), 2.32 (s, 3H); MS for C₂₈H₂₂F₂N₄O₄S: *m*/*z* 549.1 (MH+).

**N-[4-[(6,7-Dimethoxy-1,5-naphthyridin-4-yl)oxy]-3-fluorophenyl]-1-(2-fluoroethyl)-6-methyl-4-oxo-5-thiophen-3-ylpyridine-3-carboxamide (332):** Compound **90** was replaced with Compound 93. ¹H NMR (400MHz, DMSO-*d*₆) δ 13.04 (s, 1H), 8.65 (s, 1H), 8.48 (d, 1H), 7.94 (dd, 1H), 7.58 (s, 1H), 7.55 (dd, 1H), 7.39 (dd, 1H), 7.36 (ddd, 1H), 7.26 (t, 1H), 7.01 (dd, 1H), 6.75 (d, 1H), 4.82 (t, 1H), 4.70 (t, 1H), 4.64 - 4.59 (m, 1H), 4.58 - 4.52 (m, 1H), 3.90 (s, 3H), 3.87 (s, 3H), 2.25 (s, 3H); MS for C₂₉H₂₄F₂N₄O₅S: *m*/*z* 579.1 (MH+).

**N-[4-[(6,7-Dimethoxy-1,5-naphthyridin-4-yl)oxy]-3-fluorophenyl]-1-(2-fluoroethyl)-5-(furan-2-yl)-6-methyl-4-oxopyridine-3-carboxamide (333):** Compound **90** was replaced with Compound 89. ¹H NMR(400MHz, DMSO-*d*₆) δ 12.87 (s, 1H), 8.66 (s, 1H), 8.48 (d, 1H), 7.94 (dd, 1H), 7.72 (dd, 1H), 7.58 (s, 1H), 7.38 (dd, 1H), 7.28 (dd, 1H), 6.76 (d, 1H), 6.58 - 6.52 (m, 2H), 4.82 (t, 1H), 4.70 (t, 1H), 4.61 (t, 1H), 4.55 (t, 1H), 3.90 (s, 3H), 3.87 (s, 3H), 2.30 (s, 3H); MS for C₂₉H₂₄F₂N₄O₆: *m*/*z* 563.1 (MH+).

**1-(2-Fluoroethyl)-N-[3-fluoro-4-[(7-methoxy-1,5-naphthyridin-4-yl)oxy]phenyl]-5-(furan-3-yl)-6-methyl-4-oxopyridine-3-carboxamide (334):** Compound **90** was replaced with Compound 99. ¹H NMR(400MHz, DMSO-*d*₆) δ 13.14 (s, 1H), 8.75 (d, 1H), 8.73 - 8.68 (m, 2H), 8.05 (dd, 1H), 7.80 (d 1H), 7.79 - 7.74 (m, 2H), 7.48 (dd, 1H), 7.42 (dd, 1H), 6.75 (d, 1H), 6.60 - 6.56 (m, 1H), 4.88 (t, 1H), 4.76 (t, 1H), 4.69 (d, 1H), 4.62 (d, 1H), 4.00 (s, 3H), 2.42 (s, 3H); MS for C₂₈H₂₂F₂N₄O₅: *m*/*z* 533.1 (MH+).

### Example 60: N-[4-[[7-(2-Cyclobutylethoxy)-6-methoxy-1,5-naphthyridin-4-yl]oxy]-3-fluorophenyl]-5-(furan-2-yl)-1,2,6-trimethyl-4-oxopyridine-3-carboxamide (337)

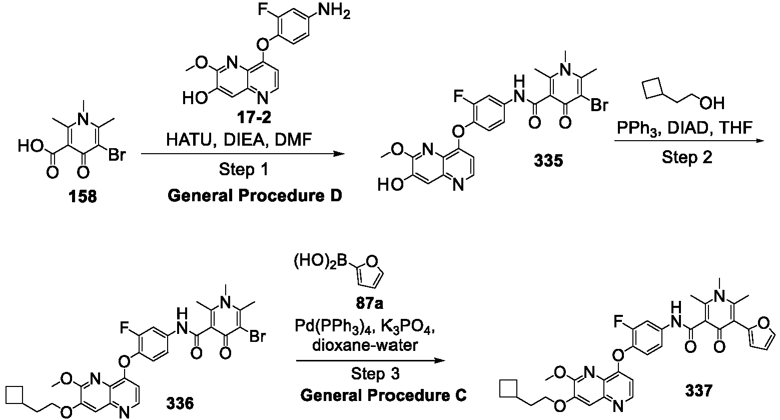

**Step 1: 5-Bromo-N-(3-fluoro-4-((7-hydroxy-6-methoxy-1,5-naphthyridin-4-yl)oxy)phenyl)-1,2,6-trimethyl-4-oxo-1,4-dihydropyridine-3-carboxamide (335):** Compound **335** was synthesized from Compound **158** and Compound 17-2 using **General Procedure D.** MS for C₂₄H₂₀BrFN₄O₅: *m*/*z* 543 (MH+).

**Step 2: 5-Bromo-N-(4-((7-(2-cyclobutylethoxy)-6-methoxy-1,5-naphthyridin-4-yl)oxy)-3-fluorophenyl)-1,2,6-trimethyl-4-oxo-1,4-dihydropyridine-3-carboxamide (336):** A mixture of Compound **335** (54 mg, 0.1 mmol, 1 eq), triphenylphosphine (78 mg, 0.30 mmol, 3.0 eq) and 2-cyclobutylethanol (11 mg, 0.11 mmol, 1.1eq) in THF (0.5 mL, 0.2M) was cooled to 0 °C in an ice bath followed by the addition of DIAD (61 mg, 0.3 mmol, 3.0 eq). The resulting mixture was allowed to warm to room temperature overnight with stirring. The reaction mixture was then concentrated under reduced pressure. EtOAc was added to the resulting residue and the solution with washed with water, dried over anhyd. Na₂SO₄ and concentrated under reduced pressure. The resulting residue was purified by flash column chromatography over silica gel (DCM/MeOH) to give Compound 336 as an oil (40 mg, 64% yield). ¹H NMR (400MHz, DMSO-*d*₆) δ 10.81 (s, 1H), 8.58 (d, 1H), 7.96 (dd, 1H), 7.55 - 7.43 (m, 2H), 7.40 (t, 1H), 6.86 (d, 1H), 4.14 (t, 2H), 4.01 (s, 3H), 3.67 (s, 3H), 2.70 (s, 3H), 2.46 (m, 1H), 2.42 (s, 3H), 2.08 (dt, 2H), 1.93 (q, 2H), 1.89 - 1.78 (m, 2H), 1.78 - 1.61 (m, 2H); MS for C₃₀H₃₀BrFN₄O₅: *m*/*z* 627 (MH+).

**Step 3: N-[4-[[7-(2-Cyclobutylethoxy)-6-methoxy-1,5-naphthyridin-4-yl]oxy]-3-fluorophenyl]-5-(furan-2-yl)-1,2,6-trimethyl-4-oxopyridine-3-carboxamide (337):** Compound **337** was synthesized from Compound **336** using **General Procedure C.** ¹H NMR (400MHz, DMSO-*d*₆) δ 10.96 (s, 1H), 8.44 (d, 1H), 7.88 (dd, 1H), 7.65 (d, 1H), 7.55 (s, 1H), 7.44 - 7.34 (m, 1H), 7.27 (t, 1H), 6.69 (dd, 1H), 6.55 - 6.34 (m, 2H), 4.04 (t, 2H), 3.91 (s, 3H), 3.56 (s, 3H), 2.42 (s, 3H), 2.38 (d, 1H), 2.27 (s, 3H), 2.01 (m, 2H), 1.85 (t, 2H), 1.83 - 1.73 (m, 2H), 1.66 (ddd, 2H); MS for C₃₄H₃₃FN₄O₆: *m*/*z* 613 (MH+).

### Kinase Assays

### Example A: Kinase Assays

Kinase activity and compound inhibition were investigated using the HTRF ^{®} KinEase assay (Cisbio Cat # 62TK0PEB) per manufacturer's instructions. In short, compounds were delivered in 300 nL volumes at 10 different concentrations in DMSO (3% final) to empty 384-well assay plates (Corning cat # 3824). A mixture of enzyme, 1 µM biotynlated peptide substrate, and buffer in 10 µL volume was added. The assay was started upon the addition of ATP (at Km). The 10 µL reaction was incubated at room temperature. The reaction was stopped upon the addition of detection buffer containing streptavidin-XL665 (5 µL) and TK antibody-Eu3+ (5 µL). After a 60 min incubation at room temperature, the fluorescence at 665 nm and 620 nm was read on an Envision microplate reader (Perkin Elmer). Kinase activity normalized to DMSO (100% activity) and reference compound at 1 µM and (0% activity) was calculated using the fluorescence ratio 620/665 x 10,000. IC₅₀ values were calculated by nonlinear regression analysis using a 4-parameter logistic curve fit in ActivityBase XE (IDBS).

### Example B: Human AXL Kinase Assay

Human AXL (residues 464-885; CarnaBio, 1 ng/ well) was incubated with enzymatic buffer (Cisbio) supplemented with 5 mM MgCl₂, 1 mM DTT, and Supplemental Enzymatic Buffer (SEB; Cisbio). The mixture was added to the pre-plated compounds. The reaction was initiated upon addition of ATP at Km (1.0 µM). The reaction was incubated at room temperature for 50 min and stopped upon the addition of SA-XL665 and TK-antibody both diluted in EDTA-containing kinase detection buffer (Cisbio). The kinase activity was calculated as stated above and the IC₅₀ values were reported.

### Example C: Human MET Kinase Assay

Human MET (residues 956-1390; CarnaBio, 0.1 ng/ well) was incubated with enzymatic buffer (Cisbio) supplemented with 5 mM MgCl₂, 1 mM DTT and 1 mM MnCl₂. The mixture was added to the pre-plated compounds. The reaction was initiated upon addition of ATP at Km (3.0 µM). The reaction was incubated at room temperature for 40 min and stopped upon the addition of SA-XL665 and TK-antibody both diluted in EDTA-containing kinase detection buffer (Cisbio). The kinase activity was calculated as stated above and the IC₅₀ values were reported.

### Example D: Human MER Kinase Assay

Human MER (residues 528-999; CarnaBio, 1 ng/ well) was incubated with enzymatic buffer (Cisbio) supplemented with 5 mM MgCl₂ and 1 mM DTT. The mixture was added to the pre-plated compounds. The reaction was initiated upon addition of ATP at Km (40 µM). The reaction was incubated at room temperature for 60 min and stopped upon the addition of SA-XL665 and TK-antibody both diluted in EDTA-containing kinase detection buffer (Cisbio). The kinase activity was calculated as stated above and the IC₅₀ values were reported.

### Example E: Human KDR Kinase Assay

Human KDR (residues 790-1356; CarnaBio, 0.1 ng/ well) was incubated with enzymatic buffer (Cisbio) supplemented with 5 mM MgCl₂, 1 mM MnCl₂, and 1 mM DTT. The mixture was added to the pre-plated compounds. The reaction was initiated upon addition of ATP at Km (4.0 µM). The reaction was incubated at room temperature for 40 min and stopped upon the addition of SA-XL665 and TK-antibody both diluted in EDTA-containing kinase detection buffer (Cisbio). The kinase activity was calculated as stated above and the IC₅₀ values were reported.

### Example F: AXL Autophosphorylation ELISA in A-172 Cells

A-172 glioblastoma cells (ATCC #CRL-1620) were seeded at 2.5 x 10⁵ cells/well onto 24-well plates (Greiner #662165), in DMEM (Thermo Fisher #11995-040) containing 10% FBS (Thermo Fisher #26140-079), 1% MEM NEAA (Thermo Fisher #11140-050), 1% GlutaMax (Thermo Fisher #35050-061), and 1% Penicillin Streptomycin (Thermo Fisher #15140-122). A-172 cells were incubated at 37°C, 5% CO₂ for 24 h and then starved for 24 h in serum-free medium. Test compounds were serially diluted to produce an 8-point dose curve in fresh serum-free medium to a final concentration of 0.3% DMSO (vehicle) and added to the cells and incubated for 1 h. Cells were then stimulated with 1 µg/mL recombinant human Gas6 (R&D Systems #885-GSB-500) for 15 min, washed with cold PBS, and immediately lysed with 150 µL of cold 1X lysis buffer [20 mM Tris, 137 mM sodium chloride, 2 mM EDTA, 10% glycerol, 1% NP-40 alternative, 1 mM activated sodium orthovanadate, 1 mM PefaBloc SC (Sigma-Aldrich #11429868001), protease/phosphatase inhibitor tablet (Thermo Fisher #A32959)]. Lysates were collected and 100 µL/well added into the human phospho-AXL DuoSet IC ELISA (R&D Systems #DYC2228-2). Assay was performed according to manufacturer's instructions and sample phospho-AXL concentrations were extrapolated using human phospho-AXL control (R&D Systems #841645) as a standard. Positive control wells (100% activity) contained Gas6-stimulated, DMSO-treated cell lysates. Negative control wells (0% activity) contained Gas6-stimulated, reference inhibitor-treated cell lysates. IC₅₀ values were calculated by nonlinear regression analysis using a 4-parameter logistic curve fit in ActivityBase XE (IDBS).

### Example G: Met Autophosphorylation ELISA in PC-3 Cells

PC-3 prostate cancer cells (ATCC #CRL-1435) were seeded at 4 x 10⁴ cells/well onto 24-well plates (Greiner #662165), in DMEM (Thermo Fisher #11995-040) containing 10% FBS (Thermo Fisher #26140-079), 1% MEM NEAA (Thermo Fisher #11140-050), 1% GlutaMax (Thermo Fisher #35050-061), and 1% Penicillin Streptomycin (Thermo Fisher #15140-122). PC-3 cells were incubated at 37°C, 5% CO₂ for 24 h and then starved for 3 h in serum-free medium. Test compounds were serially diluted to produce an 8-point dose curve in fresh serum-free medium to a final concentration of 0.3% DMSO (vehicle) and added to the cells and incubated for 1 h. Cells were then stimulated with 100 ng/mL recombinant human HGF (R&D Systems #294-HG-250) for 10 min, washed with cold PBS, and immediately lysed with 130 µL of cold 1X lysis buffer [20 mM Tris, 137 mM sodium chloride, 2 mM EDTA, 10% glycerol, 1% NP-40 alternative, 1 mM activated sodium orthovanadate, 1 mM PefaBloc SC (Sigma-Aldrich #11429868001), protease/phosphatase inhibitor tablet (Thermo Fisher #A32959)]. Lysates were clarified by centrifugation and 100 µL/well added into the PathScan phospho-Met (panTyr) Sandwich ELISA (Cell Signaling Technology #7333). Assay was performed according to manufacturer's instructions. Positive control wells (100% activity) contained HGF-stimulated, DMSO-treated cell lysates. Negative control wells (0% activity) contained HGF-stimulated, reference inhibitor-treated cell lysates. IC₅₀ values were calculated by nonlinear regression analysis using a 4-parameter logistic curve fit in ActivityBase XE (IDBS).

### Example H: KDR Autophosphorylation ELISA in HUVEC Cells

Human umbilical vein endothelial cells or HUVEC (Lonza #C2519A) were seeded at 2 x 10⁴ cells/well onto 96-well plates (Corning #3904), in EGM-2 growth medium (Lonza #CC-3162) containing 1% Penicillin Streptomycin (Thermo Fisher #15140-122). HUVEC cells were incubated at 37°C, 5% CO₂ for 24 h and then starved for 24 h in serum-free EBM-2 basal medium (Lonza #CC-3156) containing 1% Penicillin Streptomycin. Test compounds were serially diluted to produce an 8-point dose curve in fresh serum-free medium to a final concentration of 0.3% DMSO (vehicle) and added to the cells and incubated for 1 h. Cells were then stimulated with 100 ng/mL recombinant human VEGF165 (R&D Systems #293-VE-500) for 5 min, washed with cold PBS, and immediately lysed with 130 µL of cold 1X lysis buffer [20 mM Tris, 137 mM sodium chloride, 2 mM EDTA, 10% glycerol, 1% NP-40 alternative, 1 mM activated sodium orthovanadate, 1 mM PefaBloc SC (Sigma-Aldrich #11429868001), protease/phosphatase inhibitor tablet (Thermo Fisher #A32959)]. Lysates were collected and 100 µL/well added into the human phospho-KDR DuoSet IC ELISA (R&D Systems #DYC1766-2). Assay was performed according to manufacturer's instructions and sample phospho-KDR concentrations were extrapolated using human phospho-KDR control (R&D Systems #841421) as a standard. Positive control wells (100% activity) contained VEGF165-stimulated, DMSO-treated cell lysates. Negative control wells (0% activity) contained non-stimulated cell lysates. IC₅₀ values were calculated by nonlinear regression analysis using a 4-parameter logistic curve fit in ActivityBase XE (IDBS).

### Example I: Mer Autophosphorylation ELISA in Transient Transfected 293A Cells

293A cells (Thermo Fisher #R70507) were seeded at 1.5 x 10⁶ cells/well onto 100 mm dish (Greiner #664169), in DMEM (Thermo Fisher #11995-040) containing 10% FBS (Thermo Fisher #26140-079), 1% MEM NEAA (Thermo Fisher #11140-050), 1% GlutaMax (Thermo Fisher #35050-061), and 1% Penicillin Streptomycin (Thermo Fisher #15140-122). 293A cells were incubated at 37°C, 5% CO₂ for 24 h and then transfected with 6 µg MERTK DNA (Genecopoeia #EX-Z8208-M02) using TransIT LT1 transfection reagent (Mirus-Bio #MIR2305). After 24 h incubation, the transfected 293A cells were seeded at 1 x 10⁵ cells/well onto 96-well plates (Corning #3904) in DMEM growth medium overnight. Test compounds were serially diluted to produce an 8-point dose curve in fresh serum-free medium to a final concentration of 0.3% DMSO (vehicle) and added to the cells and incubated for 1 h. Cells were then immediately lysed with 150 µL of cold 1X lysis buffer [20 mM Tris, 137 mM sodium chloride, 2 mM EDTA, 10% glycerol, 1% NP-40 alternative, 1 mM activated sodium orthovanadate, 1 mM PefaBloc SC (Sigma-Aldrich #11429868001), protease/phosphatase inhibitor tablet (Thermo Fisher #A32959)]. Lysates were clarified by centrifugation and 50 µL/well added into the human phospho-Mer DuoSet IC ELISA (R&D Systems #DYC2579-2). Assay was performed according to manufacturer's instructions and sample phospho-Mer concentrations were extrapolated using human phospho-Mer control (R&D Systems #841793) as a standard. Positive control wells (100% activity) contained DMSO-treated cell lysates. Negative control wells (0% activity) contained reference inhibitor-treated cell lysates. IC₅₀ values were calculated by nonlinear regression analysis using a 4-parameter logistic curve fit in ActivityBase XE (IDBS).

Compounds of the present disclosure, as exemplified in Examples 1-60, were assessed in each of the assays (Examples A-I). Results for the test compounds are summarized in Table 2 and Table 3. A, B, and C of Tables 2 and 3 have the following meanings: A = IC₅₀ ≤ 100 nM; B = 100 nM < IC₅₀ ≤ 300 nM; C = 300 nM < IC₅₀ ≤ 1µM

**Table 2. Biological Activities of Selected Compounds**

| **Compound No.** | **Axl IC₅₀ (nM)** | **Mer IC₅₀ (nM)** | **c-Met IC₅₀ (nM)** | **KDR IC₅₀ (nM)** |
|---|---|---|---|---|
| 41 | A | A | A | A |
| 42 | A | A | A | A |
| 43 | A | A | A | A |
| 44 | A | A | A | A |
| 45 | A | A | A | B |
| 46 | A | A | A | C |
| 47 | A | A | B | C |
| 48 | A | A | A | C |
| 49 | NT | A | A | B |
| 50 | A | A | A | A |
| 51 | A | A | A | B |
| 52 | A | A | A | C |
| 53 | A | A | A | C |
| 54 | NT | NT | NT | NT |
| 54A | NT | A | B | C |
| 56 | NT | A | A | C |
| 59 | A | A | A | C |
| 68 | A | A | A | A |
| 69 | A | A | A | A |
| 70 | A | A | A | A |
| 71 | NT | A | NT | C |
| 72 | NT | B | C | C |
| 73 | NT | B | C | C |
| 85 | A | A | A | A |
| 86 | A | A | A | C |
| 214 | A | NT | A | NT |
| 216 | A | NT | A | NT |
| 217 | A | NT | A | NT |
| 218 | A | NT | A | NT |
| 220 | A | NT | A | NT |
| 221 | A | NT | A | NT |

| | | | | |
|---|---|---|---|---|
| NT means not tested | | | | |

**Table 3. Cellular Activities of Selected Compounds**

| **Compound No.** | **Axl IC₅₀ (nM)** | **Mer IC₅₀ (nM)** | **c-Met IC₅₀ (nM)** | **KDR IC₅₀ (nM)** |
|---|---|---|---|---|
| 41 | A | A | A | A |
| 42 | A | A | A | A |
| 43 | A | A | A | A |
| 44 | A | A | A | A |
| 45 | A | A | A | A |
| 46 | A | A | A | A |
| 47 | A | A | A | A |
| 48 | A | A | A | C |
| 49 | A | A | A | A |
| 50 | A | A | A | A |
| 51 | A | A | A | A |
| 52 | A | A | A | A |
| 53 | A | A | A | A |
| 54 | A | A | A | A |
| 54A | B | A | A | B |
| 55 | A | A | A | C |
| 56 | A | A | A | C |
| 59 | A | A | A | B |
| 68 | A | A | A | A |
| 69 | A | A | A | A |
| 70 | A | A | A | A |
| 71 | A | A | A | C |
| 72 | A | A | A | C |
| 73 | A | A | A | C |
| 85 | A | A | A | A |
| 86 | A | A | A | C |
| 89 | A | A | A | A |
| 90 | A | A | A | A |
| 91 | A | A | A | A |
| 92 | A | A | A | A |
| 93 | A | A | A | A |
| 94 | A | A | A | A |
| 95 | A | A | A | A |
| 96 | A | A | A | A |
| 97 | A | A | B | A |
| 98 | A | A | A | A |
| 99 | A | A | A | A |
| 100 | A | A | A | A |
| 101 | A | A | A | A |
| 102 | A | A | A | A |
| 103 | A | A | A | A |
| 104 | A | A | A | A |
| 105 | A | A | B | A |
| 106 | A | A | A | A |
| 107 | A | A | A | A |
| 108 | A | A | A | A |
| 109 | A | A | A | A |
| 110 | A | A | A | A |
| 111 | A | A | A | A |
| 112 | A | A | A | A |
| 115 | A | A | A | A |
| 116 | A | A | A | A |
| 117 | A | A | A | A |
| 118 | A | A | A | A |
| 119 | A | A | A | A |
| 120 | A | A | A | A |
| 121 | NT | A | B | A |
| 122 | NT | A | A | A |
| 123 | A | A | A | A |
| 124 | NT | A | A | A |
| 125 | A | A | A | A |
| 126 | A | A | A | A |
| 127 | A | A | A | A |
| 128 | A | A | A | A |
| 129 | A | A | A | A |
| 135 | A | A | A | A |
| 136 | B | A | A | A |
| 137 | A | A | A | A |
| 138 | A | A | A | A |
| 139 | A | A | A | A |
| 140 | A | A | A | A |
| 141 | A | A | A | A |
| 142 | A | A | A | A |
| 143 | A | A | A | A |
| 147 | A | A | A | A |
| 148 | A | A | A | A |
| 151 | A | A | A | A |
| 152 | B | A | B | A |
| 153 | A | A | B | A |
| 154 | A | A | A | A |
| 155 | A | A | A | A |
| 155A | A | A | A | A |
| 155B | A | A | A | A |
| 160 | A | A | A | B |
| 161 | NT | A | A | C |
| 162 | NT | A | A | C |
| 163 | A | A | A | C |
| 164 | A | A | A | C |
| 165 | A | A | A | C |
| 166 | A | A | A | C |
| 167 | A | A | A | C |
| 168 | A | A | A | A |
| 195 | A | A | A | A |
| 196 | NT | A | A | A |
| 197 | NT | A | A | A |
| 198 | NT | A | A | A |
| 200 | A | A | A | A |
| 212 | NT | NT | B | NT |
| 213 | NT | NT | C | NT |
| 214 | A | A | A | A |
| 215 | A | A | A | C |
| 216 | A | A | A | A |
| 217 | A | A | A | A |
| 218 | A | A | A | A |
| 219 | A | A | A | A |
| 220 | A | A | A | A |
| 221 | A | A | A | A |
| 222 | A | A | A | A |
| 223 | A | A | A | A |
| 224 | NT | A | A | A |
| 225 | NT | A | A | C |
| 226 | A | A | A | A |
| 227 | A | A | A | A |
| 228 | A | A | A | A |
| 229 | A | A | A | A |
| 230 | A | A | A | A |
| 231 | A | A | A | B |
| 232 | A | A | A | C |
| 233 | A | A | A | A |
| 234 | A | A | A | B |
| 235 | A | A | A | C |
| 236 | A | A | C | C |
| 237 | A | A | C | C |
| 238 | B | C | C | C |
| 239 | NT | C | C | C |
| 240 | A | A | C | C |
| 243 | A | A | A | B |
| 244 | A | A | A | C |
| 245 | A | A | A | A |
| 246 | A | A | A | B |
| 247 | C | A | A | B |
| 248 | C | C | A | C |
| 254 | A | A | A | A |
| 255 | A | A | A | A |
| 256 | A | A | A | A |
| 257 | A | A | A | A |
| 258 | A | A | A | A |
| 259 | A | A | A | A |
| 260 | A | A | A | A |
| 261 | A | B | C | C |
| 262 | A | A | C | C |
| 263 | A | B | C | C |
| 264 | A | A | A | C |
| 265 | NT | C | C | C |
| 266 | NT | A | A | C |
| 269 | C | A | A | C |
| 275 | NT | A | A | C |
| 297 | A | A | A | C |
| 298 | A | A | C | C |
| 299 | A | A | A | C |
| 300 | NT | A | A | B |
| 301 | NT | A | A | C |
| 302 | A | A | A | A |
| 303 | A | A | B | C |
| 304 | A | A | A | C |
| 305 | A | A | A | C |
| 306 | A | A | A | C |
| 307 | A | A | A | B |
| 308 | NT | A | A | C |
| 309 | A | A | A | C |
| 310 | A | A | C | C |
| 311 | NT | A | A | NT |
| 312 | NT | A | A | NT |
| 313 | NT | A | B | NT |
| 314 | NT | A | A | NT |
| 315 | NT | A | A | NT |
| 316 | NT | A | A | NT |
| 324 | A | A | A | A |
| 325 | NT | A | C | C |
| 326 | NT | A | A | A |
| 327 | A | A | A | B |
| 328 | A | A | A | C |
| 329 | A | A | A | C |
| 330 | A | A | A | C |
| 331 | A | A | A | C |
| 332 | A | A | A | A |
| 333 | A | A | A | B |
| 334 | A | A | A | C |
| 337 | A | A | A | A |

| | | | | |
|---|---|---|---|---|
| NT means not tested | | | | |

Unless otherwise defined, all technical and scientific terms used herein have the same meaning as commonly understood by one of ordinary skill in the art to which this invention belongs.

The inventions illustratively described herein may suitably be practiced in the absence of any element or elements, limitation or limitations, not specifically disclosed herein. Thus, for example, the terms "comprising", "including," "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed.

## Claims

1. A compound of Formula (II): 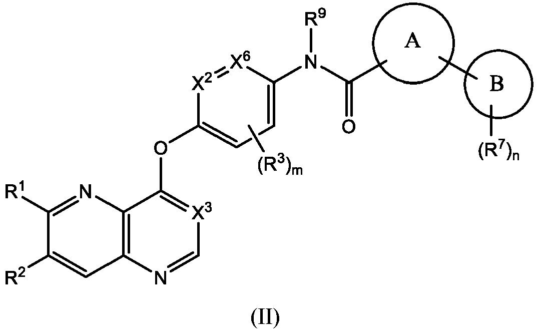 or a pharmaceutically acceptable salt or stereoisomer thereof, wherein:
ring A is 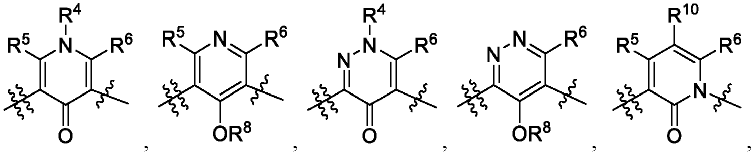 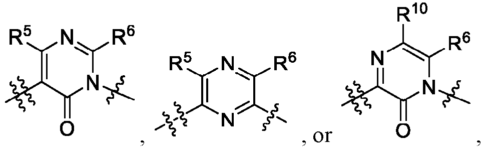 wherein the single wavy line indicates the point of attachment to the ring B and the double wavy line indicates the point of attachment to the rest of the molecule;
ring B is 5-membered heteroaryl having 1, 2 or 3 heteroatoms as ring members selected from N, O and S, 9-10-membered heteroaryl having 1, 2 or 3 heteroatoms as ring members selected from N, O and S, or 6-membered heteroaryl having 1 or 2 nitrogen atoms as ring members;
X² is CH or CR³;
X³ is CH;
X⁶ is CH or CR³;
R¹ and R² are each independently selected from H, C₁₋₆ alkoxy, and C₁₋₆ alkoxy-C₁₋₆ alkoxy;
each R³ is halo;
R⁴ is selected from H, halo, C₁₋₆ alkyl, C₂₋₆ alkenyl, C₂₋₆ alkynyl, C₁₋₆ haloalkyl, C₁₋₆ haloalkoxy, C₆₋₁₀ aryl, C₃₋₁₄ cycloalkyl, 5-14 membered heteroaryl, 4-14 membered heterocycloalkyl, C₆₋₁₀ aryl-C₁₋₄ alkylene-, C₃₋₁₄ cycloalkyl-C₁₋₄ alkylene-, (5-14 membered heteroaryl)-C₁₋₄ alkylene-, (4-14 membered heterocycloalkyl)-C₁₋₄ alkylene-, CN, NO₂, OR^{a}, SR^{a}, NHOR^{a}, C(O)R^{a}, C(O)NR^{a}R^{a}, C(O)OR^{a}, C(O)NR^{a}S(O)₂R^{a}, OC(O)R^{a}, OC(O)NR^{a}R^{a}, NHR^{a}, NR^{a}R^{a}, NR^{a}C(O)R^{a}, N=C(NR^{a}R^{a})₂, NR^{a}C(=NR^{a})R^{a}, NR^{a}C(O)OR^{a}, NR^{a}C(O)NR^{a}R^{a}, C(=NR^{a})R^{a}, C(=NOH)R^{a}, C(=NOH)NR^{a}, C(=NCN)NR^{a}R^{a} , NR^{a}C(=NCN)NR^{a}R^{a}, C(=NR^{a})NR^{a}R^{a}, NR^{a}C(=NR^{a})NR^{a}R^{a}, NR^{a}S(O)R^{a}, NR^{a}S(O)₂R^{a}, NR^{a}S(O)₂NR^{a}R^{a}, S(O)R^{a}, S(O)NR^{a}R^{a}, S(O)₂R^{a}, S(O)₂NR^{a}C(O)R^{a}, P(O)R^{a}R^{a}, P(O)(OR^{a})(OR^{a}), B(OH)₂, B(OR^{a})₂, and S(O)₂NR^{a}R^{a}, wherein the C₁₋₆ alkyl, C₂₋₆ alkenyl, C₂₋₆ alkynyl, C₆₋₁₀ aryl, C₃₋₁₄ cycloalkyl, 5-14 membered heteroaryl, 4-14 membered heterocycloalkyl, C₆₋₁₀ aryl-C₁₋₄ alkylene-, C₃₋₁₄ cycloalkyl-C₁₋₄ alkylene-, (5-14 membered heteroaryl)-C₁₋₄ alkylene-, and (4-14 membered heterocycloalkyl)-C₁₋₄ alkylene- of R⁴ are each optionally substituted with 1, 2, 3, 4 or 5 independently selected R^{b} substituents;
R⁵, R⁶ and R¹⁰ are each independently H, halo, C₁₋₆ alkyl, C₂₋₆ alkenyl, C₁₋₆ alkoxy, C₁₋₆ alkylthio, CN, C₁₋₄ haloalkyl, C₁₋₄ haloalkoxy, OH, C₁₋₄alkyl-C(O)-, C₁₋₄alkyl-OC(O)-, -CONH(C₁₋₄ alkyl), NH₂, -NHC₁₋₄alkyl, or -N(C₁₋₄ alkyl)₂, wherein the C₁₋₆ alkyl, C₂₋₆ alkenyl, C₁₋₆ alkoxy, C₁₋₆ alkylthio, C₁₋₆ alkyl-C(O)- and C₁₋₄ alkyl of -NH(C₁₋₄alkyl), or -N(C₁₋₄ alkyl)₂ of R⁵, R⁶ and R¹⁰ are each optionally substituted with 1 or 2 independently selected R^{g} substituents;
each R⁷ is independently selected from halo, OH, C(O)OR^{a}, C(O)R^{a}, C(O)NR^{a}R^{a}, CN, NH₂, - NH(C₁₋C₆ alkyl), -N(C₁₋C₆ alkyl)₂, C₁₋C₆ alkyl, C₂₋C₆ alkenyl, C₁₋C₆ alkoxy, C₁₋C₆ haloalkyl, C₁₋C₆ haloalkoxy, C(O)NR^{a}R^{a}, NR^{a}C(O)R^{a}, NR^{a}C(O)NR^{a}R^{a}, SO₂R^{a}, NR^{a}S(O)₂R^{a}, NR^{a}S(O)₂NR^{a}R^{a}, C₃₋C₆ cycloalkyl, 4- to 6-membered heterocycloalkyl, phenyl, 5- or 6-membered heteroaryl, C₃₋C₆ cycloalkyl-C₁₋C₄ alkylene-, (4- to 6-membered heterocycloalkyl)-C₁₋C₄ alkylene-, phenyl-C₁₋C₂ alkylene, and (5- or 6-membered heteroaryl)-C₁₋C₄ alkylene-; wherein the C₁₋C₆ alkyl, C₁₋C₆ alkoxy, C₃₋C₆ cycloalkyl, 4- to 6-membered heterocycloalkyl, phenyl, 5- or 6-membered heteroaryl, C₃₋C₆ cycloalkyl-C₁₋C₄ alkylene-, (4- to 6-membered heterocycloalkyl)-C₁₋C₄ alkylene-, phenyl-C₁₋C₂ alkylene, and (5- or 6-membered heteroaryl)-C₁₋C₄ alkylene- of R⁷ are each optionally substituted with 1, 2, or 3 independently selected R^{f} substituents;
R⁸ is H or C₁₋₆ alkyl optionally substituted with 1 or 2 R^{g} substituents or a hydroxy protecting group;
R⁹ is H or C₁₋₆ alkyl optionally substituted with 1, 2, or 3 independently selected R^{g} substituents;
or R⁴ and R⁵ taken together with the atoms to which they are attached form 4- to 7-membered fused heterocycloalkyl or 5- to 6-membered fused heteroaryl, wherein the 4- to 7-membered fused heterocycloalkyl and 5- to 6-membered fused heteroaryl are each optionally substituted with 1 or 2 independently selected R^{g} substituents;
or R¹⁰ and R⁵ taken together with the atoms to which they are attached form fused C₃₋₇ cycloalkyl, 4- to 6-membered fused heterocycloalkyl, fused 5- or 6-membered heteroaryl or fused phenyl, wherein the fused C₃₋₇ cycloalkyl, 4- to 6-membered fused heterocycloalkyl, 5- or 6-membered heteroaryl, or fused phenyl is each optionally substituted with 1 or 2 independently selected R^{g} substituents and wherein one or two ring carbon atoms of the fused C₃₋₇ cycloalkyl or fused heterocycloalkyl are optionally replaced by a carbonyl group;
or R⁶ and R⁵ taken together with the atoms to which they are attached form fused C₃₋₇ cycloalkyl, 4- to 6-membered fused heterocycloalkyl, 5- to 6-membered fused heteroaryl, or fused phenyl, wherein the fused C₃₋₇ cycloalkyl, 4- to 6-membered fused heterocycloalkyl, 5- to 6-membered fused heteroaryl, and fused phenyl are each optionally substituted with 1 or 2 independently selected R^{g} substituents and wherein one or two ring carbon atoms of the fused C₃₋₇ cycloalkyl or 4- to 6-membered fused heterocycloalkyl are optionally replaced by a carbonyl;
or R⁶ and R⁴ taken together with the atoms to which they are attached form 4- to 7-membered fused heterocycloalkyl or 5- to 6-membered fused heteroaryl, wherein the 4- to 7-membered fused heterocycloalkyl and 5- to 6-membered fused heteroaryl are each optionally substituted with 1 or 2 independently selected R^{g} substituents;
or R⁶ and R¹⁰ taken together with the atoms to which they are attached form fused C₃₋₇ cycloalkyl, 4- to 6-membered fused heterocycloalkyl, 5- to 6-membered fused heteroaryl, or fused heteroaryl, wherein the fused C₃₋₇ cycloalkyl, 4- to 6-membered fused heterocycloalkyl, 5- to 6-membered fused heteroaryl, and fused heteroaryl are each optionally substituted with 1 or 2 independently selected R^{g} substituents and wherein one or two ring carbon atoms of the fused C₃₋₇ cycloalkyl or 4- to 6-membered fused heterocycloalkyl are optionally replaced by a carbonyl;
each R^{a} is independently selected from the group consisting of H, CN, C₁₋C₆ alkyl, C₁₋C₆ haloalkyl, C₂-C₆ alkenyl, C₂-C₆ alkynyl, C₆-C₁₀ aryl, C₃-C₁₀ cycloalkyl, 5-14 membered heteroaryl, 4-14 membered heterocycloalkyl, C₆-C₁₀ aryl-C₁₋C₄ alkylene-, C₃-C₁₀ cycloalkyl-C₁₋C₄ alkylene-, (5-14 membered heteroaryl)-C₁₋C₄ alkylene-, and (4-14 membered heterocycloalkyl)-C₁₋C₄ alkylene-; wherein the C₁₋C₆ alkyl, C₁₋C₆ haloalkyl, C₂-C₆ alkenyl, C₂-C₆ alkynyl, C₆-C₁₀ aryl, C₃-C₁₀ cycloalkyl, 5-14 membered heteroaryl, 4-14 membered heterocycloalkyl, C₆-C₁₀ aryl-C₁₋C₄ alkylene-, C₃-C₁₀ cycloalkyl-C₁₋C₄ alkylene-, (5-14 membered heteroaryl)-C₁₋C₄ alkylene-, and (4-14 membered heterocycloalkyl)-C₁₋C₄ alkylene- of R^{a} are each optionally substituted with 1, 2, 3, 4, or 5 independently selected R^{d} substituents;
or any two R^{a} substituents together with the nitrogen atom to which they are attached form 4-, 5-, 6-, 7-, 8-, 9-, or 10-membered heterocycloalkyl, each of which is optionally substituted with 1, 2, or 3 independently selected R^{f} substituents;
each R^{b} is independently selected from the group consisting of halo, C₁-C₆ alkyl, C₂-C₆ alkenyl, C₂-C₆ alkynyl, C₁-C₆ haloalkyl, C₁-C₆ haloalkoxy, C₆-C₁₀ aryl, C₃-C₁₀ cycloalkyl, 5-10 membered heteroaryl, 4-10 membered heterocycloalkyl, C₆₋C₁₀ aryl-C₁₋C₄ alkylene-, C₃₋C₁₀ cycloalkyl-C₁₋C₄ alkylene-, (5-10 membered heteroaryl)-C₁₋C₄ alkylene-, (4-10 membered heterocycloalkyl)-C₁₋C₄ alkylene-, CN, OH, NH₂, NO₂, NHOR^{c}, OR^{c}, SR^{c}, C(O)R^{c}, C(O)NR^{c}R^{c}, C(O)OR^{c}, C(O)NR^{c}S(O)₂R^{c}, OC(O)R^{c}, OC(O)NR^{c}R^{c}, C(=NOH)R^{c}, C(=NOH)NR^{c}, C(=NCN)NR^{c}R^{c}, NR^{c}C(=NCN)NR^{c}R^{c}, C(=NR^{c})NR^{c}R^{c}, NR^{c}C(=NR^{c})NR^{c}RV, NHR^{c}, NR^{c}R^{c}, NR^{c}C(O)R^{c}, NR^{c}C(=NR^{c})R^{c}, NR^{c}C(O)OR^{c}, NR^{c}C(O)NR^{c}R^{c}, NR^{c}S(O)R^{c}, NR^{c}S(O)₂R^{c}, NR^{c}S(O)₂NR^{c}R^{c}, S(O)R^{c}, S(O)NR^{c}R^{c}, S(O)₂R^{c}, S(O)₂NR^{c}C(O)R^{c}, Si(R^{c})₃, P(O)R^{c}R^{c}, P(O)(OR^{c})(OR^{c}), B(OH)₂, B(OR^{c})₂, and S(O)₂NR^{c}R^{c}; wherein the C₁-C₆ alkyl, C₂-C₆ alkenyl, C₂-C₆ alkynyl, C₆-C₁₀ aryl, C₃-C₁₀ cycloalkyl, 5-10 membered heteroaryl, 4-10 membered heterocycloalkyl, C₆-C₁₀ aryl-C₁₋C₄ alkylene-, C₃₋C₁₀ cycloalkyl-C₁₋C₄alkylene-, (5-10 membered heteroaryl)-C₁₋C₄ alkylene-, and (4-10 membered heterocycloalkyl)-C₁-C₄ alkylene- of R^{b} are each further optionally substituted with 1, 2, or 3 independently selected R^{d} substituents;
each R^{c} is independently selected from the group consisting of H, C₁-C₆ alkyl, C₁-C₆ haloalkyl, C₂-C₆ alkenyl, C₂-C₆ alkynyl, C₆-C₁₀ aryl, C₃-C₁₀ cycloalkyl, 5-10 membered heteroaryl, 4-10 membered heterocycloalkyl, C₆-C₁₀ aryl-C₁₋C₄ alkylene-, C₃-C₁₀ cycloalkyl-C₁₋C₄ alkylene-, (5-10 membered heteroaryl)-C₁₋C₄ alkylene-, and (4-10 membered heterocycloalkyl)-C₁₋C₄ alkylene-; wherein the C₁-C₆ alkyl, C₂-C₆ alkenyl, C₂-C₆ alkynyl, C₆-C₁₀ aryl, C₃-C₁₀ cycloalkyl, 5-10 membered heteroaryl, 4-10 membered heterocycloalkyl, C₆-C₁₀ aryl-C₁₋C₄ alkylene-, C₃-C₁₀ cycloalkyl-C₁₋C₄ alkylene-, (5-10 membered heteroaryl)-C₁₋C₄ alkylene-, and (4-10 membered heterocycloalkyl)-C₁₋C₄ alkylene- of R^{c} are each optionally substituted with 1, 2, 3, 4, or 5 independently selected R^{f} substituents;
or any two R^{c} substituents together with the nitrogen atom to which they are attached form 4-, 5-, 6-, 7-, 8-, 9-, or 10-membered heterocycloalkyl, each of which is optionally substituted with 1, 2, or 3 independently selected R^{f} substituents;
each R^{d} is independently selected from the group consisting of C₁₋C₆ alkyl, C₁₋C₆ haloalkyl, halo, C₆₋C₁₀ aryl, 5-10 membered heteroaryl, C₃₋C₁₀ cycloalkyl, 4-10 membered heterocycloalkyl, C₆₋C₁₀ aryl-C₁₋C₄ alkylene-, C₃₋C₁₀ cycloalkyl-C₁₋C₄ alkylene-, (5-10 membered heteroaryl)-C₁₋C₄ alkylene-, (4-10 membered heterocycloalkyl)-C₁₋C₄ alkylene-, CN, NH₂, NHOR^{e}, OR^{e}, SR^{e}, C(O)R^{e}, C(O)NR^{e}R^{e}, C(O)OR^{e}, OC(O)R^{e}, OC(O)NR^{e}R^{e}, NHR^{e}, NR^{e}R^{e}, NR^{e}C(O)R^{e}, NR^{e}C(O)NR^{e}R^{e}, NR^{e}C(O)OR^{e}, C(=NR^{e})NR^{e}R^{e}, NR^{e}C(=NR^{e})NR^{e}R^{e}, NR^{e}C(=NOH)NR^{e}R^{e}, NR^{e}C(=NCN)NR^{e}R^{e}, S(O)R^{e}, S(O)NR^{e}R^{e}, S(O)₂R^{e}, NR^{e}S(O)₂R^{e}, NR^{e}S(O)₂NR^{e}R^{e}, and S(O)₂NR^{e}R^{e}; wherein the C₁₋C₆ alkyl, C₆₋C₁₀ aryl, 5-10 membered heteroaryl, C₃₋C₁₀ cycloalkyl, 4-10 membered heterocycloalkyl, C₆₋C₁₀ aryl-C₁₋C₄ alkylene-, C₃₋C₁₀ cycloalkyl-C₁₋C₄ alkylene-, (5-10 membered heteroaryl)-C₁₋C₄ alkylene-, and (4-10 membered heterocycloalkyl)-C₁₋C₄ alkylene- of R^{d} are each optionally substituted with 1, 2, or 3 independently selected R^{f} substituents;
each R^{e} is independently selected from the group consisting of H, C₁₋C₆ alkyl, C₁₋C₆ alkoxy, C₃₋C₆ cycloalkyl, C₃₋C₆ cycloalkyl-C₁₋C₄ alkylene-, C₆₋C₁₀ aryl, C₆₋C₁₀ aryl-C₁₋C₄ alkylene-, 5- or 6-membered heteroaryl, (5- or 6-membered heteroaryl)-C₁₋C₄ alkylene-, 4-7-membered heterocycloalkyl, (4-7-membered heterocycloalkyl)-C₁₋C₄ alkylene-, C₁₋C₆ haloalkyl, C₁₋C₆ haloalkoxy, C₂₋C₄ alkenyl, and C₂₋C₄ alkynyl, wherein the C₁₋C₆ alkyl, C₁₋C₆ alkoxy, C₃₋C₆ cycloalkyl, C₆₋C₁₀ aryl, 5 or 6-membered heteroaryl, 4-7-membered heterocycloalkyl, C₆₋C₁₀ aryl-C₁₋C₄ alkylene-, (5- or 6-membered heteroaryl)-C₁₋C₄ alkylene-, (4-7-membered heterocycloalkyl)-C₁₋C₄ alkylene-, C₂₋C₄ alkenyl, and C₂₋C₄ alkynyl of R^{e} are each optionally substituted with 1, 2, or 3 R^{f} substituents;
or any two R^{e} substituents together with the nitrogen atom to which they are attached form a 4-, 5-, 6-, 7-, 8-, 9-, or 10-membered heterocycloalkyl, each of which is optionally substituted with 1, 2, or 3 independently selected R^{f} substituents;
each R^{f} is independently selected from the group consisting of halo, OH, CN, C(O)OH, NH₂, -NH(C₁₋C₆ alkyl), -N(C₁₋C₆ alkyl)₂, C₁₋C₆ alkyl, vinyl, C₁₋C₆ alkoxy, C₁₋C₆ alkylthio, C₁₋C₆ haloalkyl, C₁₋C₆ haloalkoxy, phenyl, 5-6 membered heteroaryl, 4-6 membered heterocycloalkyl, and C₃₋C₆ cycloalkyl, wherein the C₁₋C₆ alkyl, C₁₋C₆ alkoxy, C₁₋C₆ alkylthio, phenyl, C₃₋C₆ cycloalkyl, 4-6 membered heterocycloalkyl, and 5-6 membered heteroaryl of R^{f} are each optionally substituted with 1, 2, or 3 substituents selected from halo, OH, CN, -C(O)OH, -NH₂, C₁₋C₄ alkyl, C₁₋C₄ alkoxy, C₁₋C₄ haloalkyl, C₁₋C₄ haloalkoxy, phenyl, C₃₋C₁₀ cycloalkyl, 5-6 membered heteroaryl, and 4-6 membered heterocycloalkyl
each R^{g} is independently selected from the group consisting of halo, OH, CN, C(O)OH, - C(O)O-C₁₋C₄ alkyl, NH₂, -NH(C₁₋C₆ alkyl), -N(C₁₋C₆ alkyl)₂, C₁₋C₆ alkyl, C₁₋C₆ alkoxy, C₁₋C₆ alkylthio, C₁₋C₆ haloalkyl, C₁₋C₆ haloalkoxy, phenyl, 5-6 membered heteroaryl, 4-6 membered heterocycloalkyl, and C₃₋C₆ cycloalkyl;
a ring nitrogen atom in Formula (II) is optionally oxidized;
the subscript m is 0, 1 or 2; and
the subscript n is 0, 1, 2, 3 or 4.

2. The compound of claim 1, wherein ring B is 6-membered heteroaryl having 1 or 2 nitrogen atoms as ring members.

3. The compound of claim 1 or claim 2, or a pharmaceutically acceptable salt or stereoisomer thereof, wherein (i) ring B is 2-thienyl, 3-thienyl, 2-furyl, 3-furyl, 1H-pyrazol-3-yl, 1H-pyrazol-4-yl, 1H-pyrazol-5-yl or pyrazol-1-yl; or (ii) ring B is 2-pyridyl, 3-pyridyl, 4-pyridyl, or 3-pyridazinyl.

4. The compound of any one of claims 1 to 3, having formula (IIa): 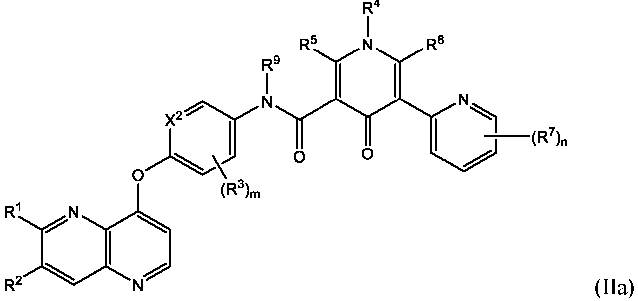 or a pharmaceutically acceptable salt or stereoisomer thereof;
said compound preferably having formula (Ia-1): 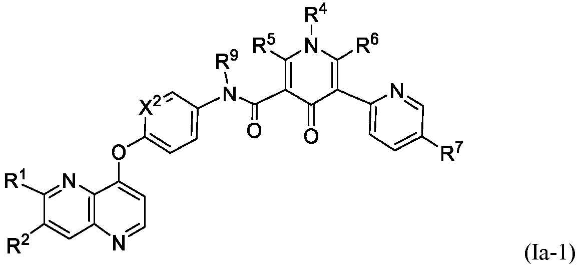 or a pharmaceutically acceptable salt or stereoisomer thereof.

5. The compound of any one of claims 1 to 3, having formula (IIb): 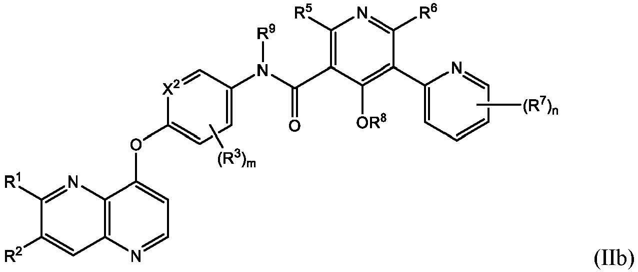 or a pharmaceutically acceptable salt or stereoisomer thereof;
said compound preferably having formula (Ib-1): 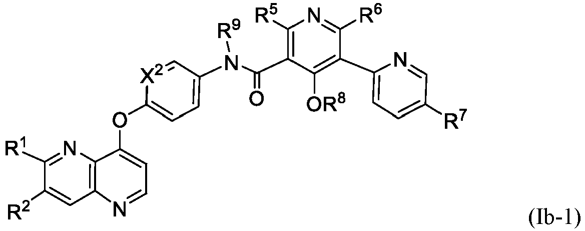 or a pharmaceutically acceptable salt or stereoisomer thereof.

6. The compound of any one of claims 1 to 3, having formula (IIc): 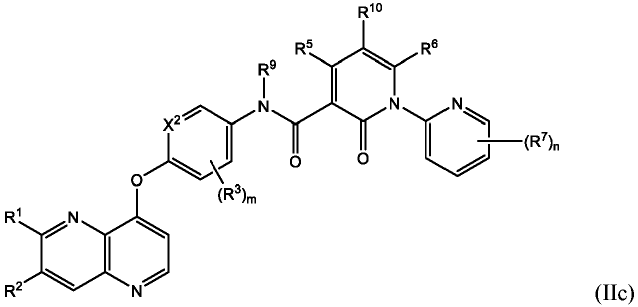 or a pharmaceutically acceptable salt or stereoisomer thereof;
said compound preferably having formula (Ic-1): 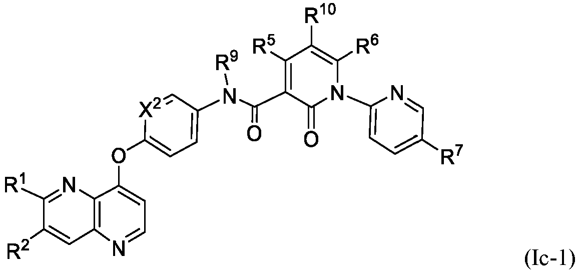 or a pharmaceutically acceptable salt or stereoisomer thereof.

7. The compound of any of one of claims 1 to 3, having:
i) f 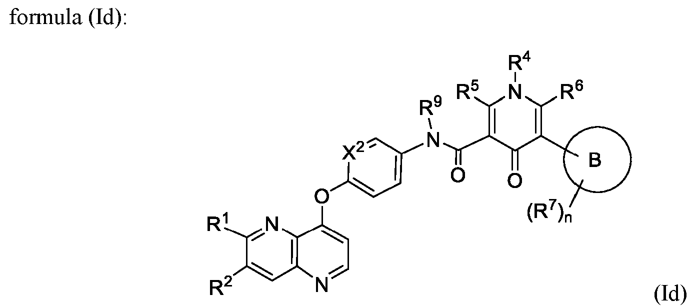 or a pharmaceutically acceptable salt or stereoisomer thereof; or
ii) 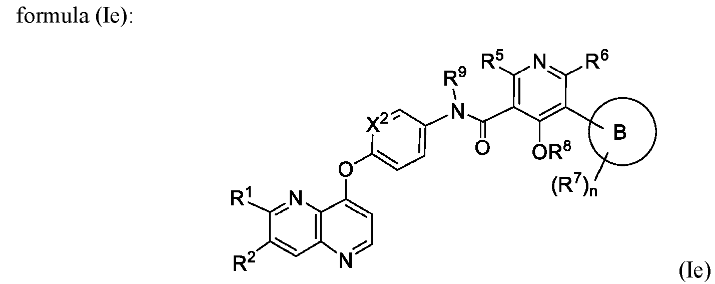 or a pharmaceutically acceptable salt or stereoisomer thereof; or
iii) 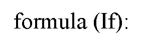 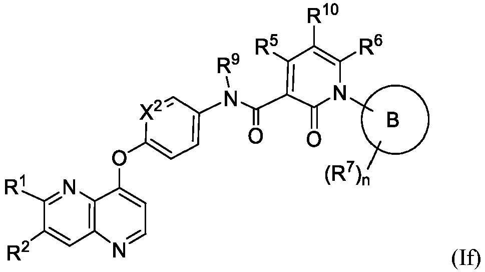 or a pharmaceutically acceptable salt or stereoisomer thereof; or
iv) 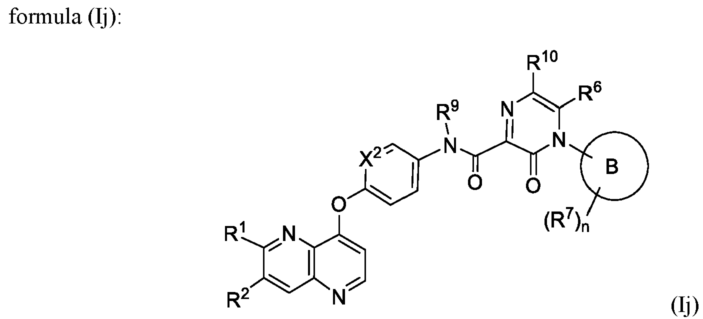 or a pharmaceutically acceptable salt or stereoisomer thereof; or
v) 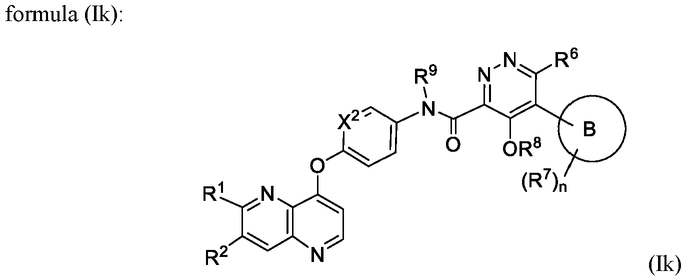 or a pharmaceutically acceptable salt or stereoisomer thereof; or
vi) 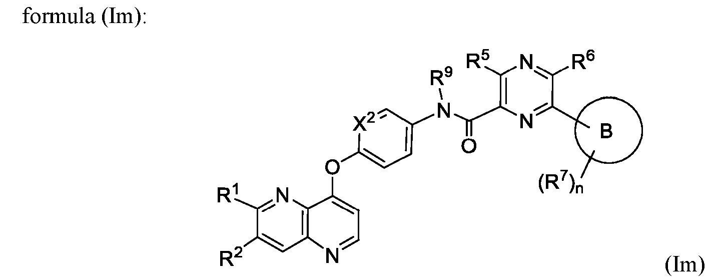 or a pharmaceutically acceptable salt or stereoisomer thereof.

8. The compound of claim 7, or a pharmaceutically acceptable salt or stereoisomer thereof, wherein ring B is:
i) 2-pyridyl, 3, pyridyl, 4-pyridyl or 5-membered heteroaryl having 1, 2 or 3 heteroatoms as ring members selected from N, O and S; or
ii) 2-thienyl, 3-thienyl, 2-furyl, 3-furyl, 2-benzofuranyl, 3-benzofuranyl, 1H-pyrazol-3-yl, 1H-pyrazol-4-yl, 1H-pyrazol-5-yl or pyrazol-1-yl.

9. The compound of any one of claims 1 to 8, or a pharmaceutically acceptable salt or stereoisomer thereof, wherein R⁹ is H or methyl.

10. The compound of any one of claims 1 to 9, or a pharmaceutically acceptable salt or stereoisomer thereof, wherein n is 0; or wherein n is 1, 2, 3, or 4, and each R⁷ is independently selected from halo, C₁₋₆ alkyl, and C₁₋₆ alkoxy.

11. The compound of any one of claims 1 to 10, or a pharmaceutically acceptable salt or stereoisomer thereof, wherein X² is CF.

12. The compound of any one of claims 1 to 11, or a pharmaceutically acceptable salt or stereoisomer thereof, wherein:
i) each R⁴ is independently selected from H, C₁₋₆ alkyl, C₁₋₆ alkoxy, OH, NH₂, -NH(C₁₋₆ alkyl), -N(C₁₋₆ alkyl)₂, C₃₋₆ cycloalkyl, C₁₋₆ haloalkyl, C₃₋₆ cycloalkyl-C₁₋₄ alkylene-, 4-6 membered heterocycloalkyl, (4-6 membered heterocycloalkyl)-C₁₋₄ alkylene-, 5-6 membered heteroaryl, (5-6 membered heteroaryl)-C₁₋₄ alkylene-, and N=C[N(C₁₋₆ alkyl)(C₁₋₆ alkyl)]₂, wherein the C₁₋₆ alkyl, C₁₋₆ alkoxy, -NH(C₁₋₆ alkyl), -N(C₁₋₆ alkyl)₂, C₃₋₆ cycloalkyl, C₃₋₆ cycloalkyl-C₁₋₄ alkylene-, 4-6 membered heterocycloalkyl, (4-6 membered heterocycloalkyl)-C₁₋₄ alkylene-, 5-6 membered heteroaryl, (5-6 membered heteroaryl)-C₁₋₄ alkylene-, and N=C[N(C₁₋₆ alkyl)(C₁₋₆ alkyl)]₂ of R⁴ are each optionally substituted with 1 or 2 independently selected R^{b} or R^{g} substituents; and/or
ii) R⁵, R⁶, and R¹⁰ are each independently selected from H, CH₃, propen-2-yl, Br, Cl, CN, methoxy, 2-fluoroethyl, isopropyl, CH₃C(O)-, OH, t-butyl, ethyl, hydroxymethyl, isopropylthio, and methoxymethyl.

13. The compound of any preceding claim, selected from
N-[4-[(6,7-dimethoxy-1,5-naphthyridin-4-yl)oxy]-3-fluorophenyl]-1-(5-fluoropyridin-2-yl)-4-methyl-2-oxopyridine-3-carboxamide;
N-[4-[(6,7-dimethoxy-1,5-naphthyridin-4-yl)oxy]-3-fluorophenyl]-1-(5-fluoropyridin-2-yl)-2-oxopyridine-3-carboxamide;
N-[4-[(6,7-dimethoxy-1,5-naphthyridin-4-yl)oxy]-3-fluorophenyl]-1-(5-fluoropyridin-2-yl)-6-methyl-2-oxopyridine-3-carboxamide;
N-[4-[(6,7-dimethoxy-1,5-naphthyridin-4-yl)oxy]phenyl]-1-(5-fluoropyridin-2-yl)-6-methyl-2-oxopyridine-3-carboxamide;
N-[3-fluoro-4-[(7-methoxy-1,5-naphthyridin-4-yl)oxy]phenyl]-1-(5-fluoropyridin-2-yl)-6-methyl-2-oxopyridine-3-carboxamide;
1-(5-fluoropyridin-2-yl)-N-[4-[(7-methoxy-1,5-naphthyridin-4-yl)oxy]phenyl]-6-methyl-2-oxopyridine-3-carboxamide;
N-[3-fluoro-4-[(6-methoxy-1,5-naphthyridin-4-yl)oxy]phenyl]-1-(5-fluoropyridin-2-yl)-6-methyl-2-oxopyridine-3-carboxamide;
N-[3-fluoro-4-(1,5-naphthyridin-4-yloxy)phenyl]-1-(5-fluoropyridin-2-yl)-6-methyl-2-oxopyridine-3-carboxamide;
N-[3-fluoro-4-[[6-methoxy-7-(2-methoxyethoxy)-1,5-naphthyridin-4-yl]oxy]phenyl]-1-(5-fluoropyridin-2-yl)-6-methyl-2-oxopyridine-3-carboxamide;
N-[4-[(6,7-dimethoxy-1,5-naphthyridin-4-yl)oxy]-3-fluorophenyl]-1-(5-fluoro-3-methylpyridin-2-yl)-6-methyl-2-oxopyridine-3-carboxamide;
N-[3-fluoro-4-[(7-methoxy-1,5-naphthyridin-4-yl)oxy]phenyl]-1-(5-fluoro-3-methylpyridin-2-yl)-6-methyl-2-oxopyridine-3-carboxamide;
N-[3-fluoro-4-[(6-methoxy-1,5-naphthyridin-4-yl)oxy]phenyl]-1-(5-fluoro-3-methylpyridin-2-yl)-6-methyl-2-oxopyridine-3-carboxamide;
5-bromo-N-[4-[(6,7-dimethoxy-1,5-naphthyridin-4-yl)oxy]-3-fluorophenyl]-1-(5-fluoropyridin-2-yl)-6-methyl-2-oxopyridine-3-carboxamide;
5-cyano-N-[4-[(6,7-dimethoxy-1,5-naphthyridin-4-yl)oxy]-3-fluorophenyl]-1-(5-fluoropyridin-2-yl)-6-methyl-2-oxopyridine-3-carboxamide;
5-acetyl-N-[4-[(6,7-dimethoxy-1,5-naphthyridin-4-yl)oxy]-3-fluorophenyl]-1-(5-fluoropyridin-2-yl)-6-methyl-2-oxopyridine-3-carboxamide;
N-[4-[(6,7-dimethoxy-1,5-naphthyridin-4-yl)oxy]-3-fluorophenyl]-5-(5-fluoropyridin-2-yl)-4-hydroxy-2-methylpyridine-3-carboxamide;
N-[4-[(6,7-dimethoxy-1,5-naphthyridin-4-yl)oxy]-3-fluorophenyl]-4-hydroxy-2-methyl-5-pyridin-2-ylpyridine-3-carboxamide;
N-[3-fluoro-4-[(7-methoxy-1,5-naphthyridin-4-yl)oxy]phenyl]-4-hydroxy-2-methyl-5-pyridin-2-ylpyridine-3-carboxamide;
N-[4-[(6,7-dimethoxy-1,5-naphthyridin-4-yl)oxy]-3-fluorophenyl]-5-(5-fluoropyridin-2-yl)-1,2-dimethyl-4-oxopyridine-3-carboxamide;
N-[4-[(6,7-dimethoxy-1,5-naphthyridin-4-yl)oxy]phenyl]-5-(5-fluoropyridin-2-yl)-1,2-dimethyl-4-oxopyridine-3-carboxamide;
N-[3-fluoro-4-[(7-methoxy-1,5-naphthyridin-4-yl)oxy]phenyl]-5-(5-fluoropyridin-2-yl)-1,2-dimethyl-4-oxopyridine-3-carboxamide;
N-[4-[(6,7-dimethoxy-1,5-naphthyridin-4-yl)oxy]-3-fluorophenyl]-5-(5-fluoropyridin-2-yl)-4-hydroxy-6-methylpyridine-3-carboxamide;
N-[4-[(6,7-dimethoxy-1,5-naphthyridin-4-yl)oxy]phenyl]-5-(5-fluoropyridin-2-yl)-1,6-dimethyl-4-oxopyridine-3-carboxamide;
N-[4-[(6,7-dimethoxy-1,5-naphthyridin-4-yl)oxy]-3-fluorophenyl]-5-(furan-2-yl)-4-hydroxy-6-methylpyridine-3-carboxamide;
N-[4-[(6,7-dimethoxy-1,5-naphthyridin-4-yl)oxy]-3-fluorophenyl]-4-hydroxy-6-methyl-5-thiophen-2-ylpyridine-3-carboxamide;
N-[4-[(6,7-dimethoxy-1,5-naphthyridin-4-yl)oxy]-3-fluorophenyl]-4-hydroxy-6-methyl-5-(4-methylthiophen-2-yl)pyridine-3-carboxamide;
N-[4-[(6,7-dimethoxy-1,5-naphthyridin-4-yl)oxy]-3-fluorophenyl]-4-hydroxy-6-methyl-5-(5-methylfuran-2-yl)pyridine-3-carboxamide;
N-[4-[(6,7-dimethoxy-1,5-naphthyridin-4-yl)oxy]-3-fluorophenyl]-4-hydroxy-6-methyl-5-thiophen-3-ylpyridine-3-carboxamide;
N-[4-[(6,7-dimethoxy-1,5-naphthyridin-4-yl)oxy]-3-fluorophenyl]-4-hydroxy-6-methyl-5-(5-methylthiophen-2-yl)pyridine-3-carboxamide;
N-[4-[(6,7-dimethoxy-1,5-naphthyridin-4-yl)oxy]-3-fluorophenyl]-5-(furan-3-yl)-4-hydroxy-6-methylpyridine-3-carboxamide;
N-[3-fluoro-4-[(7-methoxy-1,5-naphthyridin-4-yl)oxy]phenyl]-5-(furan-2-yl)-4-hydroxy-6-methylpyridine-3-carboxamide;
N-[3-fluoro-4-[(7-methoxy-1,5-naphthyridin-4-yl)oxy]phenyl]-4-hydroxy-6-methyl-5-(5-methylfuran-2-yl)pyridine-3-carboxamide;
N-[3-fluoro-4-[(7-methoxy-1,5-naphthyridin-4-yl)oxy]phenyl]-4-hydroxy-6-methyl-5-thiophen-3-ylpyridine-3-carboxamide;
N-[3-fluoro-4-[(7-methoxy-1,5-naphthyridin-4-yl)oxy]phenyl]-5-(furan-3-yl)-4-hydroxy-6-methylpyridine-3-carboxamide;
N-[3-fluoro-4-[(7-methoxy-1,5-naphthyridin-4-yl)oxy]phenyl]-4-hydroxy-6-methyl-5-thiophen-2-ylpyridine-3-carboxamide;
N-[3-fluoro-4-[(7-methoxy-1,5-naphthyridin-4-yl)oxy]phenyl]-4-hydroxy-6-methyl-5-(5-methylthiophen-2-yl)pyridine-3-carboxamide;
N-[3-fluoro-4-[(7-methoxy-1,5-naphthyridin-4-yl)oxy]phenyl]-4-hydroxy-6-methyl-5-(4-methylthiophen-2-yl)pyridine-3-carboxamide;
N-[4-[(6,7-dimethoxy-1,5-naphthyridin-4-yl)oxy]-3-fluorophenyl]-4-hydroxy-6-methyl-5-(3-methylthiophen-2-yl)pyridine-3-carboxamide;
N-[4-[(6,7-dimethoxy-1,5-naphthyridin-4-yl)oxy]-3-fluorophenyl]-4-hydroxy-6-methyl-5-(2-methylpyrazol-3-yl)pyridine-3-carboxamide;
N-[4-[(6,7-dimethoxy-1,5-naphthyridin-4-yl)oxy]-3-fluorophenyl]-4-hydroxy-6-methyl-5-[2-methyl-5-(trifluoromethyl)pyrazol-3-yl]pyridine-3-carboxamide;
N-[4-[(6,7-dimethoxy-1,5-naphthyridin-4-yl)oxy]-3-fluorophenyl]-4-hydroxy-2,6-dimethyl-5-thiophen-3-ylpyridine-3-carboxamide;
N-[4-[(6,7-dimethoxy-1,5-naphthyridin-4-yl)oxy]-3-fluorophenyl]-4-hydroxy-2,6-dimethyl-5-thiophen-2-ylpyridine-3-carboxamide;
N-[4-[(6,7-dimethoxy-1,5-naphthyridin-4-yl)oxy]-3-fluorophenyl]-4-hydroxy-2,6-dimethyl-5-(2-methylpyrazol-3-yl)pyridine-3-carboxamide;
N-[4-[(6,7-dimethoxy-1,5-naphthyridin-4-yl)oxy]-3-fluorophenyl]-5-(2,5-dimethylpyrazol-3-yl)-4-hydroxy-2,6-dimethylpyridine-3-carboxamide;
N-[4-[(6,7-dimethoxy-1,5-naphthyridin-4-yl)oxy]-3-fluorophenyl]-4-hydroxy-2,6-dimethyl-5-(5-methylfuran-2-yl)pyridine-3-carboxamide;
N-[4-[(6,7-dimethoxy-1,5-naphthyridin-4-yl)oxy]-3-fluorophenyl]-4-hydroxy-2,6-dimethyl-5-(5-methylthiophen-2-yl)pyridine-3-carboxamide;
N-[4-[(6,7-dimethoxy-1,5-naphthyridin-4-yl)oxy]-3-fluorophenyl]-5-(furan-3-yl)-4-hydroxy-2,6-dimethylpyridine-3-carboxamide;
N-[4-[(6,7-dimethoxy-1,5-naphthyridin-4-yl)oxy]-3-fluorophenyl]-4-hydroxy-2-(methoxymethyl)-6-methyl-5-thiophen-2-ylpyridine-3-carboxamide;
N-[4-[(6,7-dimethoxy-1,5-naphthyridin-4-yl)oxy]-3-fluorophenyl]-5-(furan-2-yl)-4-hydroxy-2-(methoxymethyl)-6-methylpyridine-3-carboxamide;
N-[4-[(6,7-dimethoxy-1,5-naphthyridin-4-yl)oxy]-3-fluorophenyl]-4-hydroxy-2-(methoxymethyl)-6-methyl-5-(5-methylfuran-2-yl)pyridine-3-carboxamide;
N-[3-fluoro-4-[(6-methoxy-1,5-naphthyridin-4-yl)oxy]phenyl]-5-(furan-2-yl)-4-hydroxy-6-methylpyridine-3-carboxamide;
N-[4-[(6,7-dimethoxy-1,5-naphthyridin-4-yl)oxy]-3-fluorophenyl]-5-(furan-2-yl)-4-hydroxy-2-methylpyridine-3-carboxamide;
N-[4-[(6,7-dimethoxy-1,5-naphthyridin-4-yl)oxy]-3-fluorophenyl]-4-hydroxy-2-methyl-5-(5-methylfuran-2-yl)pyridine-3-carboxamide;
N-[4-[(6,7-dimethoxy-1,5-naphthyridin-4-yl)oxy]-3-fluorophenyl]-5-(5-formylfuran-2-yl)-4-hydroxy-2-methylpyridine-3-carboxamide;
N-[4-[(6,7-dimethoxy-1,5-naphthyridin-4-yl)oxy]-3-fluorophenyl]-5-(furan-3-yl)-4-hydroxy-2-methylpyridine-3-carboxamide;
N-[4-[(6,7-dimethoxy-1,5-naphthyridin-4-yl)oxy]-3-fluorophenyl]-4-hydroxy-2-methyl-5-thiophen-2-ylpyridine-3-carboxamide;
N-[4-[(6,7-dimethoxy-1,5-naphthyridin-4-yl)oxy]-3-fluorophenyl]-4-hydroxy-2-methyl-5-(5-methylthiophen-2-yl)pyridine-3-carboxamide;
N-[4-[(6,7-dimethoxy-1,5-naphthyridin-4-yl)oxy]-3-fluorophenyl]-4-hydroxy-2-methyl-5-(4-methylthiophen-2-yl)pyridine-3-carboxamide;
N-[4-[(6,7-dimethoxy-1,5-naphthyridin-4-yl)oxy]-3-fluorophenyl]-4-hydroxy-2-methyl-5-(3-methylthiophen-2-yl)pyridine-3-carboxamide;
N-[4-[(6,7-dimethoxy-1,5-naphthyridin-4-yl)oxy]-3-fluorophenyl]-4-hydroxy-2-methyl-5-thiophen-3-ylpyridine-3-carboxamide;
N-[3-fluoro-4-[(6-methoxy-1,5-naphthyridin-4-yl)oxy]phenyl]-4-hydroxy-6-methyl-5-thiophen-2-ylpyridine-3-carboxamide;
5-(furan-2-yl)-4-hydroxy-N-[4-[(7-methoxy-1,5-naphthyridin-4-yl)oxy]phenyl]-6-methylpyridine-3-carboxamide;
N-[4-[(6,7-dimethoxy-1,5-naphthyridin-4-yl)oxy]-3-fluorophenyl]-5-(furan-2-yl)-4-hydroxy-2,6-dimethylpyridine-3-carboxamide;
N-[4-[(6,7-dimethoxy-1,5-naphthyridin-4-yl)oxy]-3-fluorophenyl]-5-(2,5-dimethylpyrazol-3-yl)-4-hydroxy-6-methylpyridine-3-carboxamide;
N-[4-[(6,7-dimethoxy-1,5-naphthyridin-4-yl)oxy]-3-fluorophenyl]-4-hydroxy-2,6-dimethyl-5-(4-methylthiophen-2-yl)pyridine-3-carboxamide;
N-[4-[(6,7-dimethoxy-1,5-naphthyridin-4-yl)oxy]-3-fluorophenyl]-4-hydroxy-2,6-dimethyl-5-[2-methyl-5-(trifluoromethyl)pyrazol-3-yl]pyridine-3-carboxamide;
N-[4-[(6,7-dimethoxy-1,5-naphthyridin-4-yl)oxy]-3-fluorophenyl]-4-hydroxy-2,6-dimethyl-5-(3-methylthiophen-2-yl)pyridine-3-carboxamide;
N-[4-[(6,7-dimethoxy-1,5-naphthyridin-4-yl)oxy]-3-fluorophenyl]-4-hydroxy-6-methyl-5-(1,2-oxazol-4-yl)pyridine-3-carboxamide;
N-[4-[(6,7-dimethoxy-1,5-naphthyridin-4-yl)oxy]-3-fluorophenyl]-4-hydroxy-6-methyl-5-(1,3-thiazol-5-yl)pyridine-3-carboxamide;
N-[4-[(6,7-dimethoxy-1,5-naphthyridin-4-yl)oxy]-3-fluorophenyl]-4-hydroxy-6-methyl-5-(1,3-thiazol-4-yl)pyridine-3-carboxamide;
N-[4-[(6,7-dimethoxy-1,5-naphthyridin-4-yl)oxy]-3-fluorophenyl]-4-hydroxy-6-methyl-5-(1,3-thiazol-2-yl)pyridine-3-carboxamide;
N-[4-[(6,7-dimethoxy-1,5-naphthyridin-4-yl)oxy]-3-fluorophenyl]-5-(5-ethylfuran-2-yl)-4-hydroxy-2,6-dimethylpyridine-3-carboxamide;
N-[4-[(6,7-dimethoxy-1,5-naphthyridin-4-yl)oxy]-3-fluorophenyl]-4-hydroxy-2,6-dimethyl-5-(5-propan-2-ylfuran-2-yl)pyridine-3-carboxamide;
N-[4-[(6,7-dimethoxy-1,5-naphthyridin-4-yl)oxy]-3-fluorophenyl]-5-(5-ethenylfuran-2-yl)-4-hydroxy-6-methylpyridine-3-carboxamide;
N-[4-[(6,7-dimethoxy-1,5-naphthyridin-4-yl)oxy]-3-fluorophenyl]-4-hydroxy-6-methyl-5-(5-prop-1-en-2-ylfuran-2-yl)pyridine-3-carboxamide;
N-[4-[(6,7-dimethoxy-1,5-naphthyridin-4-yl)oxy]-3-fluorophenyl]-5-(5-ethylfuran-2-yl)-4-hydroxy-6-methylpyridine-3-carboxamide;
N-[4-[(6,7-dimethoxy-1,5-naphthyridin-4-yl)oxy]-3-fluorophenyl]-4-hydroxy-6-methyl-5-(5-propan-2-ylfuran-2-yl)pyridine-3-carboxamide;
N-[4-[(6,7-dimethoxy-1,5-naphthyridin-4-yl)oxy]-3-fluorophenyl]-4-hydroxy-6-methyl-5-(5-prop-2-enylfuran-2-yl)pyridine-3-carboxamide;
N-[4-[(6,7-dimethoxy-1,5-naphthyridin-4-yl)oxy]-3-fluorophenyl]-5-(furan-2-yl)-1,2,6-trimethyl-4-oxopyridine-3-carboxamide;
5-(5-cyclopropylfuran-2-yl)-N-[4-[(6,7-dimethoxy-1,5-naphthyridin-4-yl)oxy]-3-fluorophenyl]-4-hydroxy-6-methylpyridine-3-carboxamide;
5-(5-cyclopropylfuran-2-yl)-N-[4-[(6,7-dimethoxy-1,5-naphthyridin-4-yl)oxy]-3-fluorophenyl]-4-hydroxy-2,6-dimethylpyridine-3-carboxamide;
5-(furan-2-yl)-N-[4-[(7-methoxy-1,5-naphthyridin-4-yl)oxy]phenyl]-1,2,6-trimethyl-4-oxopyridine-3-carboxamide;
N-[3-fluoro-4-[(7-methoxy-1,5-naphthyridin-4-yl)oxy]phenyl]-5-(furan-2-yl)-1,2,6-trimethyl-4-oxopyridine-3-carboxamide;
N-[4-[(6,7-dimethoxy-1,5-naphthyridin-4-yl)oxy]-2,5-difluorophenyl]-5-(furan-2-yl)-1,2,6-trimethyl-4-oxopyridine-3-carboxamide;
N-[4-[(6,7-dimethoxy-1,5-naphthyridin-4-yl)oxy]phenyl]-5-(furan-2-yl)-1,2,6-trimethyl-4-oxopyridine-3-carboxamide;
N-[4-[(7-methoxy-1,5-naphthyridin-4-yl)oxy]phenyl]-1,2,6-trimethyl-4-oxo-5-thiophen-2-ylpyridine-3-carboxamide;
N-[3-fluoro-4-[(7-methoxy-1,5-naphthyridin-4-yl)oxy]phenyl]-1,2,6-trimethyl-4-oxo-5-thiophen-2-ylpyridine-3-carboxamide;
N-[4-[(6,7-dimethoxy-1,5-naphthyridin-4-yl)oxy]phenyl]-1,2,6-trimethyl-4-oxo-5-thiophen-2-ylpyridine-3-carboxamide;
N-[4-[(6,7-dimethoxy-1,5-naphthyridin-4-yl)oxy]-3-fluorophenyl]-1,2,6-trimethyl-4-oxo-5-thiophen-2-ylpyridine-3-carboxamide;
N-[4-[(6,7-dimethoxy-1,5-naphthyridin-4-yl)oxy]-3-fluorophenyl]-4-hydroxy-2-(methoxymethyl)-6-methyl-5-pyridin-2-ylpyridine-3-carboxamide;
N-[4-[(6,7-dimethoxy-1,5-naphthyridin-4-yl)oxy]-3-fluorophenyl]-4-hydroxy-2-(methoxymethyl)-6-methyl-5-(3-methylpyridin-2-yl)pyridine-3-carboxamide;
N-[4-[(6,7-dimethoxy-1,5-naphthyridin-4-yl)oxy]-3-fluorophenyl]-4-hydroxy-2-(methoxymethyl)-6-methyl-5-(6-methylpyridin-2-yl)pyridine-3-carboxamide;
N-[4-[(6,7-dimethoxy-1,5-naphthyridin-4-yl)oxy]-3-fluorophenyl]-4-hydroxy-2-(methoxymethyl)-6-methyl-5-(2-methylpyridin-4-yl)pyridine-3-carboxamide;
N-[4-[(6,7-dimethoxy-1,5-naphthyridin-4-yl)oxy]-3-fluorophenyl]-5-(5-fluoro-3-methylpyridin-2-yl)-4-hydroxy-2-methylpyridine-3-carboxamide;
N-[4-[(6,7-dimethoxy-1,5-naphthyridin-4-yl)oxy]-3-fluorophenyl]-1-(5-ethoxypyridin-2-yl)-6-methyl-2-oxopyridine-3-carboxamide;
1-(5-ethoxypyridin-2-yl)-N-[3-fluoro-4-[(7-methoxy-1,5-naphthyridin-4-yl)oxy]phenyl]-6-methyl-2-oxopyridine-3-carboxamide;
N-[4-[(6,7-dimethoxy-1,5-naphthyridin-4-yl)oxy]-3-fluorophenyl]-5-(5-fluoropyridin-2-yl)-4-hydroxy-2-(methoxymethyl)-6-methylpyridine-3 -carboxamide;
N-[4-[(6,7-dimethoxy-1,5-naphthyridin-4-yl)oxy]-3-fluorophenyl]-5-(5-fluoropyridin-2-yl)-4-methoxy-6-methylpyridazine-3-carboxamide;
N-[4-[(6,7-dimethoxy-1,5-naphthyridin-4-yl)oxy]phenyl]-5-(5-fluoropyridin-2-yl)-4-hydroxy-2,6-dimethylpyridine-3-carboxamide;
N-[3-fluoro-4-[(7-methoxy-1,5-naphthyridin-4-yl)oxy]phenyl]-5-(5-fluoropyridin-2-yl)-4-hydroxy-2,6-dimethylpyridine-3-carboxamide;
N-[4-[(6,7-dimethoxy-1,5-naphthyridin-4-yl)oxy]-3-fluorophenyl]-5-(5-fluoropyridin-2-yl)-4-hydroxy-2,6-dimethylpyridine-3-carboxamide;
N-[4-[(6,7-dimethoxy-1,5-naphthyridin-4-yl)oxy]-3-fluorophenyl]-4-(5-fluoropyridin-2-yl)-5-methyl-3-oxopyrazine-2-carboxamide;
N-[4-[(6,7-dimethoxy-1,5-naphthyridin-4-yl)oxy]-3-fluorophenyl]-1-(3-fluoropyridin-4-yl)-6-methyl-2-oxopyridine-3-carboxamide;
N-[4-[(6,7-dimethoxy-1,5-naphthyridin-4-yl)oxy]-3-fluorophenyl]-6-methyl-2-oxo-1-pyridin-4-ylpyridine-3-carboxamide;
N-[4-[(6,7-dimethoxy-1,5-naphthyridin-4-yl)oxy]-3-fluorophenyl]-6-methyl-2-oxo-1-pyridin-3-ylpyridine-3-carboxamide;
N-[4-[(6,7-dimethoxy-1,5-naphthyridin-4-yl)oxy]-3-fluorophenyl]-1-[2-[2-(2-ethoxyethoxy)ethoxy]pyridin-4-yl]-6-methyl-2-oxopyridine-3-carboxamide;
N-[4-[(6,7-dimethoxy-1,5-naphthyridin-4-yl)oxy]-3-fluorophenyl]-6-methyl-2-oxo-1-(2-propan-2-yloxypyridin-4-yl)pyridine-3-carboxamide;
N-[4-[(6,7-dimethoxy-1,5-naphthyridin-4-yl)oxy]-3-fluorophenyl]-1-(2-methoxypyridin-4-yl)-6-methyl-2-oxopyridine-3-carboxamide;
N-[4-[(6,7-dimethoxy-1,5-naphthyridin-4-yl)oxy]-3-fluorophenyl]-1-(5-fluoro-6-methylpyridin-2-yl)-6-methyl-2-oxopyridine-3-carboxamide;
N-[4-[(6,7-dimethoxy-1,5-naphthyridin-4-yl)oxy]-3-fluorophenyl]-4-hydroxy-2-methyl-5-pyridin-3-ylpyridine-3-carboxamide;
N-[4-[(6,7-dimethoxy-1,5-naphthyridin-4-yl)oxy]-3-fluorophenyl]-2-oxo-1-pyridin-4-ylpyridine-3-carboxamide;
N-[4-[(6,7-dimethoxy-1,5-naphthyridin-4-yl)oxy]-3-fluorophenyl]-6-methyl-1-(2-methylpyridin-4-yl)-2-oxopyridine-3-carboxamide;
N-[4-[(6,7-dimethoxy-1,5-naphthyridin-4-yl)oxy]-3-fluorophenyl]-6-methyl-1-(1-oxidopyridin-1-ium-4-yl)-2-oxopyridine-3-carboxamide;
N-[4-[(6,7-dimethoxy-1,5-naphthyridin-4-yl)oxy]-3-fluorophenyl]-1-(2-hydroxypyridin-4-yl)-6-methyl-2-oxopyridine-3-carboxamide;
N-[4-[(6,7-dimethoxy-1,5-naphthyridin-4-yl)oxy]-3-fluorophenyl]-1-(2-fluoropyridin-4-yl)-6-methyl-2-oxopyridine-3-carboxamide;
N-[4-[(6,7-dimethoxy-1,5-naphthyridin-4-yl)oxy]-3-fluorophenyl]-6-methyl-2-oxo-1-pyridazin-3-ylpyridine-3-carboxamide;
5-acetyl-N-[4-[(6,7-dimethoxy-1,5-naphthyridin-4-yl)oxy]-3-fluorophenyl]-1-(furan-3-yl)-6-methyl-2-oxopyridine-3-carboxamide;
5-acetyl-N-[4-[(6,7-dimethoxy-1,5-naphthyridin-4-yl)oxy]-3-fluorophenyl]-1-(furan-2-yl)-6-methyl-2-oxopyridine-3-carboxamide;
5-acetyl-N-[4-[(6,7-dimethoxy-1,5-naphthyridin-4-yl)oxy]phenyl]-1-(furan-2-yl)-6-methyl-2-oxopyridine-3-carboxamide;
5-acetyl-N-[3-fluoro-4-[(7-methoxy-1,5-naphthyridin-4-yl)oxy]phenyl]-1-(furan-2-yl)-6-methyl-2-oxopyridine-3-carboxamide;
5-acetyl-N-[4-[(6,7-dimethoxy-1,5-naphthyridin-4-yl)oxy]phenyl]-1-(furan-3-yl)-6-methyl-2-oxopyridine-3-carboxamide;
5-acetyl-N-[3-fluoro-4-[(7-methoxy-1,5-naphthyridin-4-yl)oxy]phenyl]-1-(furan-3-yl)-6-methyl-2-oxopyridine-3-carboxamide;
5-acetyl-N-[4-[(6,7-dimethoxy-1,5-naphthyridin-4-yl)oxy]-3-fluorophenyl]-6-methyl-2-oxo-1-thiophen-3-ylpyridine-3-carboxamide;
5-acetyl-N-[4-[(6,7-dimethoxy-1,5-naphthyridin-4-yl)oxy]phenyl]-6-methyl-2-oxo-1-thiophen-3-ylpyridine-3-carboxamide;
N-[4-[(6,7-dimethoxy-1,5-naphthyridin-4-yl)oxy]-3-fluorophenyl]-4-ethoxy-1-(5-fluoropyridin-2-yl)-2-oxopyridine-3-carboxamide;
N-[4-[(6,7-dimethoxy-1,5-naphthyridin-4-yl)oxy]phenyl]-4-ethoxy-1-(5-fluoropyridin-2-yl)-2-oxopyridine-3-carboxamide;
N-[4-[(6,7-dimethoxy-1,5-naphthyridin-4-yl)oxy]phenyl]-1-(5-fluoropyridin-2-yl)-4,6-dimethyl-2-oxopyridine-3-carboxamide;
N-[4-[(6,7-dimethoxy-1,5-naphthyridin-4-yl)oxy]-3-fluorophenyl]-4-(5-fluoropyridin-2-yl)-3-oxopyrazine-2-carboxamide;
N-[4-[(6,7-dimethoxy-1,5-naphthyridin-4-yl)oxy]-3-fluorophenyl]-1-(1-oxidopyridin-1-ium-4-yl)-2-oxopyridine-3-carboxamide;
N-[4-[(6,7-dimethoxy-1,5-naphthyridin-4-yl)oxy]-3-fluorophenyl]-1-(2-hydroxypyridin-4-yl)-2-oxopyridine-3-carboxamide;
N-[3-fluoro-4-[[6-methoxy-7-(2-methoxyethoxy)-1,5-naphthyridin-4-yl]oxy]phenyl]-5-(furan-2-yl)-1,2,6-trimethyl-4-oxopyridine-3-carboxamide;
N-[4-[(6,7-dimethoxy-1,5-naphthyridin-4-yl)oxy]phenyl]-5-(furan-2-yl)-4-hydroxy-6-methylpyridine-3-carboxamide;
N-[4-[(6,7-dimethoxy-1,5-naphthyridin-4-yl)oxy]phenyl]-4-hydroxy-6-methyl-5-thiophen-2-ylpyridine-3-carboxamide;
4-hydroxy-N-[4-[(7-methoxy-1,5-naphthyridin-4-yl)oxy]phenyl]-6-methyl-5-thiophen-2-ylpyridine-3-carboxamide;
N-[3-fluoro-4-[[6-methoxy-7-(2-methoxyethoxy)-1,5-naphthyridin-4-yl]oxy]phenyl]-5-(furan-2-yl)-4-hydroxy-6-methylpyridine-3-carboxamide;
5-(1-benzofuran-2-yl)-N-[4-[(6,7-dimethoxy-1,5-naphthyridin-4-yl)oxy]-3-fluorophenyl]-1,2,6-trimethyl-4-oxopyridine-3-carboxamide;
5-(1-benzofuran-3-yl)-N-[4-[(6,7-dimethoxy-1,5-naphthyridin-4-yl)oxy]-3-fluorophenyl]-1,2,6-trimethyl-4-oxopyridine-3-carboxamide;
5-(1-benzofuran-2-yl)-N-[3-fluoro-4-[[6-methoxy-7-(2-methoxyethoxy)-1,5-naphthyridin-4-yl]oxy]phenyl]-1,2,6-trimethyl-4-oxopyridine-3-carboxamide;
5-(1-benzofuran-3-yl)-N-[3-fluoro-4-[[6-methoxy-7-(2-methoxyethoxy)-1,5-naphthyridin-4-yl]oxy]phenyl]-1,2,6-trimethyl-4-oxopyridine-3-carboxamide;
5-(1-benzofuran-3-yl)-N-[3-fluoro-4-[[7-(2-methoxyethoxy)-1,5-naphthyridin-4-yl]oxy]phenyl]-1,2,6-trimethyl-4-oxopyridine-3-carboxamide;
N-[3-fluoro-4-[[7-(2-methoxyethoxy)-1,5-naphthyridin-4-yl]oxy]phenyl]-5-(furan-2-yl)-1,2,6-trimethyl-4-oxopyridine-3-carboxamide;
N-[4-[(6,7-dimethoxy-1,5-naphthyridin-4-yl)oxy]-3-fluorophenyl]-6-(furan-2-yl)-5-methylpyrazine-2-carboxamide;
1-(2,2-difluoroethyl)-N-[4-[(6,7-dimethoxy-1,5-naphthyridin-4-yl)oxy]-3-fluorophenyl]-5-(furan-2-yl)-2-methyl-4-oxopyridine-3-carboxamide;
N-[4-[(6,7-dimethoxy-1,5-naphthyridin-4-yl)oxy]-3-fluorophenyl]-7-(furan-2-yl)-8-oxo-3,4-dihydro-1H-pyrido[2,1-c][1,4]oxazine-9-carboxamide;
N-[4-[(6,7-dimethoxy-1,5-naphthyridin-4-yl)oxy]phenyl]-7-(furan-2-yl)-8-oxo-3,4-dihydro-1H-pyrido[2,1-c][1,4]oxazine-9-carboxamide;
N-[4-[(6,7-dimethoxy-1,5-naphthyridin-4-yl)oxy]phenyl]-8-oxo-7-thiophen-2-yl-3,4-dihydro-1H-pyrido[2,1-c][1,4]oxazine-9-carboxamide;
N-[4-[(6,7-dimethoxy-1,5-naphthyridin-4-yl)oxy]-3-fluorophenyl]-8-oxo-7-thiophen-3-yl-3,4-dihydro-1H-pyrido[2,1-c][1,4]oxazine-9-carboxamide;
N-[4-[(6,7-dimethoxy-1,5-naphthyridin-4-yl)oxy]phenyl]-8-oxo-7-thiophen-3-yl-3,4-dihydro-1H-pyrido[2,1-c][1,4]oxazine-9-carboxamide;
N-[4-[(6,7-dimethoxy-1,5-naphthyridin-4-yl)oxy]-3-fluorophenyl]-7-(4-methylthiophen-2-yl)-8-oxo-3,4-dihydro-1H-pyrido[2,1-c][1,4]oxazine-9-carboxamide;
N-[4-[(6,7-dimethoxy-1,5-naphthyridin-4-yl)oxy]phenyl]-7-(4-methylthiophen-2-yl)-8-oxo-3,4-dihydro-1H-pyrido[2,1-c][1,4]oxazine-9-carboxamide;
N-[4-[(6,7-dimethoxy-1,5-naphthyridin-4-yl)oxy]-3-fluorophenyl]-7-(5-methylthiophen-2-yl)-8-oxo-3,4-dihydro-1H-pyrido[2,1-c][1,4]oxazine-9-carboxamide;
N-[4-[(6,7-dimethoxy-1,5-naphthyridin-4-yl)oxy]phenyl]-7-(5-methylthiophen-2-yl)-8-oxo-3,4-dihydro-1H-pyrido[2,1-c][1,4]oxazine-9-carboxamide;
N-[4-[(6,7-dimethoxy-1,5-naphthyridin-4-yl)oxy]-3-fluorophenyl]-7-(furan-3-yl)-8-oxo-3,4-dihydro-1H-pyrido[2,1-c][1,4]oxazine-9-carboxamide;
N-[4-[(6,7-dimethoxy-1,5-naphthyridin-4-yl)oxy]-3-fluorophenyl]-6-methyl-8-oxo-7-thiophen-3-yl-3,4-dihydro-1H-pyrido[2,1-c][1,4]oxazine-9-carboxamide;
N-[4-[(6,7-dimethoxy-1,5-naphthyridin-4-yl)oxy]-3-fluorophenyl]-7-(furan-3-yl)-6-methyl-8-oxo-3,4-dihydro-1H-pyrido[2,1-c][1,4]oxazine-9-carboxamide;
N-[4-[(6,7-dimethoxy-1,5-naphthyridin-4-yl)oxy]-3-fluorophenyl]-8-oxo-7-thiophen-2-yl-3,4-dihydro-1H-pyrido[2,1-c][1,4]oxazine-9-carboxamide;
N-[4-[(6,7-dimethoxy-1,5-naphthyridin-4-yl)oxy]phenyl]-7-(furan-3-yl)-8-oxo-3,4-dihydro-1H-pyrido[2,1 -c] [1,4]oxazine-9-carboxamide;
N-[4-[(6,7-dimethoxy-1,5-naphthyridin-4-yl)oxy]phenyl]-7-(furan-3-yl)-6-methyl-8-oxo-3,4-dihydro-1H-pyrido[2,1-c][1,4]oxazine-9-carboxamide;
N-[4-[(6,7-dimethoxy-1,5-naphthyridin-4-yl)oxy]phenyl]-6-methyl-8-oxo-7-thiophen-3-yl-3,4-dihydro-1H-pyrido[2,1-c][1,4]oxazine-9-carboxamide;
N-[4-[(6,7-dimethoxy-1,5-naphthyridin-4-yl)oxy]phenyl]-6-methyl-7-(4-methylthiophen-2-yl)-8-oxo-3,4-dihydro-1H-pyrido[2,1-c][1,4]oxazine-9-carboxamide;
N-[4-[(6,7-dimethoxy-1,5-naphthyridin-4-yl)oxy]-3-fluorophenyl]-7-(furan-2-yl)-6-methyl-8-oxo-3,4-dihydro-1H-pyrido[2,1-c][1,4]oxazine-9-carboxamide;
N-[4-[(6,7-dimethoxy-1,5-naphthyridin-4-yl)oxy]phenyl]-7-(furan-2-yl)-6-methyl-8-oxo-3,4-dihydro-1H-pyrido[2,1-c][1,4]oxazine-9-carboxamide;
N-[4-[(6,7-dimethoxy-1,5-naphthyridin-4-yl)oxy]phenyl]-6-methyl-8-oxo-7-thiophen-2-yl-3,4-dihydro-1H-pyrido[2,1-c][1,4]oxazine-9-carboxamide;
N-[4-[(6,7-dimethoxy-1,5-naphthyridin-4-yl)oxy]-3-fluorophenyl]-6-methyl-8-oxo-7-thiophen-2-yl-3,4-dihydro-1H-pyrido[2,1-c][1,4]oxazine-9-carboxamide;
N-[4-[(6,7-dimethoxy-1,5-naphthyridin-4-yl)oxy]-3-fluorophenyl]-6-methyl-7-(4-methylthiophen-2-yl)-8-oxo-3,4-dihydro-1H-pyrido[2,1-c][1,4]oxazine-9-carboxamide;
N-[4-[(6,7-dimethoxy-1,5-naphthyridin-4-yl)oxy]-3-fluorophenyl]-2-ethyl-5-(furan-2-yl)-1,6-dimethyl-4-oxopyridine-3-carboxamide;
N-[4-[(6,7-dimethoxy-1,5-naphthyridin-4-yl)oxy]phenyl]-2-ethyl-5-(furan-2-yl)-1,6-dimethyl-4-oxopyridine-3-carboxamide;
2-ethyl-N-[3-fluoro-4-[[6-methoxy-7-(2-methoxyethoxy)-1,5-naphthyridin-4-yl]oxy]phenyl]-5-(furan-2-yl)-1,6-dimethyl-4-oxopyridine-3-carboxamide;
N-[4-[(6,7-dimethoxy-1,5-naphthyridin-4-yl)oxy]-3-fluorophenyl]-1-(2-fluoroethyl)-6-methyl-4-oxo-5-thiophen-2-ylpyridine-3-carboxamide;
1-(2-fluoroethyl)-N-[3-fluoro-4-[(7-methoxy-1,5-naphthyridin-4-yl)oxy]phenyl]-6-methyl-5-(4-methylthiophen-2-yl)-4-oxopyridine-3-carboxamide;
1-(2-fluoroethyl)-N-[3-fluoro-4-[(7-methoxy-1,5-naphthyridin-4-yl)oxy]phenyl]-6-methyl-5-(5-methylthiophen-2-yl)-4-oxopyridine-3-carboxamide;
1-(2-fluoroethyl)-N-[3-fluoro-4-[(7-methoxy-1,5-naphthyridin-4-yl)oxy]phenyl]-6-methyl-4-oxo-5-thiophen-3-ylpyridine-3-carboxamide;
1-(2-fluoroethyl)-N-[3-fluoro-4-[(7-methoxy-1,5-naphthyridin-4-yl)oxy]phenyl]-6-methyl-4-oxo-5-thiophen-2-ylpyridine-3-carboxamide;
N-[4-[(6,7-dimethoxy-1,5-naphthyridin-4-yl)oxy]-3-fluorophenyl]-1-(2-fluoroethyl)-6-methyl-4-oxo-5-thiophen-3-ylpyridine-3-carboxamide;
N-[4-[(6,7-dimethoxy-1,5-naphthyridin-4-yl)oxy]-3-fluorophenyl]-1-(2-fluoroethyl)-5-(furan-2-yl)-6-methyl-4-oxopyridine-3-carboxamide;
1-(2-fluoroethyl)-N-[3-fluoro-4-[(7-methoxy-1,5-naphthyridin-4-yl)oxy]phenyl]-5-(furan-3-yl)-6-methyl-4-oxopyridine-3-carboxamide; and
N-[4-[[7-(2-cyclobutylethoxy)-6-methoxy-1,5-naphthyridin-4-yl]oxy]-3-fluorophenyl]-5-(furan-2-yl)-1,2,6-trimethyl-4-oxopyridine-3-carboxamide;
or a pharmaceutically acceptable salt or stereoisomer thereof.

14. A pharmaceutical composition comprising a compound according to any one of claims 1 to 13, or a pharmaceutically acceptable salt or stereoisomer thereof, and a pharmaceutically acceptable carrier or excipient.

15. A compound of any one of claims 1 to 13, or a pharmaceutically acceptable salt or stereoisomer thereof, or a pharmaceutical composition of claim 14, for use in treating a disease, disorder, or syndrome in a subject in need thereof, said use comprising administering to the subject a therapeutically effective amount of said compound or pharmaceutical composition, wherein the disease, disorder, or syndrome is mediated at least in part by modulating in vivo activity of a protein kinase; preferably wherein the protein kinase is AXL, KDR, Mer, or Met.

## Patentansprüche

1. Verbindung der Formel (II): 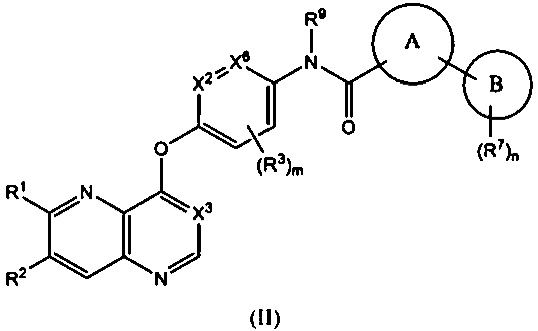 oder ein pharmazeutisch verträgliches Salz oder Stereoisomer davon, wobei:
Ring A Folgendes ist 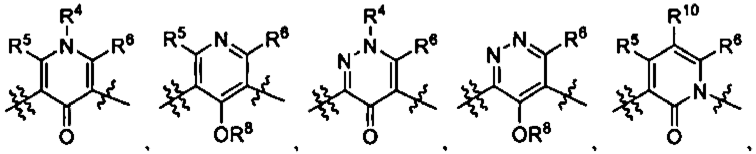 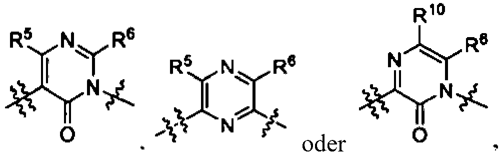
wobei die einfache Wellenlinie den Anknüpfungspunkt an Ring B und die doppelte Wellenlinie den Anknüpfungspunkt an den Rest des Moleküls angibt;
Ring B ein 5-gliedriges Heteroaryl mit 1, 2 oder 3 Heteroatomen als Ringglieder, ausgewählt aus N, O und S, ein 9- bis 10-gliedriges Heteroaryl mit 1, 2 oder 3 Heteroatomen als Ringglieder, ausgewählt aus N, O und S, oder ein 6-gliedriges Heteroaryl mit 1 oder 2 Stickstoffatomen als Ringglieder ist;
X² CH oder CR³ ist;
X³ CH ist;
X⁶ CH oder CR³ ist;
R¹ und R² jeweils unabhängig ausgewählt sind aus H, C₁₋₆-Alkoxy und C₁₋₆-Alkoxy-C₁₋₆-alkoxy;
jedes R³ Halogen ist;
R⁴ ausgewählt ist aus H, Halogen, C₁₋₆-Alkyl, C₂₋₆-Alkenyl, C₂₋₆-Alkinyl, C₁₋₆-Halogenalkyl, C₁₋₆-Halogenalkoxy, C₆₋₁₀-Aryl, C₃₋₁₄-Cycloalkyl, 5- bis 14-gliedrigem Heteroaryl, 4- bis 14-gliedrigem Heterocycloalkyl, C₆₋₁₀-Aryl-C₁₋₄-alkylen-, C₃₋₁₄-Cycloalkyl-C₁₋₄-alkylen-, (5-14-gliedrigem Heteroaryl)-C₁₋₄-alkylen-, (4-14-gliedrigem Heterocycloalkyl)-C₁₋₄-alkylen-, CN, NO₂, OR^{a}, SR^{a}, NHOR^{a}, C(O)R^{a}, C(O)NR^{a}R^{a}, C(O)OR^{a}, C(O)NR^{a}S(O)₂R^{a}, OC(O)R^{a}, OC(O)NR^{a}R^{a}, NHR^{a}, NR^{a}R^{a}, NR^{a}C(O)R^{a}, N=C(NR^{a}R^{a})₂, NR^{a}C(=NR^{a})R^{a}, NR^{a}C(O)OR^{a}, NR^{a}C(O)NR^{a}R^{a}, C(=NR^{a})R^{a}, C(=NOH)R^{a}, C(=NOH)NR^{a}, C(=NCN)NR^{a}R^{a}, NR^{a}C(=NCN)NR^{a}R^{a}, C(=NR^{a})NR^{a}R^{a}, NR^{a}C(=NR^{a})NR^{a}R^{a}, NR^{a}S(O)R^{a}, NR^{a}S(O)₂R^{a}, NR^{a}S(O)₂NR^{a}R^{a}, S(O)R^{a}, S(O)NR^{a}R^{a}, S(O)₂R^{a}, S(O)₂NR^{a}C(O)R^{a}, P(O)R^{a}R^{a}, P(O)(OR^{a})(OR^{a}), B(OH)₂, B(OR^{a})₂ und S(O)₂NR^{a}R^{a}, wobei die C₁₋₆-Alkyl, C₂₋₆-Alkenyl, C₂₋₆-Alkinyl, C₆₋₁₀-Aryl, C₃₋₁₄-Cycloalkyl, 5-14-gliedriges Heteroaryl, 4-14-gliedriges Heterocycloalkyl, C₆₋₁₀-Aryl-C₁₋₄-alkylen-, C₃₋₁₄-Cycloalkyl-C₁₋₄-alkylen-, (5-14-gliedriges Heteroaryl)-C₁₋₄-alkylen- sowie (4- bis 14-gliedriges Heterocycloalkyl)-C₁₋₄-alkylen- von R⁴ jeweils optional mit 1, 2, 3, 4 oder 5 unabhängig ausgewählten R^{b}-Substituenten substituiert sind;
R⁵, R⁶ und R¹⁰ jeweils unabhängig H, Halogen, C₁₋₆-Alkyl, C₂₋₆-Alkenyl, C₁₋₆-Alkoxy, C₁₋₆-Alkylthio, CN, C₁₋₄-Halogenalkyl, C₁₋₄-Halogenalkoxy, OH, C₁₋₄-Alkyl-C(O)-, C₁₋₄-Alkyl-OC(O)-, -CONH(C₁₋₄-Alkyl), NH₂, -NH-C₁₋₄-Alkyl oder -N(C₁₋₄-Alkyl)₂ sind, wobei das C₁₋₆-Alkyl, C₂₋₆-Alkenyl, C₁₋₆-Alkoxy, C₁₋₆-Alkylthio, C₁₋₆-Alkyl-C(O)- und C₁₋₄-Alkyl in -NH(C₁₋₄-Alkyl) oder -N(C₁₋₄-Alkyl) von R⁵, R⁶ und R¹⁰ jeweils optional mit 1 oder 2 unabhängig ausgewählten R^{g}-Substituenten substituiert sind;
jedes R⁷ unabhängig ausgewählt ist aus Halogen, OH, C(O)OR^{a}, C(O)R^{a}, C(O)NR^{a}R^{a}, CN, NH₂, -NH(C₁-C₆-Alkyl), -N(C₁-C₆-Alkyl), C₁-C₆-Alkyl, C₂-C₆-Alkenyl, C₁-C₆-Alkoxy, C₁-C₆-Halogenalkyl, C₁-C₆-Halogenalkoxy, C(O)NR^{a}R^{a}, NR^{a}C(O)R^{a}, NR^{a}C(O)NR^{a}R^{a}, SO₂R^{a}, NR^{a}S(O)₂R^{a}, NR^{a}S(O)₂NR^{a}R^{a}, C₃-C₆-Cycloalkyl, 4- bis 6-gliedrigem Heterocycloalkyl, Phenyl, 5- oder 6-gliedrigem Heteroaryl, C₃-C₆-Cycloalkyl-C₁-C₄-Alkylen-, (4- bis 6-gliedrigem Heterocycloalkyl)-C₁-C₄-Alkylen-, Phenyl-C₁-C₂-Alkylen und (5- oder 6-gliedrigem Heteroaryl)-C₁-C₄-Alkylen-; wobei das C₁-C₆-Alkyl, C₁-C₆-Alkoxy, C₃-C₆-Cycloalkyl, 4- bis 6-gliedrige Heterocycloalkyl, Phenyl, 5- oder 6-gliedrige Heteroaryl, C₃-C₆-Cycloalkyl-C₁-C₄-Alkylen-, (4- bis 6-gliedrige Heterocycloalkyl)-C₁-C₄-Alkylen-, Phenyl-C₁-C₂-Alkylen und (5- oder 6-gliedrige Heteroaryl)-C₁-C₄-Alkylen- von R⁷ jeweils optional mit 1, 2 oder 3 unabhängig ausgewählten R^{f}-Substituenten substituiert sind;
R⁸ H oder C₁₋₆-Alkyl ist, optional substituiert mit 1 oder 2 R^{g}-Substituenten oder einer Hydroxyschutzgruppe;
R⁹ H oder C₁₋₆-Alkyl ist, optional substituiert mit 1, 2 oder 3 unabhängig ausgewählten R^{g}-Substituenten;
oder R⁴ und R⁵ zusammen mit den Atomen, an die sie gebunden sind, ein 4- bis 7-gliedriges kondensiertes Heterocycloalkyl oder ein 5- bis 6-gliedriges kondensiertes Heteroaryl bilden, wobei das 4- bis 7-gliedrige kondensierte Heterocycloalkyl und das 5-bis 6-gliedrige kondensierte Heteroaryl jeweils optional mit 1 oder 2 unabhängig ausgewählten R^{g}-Substituenten substituiert sind;
oder R¹⁰ und R⁵ zusammen mit den Atomen, an die sie gebunden sind, kondensiertes C₃₋₇-Cycloalkyl, 4- bis 6-gliedriges kondensiertes Heterocycloalkyl, ein kondensiertes 5- oder 6-gliedriges Heteroaryl oder ein kondensiertes Phenyl bilden, wobei das kondensierte C₃₋₇-Cycloalkyl, das 4- bis 6- -gliedrigen kondensierten Heterocycloalkyl, das 5- oder 6-gliedrige Heteroaryl oder das kondensierte Phenyl jeweils optional mit 1 oder 2 unabhängig ausgewählten R^{g}-Substituenten substituiert sind und wobei ein oder zwei Ringkohlenstoffatome des kondensierten C₃₋₇-Cycloalkyls oder des kondensierten Heterocycloalkyls optional durch eine Carbonylgruppe ersetzt sind;
oder R⁶ und R⁵ zusammen mit den Atomen, an die sie gebunden sind, ein kondensiertes C₃₋₇-Cycloalkyl, ein 4- bis 6-gliedriges kondensiertes Heterocycloalkyl, ein 5- bis 6-gliedriges kondensiertes Heteroaryl oder ein kondensiertes Phenyl bilden, wobei das kondensierte C₃₋₇-Cycloalkyl, das 4- bis 6-gliedrige kondensierte Heterocycloalkyl, das 5- bis 6-gliedrige kondensierte Heteroaryl und das kondensierte Phenyl jeweils optional mit 1 oder 2 unabhängig ausgewählten R^{g}-Substituenten substituiert sind und wobei ein oder zwei Ringkohlenstoffatome des kondensierten C₃₋₇-Cycloalkyls oder des 4- bis 6-gliedrigen kondensierten Heterocycloalkyls optional durch eine Carbonylgruppe ersetzt sind;
oder R⁶ und R⁴ zusammen mit den Atomen, an die sie gebunden sind, ein 4- bis 7-gliedriges kondensiertes Heterocycloalkyl oder ein 5- bis 6-gliedriges kondensiertes Heteroaryl bilden, wobei das 4- bis 7-gliedrige kondensierte Heterocycloalkyl und das 5-bis 6-gliedrige kondensierte Heteroaryl jeweils optional mit 1 oder 2 unabhängig ausgewählten R^{g}-Substituenten substituiert sind;
oder R⁶ und R¹⁰ zusammen mit den Atomen, an die sie gebunden sind, ein kondensiertes C₃₋₇-Cycloalkyl, ein 4- bis 6-gliedriges kondensiertes Heterocycloalkyl, ein 5- bis 6-gliedriges kondensiertes Heteroaryl oder ein kondensiertes Heteroaryl bilden, wobei das kondensierte C₃₋₇-Cycloalkyl, das 4- bis 6-gliedrige kondensierte Heterocycloalkyl, das 5- bis 6-gliedrige kondensierte Heteroaryl und das kondensierte Heteroaryl jeweils optional mit 1 oder 2 unabhängig ausgewählten R^{g}-Substituenten substituiert sind und wobei ein oder zwei Ringkohlenstoffatome des kondensierten C₃₋₇-Cycloalkyls oder des 4- bis 6-gliedrigen kondensierten Heterocycloalkyls optional durch eine Carbonylgruppe ersetzt sind;
jedes R^{a} unabhängig ausgewählt ist aus der Gruppe bestehend aus H, CN, C₁-C₆-Alkyl, C₁-C₆-Halogenalkyl, C₂-C₆-Alkenyl, C₂-C₆-Alkinyl, C₆-C₁₀-Aryl, C₃-C₁₀-Cycloalkyl, 5- bis 14-gliedrigem Heteroaryl, 4- bis 14-gliedrigem Heterocycloalkyl, C₆-C₁₀-Aryl-C₁-C₄-Alkylen-, C₃-C₁₀-Cycloalkyl-C₁-C₄-Alkylen-, (5-14-gliedrigem Heteroaryl)-C₁-C₄-Alkylen- sowie (4-14-gliedrigem Heterocycloalkyl)-C₁-C₄-Alkylen-; wobei das C₁-C₆-Alkyl, C₁-C₆-Halogenalkyl, C₂-C₆-Alkenyl, C₂-C₆-Alkinyl, C₆-C₁₀-Aryl, C₃-C₁₀-Cycloalkyl, 5-14-gliedrige Heteroaryl, 4-14-gliedrige Heterocycloalkyl, C₆-C₁₀-Aryl-C₁-C₄-alkylen-, C₃-C₁₀-Cycloalkyl-C₁-C₄-alkylen-, (5-14-gliedrige Heteroaryl)-C₁-C₄-alkylen- und (4-14-gliedrige Heterocycloalkyl)-C₁-C₄-Alkylen- von R^{a} jeweils optional mit 1, 2, 3, 4 oder 5 unabhängig ausgewählten R^{d}-Substituenten substituiert sind;
oder beliebige zwei R^{a}-Substituenten zusammen mit dem Stickstoffatom, an das sie gebunden sind, einen 4-, 5-, 6-, 7-, 8-, 9- oder 10-gliedrigen Heterocycloalkylrest bilden, von denen jeder optional mit 1, 2 oder 3 unabhängig ausgewählten R^{f}-Substituenten substituiert ist;
jedes R^{b} unabhängig ausgewählt ist aus der Gruppe bestehend aus Halogen, C₁-C₆-Alkyl, C₂-C₆-Alkenyl, C₂-C₆-Alkinyl, C₁-C₆-Halogenalkyl, C₁-C₆-Halogenalkoxy, C₆-C₁₀-Aryl, C₃-C₁₀-Cycloalkyl, 5- bis 10-gliedrigem Heteroaryl, 4- bis 10-gliedrigem Heterocycloalkyl, C₆-C₁₀-Aryl-C₁-C₄-Alkylen-, C₃-C₁₀-Cycloalkyl-C₁-C₄-alkylen-, (5-10-gliedrigem Heteroaryl)-C₁-C₄-Alkylen-, (4-10-gliedrigem Heterocycloalkyl)-C₁-C₄-Alkylen-, CN, OH, NH₂, NO₂, NHOR^{c}, OR^{c}, SR^{c}, C(O)R^{c}, C(O)NR^{c}R^{c}, C(O)OR^{c}, C(O)NR^{c}S(O)₂ R^{c}, OC(O)R^{c}, OC(O)NR^{c}R^{c}, C(=NOH)R^{c}, C(=NOH)NR^{c}, C(=NCN)NR^{c}R^{c}, NR^{c}C(=NCN)NR^{c}R^{c}, C(=NR^{c})NR^{c}R^{c}, NR^{c}C(=NR^{c})NR^{c}R^{c}, NHR^{c}, NR^{c}R^{c}, NR^{c}C(O)R^{c}, NR^{c}C(=NR^{c})R^{c}, NR^{c}C(O)OR^{c}, NR^{c}C(O)NR^{c}R^{c}, NR^{c}S(O)R^{c}, NR^{c}S(O)₂R^{c}, NR^{c}S(O)₂NR^{c}R^{c}, S(O)R^{c}, S(O)NR^{c}R^{c}, S(O)₂R^{c}, S(O)₂NR^{c}C(O)R^{c}, Si(R^{c})₂, P(O)R^{c}R^{c}, P(O)(OR^{c})(OR^{c}), B(OH)₂, B(OR^{c})₂, und S(O)₂NR^{c}R^{c}; wobei C₁-C₆-Alkyl, C₂-C₆-Alkenyl, C₂-C₆-Alkinyl, C₆-C₁₀-Aryl, C₃-C₁₀-Cycloalkyl, 5- bis 10-gliedriges Heteroaryl-, 4- bis 10-gliedriges Heterocycloalkyl-, C₆-C₁₀-Aryl-C₁-C₄-alkylen-, C₃-C₁₀-Cycloalkyl-C₁-C₄-alkylen-, (5- bis 10-gliedriges Heteroaryl)-C₁-C₄-alkylen- und (4- bis 10-gliedriges Heterocycloalkyl)-C₁-C₄-alkylen- von R^{b} jeweils ferner optional mit 1, 2 oder 3 unabhängig ausgewählten R^{d}-Substituenten substituiert sind;
jedes R^{c} unabhängig ausgewählt ist aus der Gruppe bestehend aus H, C₁-C₆-Alkyl, C₁-C₆-Halogenalkyl, C₂-C₆-Alkenyl, C₂-C₆-Alkinyl, C₆-C₁₀-Aryl, C₃-C₁₀-Cycloalkyl, 5-bis 10-gliedrigem Heteroaryl, 4- bis 10-gliedrigem Heterocycloalkyl, C₆-C₁₀-Aryl-C₁-C₄-alkylen-, C₃-C₁₀-Cycloalkyl-C₁-C₄-alkylen-, (5- bis 10-gliedrigem Heteroaryl)-C₁-C₄-alkylen- und (4- bis 10-gliedrigem Heterocycloalkyl)-C₁-C₄-Alkylen-; wobei das C₁-C₆-Alkyl, C₂-C₆-Alkenyl, C₂-C₆-Alkinyl, C₆-C₁₀-Aryl, C₃-C₁₀-Cycloalkyl, 5- bis 10-gliedrige Heteroaryl, 4- bis 10-gliedrige Heterocycloalkyl, C₆-C₁₀-Aryl-C₁-C₄-alkylen-, C₃-C₁₀-Cycloalkyl-C₁-C₄-Alkylen-, (5- bis 10-gliedrige Heteroaryl)-C₁-C₄-Alkylen- sowie (4- bis 10-gliedrige Heterocycloalkyl)-C₁-C₄-Alkylen- von R^{c} jeweils optional mit 1, 2, 3, 4 oder 5 unabhängig ausgewählten R^{f}-Substituenten substituiert sind;
oder beliebige zwei R^{c}-Substituenten zusammen mit dem Stickstoffatom, an das sie gebunden sind, einen 4-, 5-, 6-, 7-, 8-, 9- oder 10-gliedrigen Heterocycloalkylrest bilden, von denen jeder optional mit 1, 2 oder 3 unabhängig ausgewählten R^{f}-Substituenten substituiert ist;
jedes R^{d} unabhängig ausgewählt ist aus der Gruppe bestehend aus C₁-C₆-Alkyl, C₁-C₆-Halogenalkyl, Halogen, C₆-C₁₀-Aryl, 5- bis 10-gliedrigem Heteroaryl, C₃-C₁₀-Cycloalkyl, 4- bis 10-gliedrigem Heterocycloalkyl, C₆-C₁₀-Aryl-C₁-C₄-Alkylen-, C₃-C₁₀-Cycloalkyl-C₁-C₄-Alkylen-, (5-10-gliedrigem Heteroaryl)-C₁-C₄-Alkylen-, (4-10-gliedrigem Heterocycloalkyl)-C₁-C₄-Alkylen-, CN, NH₂, NHOR^{e}, OR^{e}, SR^{e}, C(O)R^{e}, C(O)NR^{e}R^{e}, C(O)OR^{e}, OC(O)R^{e}, OC(O)NR^{e}R^{e}, NHR^{e}, NR^{e}R^{e}, NR^{e}C(O)R^{e}, NR^{e}C(O)NR^{e}R^{e}, NR^{e}C(O)OR^{e}, C(=NR^{e})NR^{e}R^{e}, NR^{e}R^{e}C(=NR^{e})NR^{e}R^{e}, NR^{e}C(=NOH)NR^{e}R^{e}, NR^{e}C(=NCN)NR^{e}R^{e}, S(O)R^{e}, S(O)NR^{e}R^{e}, S(O)₂R^{e}, NR^{e}S(O)₂R^{e}, NR^{e}S(O)₂NR^{e}R^{e} und S(O)₂NR^{e}R^{e}; wobei das C₁-C₆ -Alkyl, C₆-C₁₀-Aryl, 5-10-gliedrige Heteroaryl-, C₃-C₁₀-Cycloalkyl-, 4-10-gliedrige Heterocycloalkyl-, C₆-C₁₀-Aryl-C₁-C₄-Alkylen-, C₃-C₁₀-Cycloalkyl-C₁-C₄-Alkylen-, (5-10-gliedrige Heteroaryl)-C₁-C₄-Alkylen- und (4-10-gliedriges Heterocycloalkyl)-C₁-C₄-Alkylen- von R^{d} jeweils optional mit 1, 2 oder 3 unabhängig ausgewählten R^{f}-Substituenten substituiert sind;
jedes R^{e} ist unabhängig ausgewählt ist aus der Gruppe bestehend aus H, C₁-C₆-Alkyl, C1-C6-Alkoxy, C₃-C₆-Cycloalkyl, C₃-C₆-Cycloalkyl-C₁-C₄-Alkylen-, C₆-C₁₀-Aryl, C₆-C₁₀-Aryl-C₁-C₄-Alkylen-, 5- oder 6-gliedrigem Heteroaryl, (5- oder 6-gliedrigem Heteroaryl)-C₁-C₄-Alkylen-, 4- bis 7-gliedrigem Heterocycloalkyl, (4- bis 7-gliedrigem Heterocycloalkyl)-C₁-C₄-Alkylen-, C₁-C₆-Halogenalkyl, C₁-C₆-Halogenalkoxy, C₂-C₄-Alkenyl und C₂-C₄-Alkinyl, wobei das C₁-C₆-Alkyl, C₁-C₆-Alkoxy, C₃-C₆-Cycloalkyl, C6-C10-Aryl, 5- oder 6-gliedrige Heteroaryl, 4-7-gliedrige Heterocycloalkyl, C₆-C₁₀-Aryl-C₁-C₄-alkylen-, (5- oder 6-gliedrige Heteroaryl)-C₁-C₄-alkylen-, (4-7-gliedrige Heterocycloalkyl)-C₁-C₄-alkylen-, C₂-C₄-Alkenyl und C₂-C₄-Alkinyl von R^{e} jeweils optional mit 1, 2 oder 3 R^{f}-Substituenten substituiert sind;
oder beliebige zwei R^{e}-Substituenten zusammen mit dem Stickstoffatom, an das sie gebunden sind, einen 4-, 5-, 6-, 7-, 8-, 9- oder 10-gliedrigen Heterocycloalkylrest bilden, von denen jeder optional mit 1, 2 oder 3 unabhängig ausgewählten R^{f}-Substituenten substituiert ist;
jedes R^{f} unabhängig ausgewählt ist aus der Gruppe bestehend aus Halogen, OH, CN, C(O)OH, NH₂, -NH(C₁-C₆-Alkyl), -N(C₁-C₆-Alkyl)₂, C₁-C₆-Alkyl, Vinyl, C₁-C₆-Alkoxy, C₁-C₆-Alkylthio, C₁-C₆-Halogenalkyl, C₁-C₆-Halogenalkoxy, Phenyl, 5- bis 6-gliedrigem Heteroaryl, 4- bis 6-gliedrigem Heterocycloalkyl und C₃-C₆-Cycloalkyl, wobei das C₁-C₆-Alkyl, C₁-C₆-Alkoxy, C₁-C₆-Alkylthio, Phenyl, C₃-C₆-Cycloalkyl, 4- bis 6-gliedrige Heterocycloalkyl und 5- bis 6-gliedrige Heteroaryl von R^{f} jeweils optional mit 1, 2 oder 3 Substituenten substituiert sind, die ausgewählt sind aus Halogen, OH, CN,-C(O)OH, -NH₂, C₁-C₄-Alkyl, C₁-C₄-Alkoxy, C₁-C₄-Halogenalkyl, C₁-C₄-Halogenalkoxy, Phenyl, C₃-C₁₀-Cycloalkyl, 5- bis 6-gliedrigem Heteroaryl und 4- bis 6-gliedrigem Heterocycloalkyl;
jedes R^{g} unabhängig ausgewählt ist aus der Gruppe bestehend aus Halogen, OH, CN, C(O)OH, -C(O)O-C₁-C₄-Alkyl, NH₂, -NH(C₁-C₆-Alkyl), -N(C₁-C₆-Alkyl)₂, C₁-C₆-Alkyl, C₁-C₆-Alkoxy, C₁-C₆-Alkylthio, C₁-C₆-Halogenalkyl, C₁-C₆-Halogenalkoxy, Phenyl, 5- bis 6-gliedrigem Heteroaryl, 4- bis 6-gliedrigem Heterocycloalkyl und C₃-C₆-Cycloalkyl;
ein Ringstickstoffatom in Formel (II) optional oxidiert ist;
der Index m 0, 1 oder 2 ist; und
der Index n 0, 1, 2, 3 oder 4 ist.

2. Verbindung nach Anspruch 1, wobei Ring B ein 6-gliedriges Heteroaryl mit 1 oder 2 Stickstoffatomen als Ringglieder ist.

3. Verbindung nach Anspruch 1 oder Anspruch 2 oder ein pharmazeutisch verträgliches Salz oder Stereoisomer davon, wobei (i) Ring B 2-Thienyl, 3-Thienyl, 2-Furyl, 3-Furyl, 1H-Pyrazol-3-yl, 1H-Pyrazol-4-yl, 1H-Pyrazol-5-yl oder Pyrazol-1-yl ist; oder (ii) Ring B 2-Pyridyl, 3-Pyridyl, 4-Pyridyl oder 3-Pyridazinyl ist.

4. Verbindung nach einem der Ansprüche 1 bis 3, die die Formel (Ia) aufweist: 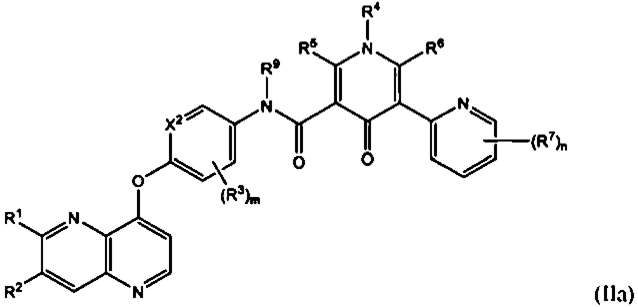 oder ein pharmazeutisch verträgliches Salz oder Stereoisomer davon;
wobei die Verbindung vorzugsweise die Formel (Ia-1) aufweist: 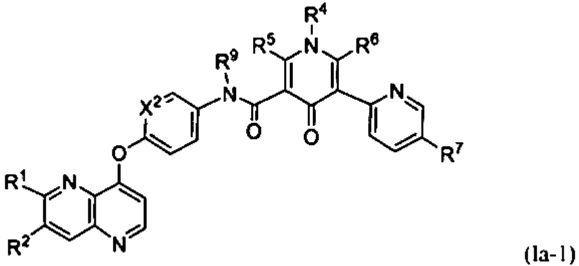 oder ein pharmazeutisch verträgliches Salz oder Stereoisomer davon.

5. Verbindung nach einem der Ansprüche 1 bis 3, die die Formel (IIb) aufweist: 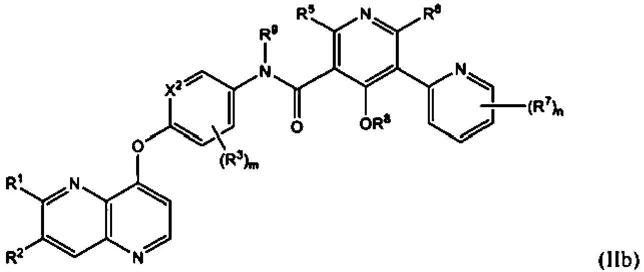 oder ein pharmazeutisch verträgliches Salz oder Stereoisomer davon;
wobei die Verbindung vorzugsweise die Formel (Ib-1) aufweist: 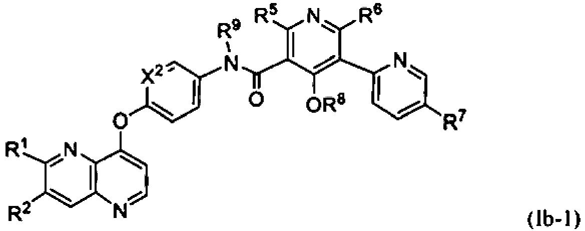 oder ein pharmazeutisch verträgliches Salz oder Stereoisomer davon.

6. Verbindung nach einem der Ansprüche 1 bis 3, die die Formel (IIc) aufweist: 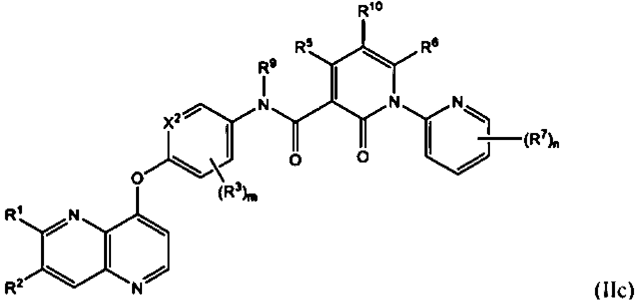 oder ein pharmazeutisch verträgliches Salz oder Stereoisomer davon;
wobei die Verbindung vorzugsweise die Formel (Ic-1) aufweist: 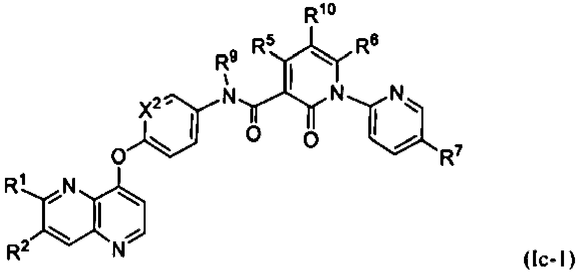 oder ein pharmazeutisch verträgliches Salz oder Stereoisomer davon.

7. Verbindung nach einem der Ansprüche 1 bis 3, die Folgendes aufweist:
i) 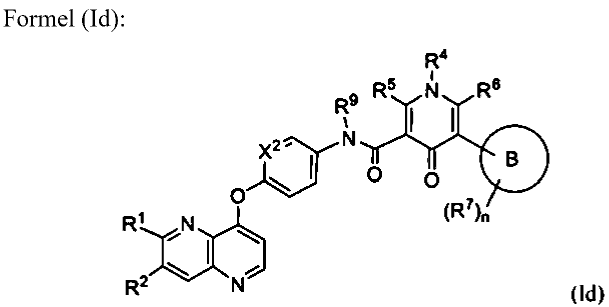 oder ein pharmazeutisch verträgliches Salz oder Stereoisomer davon; oder
ii) 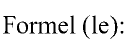 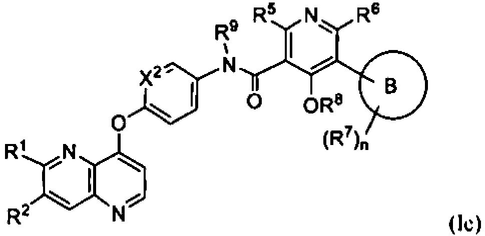 oder ein pharmazeutisch verträgliches Salz oder Stereoisomer davon; oder
iii) 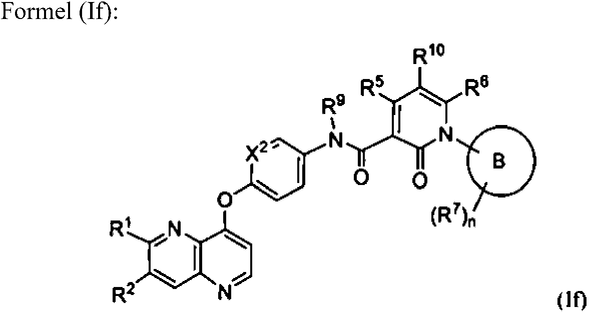 oder ein pharmazeutisch verträgliches Salz oder Stereoisomer davon; oder
iv) 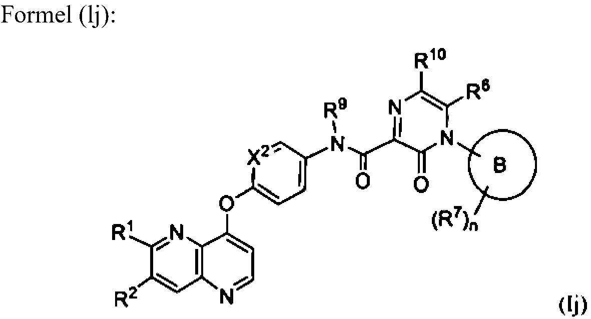 oder ein pharmazeutisch verträgliches Salz oder Stereoisomer davon; oder
v) 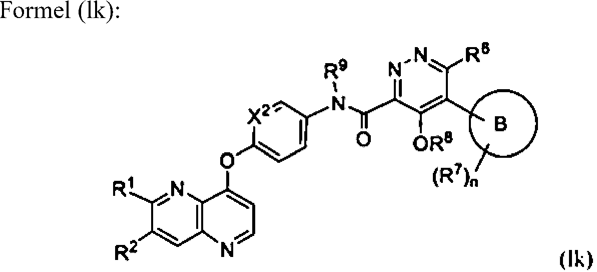 oder ein pharmazeutisch verträgliches Salz oder Stereoisomer davon; oder
vi) 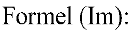 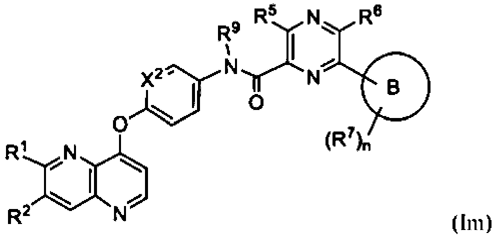 oder ein pharmazeutisch verträgliches Salz oder Stereoisomer davon.

8. Verbindung nach Anspruch 7 oder ein pharmazeutisch verträgliches Salz oder Stereoisomer davon, wobei Ring B Folgendes ist:
i) 2-Pyridyl, 3-Pyridyl, 4-Pyridyl oder ein 5-gliedriges Heteroaryl mit 1, 2 oder 3 Heteroatomen als Ringglieder, ausgewählt aus N, O und S; oder
ii) 2-Thienyl, 3-Thienyl, 2-Furyl, 3-Furyl, 2-Benzofuranyl, 3-Benzofuranyl, 1H-Pyrazol-3-yl, 1H-Pyrazol-4-yl, 1H-Pyrazol-5-yl oder Pyrazol-1-yl.

9. Verbindung nach einem der Ansprüche 1 bis 8 oder ein pharmazeutisch verträgliches Salz oder Stereoisomer davon, wobei R⁹ H oder Methyl ist.

10. Verbindung nach einem der Ansprüche 1 bis 9 oder ein pharmazeutisch verträgliches Salz oder Stereoisomer davon, wobei n O ist; oder wobei n 1, 2, 3 oder 4 ist und jedes R⁷ unabhängig aus Halogen, C₁₋₆-Alkyl und C₁₋₆-Alkoxy ausgewählt ist.

11. Verbindung nach einem der Ansprüche 1 bis 10 oder ein pharmazeutisch verträgliches Salz oder Stereoisomer davon, wobei X² CF ist.

12. Verbindung nach einem der Ansprüche 1 bis 11 oder ein pharmazeutisch verträgliches Salz oder Stereoisomer davon, wobei:
i) jedes R⁴ unabhängig ausgewählt ist aus H, C₁₋₆-Alkyl, C₁₋₆-Alkoxy, OH, NH₂, - NH(C₁₋₆-Alkyl), -N(C₁₋₆-Alkyl)₂, C₃₋₆-Cycloalkyl, C₁₋₆-Halogenalkyl, C₃₋₆-Cycloalkyl-C₁₋₄-alkylen-, 4- bis 6-gliedrigem Heterocycloalkyl, (4- bis 6-gliedrigem Heterocycloalkyl)-C₁₋₄-alkylen-, 5- bis 6-gliedrigem Heteroaryl, (5- bis 6-gliedrigem Heteroaryl)-C₁₋₄-Alkylen- sowie N=C[N(C₁₋₆-Alkyl)(C₁₋₆-Alkyl)]₂, wobei das C₁₋₆-Alkyl-, C₁₋₆-Alkoxy-, - NH(C₁₋₆-Alkyl)-, -N(C₁₋₆-Alkyl)₂, C₃₋₆-Cycloalkyl, C₃₋₆-Cycloalkyl-C₁₋₄-alkylen-, 4- bis 6-gliedrige Heterocycloalkyl, (4- bis 6-gliedrige Heterocycloalkyl)-C₁₋₄-alkylen-, 5- bis 6-gliedrige Heteroaryl, (5-6-gliedrige Heteroaryl)-C₁₋₄-alkylen- sowie N=C[N(C₁₋₆-Alkyl)(C₁₋₆-Alkyl)]₂ von R⁴ jeweils optional mit 1 oder 2 unabhängig ausgewählten R^{b}- oder R^{g}-Substituenten substituiert sind; und/oder
ii) R⁵, R⁶ und R¹⁰ jeweils unabhängig aus H, CH₃, Propen-2-yl, Br, Cl, CN, Methoxy, 2-Fluorethyl, Isopropyl, CH₃C(O)-, OH, t-Butyl, Ethyl, Hydroxymethyl, Isopropylthio und Methoxymethyl ausgewählt sind.

13. Verbindung nach einem der vorstehenden Ansprüche, ausgewählt aus
N-[4-[(6,7-Dimethoxy-1,5-naphthyridin-4-yl)oxy]-3-fluorphenyl]-1-(5-fluorpyridin-2-yl)-4-methyl-2-oxopyridin-3-carboxamid;
N-[4-[(6,7-Dimethoxy-1,5-naphthyridin-4-yl)oxy]-3-fluorphenyl]-1-(5-fluorpyridin-2-yl)-2-oxopyridin-3-carboxamid;
N-[4-[(6,7-Dimethoxy-1,5-naphthyridin-4-yl)oxy]-3-fluorphenyl]-1-(5-fluorpyridin-2-yl)-6-methyl-2-oxopyridin-3-carboxamid;
N-[4-[(6,7-Dimethoxy-1,5-naphthyridin-4-yl)oxy]phenyl]-1-(5-fluorpyridin-2-yl)-6-methyl-2-oxopyridin-3-carboxamid;
N-[3-Fluor-4-[(7-methoxy-1,5-naphthyridin-4-yl)oxy]phenyl]-1-(5-fluorpyridin-2-yl)-6-methyl-2-oxopyridin-3-carboxamid;
1-(5-Fluorpyridin-2-yl)-N-[4-[(7-methoxy-1,5-naphthyridin-4-yl)oxy]phenyl]-6-methyl-2-oxopyridin-3-carboxamid;
N-[3-Fluor-4-[(6-methoxy-1,5-naphthyridin-4-yl)oxy]phenyl]-1-(5-fluorpyridin-2-yl)-6-methyl-2-oxopyridin-3-carboxamid;
N-[3-Fluor-4-(1,5-naphthyridin-4-yloxy)phenyl]-1-(5-fluorpyridin-2-yl)-6-methyl-2-oxopyridin-3-carboxamid;
N-[3-Fluor-4-[[6-methoxy-7-(2-methoxyethoxy)-1,5-naphthyridin-4-yl]oxy]phenyl]-1-(5-fluorpyridin-2-yl)-6-methyl-2-oxopyridin-3-carboxamid;
N-[4-[(6,7-Dimethoxy-1,5-naphthyridin-4-yl)oxy]-3-fluorphenyl]-1-(5-fluor-3-methylpyridin-2-yl)-6-methyl-2-oxopyridin-3-carboxamid;
N-[3-Fluor-4-[(7-methoxy-1,5-naphthyridin-4-yl)oxy]phenyl]-1-(5-fluor-3-methylpyridin-2-yl)-6-methyl-2-oxopyridin-3-carboxamid;
N-[3-Fluor-4-[(6-methoxy-1,5-naphthyridin-4-yl)oxy]phenyl]-1-(5-fluor-3-methylpyridin-2-yl)-6-methyl-2-oxopyridin-3-carboxamid;
5-Brom-N-[4-[(6,7-dimethoxy-1,5-naphthyridin-4-yl)oxy]-3-fluorphenyl]-1-(5-fluorpyridin-2-yl)-6-methyl-2-oxopyridin-3-carboxamid;
5-Cyano-N-[4-[(6,7-dimethoxy-1,5-naphthyridin-4-yl)oxy]-3-fluorphenyl]-1-(5-fluorpyridin-2-yl)-6-methyl-2-oxopyridin-3-carboxamid;
5-Acetyl-N-[4-[(6,7-dimethoxy-1,5-naphthyridin-4-yl)oxy]-3-fluorphenyl]-1-(5-fluorpyridin-2-yl)-6-methyl-2-oxopyridin-3-carboxamid;
N-[4-[(6,7-Dimethoxy-1,5-naphthyridin-4-yl)oxy]-3-fluorphenyl]-5-(5-fluorpyridin-2-yl)-4-hydroxy-2-methylpyridin-3-carboxamid;
N-[4-[(6,7-Dimethoxy-1,5-naphthyridin-4-yl)oxy]-3-fluorphenyl]-4-hydroxy-2-methyl-5-pyridin-2-ylpyridin-3-carboxamid;
N-[3-Fluor-4-[(7-methoxy-1,5-naphthyridin-4-yl)oxy]phenyl]-4-hydroxy-2-methyl-5-pyridin-2-ylpyridin-3-carboxamid;
N-[4-[(6,7-Dimethoxy-1,5-naphthyridin-4-yl)oxy]-3-fluorphenyl]-5-(5-fluorpyridin-2-yl)-1,2-dimethyl-4-oxopyridin-3-carboxamid;
N-[4-[(6,7-Dimethoxy-1,5-naphthyridin-4-yl)oxy]phenyl]-5-(5-fluorpyridin-2-yl)-1,2-dimethyl-4-oxopyridin-3-carboxamid;
N-[3-Fluor-4-[(7-methoxy-1,5-naphthyridin-4-yl)oxy]phenyl]-5-(5-fluorpyridin-2-yl)-1,2-dimethyl-4-oxopyridin-3-carboxamid;
N-[4-[(6,7-Dimethoxy-1,5-naphthyridin-4-yl)oxy]-3-fluorphenyl]-5-(5-fluorpyridin-2-yl)-4-hydroxy-6-methylpyridin-3-carboxamid;
N-[4-[(6,7-Dimethoxy-1,5-naphthyridin-4-yl)oxy]phenyl]-5-(5-fluorpyridin-2-yl)-1,6-dimethyl-4-oxopyridin-3-carboxamid;
N-[4-[(6,7-Dimethoxy-1,5-naphthyridin-4-yl)oxy]-3-fluorphenyl]-5-(furan-2-yl)-4-hydroxy-6-methylpyridin-3-carboxamid;
N-[4-[(6,7-Dimethoxy-1,5-naphthyridin-4-yl)oxy]-3-fluorphenyl]-4-hydroxy-6-methyl-5-thiophen-2-ylpyridin-3-carboxamid;
N-[4-[(6,7-Dimethoxy-1,5-naphthyridin-4-yl)oxy]-3-fluorphenyl]-4-hydroxy-6-methyl-5-(4-methylthiophen-2-yl)pyridin-3-carboxamid;
N-[4-[(6,7-Dimethoxy-1,5-naphthyridin-4-yl)oxy]-3-fluorphenyl]-4-hydroxy-6-methyl-5-(5-methylfuran-2-yl)pyridin-3-carboxamid;
N-[4-[(6,7-Dimethoxy-1,5-naphthyridin-4-yl)oxy]-3-fluorphenyl]-4-hydroxy-6-methyl-5-thiophen-3-ylpyridin-3-carboxamid;
N-[4-[(6,7-Dimethoxy-1,5-naphthyridin-4-yl)oxy]-3-fluorphenyl]-4-hydroxy-6-methyl-5-(5-methylthiophen-2-yl)pyridin-3-carboxamid;
N-[4-[(6,7-Dimethoxy-1,5-naphthyridin-4-yl)oxy]-3-fluorphenyl]-5-(furan-3-yl)-4-hydroxy-6-methylpyridin-3-carboxamid;
N-[3-Fluor-4-[(7-methoxy-1,5-naphthyridin-4-yl)oxy]phenyl]-5-(furan-2-yl)-4-hydroxy-6-methylpyridin-3-carboxamid;
N-[3-Fluor-4-[(7-methoxy-1,5-naphthyridin-4-yl)oxy]phenyl]-4-hydroxy-6-methyl-5-(5-methylfuran-2-yl)pyridin-3-carboxamid;
N-[3-Fluor-4-[(7-methoxy-1,5-naphthyridin-4-yl)oxy]phenyl]-4-hydroxy-6-methyl-5-thiophen-3-ylpyridin-3-carboxamid;
N-[3-Fluor-4-[(7-methoxy-1,5-naphthyridin-4-yl)oxy]phenyl]-5-(furan-3-yl)-4-hydroxy-6-methylpyridin-3-carboxamid;
N-[3-Fluor-4-[(7-methoxy-1,5-naphthyridin-4-yl)oxy]phenyl]-4-hydroxy-6-methyl-5-thiophen-2-ylpyridin-3-carboxamid;
N-[3-Fluor-4-[(7-methoxy-1,5-naphthyridin-4-yl)oxy]phenyl]-4-hydroxy-6-methyl-5-(5-methylthiophen-2-yl)pyridin-3-carboxamid;
N-[3-Fluor-4-[(7-methoxy-1,5-naphthyridin-4-yl)oxy]phenyl]-4-hydroxy-6-methyl-5-(4-methylthiophen-2-yl)pyridin-3-carboxamid;
N-[4-[(6,7-Dimethoxy-1,5-naphthyridin-4-yl)oxy]-3-fluorphenyl]-4-hydroxy-6-methyl-5-(3-methylthiophen-2-yl)pyridin-3-carboxamid;
N-[4-[(6,7-Dimethoxy-1,5-naphthyridin-4-yl)oxy]-3-fluorphenyl]-4-hydroxy-6-methyl-5-(2-methylpyrazol-3-yl)pyridin-3-carboxamid;
N-[4-[(6,7-Dimethoxy-1,5-naphthyridin-4-yl)oxy]-3-fluorphenyl]-4-hydroxy-6-methyl-5-[2-methyl-5-(trifluormethyl)pyrazol-3-yl]pyridin-3-carboxamid;
N-[4-[(6,7-Dimethoxy-1,5-naphthyridin-4-yl)oxy]-3-fluorphenyl]-4-hydroxy-2,6-dimethyl-5-thiophen-3-ylpyridin-3-carboxamid;
N-[4-[(6,7-Dimethoxy-1,5-naphthyridin-4-yl)oxy]-3-fluorphenyl]-4-hydroxy-2,6-dimethyl-5-thiophen-2-ylpyridin-3-carboxamid;
N-[4-[(6,7-Dimethoxy-1,5-naphthyridin-4-yl)oxy]-3-fluorphenyl]-4-hydroxy-2,6-dimethyl-5-(2-methylpyrazol-3-yl)pyridin-3-carboxamid;
N-[4-[(6,7-Dimethoxy-1,5-naphthyridin-4-yl)oxy]-3-fluorphenyl]-5-(2,5-dimethylpyrazol-3-yl)-4-hydroxy-2,6-dimethylpyridin-3-carboxamid;
N-[4-[(6,7-Dimethoxy-1,5-naphthyridin-4-yl)oxy]-3-fluorphenyl]-4-hydroxy-2,6-dimethyl-5-(5-methylfuran-2-yl)pyridin-3-carboxamid;
N-[4-[(6,7-Dimethoxy-1,5-naphthyridin-4-yl)oxy]-3-fluorphenyl]-4-hydroxy-2,6-dimethyl-5-(5-methylthiophen-2-yl)pyridin-3-carboxamid;
N-[4-[(6,7-Dimethoxy-1,5-naphthyridin-4-yl)oxy]-3-fluorphenyl]-5-(furan-3-yl)-4-hydroxy-2,6-dimethylpyridin-3-carboxamid;
N-[4-[(6,7-Dimethoxy-1,5-naphthyridin-4-yl)oxy]-3-fluorphenyl]-4-hydroxy-2-(methoxymethyl)-6-methyl-5-thiophen-2-ylpyridin-3-carboxamid;
N-[4-[(6,7-Dimethoxy-1,5-naphthyridin-4-yl)oxy]-3-fluorphenyl]-5-(furan-2-yl)-4-hydroxy-2-(methoxymethyl)-6-methylpyridin-3-carboxamid;
N-[4-[(6,7-Dimethoxy-1,5-naphthyridin-4-yl)oxy]-3-fluorphenyl]-4-hydroxy-2-(methoxymethyl)-6-methyl-5-(5-methylfuran-2-y1)pyridin-3-carboxamid;
N-[3-Fluor-4-[(6-methoxy-1,5-naphthyridin-4-yl)oxy]phenyl]-5-(furan-2-yl)-4-hydroxy-6-methylpyridin-3-carboxamid;
N-[4-[(6,7-Dimethoxy-1,5-naphthyridin-4-yl)oxy]-3-fluorphenyl]-5-(furan-2-yl)-4-hydroxy-2-methylpyridin-3-carboxamid;
N-[4-[(6,7-Dimethoxy-1,5-naphthyridin-4-yl)oxy]-3-fluorphenyl]-4-hydroxy-2-methyl-5-(5-methylfuran-2-yl)pyridin-3-carboxamid;
N-[4-[(6,7-Dimethoxy-1,5-naphthyridin-4-yl)oxy]-3-fluorphenyl]-5-(5-formylfuran-2-yl)-4-hydroxy-2-methylpyridin-3-carboxamid;
N-[4-[(6,7-Dimethoxy-1,5-naphthyridin-4-yl)oxy]-3-fluorphenyl]-5-(furan-3-yl)-4-hydroxy-2-methylpyridin-3-carboxamid;
N-[4-[(6,7-Dimethoxy-1,5-naphthyridin-4-yl)oxy]-3-fluorphenyl]-4-hydroxy-2-methyl-5-thiophen-2-ylpyridin-3-carboxamid;
N-[4-[(6,7-Dimethoxy-1,5-naphthyridin-4-yl)oxy]-3-fluorphenyl]-4-hydroxy-2-methyl-5-(5-methylthiophen-2-yl)pyridin-3-carboxamid;
N-[4-[(6,7-Dimethoxy-1,5-naphthyridin-4-yl)oxy]-3-fluorphenyl]-4-hydroxy-2-methyl-5-(4-methylthiophen-2-yl)pyridin-3-carboxamid;
N-[4-[(6,7-Dimethoxy-1,5-naphthyridin-4-yl)oxy]-3-fluorphenyl]-4-hydroxy-2-methyl-5-(3-methylthiophen-2-yl)pyridin-3-carboxamid;
N-[4-[(6,7-Dimethoxy-1,5-naphthyridin-4-yl)oxy]-3-fluorphenyl]-4-hydroxy-2-methyl-5-thiophen-3-ylpyridin-3-carboxamid;
N-[3-Fluor-4-[(6-methoxy-1,5-naphthyridin-4-yl)oxy]phenyl]-4-hydroxy-6-methyl-5-thiophen-2-ylpyridin-3-carboxamid;
5-(Furan-2-yl)-4-hydroxy-N-[4-[(7-methoxy-1,5-naphthyridin-4-yl)oxy]phenyl]-6-methylpyridin-3-carboxamid;
N-[4-[(6,7-Dimethoxy-1,5-naphthyridin-4-yl)oxy]-3-fluorphenyl]-5-(furan-2-yl)-4-hydroxy-2,6-dimethylpyridin-3-carboxamid;
N-[4-[(6,7-Dimethoxy-1,5-naphthyridin-4-yl)oxy]-3-fluorphenyl]-5-(2,5-dimethylpyrazol-3-yl)-4-hydroxy-6-methylpyridin-3-carboxamid;
N-[4-[(6,7-Dimethoxy-1,5-naphthyridin-4-yl)oxy]-3-fluorphenyl]-4-hydroxy-2,6-dimethyl-5-(4-methylthiophen-2-yl)pyridin-3-carboxamid;
N-[4-[(6,7-Dimethoxy-1,5-naphthyridin-4-yl)oxy]-3-fluorphenyl]-4-hydroxy-2,6-dimethyl-5-[2-methyl-5-(trifluormethyl)pyrazol-3-yl]pyridin-3-carboxamid;
N-[4-[(6,7-Dimethoxy-1,5-naphthyridin-4-yl)oxy]-3-fluorphenyl]-4-hydroxy-2,6-dimethyl-5-(3-methylthiophen-2-yl)pyridin-3-carboxamid;
N-[4-[(6,7-Dimethoxy-1,5-naphthyridin-4-yl)oxy]-3-fluorphenyl]-4-hydroxy-6-methyl-5-(1,2-oxazol-4-yl)pyridin-3-carboxamid;
N-[4-[(6,7-Dimethoxy-1,5-naphthyridin-4-yl)oxy]-3-fluorphenyl]-4-hydroxy-6-methyl-5-(1,3-thiazol-5-yl)pyridin-3-carboxamid;
N-[4-[(6,7-Dimethoxy-1,5-naphthyridin-4-yl)oxy]-3-fluorphenyl]-4-hydroxy-6-methyl-5-(1,3-thiazol-4-yl)pyridin-3-carboxamid;
N-[4-[(6,7-Dimethoxy-1,5-naphthyridin-4-yl)oxy]-3-fluorphenyl]-4-hydroxy-6-methyl-5-(1,3-thiazol-2-yl)pyridin-3-carboxamid;
N-[4-[(6,7-Dimethoxy-1,5-naphthyridin-4-yl)oxy]-3-fluorphenyl]-5-(5-ethylfuran-2-yl)-4-hydroxy-2,6-dimethylpyridin-3-carboxamid;
N-[4-[(6,7-Dimethoxy-1,5-naphthyridin-4-yl)oxy]-3-fluorphenyl]-4-hydroxy-2,6-dimethyl-5-(5-propan-2-ylfuran-2-yl)pyridin-3-carboxamid;
N-[4-[(6,7-Dimethoxy-1,5-naphthyridin-4-yl)oxy]-3-fluorphenyl]-5-(5-ethenylfuran-2-yl)-4-hydroxy-6-methylpyridin-3-carboxamid;
N-[4-[(6,7-Dimethoxy-1,5-naphthyridin-4-yl)oxy]-3-fluorphenyl]-4-hydroxy-6-methyl-5-(5-prop-1-en-2-ylfuran-2-yl)pyridin-3-carboxamid;
N-[4-[(6,7-Dimethoxy-1,5-naphthyridin-4-yl)oxy]-3-fluorphenyl]-5-(5-ethylfuran-2-yl)-4-hydroxy-6-methylpyridin-3-carboxamid;
N-[4-[(6,7-Dimethoxy-1,5-naphthyridin-4-yl)oxy]-3-fluorphenyl]-4-hydroxy-6-methyl-5-(5-propan-2-ylfuran-2-yl)pyridin-3-carboxamid;
N-[4-[(6,7-Dimethoxy-1,5-naphthyridin-4-yl)oxy]-3-fluorphenyl]-4-hydroxy-6-methyl-5-(5-prop-2-enylfuran-2-yl)pyridin-3-carboxamid;
N-[4-[(6,7-Dimethoxy-1,5-naphthyridin-4-yl)oxy]-3-fluorphenyl]-5-(furan-2-yl)-1,2,6-trimethyl-4-oxopyridin-3-carboxamid;
5-(5-Cyclopropylfuran-2-yl)-N-[4-[(6,7-dimethoxy-1,5-naphthyridin-4-yl)oxy]-3-fluorphenyl]-4-hydroxy-6-methylpyridin-3-carboxamid;
5-(5-Cyclopropylfuran-2-yl)-N-[4-[(6,7-dimethoxy-1,5-naphthyridin-4-yl)oxy]-3-fluorphenyl]-4-hydroxy-2,6-dimethylpyridin-3-carboxamid;
5-(Furan-2-yl)-N-[4-[(7-methoxy-1,5-naphthyridin-4-yl)oxy]phenyl]-1,2,6-trimethyl-4-oxopyridin-3-carboxamid;
N-[3-Fluor-4-[(7-methoxy-1,5-naphthyridin-4-yl)oxy]phenyl]-5-(furan-2-yl)-1,2,6-trimethyl-4-oxopyridin-3-carboxamid;
N-[4-[(6,7-Dimethoxy-1,5-naphthyridin-4-yl)oxy]-2,5-difluorphenyl]-5-(furan-2-yl)-1,2,6-trimethyl-4-oxopyridin-3-carboxamid;
N-[4-[(6,7-Dimethoxy-1,5-naphthyridin-4-yl)oxy]phenyl]-5-(furan-2-yl)-1,2,6-trimethyl-4-oxopyridin-3-carboxamid;
N-[4-[(7-Methoxy-1,5-naphthyridin-4-yl)oxy]phenyl]-1,2,6-trimethyl-4-oxo-5-thiophen-2-ylpyridin-3-carboxamid;
N-[3-Fluor-4-[(7-methoxy-1,5-naphthyridin-4-yl)oxy]phenyl]-1,2,6-trimethyl-4-oxo-5-thiophen-2-ylpyridin-3-carboxamid;
N-[4-[(6,7-Dimethoxy-1,5-naphthyridin-4-yl)oxy]phenyl]-1,2,6-trimethyl-4-oxo-5-thiophen-2-ylpyridin-3-carboxamid;
N-[4-[(6,7-Dimethoxy-1,5-naphthyridin-4-yl)oxy]-3-fluorphenyl]-1,2,6-trimethyl-4-oxo-5-thiophen-2-ylpyridin-3-carboxamid;
N-[4-[(6,7-Dimethoxy-1,5-naphthyridin-4-yl)oxy]-3-fluorphenyl]-4-hydroxy-2-(methoxymethyl)-6-methyl-5-pyridin-2-ylpyridin-3-carboxamid;
N-[4-[(6,7-Dimethoxy-1,5-naphthyridin-4-yl)oxy]-3-fluorphenyl]-4-hydroxy-2-(methoxymethyl)-6-methyl-5-(3-methylpyridin-2-yl)pyridin-3-carboxamid;
N-[4-[(6,7-Dimethoxy-1,5-naphthyridin-4-yl)oxy]-3-fluorphenyl]-4-hydroxy-2-(methoxymethyl)-6-methyl-5-(6-methylpyridin-2-yl)pyridin-3-carboxamid;
N-[4-[(6,7-Dimethoxy-1,5-naphthyridin-4-yl)oxy]-3-fluorphenyl]-4-hydroxy-2-(methoxymethyl)-6-methyl-5-(2-methyl pyridin-4-y1)pyridin-3-carboxamid;
N-[4-[(6,7-Dimethoxy-1,5-naphthyridin-4-yl)oxy]-3-fluorphenyl]-5-(5-fluor-3-methylpyridin-2-yl)-4-hydroxy-2-methylpyridin-3-carboxamid;
N-[4-[(6,7-Dimethoxy-1,5-naphthyridin-4-yl)oxy]-3-fluorphenyl]-1-(5-ethoxypyridin-2-yl)-6-methyl-2-oxopyridin-3-carboxamid;
1-(5-Ethoxypyridin-2-yl)-N-[3-fluor-4-[(7-methoxy-1,5-naphthyridin-4-yl)oxy]phenyl]-6-methyl-2-oxopyridin-3-carboxamid;
N-[4-[(6,7-Dimethoxy-1,5-naphthyridin-4-yl)oxy]-3-fluorphenyl]-5-(5-fluorpyridin-2-yl)-4-hydroxy-2-(methoxymethyl)-6-methylpyridin-3-carboxamid;
N-[4-[(6,7-Dimethoxy-1,5-naphthyridin-4-yl)oxy]-3-fluorphenyl]-5-(5-fluorpyridin-2-yl)-4-methoxy-6-methylpyridazin-3-carboxamid;
N-[4-[(6,7-Dimethoxy-1,5-naphthyridin-4-yl)oxy]phenyl]-5-(5-fluorpyridin-2-yl)-4-hydroxy-2,6-dimethylpyridin-3-carboxamid;
N-[3-Fluor-4-[(7-methoxy-1,5-naphthyridin-4-yl)oxy]phenyl]-5-(5-fluorpyridin-2-yl)-4-hydroxy-2,6-dimethylpyridin-3-carboxamid;
N-[4-[(6,7-Dimethoxy-1,5-naphthyridin-4-yl)oxy]-3-fluorphenyl]-5-(5-fluorpyridin-2-yl)-4-hydroxy-2,6-dimethylpyridin-3-carboxamid;
N-[4-[(6,7-Dimethoxy-1,5-naphthyridin-4-yl)oxy]-3-fluorphenyl]-4-(5-fluorpyridin-2-yl)-5-methyl-3-oxopyrazine-2-carboxamid;
N-[4-[(6,7-Dimethoxy-1,5-naphthyridin-4-yl)oxy]-3-fluorphenyl]-1-(3-fluorpyridin-4-yl)-6-methyl-2-oxopyridin-3-carboxamid;
N-[4-[(6,7-Dimethoxy-1,5-naphthyridin-4-yl)oxy]-3-fluorphenyl]-6-methyl-2-oxo-1-pyridin-4-ylpyridin-3-carboxamid;
N-[4-[(6,7-Dimethoxy-1,5-naphthyridin-4-yl)oxy]-3-fluorphenyl]-6-methyl-2-oxo-1-pyridin-3-ylpyridin-3-carboxamid;
N-[4-[(6,7-Dimethoxy-1,5-naphthyridin-4-yl)oxy]-3-fluorphenyl]-1-[2-[2-(2-ethoxyethoxy)ethoxy]pyridin-4-yl]-6-methyl-2-oxopyridin-3-carboxamid;
N-[4-[(6,7-Dimethoxy-1,5-naphthyridin-4-yl)oxy]-3-fluorphenyl]-6-methyl-2-oxo-1-(2-propan-2-yloxypyridin-4-yl)pyridin-3-carboxamid;
N-[4-[(6,7-Dimethoxy-1,5-naphthyridin-4-yl)oxy]-3-fluorphenyl]-1-(2-methoxypyridin-4-yl)-6-methyl-2-oxopyridin-3-carboxamid;
N-[4-[(6,7-Dimethoxy-1,5-naphthyridin-4-yl)oxy]-3-fluorphenyl]-1-(5-fluor-6-methylpyridin-2-yl)-6-methyl-2-oxopyridin-3-carboxamid;
N-[4-[(6,7-Dimethoxy-1,5-naphthyridin-4-yl)oxy]-3-fluorphenyl]-4-hydroxy-2-methyl-5-pyridin-3-ylpyridin-3-carboxamid;
N-[4-[(6,7-Dimethoxy-1,5-naphthyridin-4-yl)oxy]-3-fluorphenyl]-2-oxo-1-pyridin-4-ylpyridin-3-carboxamid;
N-[4-[(6,7-Dimethoxy-1,5-naphthyridin-4-yl)oxy]-3-fluorphenyl]-6-methyl-1-(2-methylpyridin-4-yl)-2-oxopyridin-3-carboxamid;
N-[4-[(6,7-Dimethoxy-1,5-naphthyridin-4-yl)oxy]-3-fluorphenyl]-6-methyl-1-(1-oxidopyridin-1-ium-4-yl)-2-oxopyridin-3-carboxamid;
N-[4-[(6,7-Dimethoxy-1,5-naphthyridin-4-yl)oxy]-3-fluorphenyl]-1-(2-hydroxypyridin-4-yl)-6-methyl-2-oxopyridin-3-carboxamid;
N-[4-[(6,7-Dimethoxy-1,5-naphthyridin-4-yl)oxy]-3-fluorphenyl]-1-(2-fluorpyridin-4-yl)-6-methyl-2-oxopyridin-3-carboxamid;
N-[4-[(6,7-Dimethoxy-1,5-naphthyridin-4-yl)oxy]-3-fluorphenyl]-6-methyl-2-oxo-1-pyridazin-3-ylpyridin-3-carboxamid;
5-Acetyl-N-[4-[(6,7-dimethoxy-1,5-naphthyridin-4-yl)oxy]-3-fluorphenyl]-1-(furan-3-yl)-6-methyl-2-oxopyridin-3-carboxamid;
5-Acetyl-N-[4-[(6,7-dimethoxy-1,5-naphthyridin-4-yl)oxy]-3-fluorphenyl]-1-(furan-2-yl)-6-methyl-2-oxopyridin-3-carboxamid;
5-Acetyl-N-[4-[(6,7-dimethoxy-1,5-naphthyridin-4-yl)oxy]phenyl]-1-(furan-2-yl)-6-methyl-2-oxopyridin-3-carboxamid;
5-Acetyl-N-[3-fluor-4-[(7-methoxy-1,5-naphthyridin-4-yl)oxy]phenyl]-1-(furan-2-yl)-6-methyl-2-oxopyridin-3-carboxamid;
5-Acetyl-N-[4-[(6,7-dimethoxy-1,5-naphthyridin-4-yl)oxy]phenyl]-1-(furan-3-yl)-6-methyl-2-oxopyridin-3-carboxamid;
5-Acetyl-N-[3-fluor-4-[(7-methoxy-1,5-naphthyridin-4-yl)oxy]phenyl]-1-(furan-3-yl)-6-methyl-2-oxopyridin-3-carboxamid;
5-Acetyl-N-[4-[(6,7-dimethoxy-1,5-naphthyridin-4-yl)oxy]-3-fluorphenyl]-6-methyl-2-oxo-1-thiophen-3-ylpyridin-3-carboxamid;
5-Acetyl-N-[4-[(6,7-dimethoxy-1,5-naphthyridin-4-yl)oxy]phenyl]-6-methyl-2-oxo-1-thiophen-3-ylpyridin-3-carboxamid;
N-[4-[(6,7-Dimethoxy-1,5-naphthyridin-4-yl)oxy]-3-fluorphenyl]-4-ethoxy-1-(5-fluorpyridin-2-yl)-2-oxopyridin-3-carboxamid;
N-[4-[(6,7-Dimethoxy-1,5-naphthyridin-4-yl)oxy]phenyl]-4-ethoxy-1-(5-fluorpyridin-2-yl)-2-oxopyridin-3-carboxamid;
N-[4-[(6,7-Dimethoxy-1,5-naphthyridin-4-yl)oxy]phenyl]-1-(5-fluorpyridin-2-yl)-4,6-dimethyl-2-oxopyridin-3-carboxamid;
N-[4-[(6,7-Dimethoxy-1,5-naphthyridin-4-yl)oxy]-3-fluorphenyl]-4-(5-fluorpyridin-2-yl)-3-oxopyrazine-2-carboxamid;
N-[4-[(6,7-Dimethoxy-1,5-naphthyridin-4-yl)oxy]-3-fluorphenyl]-1-(1-oxidopyridin-1-ium-4-yl)-2-oxopyridin-3-carboxamid;
N-[4-[(6,7-Dimethoxy-1,5-naphthyridin-4-yl)oxy]-3-fluorphenyl]-1-(2-hydroxypyridin-4-yl)-2-oxopyridin-3-carboxamid;
N-[3-Fluor-4-[[6-methoxy-7-(2-methoxyethoxy)-1,5-naphthyridin-4-yl]oxy]phenyl]-5-(furan-2-yl)-1,2,6-trimethyl-4-oxopyridin-3-carboxamid;
N-[4-[(6,7-Dimethoxy-1,5-naphthyridin-4-yl)oxy]phenyl]-5-(furan-2-yl)-4-hydroxy-6-methylpyridin-3-carboxamid;
N-[4-[(6,7-Dimethoxy-1,5-naphthyridin-4-yl)oxy]phenyl]-4-hydroxy-6-methyl-5-thiophen-2-ylpyridin-3-carboxamid;
4-Hydroxy-N-[4-[(7-methoxy-1,5-naphthyridin-4-yl)oxy]phenyl]-6-methyl-5-thiophen-2-ylpyridin-3-carboxamid;
N-[3-Fluor-4-[[6-methoxy-7-(2-methoxyethoxy)-1,5-naphthyridin-4-yl]oxy]phenyl]-5-(furan-2-yl)-4-hydroxy-6-methylpyridin-3-carboxamid;
5-(1-Benzofuran-2-yl)-N-[4-[(6,7-dimethoxy-1,5-naphthyridin-4-yl)oxy]-3-fluorphenyl]-1,2,6-trimethyl-4-oxopyridin-3-carboxamid;
5-(1-Benzofuran-3-yl)-N-[4-[(6,7-dimethoxy-1,5-naphthyridin-4-yl)oxy]-3-fluorphenyl]-1,2,6-trimethyl-4-oxopyridin-3-carboxamid;
5-(1-Benzofuran-2-yl)-N-[3-fluor-4-[[6-methoxy-7-(2-methoxyethoxy)-1,5-naphthyridin-4-yl]oxy]phenyl]-1,2,6-trimethyl-4-oxopyridin-3-carboxamid;
5-(1-Benzofuran-3-yl)-N-[3-fluor-4-[[6-methoxy-7-(2-methoxyethoxy)-1,5-naphthyridin-4-yl]oxy]phenyl]-1,2,6-trimethyl-4-oxopyridin-3-carboxamid;
5-(1-Benzofuran-3-yl)-N-[3-fluor-4-[[7-(2-methoxyethoxy)-1,5-naphthyridin-4-yl]oxy]phenyl]-1,2,6-trimethyl-4-oxopyridin-3-carboxamid;
N-[3-Fluor-4-[[7-(2-methoxyethoxy)-1,5-naphthyridin-4-yl]oxy]phenyl]-5-(furan-2-yl)-1,2,6-trimethyl-4-oxopyridin-3-carboxamid;
N-[4-[(6,7-Dimethoxy-1,5-naphthyridin-4-yl)oxy]-3-fluorphenyl]-6-(furan-2-yl)-5-methylpyrazine-2-carboxamid;
1-(2,2-Difluorethyl)-N-[4-[(6,7-dimethoxy-1,5-naphthyridin-4-yl)oxy]-3-fluorphenyl]-5-(furan-2-yl)-2-methyl-4-oxopyridin-3-carboxamid;
N-[4-[(6,7-Dimethoxy-1,5-naphthyridin-4-yl)oxy]-3-fluorphenyl]-7-(furan-2-yl)-8-oxo-3,4-dihydro-1H-pyrido[2,1-c][1,4]oxazin-9-carboxamid;
N-[4-[(6,7-Dimethoxy-1,5-naphthyridin-4-yl)oxy]phenyl]-7-(furan-2-yl)-8-oxo-3,4-dihydro-1H-pyrido[2,1-c][1,4]oxazin-9-carboxamid;
N-[4-[(6,7-Dimethoxy-1,5-naphthyridin-4-yl)oxy]phenyl]-8-oxo-7-thiophen-2-yl-3,4-dihydro-1H-pyrido[2,1-c][1,4]oxazin-9-carboxamid;
N-[4-[(6,7-Dimethoxy-1,5-naphthyridin-4-yl)oxy]-3-fluorphenyl]-8-oxo-7-thiophen-3-yl-3,4-dihydro-1H-pyrido[2,1-c][1,4]oxazin-9-carboxamid;
N-[4-[(6,7-Dimethoxy-1,5-naphthyridin-4-yl)oxy]phenyl]-8-oxo-7-thiophen-3-yl-3,4-dihydro-1H-pyrido[2,1-c][1,4]oxazin-9-carboxamid;
N-[4-[(6,7-Dimethoxy-1,5-naphthyridin-4-yl)oxy]-3-fluorphenyl]-7-(4-methylthiophen-2-yl)-8-oxo-3,4-dihydro-1H-pyrido[2,1-c][1,4]oxazin-9-carboxamid;
N-[4-[(6,7-Dimethoxy-1,5-naphthyridin-4-yl)oxy]phenyl]-7-(4-methylthiophen-2-yl)-8-oxo-3,4-dihydro-1H-pyrido[2,1-c][1,4]oxazin-9-carboxamid;
N-[4-[(6,7-Dimethoxy-1,5-naphthyridin-4-yl)oxy]-3-fluorphenyl]-7-(5-methylthiophen-2-yl)-8-oxo-3,4-dihydro-1H-pyrido[2,1-c][1,4]oxazin-9-carboxamid;
N-[4-[(6,7-Dimethoxy-1,5-naphthyridin-4-yl)oxy]phenyl]-7-(5-methylthiophen-2-yl)-8-oxo-3,4-dihydro-1H-pyrido[2,1-c][1,4]oxazin-9-carboxamid;
N-[4-[(6,7-Dimethoxy-1,5-naphthyridin-4-yl)oxy]-3-fluorphenyl]-7-(furan-3-yl)-8-oxo-3,4-dihydro-1H-pyrido[2,1-c][1,4]oxazin-9-carboxamid;
N-[4-[(6,7-Dimethoxy-1,5-naphthyridin-4-yl)oxy]-3-fluorphenyl]-6-methyl-8-oxo-7-thiophen-3-yl-3,4-dihydro-1H-pyrido[2,1-c][1,4]oxazin-9-carboxamid;
N-[4-[(6,7-Dimethoxy-1,5-naphthyridin-4-yl)oxy]-3-fluorphenyl]-7-(furan-3-yl)-6-methyl-8-oxo-3,4-dihydro-1H-pyrido[2,1-c][1,4]oxazin-9-carboxamid;
N-[4-[(6,7-Dimethoxy-1,5-naphthyridin-4-yl)oxy]-3-fluorphenyl]-8-oxo-7-thiophen-2-yl-3,4-dihydro-1H-pyrido[2,1-c][1,4]oxazin-9-carboxamid;
N-[4-[(6,7-Dimethoxy-1,5-naphthyridin-4-yl)oxy]phenyl]-7-(furan-3-yl)-8-oxo-3,4-dihydro-1H-pyrido[2,1-c][1,4]oxazin-9-carboxamid;
N-[4-[(6,7-Dimethoxy-1,5-naphthyridin-4-yl)oxy]phenyl]-7-(furan-3-yl)-6-methyl-8-oxo-3,4-dihydro-1H-pyrido[2,1-c][1,4]oxazin-9-carboxamid;
N-[4-[(6,7-Dimethoxy-1,5-naphthyridin-4-yl)oxy]phenyl]-6-methyl-8-oxo-7-thiophen-3-yl-3,4-dihydro-1H-pyrido[2,1-c][1,4]oxazin-9-carboxamid;
N-[4-[(6,7-Dimethoxy-1,5-naphthyridin-4-yl)oxy]phenyl]-6-methyl-7-(4-methylthiophen-2-yl)-8-oxo-3,4-dihydro-1H-pyrido[2,1-c][1,4]oxazin-9-carboxamid;
N-[4-[(6,7-Dimethoxy-1,5-naphthyridin-4-yl)oxy]-3-fluorphenyl]-7-(furan-2-yl)-6-methyl-8-oxo-3,4-dihydro-1H-pyrido[2,1-c][1,4]oxazin-9-carboxamid;
N-[4-[(6,7-Dimethoxy-1,5-naphthyridin-4-yl)oxy]phenyl]-7-(furan-2-yl)-6-methyl-8-oxo-3,4-dihydro-1H-pyrido[2,1-c][1,4]oxazin-9-carboxamid;
N-[4-[(6,7-Dimethoxy-1,5-naphthyridin-4-yl)oxy]phenyl]-6-methyl-8-oxo-7-thiophen-2-yl-3,4-dihydro-1H-pyrido[2,1-c][1,4]oxazin-9-carboxamid;
N-[4-[(6,7-Dimethoxy-1,5-naphthyridin-4-yl)oxy]-3-fluorphenyl]-6-methyl-8-oxo-7-thiophen-2-yl-3,4-dihydro-1H-pyrido[2,1-c][1,4]oxazin-9-carboxamid;
N-[4-[(6,7-Dimethoxy-1,5-naphthyridin-4-yl)oxy]-3-fluorphenyl]-6-methyl-7-(4-methylthiophen-2-yl)-8-oxo-3,4-dihydro-1H-pyrido[2,1-c][1,4]oxazin-9-carboxamid;
N-[4-[(6,7-Dimethoxy-1,5-naphthyridin-4-yl)oxy]-3-fluorphenyl]-2-ethyl-5-(furan-2-yl)-1,6-dimethyl-4-oxopyridin-3-carboxamid;
N-[4-[(6,7-Dimethoxy-1,5-naphthyridin-4-yl)oxy]phenyl]-2-ethyl-5-(furan-2-yl)-1,6-dimethyl-4-oxopyridin-3-carboxamid;
2-Ethyl-N-[3-fluor-4-[[6-methoxy-7-(2-methoxyethoxy)-1,5-naphthyridin-4-yl]oxy]phenyl]-5-(furan-2-yl)-1,6-dimethyl-4-oxopyridin-3-carboxamid;
N-[4-[(6,7-Dimethoxy-1,5-naphthyridin-4-yl)oxy]-3-fluorphenyl]-1-(2-fluorethyl)-6-methyl-4-oxo-5-thiophen-2-ylpyridin-3-carboxamid;
1-(2-Fluorethyl)-N-[3-fluor-4-[(7-methoxy-1,5-naphthyridin-4-yl)oxy]phenyl]-6-methyl-5-(4-methylthiophen-2-yl)-4-oxopyridin-3-carboxamid;
1-(2-Fluorethyl)-N-[3-fluor-4-[(7-methoxy-1,5-naphthyridin-4-yl)oxy]phenyl]-6-methyl-5-(5-methylthiophen-2-yl)-4-oxopyridin-3-carboxamid;
1-(2-Fluorethyl)-N-[3-fluor-4-[(7-methoxy-1,5-naphthyridin-4-yl)oxy]phenyl]-6-methyl-4-oxo-5-thiophen-3-ylpyridin-3-carboxamid;
1-(2-Fluorethyl)-N-[3-fluor-4-[(7-methoxy-1,5-naphthyridin-4-yl)oxy]phenyl]-6-methyl-4-oxo-5-thiophen-2-ylpyridin-3-carboxamid;
N-[4-[(6,7-Dimethoxy-1,5-naphthyridin-4-yl)oxy]-3-fluorphenyl]-1-(2-fluorethyl)-6-methyl-4-oxo-5-thiophen-3-ylpyridin-3-carboxamid;
N-[4-[(6,7-Dimethoxy-1,5-naphthyridin-4-yl)oxy]-3-fluorphenyl]-1-(2-fluorethyl)-5-(furan-2-yl)-6-methyl-4-oxopyridin-3-carboxamid;
1-(2-Fluorethyl)-N-[3-fluor-4-[(7-methoxy-1,5-naphthyridin-4-yl)oxy]phenyl]-5-(furan-3-yl)-6-methyl-4-oxopyridin-3-carboxamid; und
N-[4-[[7-(2-Cyclobutylethoxy)-6-methoxy-1,5-naphthyridin-4-yl]oxy]-3-fluorphenyl]-5-(furan-2-yl)-1,2,6-trimethyl-4-oxopyridin-3-carboxamid;
oder einem pharmazeutisch verträglichen Salz oder Stereoisomer davon.

14. Pharmazeutische Zusammensetzung, die eine Verbindung nach einem der Ansprüche 1 bis 13 oder ein pharmazeutisch verträgliches Salz oder Stereoisomer davon sowie einen pharmazeutisch verträglichen Träger oder Hilfsstoff umfasst.

15. Verbindung nach einem der Ansprüche 1 bis 13 oder ein pharmazeutisch verträgliches Salz oder Stereoisomer davon oder eine pharmazeutische Zusammensetzung nach Anspruch 14, zur Verwendung bei der Behandlung einer Krankheit, einer Störung oder eines Syndroms bei einem Patienten, der einer solchen Behandlung bedarf, wobei die Verwendung die Verabreichung einer therapeutisch wirksamen Menge der Verbindung oder der pharmazeutischen Zusammensetzung an den Patienten umfasst, wobei die Krankheit, Störung oder das Syndrom zumindest teilweise durch die Modulation der In-vivo-Aktivität einer Proteinkinase vermittelt wird; vorzugsweise wobei die Proteinkinase AXL, KDR, Mer oder Met ist.

## Revendications

1. Composé de la formule (II) : 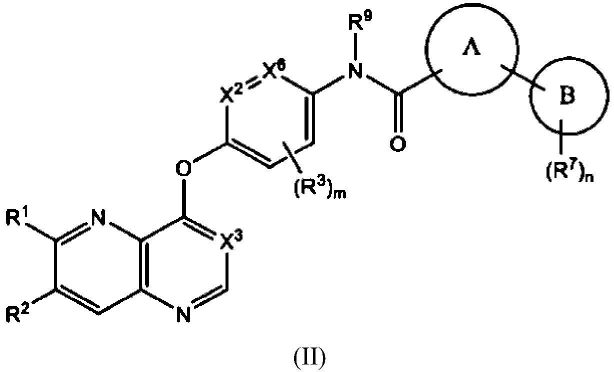
ou sel pharmaceutiquement acceptable ou stéréoisomère de celui-ci, dans lequel :
l'anneau A est 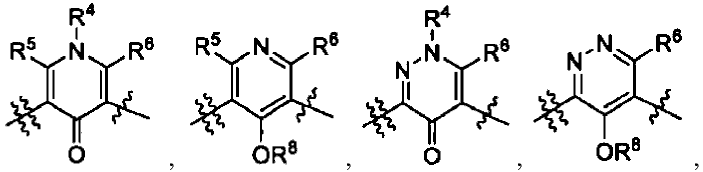 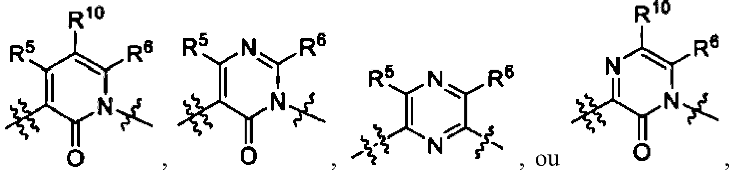 dans lequel la ligne ondulée unique indique le point d'attache à l'anneau B et la ligne ondulée double indique le point d'attache au reste de la molécule ;
l'anneau B est hétéroaryle à 5 membres ayant 1, 2, ou 3 hétéroatomes en tant que membres d'anneau sélectionnés parmi N, O, et S, hétéroaryle à 9 à 10 membres ayant 1, 2, ou 3 hétéroatomes en tant que membres d'anneau sélectionnés parmi N, O, et S, ou hétéroaryle à 6 membres ayant 1 ou 2 atomes d'azote en tant que membres d'anneau ;
X² est CH ou CR³ ;
X³ est CH ;
X⁶ est CH ou CR³ ;
R¹ et R² sont chacun indépendamment sélectionnés parmi H, alkoxy en C₁₋₆, et alkoxy en C₁₋₆-alkoxy en C₁₋₆ ;
chaque R³ est halo ;
R⁴ est sélectionné parmi H, halo, alkyle en C₁₋₆, alcényle en C₂₋₆, alkynyle en C₂₋₆, haloalkyle en C₁₋₆, haloalkoxy en C₁₋₆, aryle en C₆₋₁₀, cycloalkyle en C₃₋₁₄, hétéroaryle à 5 à 14 membres, hétérocycloalkyle à 4 à 14 membres, aryle en C₆₋₁₀-alkylène en C₁₋₄-, cycloalkyle en C₃₋₁₄-alkylène en C₁₋₄-, (hétéroaryle à 5 à 14 membres)-alkylène en C₁₋₄-, (hétérocycloalkyle à 4 à 14 membres)-alkylène en C₁₋₄-, CN, NO₂, OR^{a}, SR^{a}, NHOR^{a}, C(O)R^{a}, C(O)NR^{a}R^{a}, C(O)OR^{a}, C(O)NR^{a}S(O)₂R^{a}, OC(O)R^{a}, OC(O)NR^{a}R^{a}, NHR^{a}, NR^{a}R^{a}, NR^{a}C(O)R^{a}, N=C(NR^{a}R^{a})₂, NR^{a}C(=NR^{a})R^{a}, NR^{a}C(O)OR^{a}, NR^{a}C(O)NR^{a}R^{a}, C(=NR^{a})R^{a}, C(=NOH)R^{a}, C(=NOH)NR^{a}, C(=NCN)NR^{a}R^{a}, NR^{a}C(=NCN)NR^{a}R^{a}, C(=NR^{a})NR^{a}R^{a}, NR^{a}C(=NR^{a})NR^{a}R^{a}, NR^{a}S(O)R^{a}, NR^{a}S(O)₂R^{a}, NR^{a}S(O)₂NR^{a}R^{a}, S(O)R^{a}, S(O)NR^{a}R^{a}, S(O)₂R^{a}, S(O)₂NR^{a}C(O)R^{a}, P(O)R^{a}R^{a}, P(O)(OR^{a})(OR^{a}), B(OH)₂, B(OR^{a})₂, et S(O)₂NR^{a}R^{a}, dans lequel l'alkyle en C₁₋₆, alcényle en C₂₋₆, alkynyle en C₂₋₆, aryle en C₆₋₁₀, cycloalkyle en C₃₋₁₄, hétéroaryle à 5 à 14 membres, hétérocycloalkyle à 4 à 14 membres, aryle en C₆₋₁₀-alkylène en C₁₋₄-, cycloalkyle en C₃₋₁₄-alkylène en C₁₋₄-, (hétéroaryle à 5 à 14 membres)-alkylène en C₁₋₄-, et (hétérocycloalkyle à 4 à 14 membres)-alkylène en C₁₋₄- de R⁴ sont chacun facultativement substitués avec 1, 2, 3, 4, ou 5 substituants R^{b} indépendamment sélectionnés ;
R⁵, R⁶ et R¹⁰ sont chacun indépendamment H, halo, alkyle en C₁₋₆, alcényle en C₂₋₆, alkoxy en C₁₋₆, alkylthio en C₁₋₆, CN, haloalkyle en C₁₋₄, haloalkoxy en C₁₋₄, OH, alkyle en C₁₋₄-C(O)-, alkyle en C₁₋₄-OC(O)-, -CONH(alkyle en C₁₋₄), NH₂, -NHalkyle en C₁₋₄, ou -N(alkyle en C₁₋₄), dans lequel l'alkyle en C₁₋₆, alcényle en C₂₋₆, alkoxy en C₁₋₆, alkylthio en C₁₋₆, alkyle en C₁₋₆-C(O)-, et alkyle en C₁₋₄ de -NH(alkyle en C₁₋₄), ou de -N(alkyle en C₁₋₄ ) de R⁵, de R⁶, et de R¹⁰ sont chacun facultativement substitués avec 1 ou 2 substituants R^{g} indépendamment sélectionnés ;
chaque R⁷ est indépendamment sélectionné parmi halo, OH, C(O)OR^{a}, C(O)R^{a}, C(O)NR^{a}R^{a}, CN, NH₂, -NH(alkyle en C₁-C₆), -N(alkyle en C₁-C₆), alkyle en C₁-C₆, alcényle en C₂-C₆, alkoxy en C₁-C₆, haloalkyle en C₁-C₆, haloalkoxy en C₁-C₆, C(O)NR^{a}R^{a}, NR^{a}C(O)R^{a}, NR^{a}C(O)NR^{a}R^{a}, SO₂R^{a}, NR^{a}S(O)₂R^{a}, NR^{a}S(O)₂NR^{a}R^{a}, cycloalkyle en C₃-C₆, hétérocycloalkyle à 4 à 6 membres, phényle, hétéroaryle à 5 ou 6 membres, cycloalkyle en C₃-C₆-alkylène en C₁-C₄-, (hétérocycloalkyle à 4 à 6 membres)-alkylène en C₁-C₄-, phényle-alkylène en C₁-C₂, et (hétéroaryle à 5 ou 6 membres)-alkylène en C₁₋₄- ; dans lequel l'alkyle en C₁₋₄, alkoxy en C₁-C₆, cycloalkyle en C₃-C₆, hétérocycloalkyle à 4 à 6 membres, phényle, hétéroaryle à 5 ou 6 membres, cycloalkyle en C₃-C₆-alkylène en C₁-C₄-, (hétérocycloalkyle à 4 à 6 membres)-alkylène en C₁-C₄-, phényl-alkylène en C₁-C₂, et (hétéroaryle à 5 ou 6 membres)-alkylène en C₁-C₄- de R⁷ sont chacun facultativement substitués avec 1, 2, ou 3 substituants R^{f} indépendamment sélectionnés ;
R⁸ est H ou alkyle en C₁₋₆ facultativement substitué avec 1 ou 2 substituants R^{g} ou un groupe protecteur hydroxy ;
R⁹ est H ou alkyle en C₁₋₆ facultativement substitué avec 1, 2, ou 3 substituants R^{g} indépendamment sélectionnés ;
ou R⁴ et R⁵ pris conjointement avec les atomes auxquels ils sont attachés forment hétérocycloalkyle fusionné à 4 à 7 membres ou hétéroaryle fusionné à 5 à 6 membres, dans lequel l'hétérocycloalkyle fusionné à 4 à 7 membres et hétéroaryle fusionné à 5 à 6 membres sont chacun facultativement substitués avec 1 ou 2 substituants R^{g} indépendamment sélectionnés ;
ou R¹⁰ et R⁵ pris conjointement avec les atomes auxquels ils sont attachés forment cycloalkyle en C₃₋₇ fusionné, hétérocycloalkyle fusionné à 4 à 6 membres, hétéroaryle fusionné à 5 ou 6 membres, ou phényle fusionné, dans lequel le cycloalkyle en C₃₋₇ fusionné, hétérocycloalkyle fusionné à 4 à 6 membres, hétéroaryle à 5 ou 6 membres, ou phényle fusionné sont chacun facultativement substitués avec 1 ou 2 substituants R^{g} indépendamment sélectionnés et dans lequel un ou deux atomes de carbone d'anneau du cycloalkyle en C₃₋₇ fusionné ou de l'hétérocycloalkyle fusionné sont facultativement remplacés par un groupe carbonyle ;
ou R⁶ et R⁵ pris conjointement avec les atomes auxquels ils sont attachés forment cycloalkyle en C₃₋₇ fusionné, hétérocycloalkyle fusionné à 4 à 6 membres, hétéroaryle fusionné à 5 à 6 membres, ou phényle fusionné, dans lequel le cycloalkyle en C₃₋₇ fusionné, hétérocycloalkyle fusionné à 4 à 6 membres, hétéroaryle fusionné à 5 à 6 membres, et phényle fusionné sont chacun facultativement substitués avec 1 ou 2 substituants R^{g} indépendamment sélectionnés, et dans lequel un ou deux atomes de carbone d'anneau du cycloalkyle en C₃₋₇ fusionné ou de l'hétérocycloalkyle fusionné à 4 à 6 membres sont facultativement remplacés par un carbonyle ;
ou R⁶ et R⁴ pris conjointement avec les atomes auxquels ils sont attachés forment hétérocycloalkyle fusionné à 4 à 7 membres ou hétéroaryle fusionné à 5 à 6 membres, dans lequel l'hétérocycloalkyle fusionné à 4 à 7 membres et hétéroaryle fusionné à 5 à 6 membres sont chacun facultativement substitués avec 1 ou 2 substituants R^{g} indépendamment sélectionnés ;
ou R⁶ et R¹⁰ pris conjointement avec les atomes auxquels ils sont attachés forment cycloalkyle en C₃₋₇ fusionné, hétérocycloalkyle fusionné à 4 à 6 membres, hétéroaryle fusionné à 5 à 6 membres, ou hétéroaryle fusionné, dans lequel le cycloalkyle en C₃₋₇ fusionné, hétérocycloalkyle fusionné à 4 à 6 membres, hétéroaryle fusionné à 5 à 6 membres, et hétéroaryle fusionné sont chacun facultativement substitués avec 1 ou 2 substituants R^{g} indépendamment sélectionnés, et dans lequel un ou deux atomes de carbone d'anneau du cycloalkyle en C₃₋₇ fusionné ou hétérocycloalkyle fusionné à 4 à 6 membres sont facultativement remplacés par un carbonyle ;
chaque R^{a} est indépendamment sélectionné parmi le groupe constitué de : H, CN, alkyle en C₁-C₆, haloalkyle en C₁-C₆, alcényle en C₂-C₆, alkynyle en C₂-C₆, aryle en C₆-C₁₀, cycloalkyle en C₃-C₁₀, hétéroaryle à 5 à 14 membres, hétérocycloalkyle à 4 à 14 membres, aryle en C₆-C₁₀-alkylène en C₁-C₄-, cycloalkyle en C₃-C₁₀-alkylène en C₁-C₄-, (hétéroaryle à 5 à 14 membres)-alkylène en C₁-C₄-, et (hétérocycloalkyle à 4 à 14 membres)-alkylène en C₁-C₄- ; dans lequel l'alkyle en C₁-C₆, haloalkyle en C₁-C₆, alcényle en C₂-C₆, alkynyle en C₂-C₆, aryle en C₆-C₁₀, cycloalkyle en C₃-C₁₀, hétéroaryle à 5 à 14 membres, hétérocycloalkyle à 4 à 14 membres, aryle en C₆-C₁₀-alkylène en C₁-C₄-, cycloalkyle en C₃-C₁₀-alkylène en C₁-C₄-, (hétéroaryle à 5 à 14 membres)-alkylène en C₁-C₄-, et (hétérocycloalkyle à 4 à 14 membres)-alkylène en C₁-C₄- de R^{a} sont chacun facultativement substitués avec 1, 2, 3, 4, ou 5 substituants R^{d} indépendamment sélectionnés ;
ou deux quelconques substituants R^{a} conjointement avec l'atome d'azote auxquels ils sont attachés forment hétérocycloalkyle à 4, 5, 6, 7, 8, 9, ou 10 membres, dont chacun est facultativement substitué avec 1, 2, ou 3 substituants R^{f} indépendamment sélectionnés ;
chaque R^{b} est indépendamment sélectionné parmi le groupe constitué de : halo, alkyle en C₁-C₆, alcényle en C₂-C₆, alkynyle en C₂-C₆, haloalkyle en C₁-C₆, haloalkoxy en C₁-C₆, aryle en C₆-C₁₀, cycloalkyle en C₃-C₁₀, hétéroaryle à 5 à 10 membres, hétérocycloalkyle à 4 à 10 membres, aryle en C₆-C₁₀-alkylène en C₁-C₄-, cycloalkyle en C₃-C₁₀-alkylène en C₁-C₄-, (hétéroaryle à 5 à 10 membres)-alkylène en C₁-C₄-, (hétérocycloalkyle à 4 à 10 membres)-alkylène en C₁-C₄-, CN, OH, NH₂, NO₂, NHOR^{c}, OR^{c}, SR^{c}, C(O)R^{c}, C(O)NR^{c}R^{c}, C(O)OR^{c}, C(O)NR^{c}S(O)₂R^{c}, OC(O)R^{c}, OC(O)NR^{c}R^{c}, C(=NOH)R^{c}, C(=NOH)NR^{c}, C(=NCN)NR^{c}R^{c}, NR^{c}C(=NCN)NR^{c}R^{c}, C(=NR^{c})NR^{c}R^{c}, NR^{c}C(=NR^{c})NR^{c}R^{c}, NHR^{c}, NR^{c}R^{c}, NR^{c}C(O)R^{c}, NR^{c}C(=NR^{c})R^{c}, NR^{c}C(O)OR^{c}, NR^{c}C(O)NR^{c}R^{c}, NR^{c}S(O)R^{c}, NR^{c}S(O)₂R^{c}, NR^{c}S(O)₂NR^{c}R^{c}, S(O)R^{c}, S(O)NR^{c}R^{c}, S(O)₂R^{c}, S(O)₂NR^{c}C(O)R^{c}, Si(R^{c})₃, P(O)R^{c}R^{c}, P(O)(OR^{c})(OR^{c}), B(OH)₂, B(OR^{c})₂, et S(O)₂NR^{c}R^{c} ; dans lequel l'alkyle en C₁-C₆, alcényle en C₂-C₆, alkynyle en C₂-C₆, aryle en C₆-C₁₀, cycloalkyle en C₃-C₁₀, hétéroaryle à 5 à 10 membres, hétérocycloalkyle à 4 à 10 membres, aryle en C₆-C₁₀-alkylène en C₁-C₄-, cycloalkyle en C₃-C₁₀-alkylène en C₁-C₄-, (hétéroaryle à 5 à 10 membres)-alkylène en C₁-C₄-, et (hétérocycloalkyle à 4 à 10 membres)-alkylène en C₁-C₄- de R^{b} sont chacun en outre facultativement substitué avec 1, 2, ou 3 substituants R^{d} indépendamment sélectionnés ;
chaque R^{c} est indépendamment sélectionné parmi le groupe constitué de : H, alkyle en C₁-C₆, haloalkyle en C₁-C₆, alcényle en C₂-C₆, alkynyle en C₂-C₆, aryle en C₆-C₁₀, cycloalkyle en C₃-C₁₀, hétéroaryle à 5 à 10 membres, hétérocycloalkyle à 4 à 10 membres, aryle en C₆-C₁₀-alkylène en C₁-C₄-, cycloalkyle en C₃-C₁₀-alkylène en C₁-C₄-, (hétéroaryle à 5 à 10 membres)-alkylène en C₁-C₄-, et (hétérocycloalkyle à 4 à 10 membres)-alkylène en C₁-C₄- ; dans lequel l'alkyle en C₁-C₆, alcényle en C₂-C₆, alkynyle en C₂-C₆, aryle en C₆-C₁₀, cycloalkyle en C₃-C₁₀, hétéroaryle à 5 à 10 membres, hétérocycloalkyle à 4 à 10 membres, aryle en C₆-C₁₀-alkylène en C₁-C₄-, cycloalkyle en C₃-C₁₀-alkylène en C₁-C₄-, (hétéroaryle à 5 à 10 membres)-alkylène en C₁-C₄-, et (hétérocycloalkyle à 4 à 10 membres)-alkylène en C₁-C₄- de R^{c} sont chacun facultativement substitués avec 1, 2, 3, 4, ou 5 substituants R^{f} indépendamment sélectionnés ;
ou deux quelconques substituants R^{c} conjointement avec l'atome d'azote auxquels ils sont attachés forment hétérocycloalkyle à 4, 5, 6, 7, 8, 9, ou 10 membres, dont chacun est facultativement substitué avec 1, 2, ou 3 substituants R^{f} indépendamment sélectionnés ;
chaque R^{d} est indépendamment sélectionné parmi le groupe constitué de : alkyle en C₁-C₆, haloalkyle en C₁-C₆, halo, aryle en C₆-C₁₀, hétéroaryle à 5 à 10 membres, cycloalkyle en C₃-C₁₀, hétérocycloalkyle à 4 à 10 membres, aryle en C₆-C₁₀-alkylène en C₁-C₄-, cycloalkyle en C₃-C₁₀-alkylène en C₁-C₄-, (hétéroaryle à 5 à 10 membres)-alkylène en C₁-C₄-, (hétérocycloalkyle à 4 à 10 membres)-alkylène en C₁-C₄-, CN, NH₂, NHOR^{e}, OR^{e}, SR^{e}, C(O)R^{e}, C(O)NR^{e}R^{e}, C(O)OR^{e}, OC(O)R^{e}, OC(O)NR^{e}R^{e}, NHR^{e}, NR^{e}R^{e}, NR^{e}C(O)R^{e}, NR^{e}C(O)NR^{e}R^{e}, NR^{e}C(O)OR^{e}, C(=NR^{e})NR^{e}R^{e}, NR^{e}C(=NR^{e})NR^{e}R^{e}, NR^{e}C(=NOH)NR^{e}R^{e}, NR^{e}C(=NCN)NR^{e}R^{e}, S(O)R^{e}, S(O)NR^{e}R^{e}, S(O)₂R^{e}, NR^{e}S(O)₂R^{e}, NR^{e}S(O)₂NR^{e}R^{e}, et S(O)₂NR^{e}R^{e} ; dans lequel l'alkyle en C₁-C₆, aryle en C₆-C₁₀, hétéroaryle à 5 à 10 membres, cycloalkyle en C₃-C₁₀, hétérocycloalkyle à 4 à 10 membres, aryle en C₆-C₁₀-alkylène en C₁-C₄-, cycloalkyle en C₃-C₁₀-alkylène en C₁-C₄-, (hétéroaryle à 5 à 10 membres)-alkylène en C₁-C₄-, et (hétérocycloalkyle à 4 à 10 membres)-alkylène en C₁-C₄- de R^{d} sont chacun facultativement substitués avec 1, 2, ou 3 substituants R^{f} indépendamment sélectionnés ;
chaque R^{e} est indépendamment sélectionné parmi le groupe constitué de : H, alkyle en C₁-C₆, alkoxy en C₁-C₆, cycloalkyle en C₃-C₆, cycloalkyle en C₃-C₆-alkylène en C₁-C₄-, aryle en C₆-C₁₀, aryle en C₆-C₁₀-alkylène en C₁-C₄-, hétéroaryle à 5 ou 6 membres, (hétéroaryle à 5 ou 6 membres)-alkylène en C₁-C₄-, hétérocycloalkyle à 4 à 7 membres, (hétérocycloalkyle à 4 à 7 membres)-alkylène en C₁-C₄-, haloalkyle en C₁-C₆, haloalkoxy en C₁-C₆, alcényle en C₂-C₄, et alkynyle en C₂-C₄, dans lequel l'alkyle en C₁-C₆, alkoxy en C₁-C₆, cycloalkyle en C₃-C₆, aryle en C₆-C₁₀, hétéroaryle à 5 ou 6 membres, hétérocycloalkyle à 4 à 7 membres, aryle en C₆-C₁₀-alkylène en C₁-C₄-, (hétéroaryle à 5 ou 6 membres)-alkylène en C₁-C₄-, (hétérocycloalkyle à 4 à 7 membres)-alkylène en C₁-C₄-, alcényle en C₂-C₄, et alkynyle en C₂-C₄ de R^{e} sont chacun facultativement substitués avec 1, 2, ou 3 substituants R^{f} ;
ou deux quelconques substituants R^{e} conjointement avec l'atome d'azote auxquels ils sont attachés forment un hétérocycloalkyle à 4, 5, 6, 7, 8, 9, ou 10 membres, dont chacun est facultativement substitué avec 1, 2, ou 3 substituants R^{f} indépendamment sélectionnés ;
chaque R^{f} est indépendamment sélectionné parmi le groupe constitué de : halo, OH, CN, C(O)OH, NH₂, -NH(alkyle en C₁-C₆), -N(alkyle en C₁-C₆), alkyle en C₁-C₆, vinyle, alkoxy en C₁-C₆, alkylthio en C₁-C₆, haloalkyle en C₁-C₆, haloalkoxy en C₁-C₆, phényle, hétéroaryle à 5 à 6 membres, hétérocycloalkyle à 4 à 6 membres, et cycloalkyle en C₃-C₆, dans lequel l'alkyle en C₁-C₆, alkoxy en C₁-C₆, alkylthio en C₁-C₆, phényle, cycloalkyle en C₃-C₆-hétérocycloalkyle à 4 à 6 membres, et hétéroaryle à 5 à 6 membres de R^{f} sont chacun facultativement substitués avec 1, 2, ou 3 substituants sélectionnés parmi halo, OH, CN, - C(O)OH, -NH₂, alkyle en C₁-C₄, alkoxy en C₁-C₄, haloalkyle en C₁-C₄, haloalkoxy en C₁-C₄, phényle, cycloalkyle en C₃-C₁₀, hétéroaryle à 5 à 6 membres, et hétérocycloalkyle à 4 à 6 membres ;
chaque R^{g} est indépendamment sélectionné parmi le groupe constitué de : halo, OH, CN, C(O)OH, -C(O)O-alkyle en C₁-C₄, NH₂, -NH(alkyle en C₁-C₆), -N(alkyle en C₁-C₆)₂, alkyle en C₁-C₆, alkoxy en C₁-C₆, alkylthio en C₁-C₆, haloalkyle en C₁-C₆, haloalkoxy en C₁-C₆, phényle, hétéroaryle à 5 à 6 membres, hétérocycloalkyle à 4 à 6 membres, et cycloalkyle en C₃₋C₆ ;
un atome d'azote d'anneau dans la formule (II) est facultativement oxydé ;
l'indice m est 0, 1, ou 2 ; et
l'indice n est 0, 1, 2, 3, ou 4.

2. Composé de la revendication 1, dans lequel l'anneau B est hétéroaryle à 6 membres ayant 1 ou 2 atomes d'azote en tant que membres d'anneau.

3. Composé de la revendication 1 ou de la revendication 2, ou sel pharmaceutiquement acceptable ou stéréoisomère de celui-ci, dans lequel (i) l'anneau B est 2-thiényle, 3-thiényle, 2-furyle, 3-furyle, 1H-pyrazol-3-yle, 1H-pyrazol-4-yle, 1H-pyrazol-5-yle, ou pyrazol-1-yle ; ou (ii) l'anneau B est 2-pyridyle, 3-pyridyle, 4-pyridyle, ou 3-pyridazinyle.

4. Composé de l'une quelconque des revendications 1 à 3, ayant la formule (IIa) : 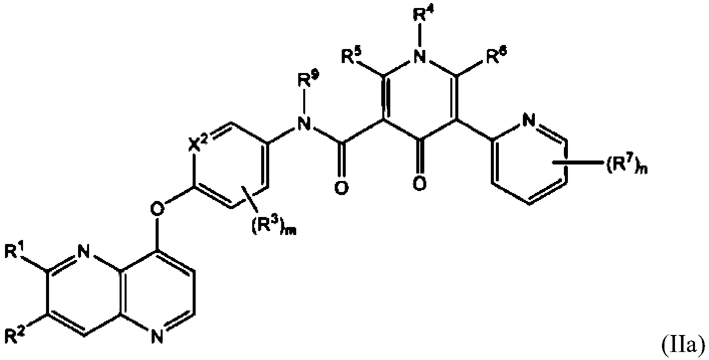 ou sel pharmaceutiquement acceptable ou stéréoisomère de celui-ci ;
ledit composé ayant de préférence la formule (Ia-1) : 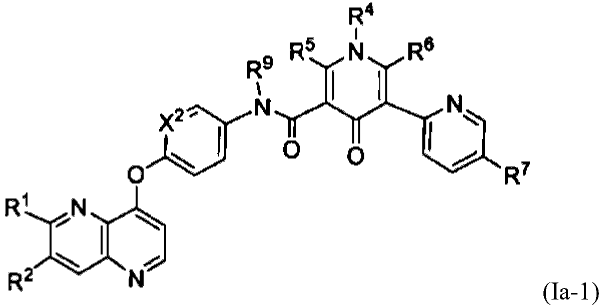 ou sel pharmaceutiquement acceptable ou stéréoisomère de celui-ci.

5. Composé de l'une quelconque des revendications 1 à 3, ayant la formule (IIb) : 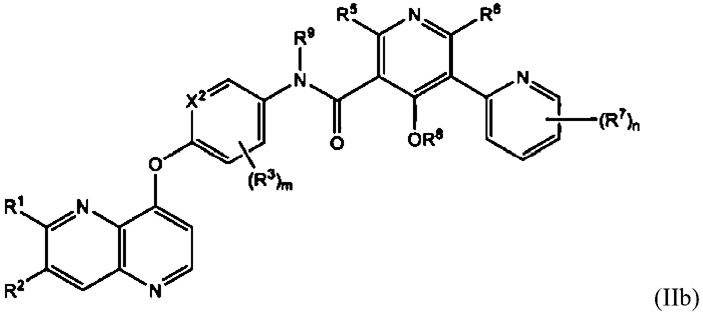 ou sel pharmaceutiquement acceptable ou stéréoisomère de celui-ci ;
ledit composé ayant de préférence la formule (Ib-1) : 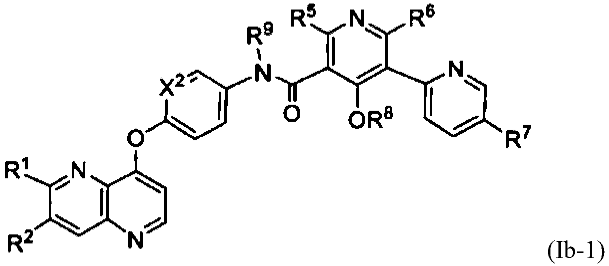 ou sel pharmaceutiquement acceptable ou stéréoisomère de celui-ci.

6. Composé de l'une quelconque des revendications 1 à 3, ayant la formule (IIc) : 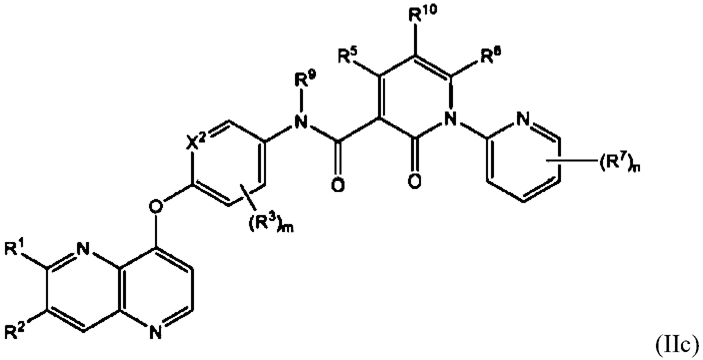 ou sel pharmaceutiquement acceptable ou stéréoisomère de celui-ci ; ledit composé ayant de préférence la formule (Ic-1) : 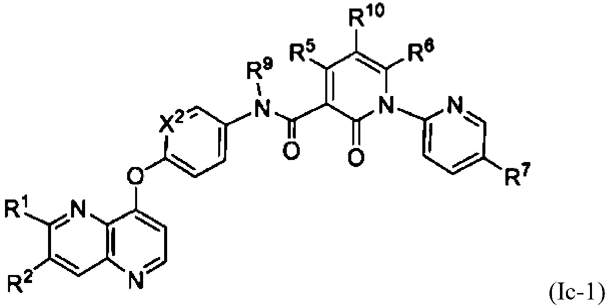 ou sel pharmaceutiquement acceptable ou stéréoisomère de celui-ci.

7. Composé de d'une quelconque des revendications 1 à 3, ayant :
i) 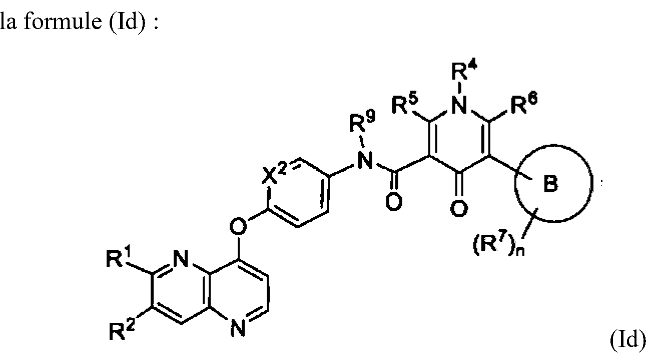 ou sel pharmaceutiquement acceptable ou stéréoisomère de celui-ci ; ou
ii) 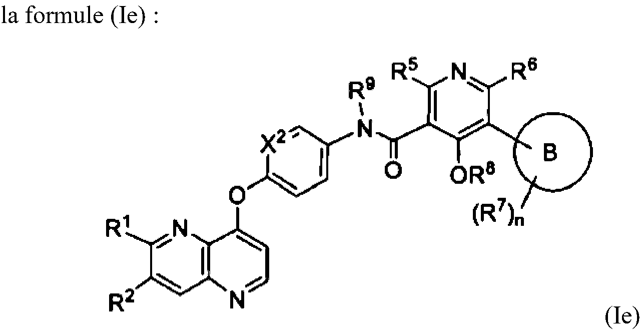 ou sel pharmaceutiquement acceptable ou stéréoisomère de celui-ci ; ou
iii) 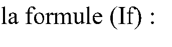 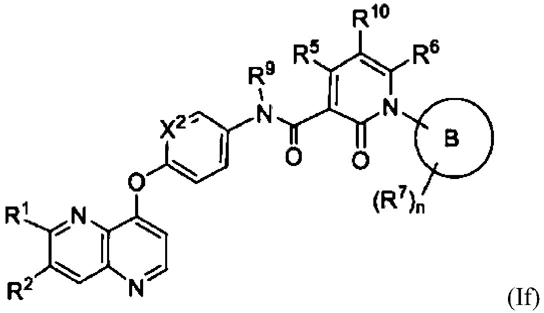 ou sel pharmaceutiquement acceptable ou stéréoisomère de celui-ci ; ou
iv) 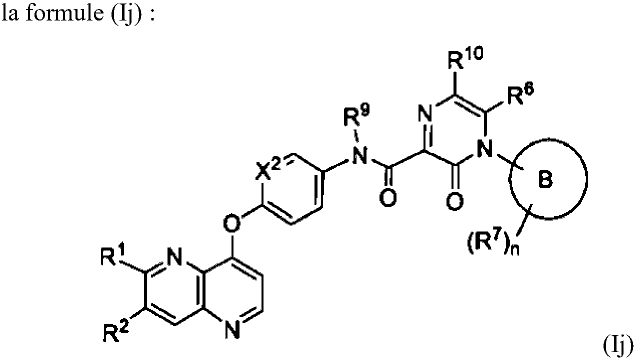 ou sel pharmaceutiquement acceptable ou stéréoisomère de celui-ci ; ou
v) 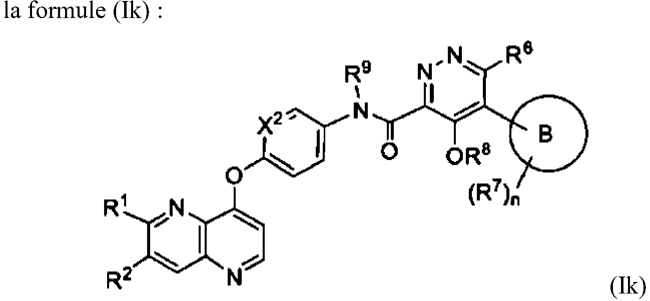 ou sel pharmaceutiquement acceptable ou stéréoisomère de celui-ci ; ou
vi) 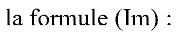 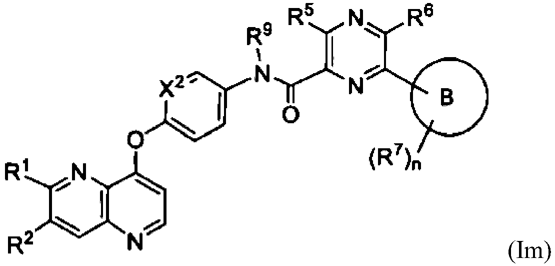 ou sel pharmaceutiquement acceptable ou stéréoisomère de celui-ci.

8. Composé de la revendication 7, ou sel pharmaceutiquement acceptable ou stéréoisomère de celui-ci, dans lequel l'anneau B est :
i) 2-pyridyle, 3-pyridyle, 4-pyridyle, ou hétéroaryle à 5 membres ayant 1, 2, ou 3 hétéroatomes en tant que membres d'anneau sélectionnés parmi N, O, et S ; ou
ii) 2-thiényle, 3-thiényle, 2-furyle, 3-furyle, 2-benzofuranyle, 3-benzofuranyle, 1H-pyrazol-3-yle, 1H-pyrazol-4-yle, 1H-pyrazol-5-yle, ou pyrazol-1-yle.

9. Composé de l'une quelconque des revendications 1 à 8, ou sel pharmaceutiquement acceptable ou stéréoisomère de celui-ci, dans lequel R⁹ est H ou méthyle.

10. Composé de l'une quelconque des revendications 1 à 9, ou sel pharmaceutiquement acceptable ou stéréoisomère de celui-ci, dans lequel n est O ; ou dans lequel n est 1, 2, 3, ou 4, et chaque R⁷ est indépendamment sélectionné parmi halo, alkyle en C₁₋₆, et alkoxy en C₁₋₆.

11. Composé de l'une quelconque des revendications 1 à 10, ou sel pharmaceutiquement acceptable ou stéréoisomère de celui-ci, dans lequel X² est CF.

12. Composé de l'une quelconque des revendications 1 à 11, ou sel pharmaceutiquement acceptable ou stéréoisomère de celui-ci, dans lequel :
i) chaque R⁴ est indépendamment sélectionné parmi H, alkyle en C₁₋₆, alkoxy en C₁₋₆, OH, NH₂, -NH(alkyle en C₁₋₆), -N(alkyle en C₁₋₆)₂, cycloalkyle en C₃₋₆, haloalkyle en C₁₋₆, cycloalkyle en C₃₋₆-alkylène en C₁₋₄-, hétérocycloalkyle à 4 à 6 membres, (hétérocycloalkyle à 4 à 6 membres)-alkylène en C₁₋₄-, hétéroaryle à 5 à 6 membres, (hétéroaryle à 5 à 6 membres)-alkylène en C₁₋₄-, et N=C[N(alkyle en C₁₋₆)(alkyle en C₁₋₆)]₂, dans lequel l'alkyle en C₁₋₆, alkoxy en C₁₋₆, -NH(alkyle en C₁₋₆), -N(alkyle en C₁₋₆)₂, cycloalkyle en C₃₋₆, cycloalkyle en C₃₋₆-alkylène en C₁₋₄-, hétérocycloalkyle à 4 à 6 membres, (hétérocycloalkyle à 4 à 6 membres)-alkylène en C₁₋₄-, hétéroaryle à 5 à 6 membres, (hétéroaryle à 5 à 6 membres)-alkylène en C₁₋₄-, et N=C[N(alkyle en C₁₋₆)(alkyle en C₁₋₆)]₂ de R⁴ sont chacun facultativement substitués avec 1 ou 2 substituants R^{b} ou R^{g} indépendamment sélectionnés ; et/ou
ii) R⁵, R⁶, et R¹⁰ sont chacun indépendamment sélectionnés parmi H, CH₃, propèn-2-yle, Br, Cl, CN, méthoxy, 2-fluoroéthyle, isopropyle, CH₃C(O)-, OH, t-butyle, éthyle, hydroxyméthyle, isopropylthio, et méthoxyméthyle.

13. Composé d'une quelconque revendication précédente, sélectionnés parmi
N-[4-[(6,7-diméthoxy-1,5-naphtyridin-4-yl)oxy]-3-fluorophényl]-1-(5-fluoropyridin-2-yl)-4-méthyl-2-oxopyridine-3-carboxamide ;
N-[4-[(6,7-diméthoxy-1,5-naphtyridin-4-yl)oxy]-3-fluorophényl]-1-(5-fluoropyridin-2-yl)-2-oxopyridine-3-carboxamide ;
N-[4-[(6,7-diméthoxy-1,5-naphtyridin-4-yl)oxy]-3-fluorophényl]-1-(5-fluoropyridin-2-yl)-6-méthyl-2-oxopyridine-3-carboxamide ;
N-[4-[(6,7-diméthoxy-1,5-naphtyridin-4-yl)oxy]phényl]-1-(5-fluoropyridin-2-yl)-6-méthyl-2-oxopyridine-3-carboxamide ;
N-[3-fluoro-4-[(7-méthoxy-1,5-naphtyridin-4-yl)oxy]phényl]-1-(5-fluoropyridin-2-yl)-6-méthyl-2-oxopyridine-3-carboxamide ;
1-(5-fluoropyridin-2-yl)-N-[4-[(7-méthoxy-1,5-naphtyridin-4-yl)oxy]phényl]-6-méthyl-2-oxopyridine-3-carboxamide ;
N-[3-fluoro-4-[(6-méthoxy-1,5-naphtyridin-4-yl)oxy]phényl]-1-(5-fluoropyridin-2-yl)-6-méthyl-2-oxopyridine-3-carboxamide ;
N-[3-fluoro-4-(1,5-naphtyridin-4-yloxy)phényl]-1-(5-fluoropyridin-2-yl)-6-méthyl-2-oxopyridine-3-carboxamide ;
N-[3-fluoro-4-[[6-méthoxy-7-(2-méthoxyéthoxy)-1,5-naphtyridin-4-yl]oxy]phényl]-1-(5-fluoropyridin-2-yl)-6-méthyl-2-oxopyridine-3-carboxamide ;
N-[4-[(6,7-diméthoxy-1,5-naphtyridin-4-yl)oxy]-3-fluorophényl]-1-(5-fluoro-3-méthylpyridin-2-yl)-6-méthyl-2-oxopyridine-3-carboxamide ;
N-[3-fluoro-4-[(7-méthoxy-1,5-naphtyridin-4-yl)oxy]phényl]-1-(5-fluoro-3-méthylpyridin-2-yl)-6-méthyl-2-oxopyridine-3-carboxamide ;
N-[3-fluoro-4-[(6-méthoxy-1,5-naphtyridin-4-yl)oxy]phényl]-1-(5-fluoro-3-méthylpyridin-2-yl)-6-méthyl-2-oxopyridine-3-carboxamide ;
5-bromo-N-[4-[(6,7-diméthoxy-1,5-naphtyridin-4-yl)oxy]-3-fluorophényl]-1-(5-fluoropyridin-2-yl)-6-méthyl-2-oxopyridine-3-carboxamide ;
5-cyano-N-[4-[(6,7-diméthoxy-1,5-naphtyridin-4-yl)oxy]-3-fluorophényl]-1-(5-fluoropyridin-2-yl)-6-méthyl-2-oxopyridine-3-carboxamide ;
5-acétyl-N-[4-[(6,7-diméthoxy-1,5-naphtyridin-4-yl)oxy]-3-fluorophényl]-1-(5-fluoropyridin-2-yl)-6-méthyl-2-oxopyridine-3-carboxamide ;
N-[4-[(6,7-diméthoxy-1,5-naphtyridin-4-yl)oxy]-3-fluorophényl]-5-(5-fluoropyridin-2-yl)-4-hydroxy-2-méthylpyridine-3-carboxamide ;
N-[4-[(6,7-diméthoxy-1,5-naphtyridin-4-yl)oxy]-3-fluorophényl]-4-hydroxy-2-méthyl-5-pyridin-2-ylpyridine-3-carboxamide ;
N-[3-fluoro-4-[(7-méthoxy-1,5-naphtyridin-4-yl)oxy]phényl]-4-hydroxy-2-méthyl-5-pyridin-2-ylpyridine-3-carboxamide ;
N-[4-[(6,7-diméthoxy-1,5-naphtyridin-4-yl)oxy]-3-fluorophényl]-5-(5-fluoropyridin-2-yl)-1,2-diméthyl-4-oxopyridine-3-carboxamide ;
N-[4-[(6,7-diméthoxy-1,5-naphtyridin-4-yl)oxy]phényl]-5-(5-fluoropyridin-2-yl)-1,2-diméthyl-4-oxopyridine-3-carboxamide ;
N-[3-fluoro-4-[(7-méthoxy-1,5-naphtyridin-4-yl)oxy]phényl]-5-(5-fluoropyridin-2-yl)-l,2-diméthyl-4-oxopyridine-3-carboxamide ;
N-[4-[(6,7-diméthoxy-1,5-naphtyridin-4-yl)oxy]-3-fluorophényl]-5-(5-fluoropyridin-2-yl)-4-hydroxy-6-méthylpyridine-3-carboxamide ;
N-[4-[(6,7-diméthoxy-1,5-naphtyridin-4-yl)oxy]phényl]-5-(5-fluoropyridin-2-yl)-1,6-diméthyl-4-oxopyridine-3-carboxamide ;
N-[4-[(6,7-diméthoxy-1,5-naphtyridin-4-yl)oxy]-3-fluorophényl]-5-(furan-2-yl)-4-hydroxy-6-méthylpyridine-3-carboxamide ;
N-[4-[(6,7-diméthoxy-1,5-naphtyridin-4-yl)oxy]-3-fluorophényl]-4-hydroxy-6-méthyl-5-thiophén-2-ylpyridine-3-carboxamide ;
N-[4-[(6,7-diméthoxy-1,5-naphtyridin-4-yl)oxy]-3-fluorophényl]-4-hydroxy-6-méthyl-5-(4-méthylthiophén-2-yl)pyridine-3-carboxamide ;
N-[4-[(6,7-diméthoxy-1,5-naphtyridin-4-yl)oxy]-3-fluorophényl]-4-hydroxy-6-méthyl-5-(5-méthylfuran-2-yl)pyridine-3-carboxamide ;
N-[4-[(6,7-diméthoxy-1,5-naphtyridin-4-yl)oxy]-3-fluorophényl]-4-hydroxy-6-méthyl-5-thiophén-3-ylpyridine-3-carboxamide ;
N-[4-[(6,7-diméthoxy-1,5-naphtyridin-4-yl)oxy]-3-fluorophényl]-4-hydroxy-6-méthyl-5-(5-méthylthiophén-2-yl)pyridine-3-carboxamide ;
N-[4-[(6,7-diméthoxy-1,5-naphtyridin-4-yl)oxy]-3-fluorophényl]-5-(furan-3-yl)-4-hydroxy-6-méthylpyridine-3-carboxamide ;
N-[3-fluoro-4-[(7-méthoxy-1,5-naphtyridin-4-yl)oxy]phényl]-5-(furan-2-yl)-4-hydroxy-6-méthylpyridine-3-carboxamide ;
N-[3-fluoro-4-[(7-méthoxy-1,5-naphtyridin-4-yl)oxy]phényl]-4-hydroxy-6-méthyl-5-(5-méthylfuran-2-yl)pyridine-3-carboxamide ;
N-[3-fluoro-4-[(7-méthoxy-1,5-naphtyridin-4-yl)oxy]phényl]-4-hydroxy-6-méthyl-5-thiophén-3-ylpyridine-3-carboxamide ;
N-[3-fluoro-4-[(7-méthoxy-1,5-naphtyridin-4-yl)oxy]phényl]-5-(furan-3-yl)-4-hydroxy-6-méthylpyridine-3-carboxamide ;
N-[3-fluoro-4-[(7-méthoxy-1,5-naphtyridin-4-yl)oxy]phényl]-4-hydroxy-6-méthyl-5-thiophén-2-ylpyridine-3-carboxamide ;
N-[3-fluoro-4-[(7-méthoxy-1,5-naphtyridin-4-yl)oxy]phényl]-4-hydroxy-6-méthyl-5-(5-méthylthiophén-2-yl)pyridine-3-carboxamide ;
N-[3-fluoro-4-[(7-méthoxy-1,5-naphtyridin-4-yl)oxy]phényl]-4-hydroxy-6-méthyl-5-(4-méthylthiophén-2-yl)pyridine-3-carboxamide ;
N-[4-[(6,7-diméthoxy-1,5-naphtyridin-4-yl)oxy]-3-fluorophényl]-4-hydroxy-6-méthyl-5-(3-méthylthiophén-2-yl)pyridine-3-carboxamide ;
N-[4-[(6,7-diméthoxy-1,5-naphtyridin-4-yl)oxy]-3-fluorophényl]-4-hydroxy-6-méthyl-5-(2-méthylpyrazol-3-yl)pyridine-3-carboxamide ;
N-[4-[(6,7-diméthoxy-1,5-naphtyridin-4-yl)oxy]-3-fluorophényl]-4-hydroxy-6-méthyl-5-[2-méthyl-5-(trifluorométhyl)pyrazol-3-yl]pyridine-3-carboxamide ;
N-[4-[(6,7-diméthoxy-1,5-naphtyridin-4-yl)oxy]-3-fluorophényl]-4-hydroxy-2,6-diméthyl-5-thiophén-3-ylpyridine-3-carboxamide ;
N-[4-[(6,7-diméthoxy-1,5-naphtyridin-4-yl)oxy]-3-fluorophényl]-4-hydroxy-2,6-diméthyl-5-thiophén-2-ylpyridine-3-carboxamide ;
N-[4-[(6,7-diméthoxy-1,5-naphtyridin-4-yl)oxy]-3-fluorophényl]-4-hydroxy-2,6-diméthyl-5-(2-méthylpyrazol-3-yl)pyridine-3-carboxamide ;
N-[4-[(6,7-diméthoxy-1,5-naphtyridin-4-yl)oxy]-3-fluorophényl]-5-(2,5-diméthylpyrazol-3-yl)-4-hydroxy-2,6-diméthylpyridine-3-carboxamide ;
N-[4-[(6,7-diméthoxy-1,5-naphtyridin-4-yl)oxy]-3-fluorophényl]-4-hydroxy-2,6-diméthyl-5-(5-méthylfuran-2-yl)pyridine-3-carboxamide ;
N-[4-[(6,7-diméthoxy-1,5-naphtyridin-4-yl)oxy]-3-fluorophényl]-4-hydroxy-2,6-diméthyl-5-(5-méthylthiophén-2-yl)pyridine-3-carboxamide ;
N-[4-[(6,7-diméthoxy-1,5-naphtyridin-4-yl)oxy]-3-fluorophényl]-5-(furan-3-yl)-4-hydroxy-2,6-diméthylpyridine-3-carboxamide ;
N-[4-[(6,7-diméthoxy-1,5-naphtyridin-4-yl)oxy]-3-fluorophényl]-4-hydroxy-2-(méthoxyméthyl)-6-méthyl-5-thiophén-2-ylpyridine-3-carboxamide ;
N-[4-[(6,7-diméthoxy-1,5-naphtyridin-4-yl)oxy]-3-fluorophényl]-5-(furan-2-yl)-4-hydroxy-2-(méthoxyméthyl)-6-méthylpyridine-3-carboxamide ;
N-[4-[(6,7-diméthoxy-1,5-naphtyridin-4-yl)oxy]-3-fluorophényl]-4-hydroxy-2-(méthoxyméthyl)-6-méthyl-5-(5-méthylfuran-2-yl)pyridine-3-carboxamide ;
N-[3-fluoro-4-[(6-méthoxy-1,5-naphtyridin-4-yl)oxy]phényl]-5-(furan-2-yl)-4-hydroxy-6-méthylpyridine-3-carboxamide ;
N-[4-[(6,7-diméthoxy-1,5-naphtyridin-4-yl)oxy]-3-fluorophényl]-5-(furan-2-yl)-4-hydroxy-2-méthylpyridine-3-carboxamide ;
N-[4-[(6,7-diméthoxy-1,5-naphtyridin-4-yl)oxy]-3-fluorophényl]-4-hydroxy-2-méthyl-5-(5-méthylfuran-2-yl)pyridine-3-carboxamide ;
N-[4-[(6,7-diméthoxy-1,5-naphtyridin-4-yl)oxy]-3-fluorophényl]-5-(5-formylfuran-2-yl)-4-hydroxy-2-méthylpyridine-3-carboxamide ;
N-[4-[(6,7-diméthoxy-1,5-naphtyridin-4-yl)oxy]-3-fluorophényl]-5-(furan-3-yl)-4-hydroxy-2-méthylpyridine-3-carboxamide ;
N-[4-[(6,7-diméthoxy-1,5-naphtyridin-4-yl)oxy]-3-fluorophényl]-4-hydroxy-2-méthyl-5-thiophén-2-ylpyridine-3-carboxamide ;
N-[4-[(6,7-diméthoxy-1,5-naphtyridin-4-yl)oxy]-3-fluorophényl]-4-hydroxy-2-méthyl-5-(5-méthylthiophén-2-yl)pyridine-3-carboxamide ;
N-[4-[(6,7-diméthoxy-1,5-naphtyridin-4-yl)oxy]-3-fluorophényl]-4-hydroxy-2-méthyl-5-(4-méthylthiophén-2-yl)pyridine-3-carboxamide ;
N-[4-[(6,7-diméthoxy-1,5-naphtyridin-4-yl)oxy]-3-fluorophényl]-4-hydroxy-2-méthyl-5-(3-méthylthiophén-2-yl)pyridine-3-carboxamide ;
N-[4-[(6,7-diméthoxy-1,5-naphtyridin-4-yl)oxy]-3-fluorophényl]-4-hydroxy-2-méthyl-5-thiophén-3-ylpyridine-3-carboxamide ;
N-[3-fluoro-4-[(6-méthoxy-1,5-naphtyridin-4-yl)oxy]phényl]-4-hydroxy-6-méthyl-5-thiophén-2-ylpyridine-3-carboxamide ;
5-(furan-2-yl)-4-hydroxy-N-[4-[(7-méthoxy-1,5-naphtyridin-4-yl)oxy]phényl]-6-méthylpyridine-3-carboxamide ;
N-[4-[(6,7-diméthoxy-1,5-naphtyridin-4-yl)oxy]-3-fluorophényl]-5-(furan-2-yl)-4-hydroxy-2,6-diméthylpyridine-3-carboxamide ;
N-[4-[(6,7-diméthoxy-1,5-naphtyridin-4-yl)oxy]-3-fluorophényl]-5-(2,5-diméthylpyrazol-3-yl)-4-hydroxy-6-méthylpyridine-3-carboxamide ;
N-[4-[(6,7-diméthoxy-1,5-naphtyridin-4-yl)oxy]-3-fluorophényl]-4-hydroxy-2,6-diméthyl-5-(4-méthylthiophén-2-yl)pyridine-3-carboxamide ;
N-[4-[(6,7-diméthoxy-1,5-naphtyridin-4-yl)oxy]-3-fluorophényl]-4-hydroxy-2,6-diméthyl-5-[2-méthyl-5-(trifluorométhyl)pyrazol-3-yl]pyridine-3-carboxamide ;
N-[4-[(6,7-diméthoxy-1,5-naphtyridin-4-yl)oxy]-3-fluorophényl]-4-hydroxy-2,6-diméthyl-5-(3-méthylthiophén-2-yl)pyridine-3-carboxamide ;
N-[4-[(6,7-diméthoxy-1,5-naphtyridin-4-yl)oxy]-3-fluorophényl]-4-hydroxy-6-méthyl-5-(1,2-oxazol-4-yl)pyridine-3-carboxamide ;
N-[4-[(6,7-diméthoxy-1,5-naphtyridin-4-yl)oxy]-3-fluorophényl]-4-hydroxy-6-méthyl-5-(1,3-thiazol-5-yl)pyridine-3-carboxamide ;
N-[4-[(6,7-diméthoxy-1,5-naphtyridin-4-yl)oxy]-3-fluorophényl]-4-hydroxy-6-méthyl-5-(1,3-thiazol-4-yl)pyridine-3-carboxamide ;
N-[4-[(6,7-diméthoxy-1,5-naphtyridin-4-yl)oxy]-3-fluorophényl]-4-hydroxy-6-méthyl-5-(1,3-thiazol-2-yl)pyridine-3-carboxamide ;
N-[4-[(6,7-diméthoxy-1,5-naphtyridin-4-yl)oxy]-3-fluorophényl]-5-(5-éthylfuran-2-yl)-4-hydroxy-2,6-diméthylpyridine-3-carboxamide ;
N-[4-[(6,7-diméthoxy-1,5-naphtyridin-4-yl)oxy]-3-fluorophényl]-4-hydroxy-2,6-diméthyl-5-(5-propan-2-ylfuran-2-yl)pyridine-3-carboxamide ;
N-[4-[(6,7-diméthoxy-1,5-naphtyridin-4-yl)oxy]-3-fluorophényl]-5-(5-ethenylfuran-2-yl)-4-hydroxy-6-méthylpyridine-3-carboxamide ;
N-[4-[(6,7-diméthoxy-1,5-naphtyridin-4-yl)oxy]-3-fluorophényl]-4-hydroxy-6-méthyl-5-(5-prop-1-én-2-ylfuran-2-yl)pyridine-3-carboxamide ;
N-[4-[(6,7-diméthoxy-1,5-naphtyridin-4-yl)oxy]-3-fluorophényl]-5-(5-éthylfuran-2-yl)-4-hydroxy-6-méthylpyridine-3-carboxamide ;
N-[4-[(6,7-diméthoxy-1,5-naphtyridin-4-yl)oxy]-3-fluorophényl]-4-hydroxy-6-méthyl-5-(5-propan-2-ylfuran-2-yl)pyridine-3-carboxamide ;
N-[4-[(6,7-diméthoxy-1,5-naphtyridin-4-yl)oxy]-3-fluorophényl]-4-hydroxy-6-méthyl-5-(5-prop-2-enylfuran-2-yl)pyridine-3-carboxamide ;
N-[4-[(6,7-diméthoxy-1,5-naphtyridin-4-yl)oxy]-3-fluorophényl]-5-(furan-2-yl)-1,2,6-triméthyl-4-oxopyridine-3-carboxamide ;
5-(5-cyclopropylfuran-2-yl)-N-[4-[(6,7-diméthoxy-1,5-naphtyridin-4-yl)oxy]-3-fluorophényl]-4-hydroxy-6-méthylpyridine-3-carboxamide ;
5-(5-cyclopropylfuran-2-yl)-N-[4-[(6,7-diméthoxy-1,5-naphtyridin-4-yl)oxy]-3-fluorophényl]-4-hydroxy-2,6-diméthylpyridine-3-carboxamide ;
5-(furan-2-yl)-N-[4-[(7-méthoxy-1,5-naphtyridin-4-yl)oxy]phényl]-1,2,6-triméthyl-4-oxopyridine-3-carboxamide ;
N-[3-fluoro-4-[(7-méthoxy-1,5-naphtyridin-4-yl)oxy]phényl]-5-(furan-2-yl)-1,2,6-triméthyl-4-oxopyridine-3-carboxamide ;
N-[4-[(6,7-diméthoxy-1,5-naphtyridin-4-yl)oxy]-2,5-difluorophényl]-5-(furan-2-yl)-1,2,6-triméthyl-4-oxopyridine-3-carboxamide ;
N-[4-[(6,7-diméthoxy-1,5-naphtyridin-4-yl)oxy]phényl]-5-(furan-2-yl)-1,2,6-triméthyl-4-oxopyridine-3-carboxamide ;
N-[4-[(7-méthoxy-1,5-naphtyridin-4-yl)oxy]phényl]-1,2,6-triméthyl-4-oxo-5-thiophén-2-ylpyridine-3-carboxamide ;
N-[3-fluoro-4-[(7-méthoxy-1,5-naphtyridin-4-yl)oxy]phényl]-1,2,6-triméthyl-4-oxo-5-thiophén-2-ylpyridine-3-carboxamide ;
N-[4-[(6,7-diméthoxy-1,5-naphtyridin-4-yl)oxy]phényl]-1,2,6-triméthyl-4-oxo-5-thiophén-2-ylpyridine-3-carboxamide ;
N-[4-[(6,7-diméthoxy-1,5-naphtyridin-4-yl)oxy]-3-fluorophényl]-1,2,6-triméthyl-4-oxo-5-thiophén-2-ylpyridine-3-carboxamide ;
N-[4-[(6,7-diméthoxy-1,5-naphtyridin-4-yl)oxy]-3-fluorophényl]-4-hydroxy-2-(méthoxyméthyl)-6-méthyl-5-pyridin-2-y]pyridine-3-carboxamide ;
N-[4-[(6,7-diméthoxy-1,5-naphtyridin-4-yl)oxy]-3-fluorophényl]-4-hydroxy-2-(méthoxyméthyl)-6-méthyl-5-(3-méthylpyridin-2-yl)pyridine-3-carboxamide ;
N-[4-[(6,7-diméthoxy-1,5-naphtyridin-4-yl)oxy]-3-fluorophényl]-4-hydroxy-2-(méthoxyméthyl)-6-méthyl-5-(6-méthylpyridin-2-yl)pyridine-3-carboxamide ;
N-[4-[(6,7-diméthoxy-1,5-naphtyridin-4-yl)oxy]-3-fluorophényl]-4-hydroxy-2-(méthoxyméthyl)-6-méthyl-5-(2-méthyl pyridin-4-yl)pyridine-3-carboxamide ;
N-[4-[(6,7-diméthoxy-1,5-naphtyridin-4-yl)oxy]-3-fluorophényl]-5-(5-fluoro-3-méthylpyridin-2-yl)-4-hydroxy-2-méthylpyridine-3-carboxamide ;
N-[4-[(6,7-diméthoxy-1,5-naphtyridin-4-yl)oxy]-3-fluorophényl]-1-(5-éthoxypyridin-2-yl)-6-méthyl-2-oxopyridine-3-carboxamide ;
1-(5-éthoxypyridin-2-yl)-N-[3-fluoro-4-[(7-méthoxy-1,5-naphtyridin-4-yl)oxy]phényl]-6-méthyl-2-oxopyridine-3-carboxamide ;
N-[4-[(6,7-diméthoxy-1,5-naphtyridin-4-yl)oxy]-3-fluorophényl]-5-(5-fluoropyridin-2-yl)-4-hydroxy-2-(méthoxyméthyl)-6-méthylpyridine-3-carboxamide ;
N-[4-[(6,7-diméthoxy-1,5-naphtyridin-4-yl)oxy]-3-fluorophényl]-5-(5-fluoropyridin-2-yl)-4-méthoxy-6-méthylpyridazine-3-carboxamide ;
N-[4-[(6,7-diméthoxy-1,5-naphtyridin-4-yl)oxy]phényl]-5-(5-fluoropyridin-2-yl)-4-hydroxy-2,6-diméthylpyridine-3-carboxamide ;
N-[3-fluoro-4-[(7-méthoxy-1,5-naphtyridin-4-yl)oxy]phényl]-5-(5-fluoropyridin-2-yl)-4-hydroxy-2,6-diméthylpyridine-3-carboxamide ;
N-[4-[(6,7-diméthoxy-1,5-naphtyridin-4-yl)oxy]-3-fluorophényl]-5-(5-fluoropyridin-2-yl)-4-hydroxy-2,6-diméthylpyridine-3-carboxamide ;
N-[4-[(6,7-diméthoxy-1,5-naphtyridin-4-yl)oxy]-3-fluorophényl]-4-(5-fluoropyridin-2-yl)-5-méthyl-3-oxopyrazine-2-carboxamide ;
N-[4-[(6,7-diméthoxy-1,5-naphtyridin-4-yl)oxy]-3-fluorophényl]-1-(3-fluoropyridin-4-yl)-6-méthyl-2-oxopyridine-3-carboxamide ;
N-[4-[(6,7-diméthoxy-1,5-naphtyridin-4-yl)oxy]-3-fluorophényl]-6-méthyl-2-oxo-1-pyridin-4-ylpyridine-3-carboxamide ;
N-[4-[(6,7-diméthoxy-1,5-naphtyridin-4-yl)oxy]-3-fluorophényl]-6-méthyl-2-oxo-1-pyridin-3-ylpyridine-3-carboxamide ;
N-[4-[(6,7-diméthoxy-1,5-naphtyridin-4-yl)oxy]-3-fluorophényl]-1-[2-[2-(2-éthoxyéthoxy)éthoxy]pyridin-4-yl]-6-méthyl-2-oxopyridine-3-carboxamide ;
N-[4-[(6,7-diméthoxy-1,5-naphtyridin-4-yl)oxy]-3-fluorophényl]-6-méthyl-2-oxo-1-(2-propan-2-yloxypyridin-4-yl)pyridine-3-carboxamide ;
N-[4-[(6,7-diméthoxy-1,5-naphtyridin-4-yl)oxy]-3-fluorophényl]-1-(2-méthoxypyridin-4-yl)-6-méthyl-2-oxopyridine-3-carboxamide ;
N-[4-[(6,7-diméthoxy-1,5-naphtyridin-4-yl)oxy]-3-fluorophényl]-1-(5-fluoro-6-méthylpyridin-2-yl)-6-méthyl-2-oxopyridine-3-carboxamide ;
N-[4-[(6,7-diméthoxy-1,5-naphtyridin-4-yl)oxy]-3-fluorophényl]-4-hydroxy-2-méthyl-5-pyridin-3-ylpyridine-3-carboxamide ;
N-[4-[(6,7-diméthoxy-1,5-naphtyridin-4-yl)oxy]-3-fluorophényl]-2-oxo-1-pyridin-4-ylpyridine-3-carboxamide ;
N-[4-[(6,7-diméthoxy-1,5-naphtyridin-4-yl)oxy]-3-fluorophényl]-6-méthyl-1-(2-méthylpyridin-4-yl)-2-oxopyridine-3-carboxamide ;
N-[4-[(6,7-diméthoxy-1,5-naphtyridin-4-yl)oxy]-3-fluorophényl]-6-méthyl-1-(1-oxydopyridin-1-ium-4-yl)-2-oxopyridine-3-carboxamide ;
N-[4-[(6,7-diméthoxy-1,5-naphtyridin-4-yl)oxy]-3-fluorophényl]-1-(2-hydroxypyridin-4-yl)-6-méthyl-2-oxopyridine-3-carboxamide ;
N-[4-[(6,7-diméthoxy-1,5-naphtyridin-4-yl)oxy]-3-fluorophényl]-1-(2-fluoropyridin-4-yl)-6-méthyl-2-oxopyridine-3-carboxamide ;
N-[4-[(6,7-diméthoxy-1,5-naphtyridin-4-yl)oxy]-3-fluorophényl]-6-méthyl-2-oxo-1-pyridazin-3-ylpyridine-3-carboxamide ;
5-acétyl-N-[4-[(6,7-diméthoxy-1,5-naphtyridin-4-yl)oxy]-3-fluorophényl]-1-(furan-3-yl)-6-méthyl-2-oxopyridine-3-carboxamide ;
5-acétyl-N-[4-[(6,7-diméthoxy-1,5-naphtyridin-4-yl)oxy]-3-fluorophényl]-1-(furan-2-yl)-6-méthyl-2-oxopyridine-3-carboxamide ;
5-acétyl-N-[4-[(6,7-diméthoxy-1,5-naphtyridin-4-yl)oxy]phényl]-1-(furan-2-yl)-6-méthyl-2-oxopyridine-3-carboxamide ;
5-acétyl-N-[3-fluoro-4-[(7-méthoxy-1,5-naphtyridin-4-yl)oxy]phényl]-1-(furan-2-yl)-6-méthyl-2-oxopyridine-3-carboxamide ;
5-acétyl-N-[4-[(6,7-diméthoxy-1,5-naphtyridin-4-yl)oxy]phényl]-1-(furan-3-yl)-6-méthyl-2-oxopyridine-3-carboxamide ;
5-acétyl-N-[3-fluoro-4-[(7-méthoxy-1,5-naphtyridin-4-yl)oxy]phényl]-1-(furan-3-yl)-6-méthyl-2-oxopyridine-3-carboxamide ;
5-acétyl-N-[4-[(6,7-diméthoxy-1,5-naphtyridin-4-yl)oxy]-3-fluorophényl]-6-méthyl-2-oxo-lthiophén-3-ylpyridine-3-carboxamide ;
5-acétyl-N-[4-[(6,7-diméthoxy-1,5-naphtyridin-4-yl)oxy]phényl]-6-méthyl-2-oxo-1-thiophén-3-ylpyridine-3-carboxamide ;
N-[4-[(6,7-diméthoxy-1,5-naphtyridin-4-yl)oxy]-3-fluorophényl]-4-éthoxy-1-(5-fluoropyridin-2-yl)-2-oxopyridine-3-carboxamide ;
N-[4-[(6,7-diméthoxy-1,5-naphtyridin-4-yl)oxy]phényl]-4-éthoxy-1-(5-fluoropyridin-2-yl)-2-oxopyridine-3-carboxamide ;
N-[4-[(6,7-diméthoxy-1,5-naphtyridin-4-yl)oxy]phényl]-1-(5-fluoropyridin-2-yl)-4,6-diméthyl-2-oxopyridine-3-carboxamide ;
N-[4-[(6,7-diméthoxy-1,5-naphtyridin-4-yl)oxy]-3-fluorophényl]-4-(5-fluoropyridin-2-yl)-3-oxopyrazine-2-carboxamide ;
N-[4-[(6,7-diméthoxy-1,5-naphtyridin-4-yl)oxy]-3-fluorophényl]-1-(1-oxydopyridin-1-ium-4-yl)-2-oxopyridine-3-carboxamide ;
N-[4-[(6,7-diméthoxy-1,5-naphtyridin-4-yl)oxy]-3-fluorophényl]-1-(2-hydroxypyridin-4-yl)-2-oxopyridine-3-carboxamide ;
N-[3-fluoro-4-[[6-méthoxy-7-(2-méthoxyéthoxy)-1,5-naphtyridin-4-yl]oxy]phényl]-5-(furan-2-yl)-1,2,6-triméthyl-4-oxopyridine-3-carboxamide ;
N-[4-[(6,7-diméthoxy-1,5-naphtyridin-4-yl)oxy]phényl]-5-(furan-2-yl)-4-hydroxy-6-méthylpyridine-3-carboxamide ;
N-[4-[(6,7-diméthoxy-1,5-naphtyridin-4-yl)oxy]phényl]-4-hydroxy-6-méthyl-5-thiophén-2-ylpyridine-3-carboxamide ;
4-hydroxy-N-[4-[(7-méthoxy-1,5-naphtyridin-4-yl)oxy]phényl]-6-méthyl-5-thiophén-2-ylpyridine-3-carboxamide ;
N-[3-fluoro-4-[[6-méthoxy-7-(2-méthoxyéthoxy)-1,5-naphtyridin-4-yl]oxy]phényl]-5-(furan-2-yl)-4-hydroxy-6-méthylpyridine-3-carboxamide ;
5-(1-benzofuran-2-yl)-N-[4-[(6,7-diméthoxy-1,5-naphtyridin-4-yl)oxy]-3-fluorophényl]-1,2,6-triméthyl-4-oxopyridine-3-carboxamide ;
5-(1-benzofuran-3-yl)-N-[4-[(6,7-diméthoxy-1,5-naphtyridin-4-yl)oxy]-3-fluorophényl]-1,2,6-triméthyl-4-oxopyridine-3-carboxamide ;
5-(1-benzofuran-2-yl)-N-[3-fluoro-4-[[6-méthoxy-7-(2-méthoxyéthoxy)-1,5-naphtyridin-4-yl]oxy]phényl]-1,2,6-triméthyl-4-oxopyridine-3-carboxamide ;
5-(1-benzofuran-3-yl)-N-[3-fluoro-4-[[6-méthoxy-7-(2-méthoxyéthoxy)-1,5-naphtyridin-4-yl]oxy]phényl]-1,2,6-triméthyl-4-oxopyridine-3-carboxamide ;
5-(1-benzofuran-3-yl)-N-[3-fluoro-4-[[7-(2-méthoxyéthoxy)-1,5-naphtyridin-4-yl]oxy]phényl]-1,2,6-triméthyl-4-oxopyridine-3-carboxamide ;
N-[3-fluoro-4-[[7-(2-méthoxyéthoxy)-1,5-naphtyridin-4-yl]oxy]phényl]-5-(furan-2-yl)-1,2,6-triméthyl-4-oxopyridine-3-carboxamide ;
N-[4-[(6,7-diméthoxy-1,5-naphtyridin-4-yl)oxy]-3-fluorophényl]-6-(furan-2-yl)-5-méthylpyrazine-2-carboxamide ;
1-(2,2-difluoroéthyl)-N-[4-[(6,7-diméthoxy-1,5-naphtyridin-4-yl)oxy]-3-fluorophényl]-5-(furan-2-yl)-2-méthyl-4-oxopyridine-3-carboxamide ;
N-[4-[(6,7-diméthoxy-1,5-naphtyridin-4-yl)oxy]-3-fluorophényl]-7-(furan-2-yl)-8-oxo-3,4-dihydro-1H-pyrido[2,1-c][1,4]oxazine-9-carboxamide ;
N-[4-[(6,7-diméthoxy-1,5-naphtyridin-4-yl)oxy]phényl]-7-(furan-2-yl)-8-oxo-3,4-dihydro-1H-pyrido[2,1-c][1,4]oxazine-9-carboxamide ;
N-[4-[(6,7-diméthoxy-1,5-naphtyridin-4-yl)oxy]phényl]-8-oxo-7-thiophén-2-yl-3,4-dihydro-1H-pyrido[2,1-c][1,4]oxazine-9-carboxamide ;
N-[4-[(6,7-diméthoxy-1,5-naphtyridin-4-yl)oxy]-3-fluorophényl]-8-oxo-7-thiophén-3-yl-3,4-dihydro-1H-pyrido[2,1-c][1,4]oxazine-9-carboxamide ;
N-[4-[(6,7-diméthoxy-1,5-naphtyridin-4-yl)oxy]phényl]-8-oxo-7-thiophén-3-yl-3,4-dihydro-1H-pyrido[2,1-c][1,4]oxazine-9-carboxamide ;
N-[4-[(6,7-diméthoxy-1,5-naphtyridin-4-yl)oxy]-3-fluorophényl]-7-(4-méthylthiophén-2-yl)-8-oxo-3,4-dihydro-1H-pyrido[2,1-c][1,4]oxazine-9-carboxamide ;
N-[4-[(6,7-diméthoxy-1,5-naphtyridin-4-yl)oxy]phényl]-7-(4-méthylthiophén-2-yl)-8-oxo-3,4-dihydro-1H-pyrido[2,1-c][1,4]oxazine-9-carboxamide ;
N-[4-[(6,7-diméthoxy-1,5-naphtyridin-4-yl)oxy]-3-fluorophényl]-7-(5-méthylthiophén-2-yl)-8-oxo-3,4-dihydro-1H-pyrido[2,1-c][1,4]oxazine-9-carboxamide ;
N-[4-[(6,7-diméthoxy-1,5-naphtyridin-4-yl)oxy]phényl]-7-(5-méthylthiophén-2-yl)-8-oxo-3,4-dihydro-1H-pyrido[2,1-c][1,4]oxazine-9-carboxamide ;
N-[4-[(6,7-diméthoxy-1,5-naphtyridin-4-yl)oxy]-3-fluorophényl]-7-(furan-3-yl)-8-oxo-3,4-dihydro-1H-pyrido[2,1-c][1,4]oxazine-9-carboxamide ;
N-[4-[(6,7-diméthoxy-1,5-naphtyridin-4-yl)oxy]-3-fluorophényl]-6-méthyl-8-oxo-7-thiophén-3-yl-3,4-dihydro-1H-pyrido[2,1-c][1,4]oxazine-9-carboxamide ;
N-[4-[(6,7-diméthoxy-1,5-naphtyridin-4-yl)oxy]-3-fluorophényl]-7-(furan-3-yl)-6-méthyl-8-oxo-3,4-dihydro-1H-pyrido[2,1-c][1,4]oxazine-9-carboxamide ;
N-[4-[(6,7-diméthoxy-1,5-naphtyridin-4-yl)oxy]-3-fluorophényl]-8-oxo-7-thiophén-2-yl-3,4-dihydro-1H-pyrido[2,1-c][1,4]oxazine-9-carboxamide ;
N-[4-[(6,7-diméthoxy-1,5-naphtyridin-4-yl)oxy]phényl]-7-(furan-3-yl)-8-oxo-3,4-dihydro-1H-pyrido[2,1-c][1,4]oxazine-9-carboxamide ;
N-[4-[(6,7-diméthoxy-1,5-naphtyridin-4-yl)oxy]phényl]-7-(furan-3-yl)-6-méthyl-8-oxo-3,4-dihydro-1H-pyrido[2,1-c][1,4]oxazine-9-carboxamide ;
N-[4-[(6,7-diméthoxy-1,5-naphtyridin-4-yl)oxy]phényl]-6-méthyl-8-oxo-7-thiophén-3-yl-3,4-dihydro-1H-pyrido[2,1-c][1,4]oxazine-9-carboxamide ;
N-[4-[(6,7-diméthoxy-1,5-naphtyridin-4-yl)oxy]phényl]-6-méthyl-7-(4-méthylthiophén-2-yl)-8-oxo-3,4-dihydro-1H-pyrido[2,1-c][1,4]oxazine-9-carboxamide ;
N-[4-[(6,7-diméthoxy-1,5-naphtyridin-4-yl)oxy]-3-fluorophényl]-7-(furan-2-yl)-6-méthyl-8-oxo-3,4-dihydro-1H-pyrido[2,1-c][1,4]oxazine-9-carboxamide ;
N-[4-[(6,7-diméthoxy-1,5-naphtyridin-4-yl)oxy]phényl]-7-(furan-2-yl)-6-méthyl-8-oxo-3,4-dihydro-1H-pyrido[2,1-c][1,4]oxazine-9-carboxamide ;
N-[4-[(6,7-diméthoxy-1,5-naphtyridin-4-yl)oxy]phényl]-6-méthyl-8-oxo-7-thiophén-2-yl-3,4-dihydro-1H-pyrido[2,1-c][1,4]oxazine-9-carboxamide ;
N-[4-[(6,7-diméthoxy-1,5-naphtyridin-4-yl)oxy]-3-fluorophényl]-6-méthyl-8-oxo-7-thiophén-2-yl-3,4-dihydro-1H-pyrido[2,1-c][1,4]oxazine-9-carboxamide ;
N-[4-[(6,7-diméthoxy-1,5-naphtyridin-4-yl)oxy]-3-fluorophényl]-6-méthyl-7-(4-méthylthiophén-2-yl)-8-oxo-3,4-dihydro-1H-pyrido[2,1-c][1,4]oxazine-9-carboxamide ;
N-[4-[(6,7-diméthoxy-1,5-naphtyridin-4-yl)oxy]-3-fluorophényl]-2-éthyl-5-(furan-2-yl)-1,6-diméthyl-4-oxopyridine-3-carboxamide ;
N-[4-[(6,7-diméthoxy-1,5-naphtyridin-4-yl)oxy]phényl]-2-éthyl-5-(furan-2-yl)-1,6-diméthyl-4-oxopyridine-3-carboxamide ;
2-éthyl-N-[3-fluoro-4-[[6-méthoxy-7-(2-méthoxyéthoxy)-1,5-naphtyridin-4-yl]oxy]phényl]-5-(furan-2-yl)-1,6-diméthyl-4-oxopyridine-3-carboxamide ;
N-[4-[(6,7-diméthoxy-1,5-naphtyridin-4-yl)oxy]-3-fluorophényl]-1-(2-fluoroéthyl)-6-méthyl-4-oxo-5-thiophén-2-ylpyridine-3-carboxamide ;
1-(2-fluoroéthyl)-N-[3-fluoro-4-[(7-méthoxy-1,5-naphtyridin-4-yl)oxy]phényl]-6-méthyl-5-(4-méthylthiophén-2-yl)-4-oxopyridine-3-carboxamide ;
1-(2-fluoroéthyl)-N-[3-fluoro-4-[(7-méthoxy-1,5-naphtyridin-4-yl)oxy]phényl]-6-méthyl-5-(5-méthylthiophén-2-yl)-4-oxopyridine-3-carboxamide ;
1-(2-fluoroéthyl)-N-[3-fluoro-4-[(7-méthoxy-1,5-naphtyridin-4-yl)oxy]phényl]-6-méthyl-4-oxo-5-thiophén-3-ylpyridine-3-carboxamide ;
1-(2-fluoroéthyl)-N-[3-fluoro-4-[(7-méthoxy-1,5-naphtyridin-4-yl)oxy]phényl]-6-méthyl-4-oxo-5-thiophén-2-ylpyridine-3-carboxamide ;
N-[4-[(6,7-diméthoxy-1,5-naphtyridin-4-yl)oxy]-3-fluorophényl]-1-(2-fluoroéthyl)-6-méthyl-4-oxo-5-thiophén-3-ylpyridine-3-carboxamide ;
N-[4-[(6,7-diméthoxy-1,5-naphtyridin-4-yl)oxy]-3-fluorophényl]-1-(2-fluoroéthyl)-5-(furan-2-yl)-6-méthyl-4-oxopyridine-3-carboxamide ;
1-(2-fluoroéthyl)-N-[3-fluoro-4-[(7-méthoxy-1,5-naphtyridin-4-yl)oxy]phényl]-5-(furan-3-yl)-6-méthyl-4-oxopyridine-3-carboxamide ; et
N-[4-[[7-(2-cyclobutyléthoxy)-6-méthoxy-1,5-naphtyridin-4-yl]oxy]-3-fluorophényl]-5-(furan-2-yl)-1,2,6-triméthyl-4-oxopyridine-3-carboxamide ;
ou sel pharmaceutiquement acceptable ou stéréoisomère de celui-ci.

14. Composition pharmaceutique comprenant un composé selon l'une quelconque des revendications 1 à 13, ou un sel pharmaceutiquement acceptable ou stéréoisomère de celui-ci, et un vecteur ou excipient pharmaceutiquement acceptable.

15. Composé de l'une quelconque des revendications 1 à 13, ou sel pharmaceutiquement acceptable ou stéréoisomère de celui-ci, ou composition pharmaceutique de la revendication 14, pour utilisation dans le traitement d'une maladie, d'un trouble, ou d'un syndrome chez un sujet en ayant besoin, ladite utilisation comprenant l'administration, au sujet, d'une quantité thérapeutiquement efficace dudit composé ou de ladite composition pharmaceutique, dans lequel ou laquelle la maladie, le trouble, ou le syndrome est à médiation au moins en partie par modulation de l'activité in vivo d'une protéine-kinase ; de préférence dans lequel ou laquelle la protéine-kinase est AXL, KDR, Mer, ou Met.
